# EUROPEAN PATENT SPECIFICATION

(11) **EP 0 922 050 B1**
(45) Date of publication and mention of the grant of the patent: **30.07.2003**
(21) Application number: 97921411.1
(22) Date of filing: 25.04.1997
(51) Int. Cl.: C07H 17/08, A61K 31/70

(54) **3-DESCLADINOSE-2,3-ANHYDROERYTHROMYCIN DERIVATIVES**
3-DESCLADINOSE-2,3-ANHYDROERYTHROMYCIN-DERIVATE
DERIVES DE LA 3-DESCLADINOSE-2,3-ANHYDRO-ERYTHROMYCINE

(30) Priority: 07.05.1996 US 646418; 04.04.1997 US 832741
(43) Date of publication of application: 16.06.1999
(73) Proprietor: ABBOTT LABORATORIES, Abbott Park, Illinois 60064-3500 (US)
(72) Inventor: ELLIOTT, Richard, L., E. Lyme, Connecticut 06333 (US); OR, Yat, Sun, Libertyville, IL 60048 (US); CHU, Daniel, T., Santa Clara, CA 95051 (US); GRIESGRABER, George, W., Libertyville, IL 60048 (US); PLATTNER, Jacob, J., Libertyville, IL 60048 (US); PIREH, Daisy, Lincolnshire, IL 60069 (US)
(74) Representative: Modiano, Guido, Dr.-Ing.
(86) International application number: US9707051
(87) International publication number: WO97042205

(56) References cited:
- EP-A- 0 487 411
- EP-A- 0 676 409
- JOURNAL OF ANTIBIOTICS, vol. 43, no. 11, 1990, TOKYO JP, pages 1508-1511, XP000676680 H.SUZUKI ET AL.: "Biosynthesis of Mycinamicins by a Blocked Mutant of Micromonospora Griseorubida."
- JOURNAL OF THE CHEMICAL SOCIETY, PERKIN TRANSACTIONS 1, no. 6, 1987, LETCHWORTH GB, page 1189-1209 XP000676687 A.G.FISHMAN ET AL.: "Novel Semisynthetic Oxo and Alkyl Macrolide Antibacterials and Related Derivatives."

## Description

### Technical Field

The present invention relates to novel semisynthetic macrolides having antibacterial activity and useful in the treatment and prevention of bacterial infections. More particularly, the invention relates to 3-descladiose-2,3-anhydroerythromycin derivatives, compositions containing such compounds and the use thereof as well as processes for making such compounds.

### Background Of The Invention

Erythromycins A through D, represented by formula (E):

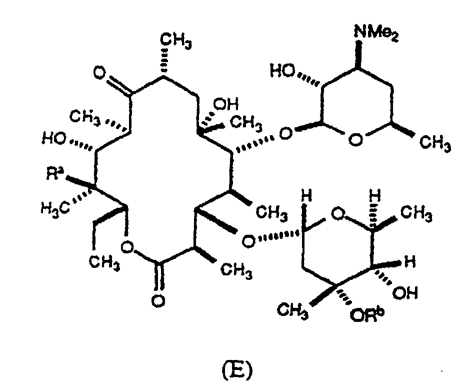

| | | |
|---|---|---|
| Erythromycin | R^{a} | R^{b} |
| A | -OH | -CH₃ |
| B | -H | -CH₃ |
| C | -OH | -H |
| D | -H | -H |

are well-known and potent antibacterial agents, used widely to treat and prevent bacterial infection. As with other antibacterial agents, however, bacterial strains having resistance or insufficient susceptibility to erythromycin have been identified. Also, erythromycin A has only weak activity against Gram-negative bacteria. Therefore, there is a continuing need to identify new erythromycin derivative compounds which possess improved antibacterial activity, which have less potential for developing resistance, which possess the desired Gram-negative activity, or which possess unexpected selectivity against target microorganisms. Consequently, numerous investigators have prepared chemical derivatives of erythromycin in an attempt to obtain analogs having modified or improved profiles of antibiotic activity.

Although Agouridas. *et al*. (U.S. Patent 5,444,051, issued August 22, 1995) have disclosed a 9-O-((2-methoxyethoxy)methyl)oxime of 2-deoxy-2,3-anhydro-3-O-des(2,6-dideoxy-3-C-methyl-3-O-methyl-α-L-ribo-hexopyranosyl)-6-O-methyl-erythromycin, no utility nor method of preparation was disclosed.

We have, however, discovered novel 3-descladinose-2,3-anhydroerythromycin derivatives that possess significant activity against selected microorganisms.

Likewise, various C3-modified erythromycin compounds are known, but none possess the C2-C3 modifications of the present invention (see, for example, Agouridas, *et al*., European application EP 676409, published October 11, 1995; Kashimura, *et al*., European application EP 559896, published September 15, 1993; and Asaka, *et al*., PCT application WO 93/21200, published October 28, 1993).

### Summary Of The Invention

The present invention provides a novel class of 3-descladinose-2,3-anhydro-erythromycin compounds which possess antibacterial activity.

In one aspect of the present invention are disclosed novel 3-descladinose-2,3-anhydroerythromycin compounds selected from the group consisting of: 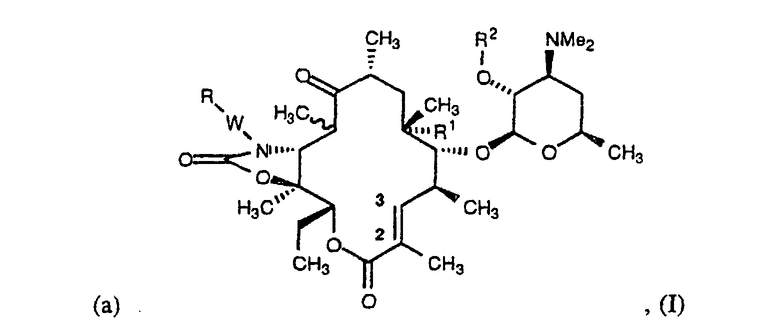 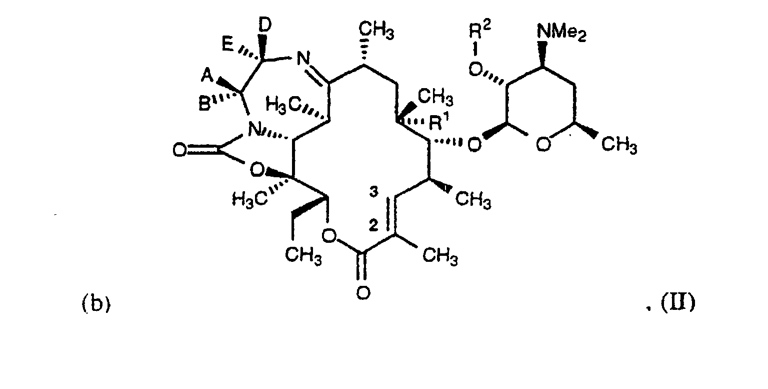 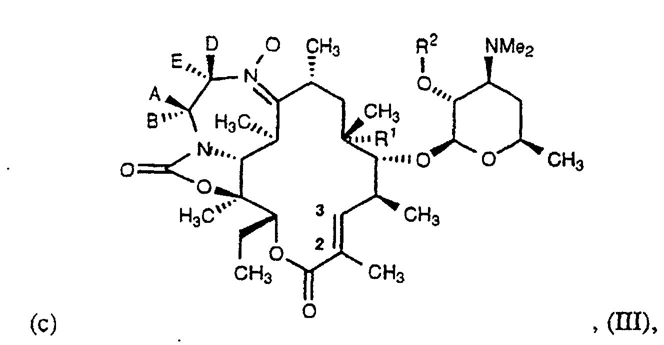 and 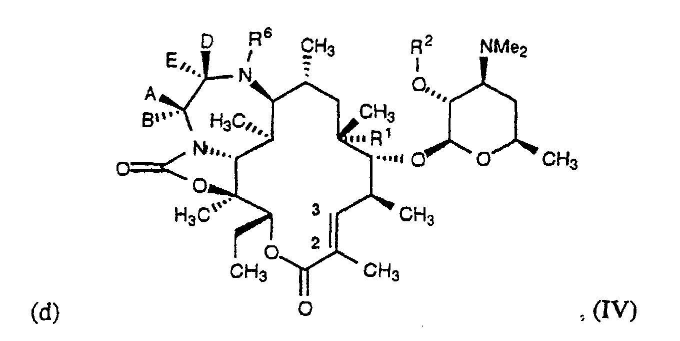 or pharmaceutically acceptable salts and esters thereof.

In formulas (I) - (IV) above,
R¹ is selected from the group consisting of:
   (a) hydrogen;
   (b) hydroxy;
   (c) O-C₁-C₁₂-alkyl;
   (d) O-CO-C₁-C₆-alkyl;
   (e) O-CO-NH₂;
   (f) O-CO-NH-CO-C₁-C₁₂-alkyl; and
   (g) O-CO-NH-SO₂-C₁-C₁₂-alkyl;
R² is hydrogen or a hydroxy protecting group;
R⁶ is hydrogen or C₁-C₆-alkyl;
R is selected from the group consisting of:
   (a) hydrogen;
   (b) C₁-C₆-alkyl optionally substituted with one or more substituents selected from the group consisting of:
      (i) aryl;
      (ii) substituted-aryl;
      (iii) heteroaryl;
      (iv) substituted-heteroaryl;
      (v) hydroxy;
      (vi) C₁-C₆-alkoxy;
      (vii) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
      (viii) -CH₂-M-R⁵, wherein M is selected from the group consisting of:
         (aa) -C(O)-NH-;
         (bb) -NH-C(O)-;
         (cc) -NH-
         (dd) -N=;
         (ee) -N(CH₃)-
         (ff) -O-
         (gg) -S(O)ₙ-, wherein n is 0, 1 or 2;
         (hh) -CO-O-
         (ii) -O-CO-
         (jj) -CO- ; and
         R⁵ is selected from the group consisting of:
         (aaa) C₁-C₆-alkyl, optionally substituted with a substituent selected from the group consisting of:
            (i) aryl;
            (ii) substituted-aryl;
            (iii) heteroaryl; and
            (iv) substituted-heteroaryl;
         (bbb) aryl;
         (ccc) substituted-aryl;
         (ddd) heteroaryl;
         (eee) substituted-heteroaryl; and
         (fff) heterocycloalkyl; and
   (c) C₃-C₇-cycloalkyl;
   (d) aryl;
   (e) substituted-aryl;
   (f) heteroaryl;
   (g) substituted-heteroaryl;
W is absent or selected from the group consisting of -O-, -NH-CO-, -N=CH-, -NH-and -N(C₁-C₆-alkyl)-;
A, B, D and E are independently selected from the group consisting of:
   (a) hydrogen;
   (b) C₁-C₆-alkyl, optionally substituted with one or more substituents selected from the group consisting of:
      (i) aryl;
      (ii) substituted-aryl;
      (iii) heteroaryl;
      (iv) substituted-heteroaryl;
      (v) heterocycloalkyl;
      (vi) hydroxy;
      (vii) C₁-C₆-alkoxy;
      (viii) halogen consisting of Br, Cl, F or I; and
      (ix) NR³R⁴, where R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, -N(aryl-C₁-C₆-alkyl-)-, -N(substituted-aryl-C₁-C₆-alkyl-)-, -N(heteroaryl-C₁-C₆-alkyl-)-, -N(substituted-heteroaryl-C₁-C₆-alkyl-)-, -S-or -S(O)ₙ-, wherein n is 1 or 2;
   (c) C₃-C₇-cycloalkyl;
   (d) aryl;
   (e) substituted-aryl;
   (f) heteroaryl;
   (g) substituted-heteroaryl;
   (h) heterocycloalkyl; and
   (i) a group selected from option (b) above further substituted with -M-R⁵, wherein M and R⁵ are as defined above; or any one pair of substituents, consisting of AB, AD, AE, BD, BE or DE, is taken together with the atom or atoms to which they are attached to form a 3- to 7-membered ring optionally containing a hetero function consisting of:
      -O-,
      -NH-,
      -N(C₁-C₆-alkyl-)-,
      -N(aryl-C₁-C₆-alkyl-)-,
      -N(substituted-aryl-C₁-C₆-alkyl-)-,
      -N(heteroaryl-C₁-C₆-alkyl-)-,
      -N(substituted-heteroaryl-C₁-C₆-alkyl-)-,
      -S- or -S(O)ₙ-, wherein n is 1 or 2;
      -C(O)-NH-;
      -C(O)-NR⁵-, wherein R⁵ is as defined above;
      -NH-C(O)-;
      -NR⁵-C(O)-, wherein R⁵ is as defined above; and
      -C(=NH)-NH-.

The compounds and compositions of the present invention have antibacterial activity.

In another aspect of the present invention are disclosed pharmaceutical compositions for treating bacterial infections comprising a therapeutically effective amount of a compound of the invention in combination with a pharmaceutically acceptable carrier. Suitable carriers and methods of formulation are also disclosed.

Still another aspect of this invention is the use of a pharmaceutical composition containing a therapeutically effective amount of a compound of the invention for manufacturing a medicament for treating bacterial infections by administration to a mammal in need of such treatment.

In a further aspect of the invention are provided processes for the preparation of tricyclic macrolide derivatives of Formulas (I) - (IV) above.

In another aspect of the invention are provided novel compounds (*cf.* compound (4) of Scheme 1) having use as intermediates in the preparation of compounds of Formulas (I)-(IV) above.

In still another aspect of the invention is a process for the preparation of a novel 3-descladinose-2.3-anhydroerythromycin intermediate compound having the Formula of Compound (4) of Scheme 1; R¹=OMe (*cf.* Scheme 1 below).

### Detailed Description Of The Invention

In one preferred embodiment of the invention are compounds having the formula (I): 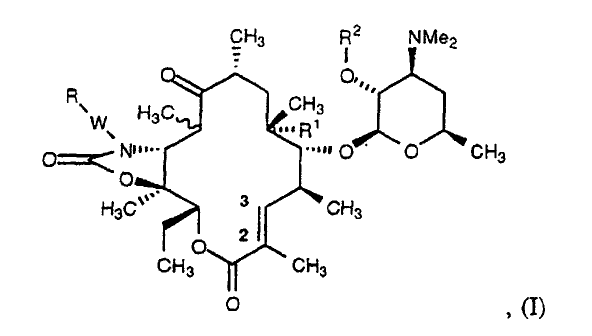 wherein R¹, R², R, and W are as defined above.

In a more preferred embodiment of the invention are compounds of formula (I) wherein W is absent or is an -NH- grouping.

In another preferred embodiment of the invention are compounds having the formula (II): 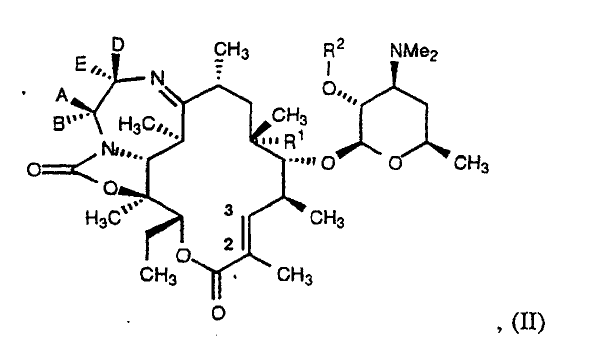 wherein R¹, R², A, B, D, and E are as defined above.

In a further embodiment of the invention are compounds having the formula (III): 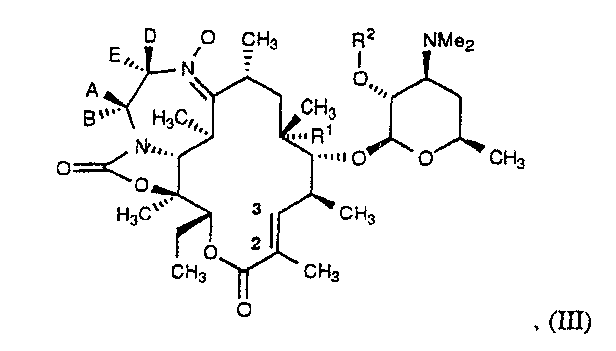 wherein R¹, R², A, B, D, and E are as defined above.

In still another embodiment of the invention are compounds having the formula (IV): 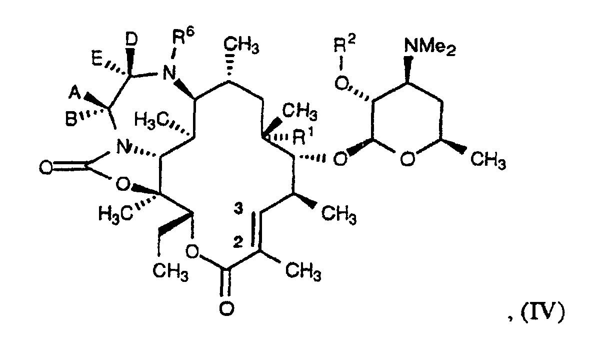 wherein R¹, R², R⁶, A, B, D, and E are as defined above.

Representative compounds of the invention include:
Compound of Formula (I); R¹=H; R²=H; W is absent; R=4-phenylbutyl;
Compound of Formula (I); R¹=methoxy; R²=H; W is absent; R=4-phenylbutyl;
Compound of Formula (I); R¹=methoxy; R²=H; W is absent; R=3-phenoxypropyl;
Compound of Formula (I); R¹=methoxy; R²=H; W is absent; R=2-((phenylmethyl)amino)ethyl:
Compound of Formula (I); R¹=methoxy; R²=H; W is absent; R=3-(N-methyl-N-phenylamino)propyl;
Compound of Formula (I); R¹=methoxy; R²=H; W is absent; R=3-(4-chlorophenoxy)propyl;
Compound of Formula (II); R¹=methoxy; R²=H; A=B=C=D=H;
Compound of Formula (I); R¹=methoxy; R²=H; W is absent; R=3-(1-quinoyloxy)propyl;
Compound of Formula (I); R¹=methoxy; R²=H; W is absent; R=4-(4-chlorophenyl)-3(Z)-butenyl;
Compound of Formula (I); R¹=methoxy; R²=H; W is absent; R=2-phenylethyl;
Compound of Formula (I); R¹=methoxy; R²=H; W is absent; R=2-(3,4-dichlorophenyl)ethyl;
Compound of Formula (I); R¹=methoxy; R²=H; W is absent; R=phenylmethyl;
Compound of Formula (I); R¹=methoxy; R²=H; W is -NH-; R=3-phenylpropyl;
Compound of Formula (I); R¹=methoxy; R²=H; W is absent; R=3-(4-phenoxyphenyl)ethyl;
Compound of Formula (I); R¹=methoxy; R²=H; W is absent; R=3-phenylpropyl;
Compound of Formula (I); R¹=methoxy; R²=H; W is absent; R=2,2-diphenylethyl;
Compound of Formula (I); R¹=methoxy; R²=H; W is absent; R=H;
Compound of Formula (IV); R¹=methoxy; R²=H; A=B=C=D=H; R=H;
Compound of Formula (I); R¹=methoxy; R²=H; W is -NH-; R=H; C10 methyl is *epi*-isomer;
Compound of Formula (I); R¹=methoxy; R²=H; W is -NH-; R=H; C10 methyl is natural isomer;
Compound of Formula (I); R¹=methoxy; R²=H; W is -NH-; R=(4-quinolinyl)propyl; C10 methyl is natural isomer;
Compound of Formula (I); R¹=methoxy; R²=H; W is absent; R=3-(2-naphthyloxy)propyl;
Compound of Formula (I); R¹=methoxy; R²=H; W is absent; R=3-(3-pyridyloxy)propyl;
Compound of Formula (I); R¹=methoxy: R²=H; W is absent; R=3-(2-pyridyloxy)propyl:
Compound of Formula (I); R¹=OH; R²=H; W is absent; R=4-phenylbutyl;
Compound of Formula (I); R¹=OCONH²; R²=H; W is absent; R=4-phenylbutyl;
Compound of Formula (I); R¹=OCONHCO-methyl; R²=H; W is absent; R=4-phenylbutyl;
Compound of Formula (I); R¹=OCONHSO²-methyl; R²=H; W is absent; R=4-phenylbutyl;
Compound of Formula (I); R¹=OMe; R²=H; W is absent; R=phenyl;
Compound of Formula (I); R¹=OMe; R²=H; W is absent; R=3-pyridyl;
Compound of Formula (I); R¹=OMe; R²=H; W is -O-; R=H;
Compound of Formula (I); R¹=OMe; R²=H; W is -O-; R=Me;
Compound of Formula (I); R¹=OMe; R²=H; W is -NH-CO-;
R=phenyl;
Compound of Formula (II); R¹=OMe; R²=H; A=benzyl; B,D,E=H;
Compound of Formula (II); R¹=OMe; R²=H; A,D=3,4-pyrrolidinyl; B,E=H;
Compound of Formula (III); R¹=OMe; R²=H; A,B,D,E=H;
Compound of Formula (IV); R¹=OMe; R²=H; A=benzyl; B,D,E=H; R=H;
Compound of Formula (IV); R¹=OMe; R²=H; A,D=3,4-pyrrolidinyl; B,E=H; R=H;
Compound of Formula (IV); R¹=OMe; R²=H; A,B,D,E=H, R=CH₂CH₂CH₂C₆H₅;
Compound of Formula (IV); R¹=OMe; R²=H; A,B,D,E=H, R=2,4-dinitrobenzene;
Compound of Formula (IV); R¹=OMe; R²=H; A,B,D,E=H, R=4-quinolyl;
Compound of Formula (I); R¹=methoxy; R²=H; W is absent; R=(4H-4-oxo-1-quinolyl)propyl;
Compound of Formula (I); R¹=methoxy; R²=H; W is absent; R=2-(4-nitrophenyl)ethyl;
Compound of Formula (I); R¹=methoxy; R²=H; W is absent; R=2-(4-aminophenyl)ethyl:
Compound of Formula (I); R¹=methoxy; R²=H; W is absent; R=3-ethoxypropyl;
Compound of Formula (I); R¹=methoxy; R²=H; W is absent; R=isopropyl;
Compound of Formula (I); R¹=methoxy; R²=H; W is absent; R=2-(4-bromophenyl)ethyl;
Compound of Formula (I); R¹=methoxy; R²=H; W is absent; R=2-(4-hydroxylphenyl )ethyl;
Compound of Formula (I): R¹=methoxy; R²=H; W is absent; R=2-(4-fluorophenyl)ethyl;
Compound of Formula (I); R¹=methoxy; R²=H; W is absent; R=2-(3-methoxyphenyl)ethyl;
Compound of Formula (I); R¹=methoxy; R²=H; W is absent; R=3-vinyloxypropyl;
Compound of Formula (I); R¹=methoxy; R²=H; W is absent; R=2-(3-trifluoromethyl)phenylethyl;
Compound of Formula (I); R¹=methoxy; R²=H; W is absent; R=2-thienylethyl;
Compound of Formula (I); R¹=methoxy; R²=H; W is absent; R=2-(3,4-di-benzyloxyphenyl)ethyl;
Compound of Formula (I); R¹=methoxy; R²=H; W is absent; R=2-(4-methylphenyl)ethyl;
Compound of Formula (I); R¹=methoxy; R²=H; W is absent; R=allyl;
Compound of Formula (I); R¹=methoxy; R²=H; W is absent; R=1,3-dihydroxypropyl;
Compound of Formula (I); R¹=methoxy; R²=H; W is absent; R=1,3-dihydroxypropyl (10-*epi*);
Compound of Formula (I); R¹=methoxy; R²=H; W is absent; R=3-hydroxypropyl;
Compound of Formula (I); R¹=methoxy; R²=H; W is absent; R=3-hydroxypropyl (10-*epi*);
Compound of Formula (I); R¹=methoxy; R²=H; W is absent; R=propyl;
Compound of Formula (I); R¹=methoxy; R²=H; W is absent; R=isobutyl;
Compound of Formula (I); R¹=methoxy; R²=H; W is absent; R=2-(benzoylamino)ethyl;
Compound of Formula (I); R¹=methoxy; R²=H; W is absent; R=3-(benzoylamino)propyl;
Compound of Formula (I); R¹=methoxy; R²=H; W is absent; R=3-(acetylamino)propyl;
Compound of Formula (I); R¹=methoxy; R²=H; W is absent; R=H (10-*epi*);
Compound of Formula (I); R¹=methoxy; R²=H; W is absent; R=3-phenylpropyl (10-*epi*);
Compound of Formula (I): R¹=methoxy; R²=H; W is absent; R=3-(4-phenoxyphenyl)ethyl (10*-epi*);
Compound of Formula (I); R¹=methoxy; R²=H; W is -NH-; R=3-(4-chlorophenyl)propyl;
Compound of Formula (I); R¹=methoxy; R²=H; W is -NH-; R=3-(3-chlorophenyl)propyl;
Compound of Formula (I); R¹=methoxy; R²=H; W is -NH-; R=3-(2-chlorophenyl)propyl;
Compound of Formula (I); R¹=methoxy; R²=H; W is -NH-; R=3-(2,4-dichlorophenyl)propyl;
Compound of Formula (I); R¹=methoxy; R²=H; W is -NH-; R=3-(4-hydroxyphenyl)propyl;
Compound of Formula (I); R¹=methoxy; R²=H; W is -NH-; R=3-(3-hydroxyphenyl)propyl;
Compound of Formula (I); R¹=methoxy; R²=H; W is -NH-; R=3-(2-hydroxyphenyl)propyl;
Compound of Formula (I); R¹=methoxy; R²=H; W is -NH-; R=3-(4-methoxyphenyl)propyl;
Compound of Formula (I); R¹=methoxy; R²=H; W is -NH-; R=3-(4-nitrophenyl)propyl;
Compound of Formula (I); R¹=methoxy; R²=H; W is -NH-; R=3-(3-nitrophenyl)propyl;
Compound of Formula (I); R¹=methoxy; R²=H; W is -NH-; R=3-(2-nitrophenyl)propyl;
Compound of Formula (I); R¹=methoxy; R²=H; W is -NH-; R=3-((4-(acetylamino)phenyl)propyl;
Compound of Formula (I); R¹=methoxy; R²=H; W is -NH-; R=*trans*-3-phenylprop-2-enyl;
Compound of Formula (I); R¹=methoxy; R²=H; W is -NH-; R=2-phenylethyl;
Compound of Formula (I); R¹=methoxy; R²=H; W is -NH-; R=phenylmethyl;
Compound of Formula (I); R¹=methoxy; R²=H; W is -NH-; R=(3-indolyl)methyl;
Compound of Formula (I); R¹=methoxy; R²=H; W is -NH-; R=(4-methoxyphenyl)methyl;
Compound of Formula (I); R¹=methoxy; R²=H; W is -NH-; R=(4-acetylaminophenyl)methyl;
Compound of Formula (I); R¹=methoxy; R²=H; W is -NH-; R=(4-chlorophenyl)methyl;
Compound of Formula (I); R¹=methoxy; R²=H; W is -NH-; R=(4-dimethylaminophenyl)methyl:
Compound of Formula (I); R¹=methoxy; R²=H; W is -NH-; R=*trans*-3-(4-nitrophenyl)prop-2-enyl;
Compound of Formula (I); R¹=methoxy; R²=H; W is -NH-; R=(4-nitrophenyl)methyl;
Compound of Formula (I); R¹=methoxy; R²=H; W is -NH-; R=(3,4-dihydroxyphenyl)methyl;
Compound of Formula (I); R¹=methoxy; R²=H; W is -NH-; R=(2,5-dihydroxyphenyl)methyl;
Compound of Formula (I); R¹=methoxy; R²=H; W is -NH-; R=(2-hydroxy-5-nitrophenyl)methyl;
Compound of Formula (I); R¹=methoxy; R²=H; W is -NH-; R=(4-hydroxymethylphenyl)methyl;
Compound of Formula (I); R¹=methoxy; R²=H; W is -NH-; R=*trans*-3-(5-nitro-2-furanyl)prop-2-enyl;
Compound of Formula (I); R¹=methoxy; R²=H; W is -NH-; R=(4-hydroxyphenyl)methyl;
Compound of Formula (I); R¹=methoxy; R²=H; W is -NH-; R=(3-hydroxyphenyl)methyl;
Compound of Formula (I); R¹=methoxy; R²=H; W is -NH-; R=(2-hydroxyphenyl)methyl;
Compound of Formula (I); R¹=methoxy; R²=H; W is -NH-; R=(4-trifluoromethylphenyl)methyl;
Compound of Formula (I); R¹=methoxy; R²=H; W is -NH-; R=(4-cyanophenyl)methyl;
Compound of Formula (I); R¹=methoxy; R²=H; W is -NH-; R=(2-pyridyl)methyl;
Compound of Formula (I); R¹=methoxy; R²=H; W is -NH-; R=(3-pyridyl)methyl;
Compound of Formula (I); R¹=methoxy; R²=H; W is -NH-; R=(4-pyridyl)methyl;
Compound of Formula (I); R¹=methoxy; R²=H; W is -NH-; R=(2-hydroxy-1-naphthyl)methyl;
Compound of Formula (I); R¹=methoxy; R²=H; W is -NH-; R=(4-dimethylamino-1-naphthyl)methyl;
Compound of Formula (I); R¹=methoxy; R²=H; W is -NH-; R=(4-(methylthio)phenyl)methyl;
Compound of Formula (I); R¹=methoxy; R²=H; W is -NH-; R=(4-phenoxyphenyl)methyl;
Compound of Formula (I): R¹=methoxy; R²=H; W is -NH-; R=3-(4-fluorophenyl)propyl;
Compound of Formula (I); R¹=methoxy; R²=H; W is -NH-; R=(*trans*-3-(4-nitrophenyl)prop-2-enyl;
Compound of Formula (I); R¹=methoxy; R²=H; W is -NH-; R=(4-aminophenyl)methyl;
Compound of Formula (I); R¹=methoxy; R²=H; W is -NH-; R=3-(4-aminophenyl)propyl;
Compound of Formula (I); R¹=methoxy; R²=H; W is -NH-; R=3-(3-aminophenyl)propyl;
Compound of Formula (I); R¹=methoxy; R²=H; W is -NH-; R=3-(2-aminophenyl)propyl;
Compound of Formula (I); R¹=methoxy; R²=H; W is -NH-; R=*trans*-3-(4-acetylaminophenyl)prop-2-enyl;
Compound of Formula (I); R¹=methoxy; R²=H; W is -NH-; R=*trans*-3-(4-(4-nitrobenzoylammo)phenyl)prop-2-enyl;
Compound of Formula (I); R¹=methoxy; R²=H; W is -NH-; R=3-(2-benztriazolyl)propyl;
Compound of Formula (I); R¹=methoxy; R²=H; W is -NH-; R=3-(1-benztriazolyl)propyl;
Compound of Formula (I); R¹=methoxy; R²=H; W is -NH-; R=3-(4-phenylimidazolyl)propyl;
Compound of Formula (I); R¹=methoxy; R²=H; W is -NH-; R=3-(1-anhydro- I -cladinosyl)propyl;
Compound of Formula (I); R¹=methoxy; R²=H; W is -NH-; R=3-phenylpropyl (10-*epi*);
Compound of Formula (I); R¹=methoxy; R²=H; W is -NH-; R=isopropyl;
Compound of Formula (I); R¹=methoxy; R²=H; W is -NH-; R=1,3-diphenyl-2-propyl; and
Compound of Formula (I); R¹=methoxy; R²=H; W is -NH-; R=3-pentyl.

The present invention provides a process for the preparation of a compound having the Formula (I): 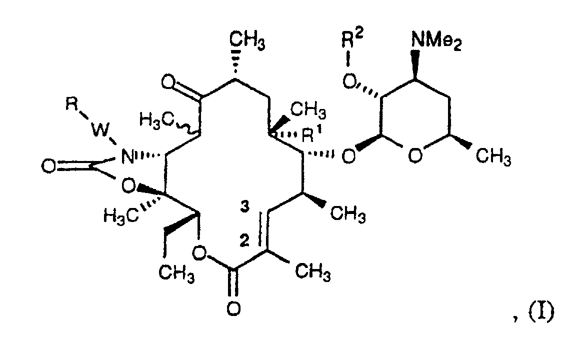 wherein
R¹ is selected from the group consisting of:
   (a) hydrogen;
   (b) hydroxy;
   (c) O-C₁-C₁₂-alkyl;
   (d) O-CO-C₁-C₆-alkyl;
   (e) O-CO-NH₂;
   (f) O-CO-NH-CO-C₁-C₁₂-alkyl; and
   (g) O-CO-NH-SO₂-C₁-C₁₂-alkyl;
R² is hydrogen or a hydroxy protecting group;
R is selected from the group consisting of:
   (a) hydrogen;
   (b) C₁-C₆-alkyl optionally substituted with one or more substituents selected from the group consisting of:
      (i) aryl;
      (ii) substituted-aryl;
      (iii) heteroaryl;
      (iv) substituted-heteroaryl;
      (v) hydroxy;
      (vi) C₁-C₆-alkoxy;
      (vii) NR³R⁴, where R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, -N(aryl-C₁-C₆-alkyl-)-, -N(substituted-aryl-C₁-C₆-alkyl-)-, -N(heteroaryl-C₁-C₆-alkyl-)-, -N(substituted-heteroaryl-C₁-C₆-alkyl-)-, -S-or -S(O)ₙ-, wherein n is 1 or 2;
      (viii) -CH₂-M-R⁵,
      wherein M is selected from the group consisting of:
      (aa) -C(O)-NH-;
      (bb) -NH-C(O)-;
      (cc) -NH-
      (dd) -N=;
      (ee) -N(CH₃)-
      (ff) -O-
      (gg) -S(O)ₙ-, wherein n is 0, 1 or 2;
      (hh) -CO-O-
      (ii) -O-CO-
      (jj) -CO-; and
      R⁵ is selected from the group consisting of:
      (aaa) C₁-C₆-alkyl, optionally substituted with a substituent selected from the group consisting of:
         (i) aryl;
         (ii) substituted-aryl;
         (iii) heteroaryl; and
         (iv) substituted-heteroaryl;
      (bbb) aryl;
      (ccc) substituted-aryl;
      (ddd) heteroaryl;
      (eee) substituted-heteroaryl; and
      (fff) heterocycloalkyl; and
   (c) C₃-C₇-cycloalkyl;
   (d) aryl;
   (e) substituted-aryl;
   (f) heteroaryl;
   (g) substituted-heteroaryl; and
W is absent;
   the method comprising:
   (a) treating a compound having the formula: 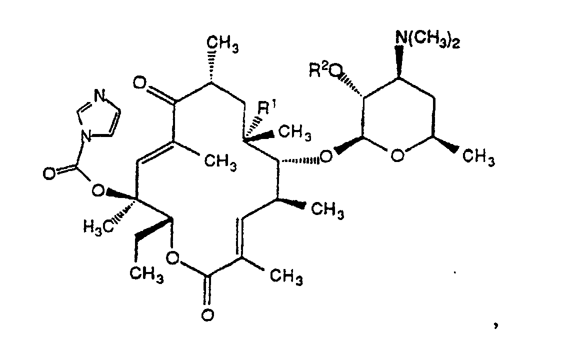
   wherein R¹ is selected from the group consisting of hydrogen, protected hydroxy, O-CO-C₁-C₆-alkyl, O-C₁-C₁₂-alkyl, O-CO-NH₂, O-CO-NH-CO-C₁-C₁₂-alkyl, and O-CO-NH-SO₂-C₁-C₁₂-alkyl; and R² is a hydroxy protecting group, with a primary amine RNH₂,
   wherein R is as defined above, in a suitable organic solvent at room temperature to reflux temperature for about 4 to about 48 hours, extracting, optionally deprotecting, and isolating the desired compound.

In a preferred embodiment of the process immediately above R is hydrogen, C₁-C₆-alkyl, C₃-C₇-cycloalkyl, aryl, substituted-aryl, heteroaryl or substituted-heteroaryl, and the solvent is selected from the group consisting of methylene chloride, tetrahydrofuran, N-methyl-pyrrolidinone, diethyl ether, bis-methoxymethyl ether, dimethyl formamide, acetonitrile, acetone and aqueous mixtures thereof.

The present invention also provides an alternate process for the preparation of a compound having the Formula (I): 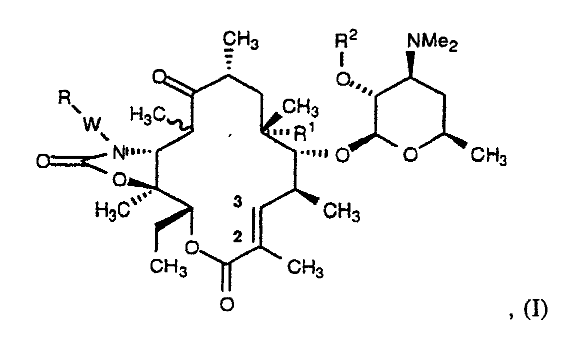 wherein
R¹ is selected from the group consisting of:
   (a) hydrogen:
   (b) hydroxy;
   (c) O-C₁-C₁₂-alkyl;
   (d) O-CO-C₁-C₆-alkyl;
   (e) O-CO-NH₂;
   (f) O-CO-NH-CO-C₁-C₁₂-alkyl; and
   (g) O-CO-NH-SO₂-C₁-C₁₂-alkyl;
R² is hydrogen or a hydroxy protecting group;
R is selected from the group consisting of:
   (a) hydrogen;
   (b) C₁-C₆-alkyl optionally substituted with one or more substituents selected from the group consisting of:
      (i) aryl;
      (ii) substituted-aryl;
      (iii) heteroaryl;
      (iv) substituted-heteroaryl;
      (v) hydroxy;
      (vi) C₁-C₆-alkoxy;
      (vii) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
      (viii) -CH₂-M-R⁵,
      wherein M is selected from the group consisting of:
      (aa) -C(O)-NH-;
      (bb) -NH-C(O)-;
      (cc) -NH-
      (dd) -N=;
      (ee) -N(CH₃)-
      (ff) -O-
      (gg) -S(O)ₙ-, wherein n is 0, 1 or 2;
      (hh) -CO-O-
      (ii) -O-CO-
      (jj) -CO-; and
      R⁵ is selected from the group consisting of:
      (aaa) C₁-C₆-alkyl, optionally substituted with a substituent selected from the group consisting of:
         (i) aryl;
         (ii) substituted-aryl;
         (iii) heteroaryl; and
         (iv) substituted-heteroaryl;
      (bbb) aryl;
      (ccc) substituted-aryl;
      (ddd) heteroaryl;
      (eee) substituted-heteroaryl; and
      (fff) heterocycloalkyl; and
   (c) C₃-C₇-cycloalkyl;
   (d) aryl;
   (e) substituted-aryl;
   (f) heteroaryl;
   (g) substituted-heteroaryl; and
W is selected from the group consisting of -NH-CO-, -N=CH-, -NH- and -N(C₁-C₆-alkyl)-;
   the method comprising:
   (a) treating a compound having the formula: 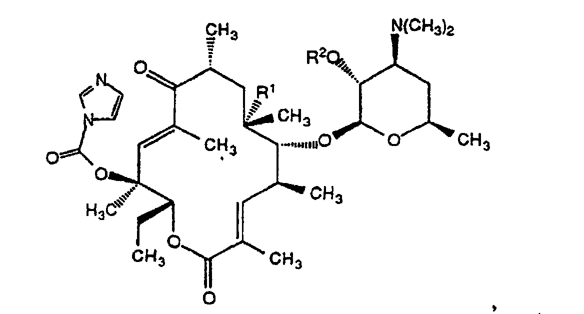 wherein R¹ is selected from the group consisting of hydrogen, protected hydroxy, O-CO-C₁-C₆-alkyl, O-C₁-C₁₂-alkyl, O-CO-NH₂, O-CO-NH-CO-C₁-C₁₂-alkyl, or O-CO-NH-SO₂-C₁-C₁₂-alkyl; and R² is a hydroxy protecting group, with a reagent selected from the group consisting of hydrazine and a substituted hydrazine in a suitable organic solvent at room temperature to reflux for about 4 to about 48 hours to afford the desired compound;
   (b) optionally acylating the compound of Formula (I) obtained in step (a)
      wherein W is -NH- and R is H with an acylating agent to afford a compound of Formula (I)
      wherein W is -NH-CO-;
   (c) optionally condensing the compound of Formula (I) obtained in step (a)
      wherein W is -NH- and R is H with an aldehyde to afford a compound of Formula (I)
      wherein W is -N=CH-;
   (d) optionally reducing the compound of Formula (I) obtained in step (c)
      wherein W is -N=CH- with a reducing agent to afford a compound of Formula (I) wherein W is -NH-;
   (e) and extracting, optionally deprotecting, and isolating the desired compound.

A preferred embodiment of the process immediately above is the one wherein the solvent is selected from the group consisting of methanol, ethanol, propanol, isopropanol, butanol, t-butanol, methylene chloride, tetrahydrofuran, N-methyl-pyrrolidinone, diethyl ether, bis-methoxymethyl ether, dimethyl formamide, acetone, aqueous acetonitrile, aqueous DMF, and aqueous acetone.

In one embodiment of the process immediately above the product is a compound of Formula (I) wherein W is -NH- and R is H, and the hydrazine reagent is hydrazine.

In another embodiment of the process immediately above the product is a compound of Formula (I) wherein W is -N(C₁-C₆-alkyl)-, the hydrazine reagent is a substituted hydrazine RR⁴NNH₂, wherein R is as defined for Formula (I) and R⁴ is C₁-C₆-alkyl.

In still another embodiment of the process immediately above the product is a compound of Formula (I) wherein W is -NH-CO-, the hydrazine reagent is hydrazine, and the product obtained in step (a) having the Formula (I) wherein W is -NH- and R is H is treated with an R-acyl acylating agent, wherein R is as defined for Formula (I). In a preferred embodiment the acylating agent is selected from the group consisting of an acid chloride, an acid fluoride, an acid anhydride, a carboxylic acid in the presence of carbonyldiimidazole, and a carboxylic acid in the presence of 1-(3-dimethylaminopropyl)-3-ethylcarbodiimide hydrochloride.

In yet another embodiment of the process immediately above the product is a compound of Formula (I) wherein W is -N=CH-, the hydrazine reagent is hydrazine, and the product obtained in step (a) having the Formula (I) wherein W is -NH- and R is H is treated with an aldehyde having the formula R-CHO, wherein R is as defined for Formula (I).

In an additional embodiment of the process immediately above the product is a compound of Formula (I) wherein W is -NH- and R is not H, the hydrazine reagent is hydrazine. the product obtained in step (a) having the Formula (I) wherein W is -NH- and R is H is treated with an aldehyde having the formula R-CHO. wherein R is as defined for Formula (I), and the product obtained in step (c) having the Formula (I) wherein W is -N=CH- is treated with a reducing agent. In a preferred embodiment of this process the reducing agent is selected from the group consisting of sodium cyanoborohydride, sodium borohydride, sodium triacetoxyborohydride, borane-tetrahydrofuran complex, and borane-piperidine complex.

The present invention further provides a process for the preparation of a compound having the Formula (I): 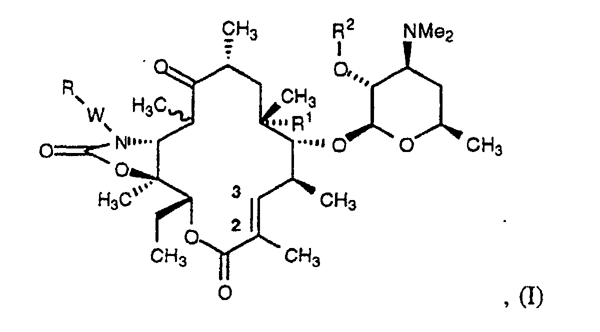 wherein
R¹ is selected from the group consisting of:
   (a) hydrogen;
   (b) hydroxy;
   (c) O-C₁-C₁₂-alkyl;
   (d) O-CO-C₁-C₆-alkyl;
   (e) O-CO-NH₂;
   (f) O-CO-NH-CO-C₁-C₁₂-alkyl; and
   (g) O-CO-NH-SO₂-C₁-C₁₂-alkyl;
R² is hydrogen or a hydroxy protecting group;
R is selected from the group consisting of:
   (a) hydrogen;
   (b) C₁-C₆-alkyl optionally substituted with one or more substituents selected from the group consisting of:
      (i) aryl;
      (ii) substituted-aryl;
      (iii) heteroaryl;
      (iv) substituted-heteroaryl;
      (v) hydroxy;
      (vi) C₁-C₆-alkoxy;
      (vii) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
      (viii) -CH₂-M-R⁵,
      wherein M is selected from the group consisting of:
      (aa) -C(O)-NH-;
      (bb) -NH-C(O);
      (cc) -NH-
      (dd) -N=;
      (ee) -N(CH₃)-
      (ff) -O-
      (gg) -S(O)ₙ-, wherein n is 0, 1 or 2;
      (hh) -CO-O-
      (ii) -O-CO-
      (jj) -CO-; and
      R⁵ is selected from the group consisting of:
      (aaa) C₁-C₆-alkyl, optionally substituted with a substituent selected from the group consisting of:
         (i) aryl;
         (ii) substituted-aryl;
         (iii) heteroaryl; and
         (iv) substituted-heteroaryl;
      (bbb) aryl;
      (ccc) substituted-aryl;
      (ddd) heteroaryl;
      (eee) substituted-heteroaryl; and
      (fff) heterocycloalkyl; and
   (c) C₃-C₇-cycloalkyl;
   (d) aryl;
   (e) substituted-aryl;
   (f) heteroaryl;
   (g) substituted-heteroaryl; and
W is -O-;
the method comprising:
(a) treating a compound having the formula: 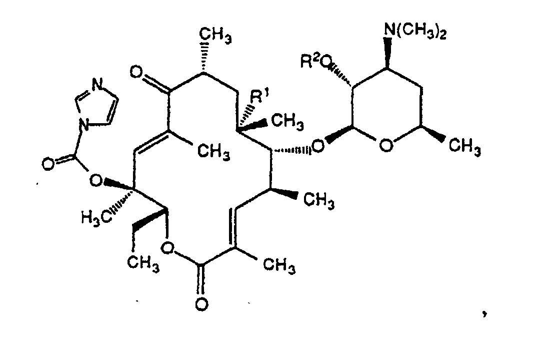 wherein R¹ is selected from the group consisting of hydrogen, protected hydroxy, O-C₁-C₁₂-alkyl, O-CO-C₁-C₆-alkyl, O-CO-NH₂, O-CO-NH-CO-C₁-C₁₂-alkyl, or O-CO-NH-SO₂-C₁-C₁₂-alkyl; and R² is a hydroxy protecting group, with a hydroxylamine reagent selected from the group consisting of unsubstituted hydroxylamine and an *O*-C₁-C₆-alkylated hydroxylamine in a suitable organic solvent at room temperature to reflux for about 4 to about 48 hours, to give the desired compound;
(b) optionally treating the product obtained in step (a) having the Formula (I)
   wherein W is -O- and R is H with a suitable base and an appropriate electrophile having the formula R-L, wherein R is selected from the group consisting of C₁-C₆-alkyl, C₃-C₇-cycloalkyl, aryl, substituted-aryl, heteroaryl and a substituted-heteroaryl group, wherein these terms are as defined for compounds of Formula (I) above and L is suitable leaving group, to give the desired compound of formula (I) wherein W is -O- and R is selected from the group consisting of C₁-C₆-alkyl, C₃-C₇-cycloalkyl, aryl, substituted-aryl, heteroaryl and a substituted-heteroaryl group; and
(c) extracting, optionally deprotecting, and isolating the desired compound.

In one embodiment of the process immediately above the product is a compound of Formula (I) wherein W is -O- and R is H and the hydroxylamine reagent is unsubstituted hydroxylamine.

In one embodiment of the process immediately above the product is a compound of Formula (I) wherein W is -O- and R is *O*-C₁-C₆-alkyl and the hydroxylamine reagent is an *O*-C₁-C₆-alkylated hydroxylamine.

In still another embodiment of the process immediately above the final product is a compound of Formula (I) wherein W is -O- and R=C₁-C₆-alkyl, the hydroxylamine reagent is unsubstituted hydroxylamine, and the intermediate product having the Formula (I)
wherein W =O and R is H is treated with a suitable base and an alkyl halide.

In still another embodiment of the process immediately above the final product is a compound of Formula (I) wherein W is -O- and R is selected from the group consisting of C₃-C₇-cycloalkyl, aryl, substituted-aryl, heteroaryl and a substituted-heteroaryl group, and the intermediate product having the Formula (I) wherein W is -O- and R is H is treated with a suitable base and an electrophile having the formula R-L, wherein R is as defined above and L is a suitable leaving group. In a preferred embodiment of this process the base is selected from the group consisting of sodium hydride, potassium hydride, lithium hydride, lithium diethylamide, and butyllithium, and L is selected from the group consisting of halide, methanesulfonyl and p-toluenesulfonyl.

The present invention also provides a process for the preparation of a compound having the Formula (II): 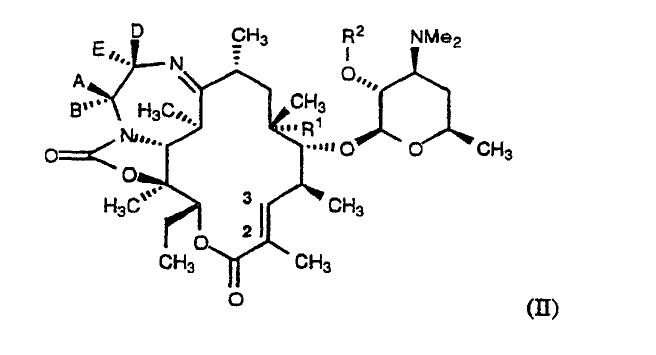 wherein
R¹ is selected from the group consisting of:
   (a) hydrogen;
   (b) hydroxy;
   (c) O-C₁-C₁₂-alkyl;
   (d) O-CO-C₁-C₆-alkyl;
   (e) O-CO-NH₂;
   (f) O-CO-NH-CO-C₁-C₁₂-alkyl; and
   (g) O-CO-NH-SO₂-C₁-C₁₂-alkyl;
R² is hydrogen or a hydroxy protecting group;
A, B, D and E are independently selected from the group consisting of:
   (a) hydrogen;
   (b) C₁-C₆-alkyl, optionally substituted with one or more substituents selected from the group consisting of:
      (i) aryl;
      (ii) substituted-aryl;
      (iii) heteroaryl;
      (iv) substituted-heteroaryl;
      (v) heterocycloalkyl;
      (vi) hydroxy;
      (vii) C₁-C₆-alkoxy;
      (viii) halogen consisting of Br, Cl, F or I; and
      (ix) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
   (c) C₃-C₇-cycloalkyl;
   (d) aryl;
   (e) substituted-aryl;
   (f) heteroaryl;
   (g) substituted-heteroaryl;
   (h) heterocycloalkyl; and
   (i) a group selected from option (b) above further substituted with -M- R⁵, wherein M is selected from the group consisting of
      (aa) -C(O)-NH-;
      (bb) -NH-C(O)-;
      (cc) -NH-
      (dd) -N(CH₃)-
      (ee) -O-
      (ff) -S(O)ₙ-, wherein n is 0, 1 or 2;
      (gg) -C(=NH)-NH-;
      (hh) -CO-O-
      (ii) -O-CO-
      (jj) -CO- ;
   and R⁵ is selected from the group consisting of:
   (aaa) C₁-C₆-alkyl, optionally substituted with a substituent selected from the group consisting of:
      (i) aryl;
      (ii) substituted-aryl;
      (iii) heteroaryl; and
      (iv) substituted-heteroaryl;
   (bbb) aryl;
   (ccc) substituted-aryl;
   (ddd) heteroaryl;
   (eee) substituted-heteroaryl; and
   (fff) heterocycloalkyl;
or any one pair of substituents, consisting of AB, AD, AE, BD, BE or DE, is taken together with the atom or atoms to which they are attached to form a 3- to 7-membered ring optionally containing a hetero function consisting of:
-O-,
-NH-,
-N(C₁-C₆-alkyl-)-,
-N(aryl-C₁-C₆-alkyl-)-,
-N(substituted-aryl-C₁-C₆-alkyl-)-,
-N(heteroaryl-C₁-C₆-alkyl-)-,
-N(substituted-heteroaryl-C₁-C₆-alkyl-)-,
-S- or -S(O)ₙ-, wherein n is 1 or 2;
-C(O)-NH-;
-C(O)-NR⁵-, wherein R⁵ is as defined above;
-NH-C(O)-;
-NR⁵-C(O)-, wherein R⁵ is as defined above; and
-C(=NH)-NH-;
the method comprising:
(a) treating a compound having the formula: 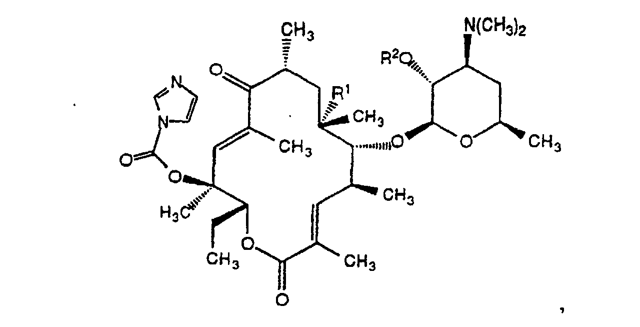 wherein R¹ is selected from the group consisting of hydrogen, protected hydroxy, O-C₁-C₁₂-alkyl, O-CO-C₁-C₆-alkyl, O-CO-NH₂, O-CO-NH-CO-C₁-C₁₂-alkyl, or O-CO-NH-SO₂-C₁-C₁₂-alkyl; and R² is a hydroxy protecting group, with a compound having the formula: 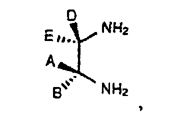 wherein A, B, D, and E are as defined for compounds of Formula (II) above, in a suitable solvent at room temperature to reflux temperature for about 4 to about 48 hours to give the bicyclic intermediate compound having the formula: 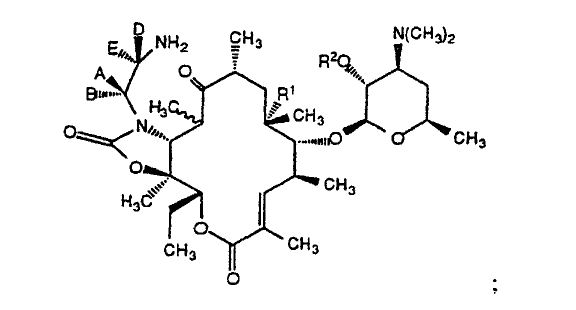
(b) deprotecting said bicyclic intermediate compounds to give the second intermediate compounds having the formula: 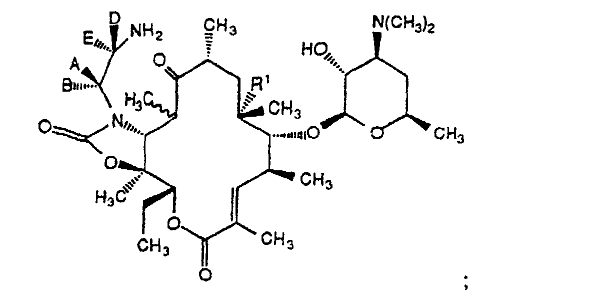
(c) cyclizing said second intermediate compounds by treatment with dilute concentration of a strong acid in a suitable organic solvent for a period of from about 4 hours to about 10 days at a temperature from ambient to reflux temperature of the solvent to give the desired compounds; and
(d) extracting, optionally deprotecting, and isolating the desired compound.

A preferred embodiment of the process immediately above is the one wherein in step (a) the solvent is selected from the group consisting of methanol, ethanol, propanol, isopropanol, butanol, t-butanol, methylene chloride, tetrahydrofuran, N-methyl-pyrrolidinone, diethyl ether, bis-methoxymethyl ether, dimethyl formamide, acetone, aqueous acetonitrile, aqueous DMF, and aqueous acetone; and in step (c) the solvent is selected the group consisting of methanol, ethanol, propanol, iso-propanol, butanol, *iso*-butanol and *t*-butanol.

The present invention provides an alternate process for the preparation of a compound having the Formula (II): 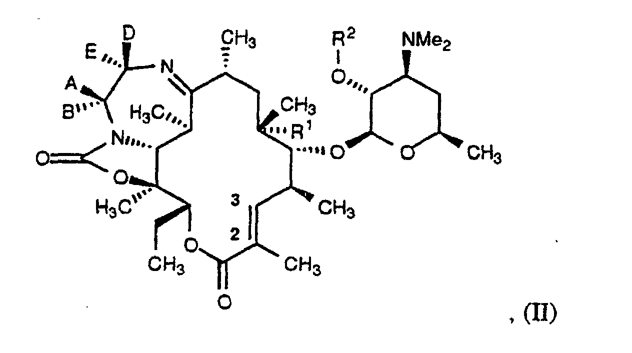 wherein A, B, D, E, R¹ and R² are as defined for Formula (II) above, the method comprising:
(a) treating a compound having the formula: 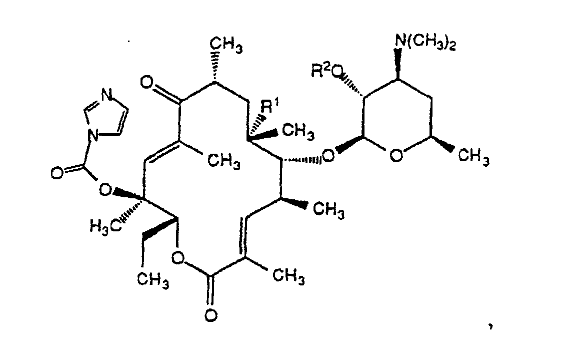 wherein R¹ is selected from the group consisting of hydrogen, protected hydroxy, O-C₁-C₁₂-alkyl, O-CO-C₁-C₆-alkyl, O-CO-NH₂, O-CO-NH-CO-C₁-C₁₂-alkyl, or O-CO-NH-SO₂-C₁-C₁₂-alkyl; and R² is a hydroxy protecting group, with a compound having the formula: 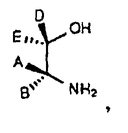 wherein A, B, D, and E are as defined above, in a suitable solvent at 0 - 70 °C for about 4 to about 48 hours to give a bicyclic intermediate compound having the formula: 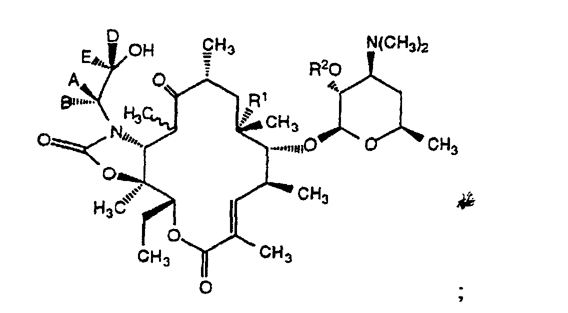
(b) treating the bicyclic intermediate compound from step (a) with triphenylphosphine and diphenylphosphoryl azide-diethylazodicarboxylate in tetrahydrofuran under Mitsunobu reaction conditions to prepare the second intermediate azide compound having the formula: 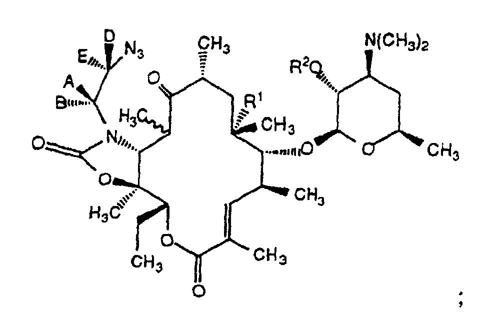
(c) reducing the second intermediate azide compound to prepare the third intermediate compound having the formula: 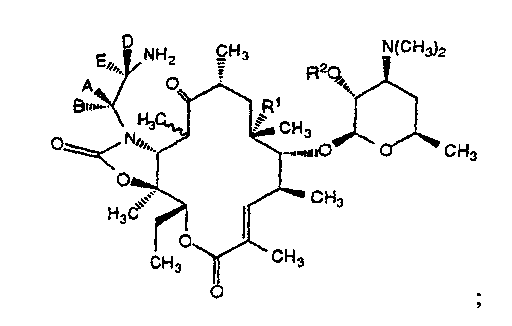
(d) cyclizing said third intermediate compound by treatment with a dilute concentration of a strong acid at ambient temperature to reflux temperature for about 4 hours to about 10 days in a aqueous alcohol solvent to give the desired compounds; and
(e) extracting, optionally deprotecting, and isolating the desired compound.

In a preferred embodiment of the process described immediately above in step (a) the solvent is selected from the group consisting of methanol, ethanol, propanol, isopropanol, butanol, t-butanol, methylene chloride, tetrahydrofuran, N-methyl-pyrrolidinone, diethyl ether, bis-methoxymethyl ether, dimethyl formamide, acetone, aqueous acetonitrile, aqueous DMF and aqueous acetone; in step (c) the reducing agent is selected from the group consisting of triphenylphosphine-water, hydrogen with a catalyst, sodium borohydride, and dialkylaluminum hydride: and in step (d) the solvent is selected the group consisting of methanol, ethanol, propanol, *iso*-propanol, butanol, *iso*-butanol and *t*-butanol.

In an alternate embodiment of the alternate process described immediately above, step (b) thereof is replaced with two steps consisting of:
(b') reacting the hydroxy group of the bicyclic intermediate compound with a sulfonating agent selected from the group consisting of sulfonyl chloride, alkyl sulfonic anhydride, aryl sulfonic anhydride, and tifluoromethanesulfonic anhydride, in an aprotic solvent at -78°C to room temperature to give an intermediate compound wherein the hydroxyl group has been replaced with a sulfonate ester moiety; and
(b") reacting the sulfonate ester of step (b') with an alkali metal azide in an aprotic solvent at from about 0°C to about 100°C to give the second intermediate azide compound.

The present invention also provides a process for the preparation of a compound having the Formula (III): 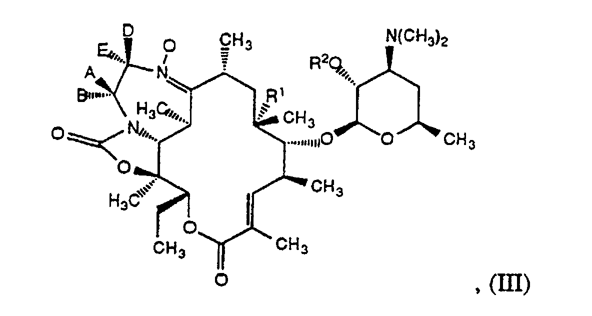 wherein
A, B, D and E are independently selected from the group consisting of:
   (a) hydrogen;
   (b) C₁-C₆-alkyl, optionally substituted with one or more substituents selected from the group consisting of:
      (i) aryl;
      (ii) substituted-aryl;
      (iii) heteroaryl;
      (iv) substituted-heteroaryl;
      (v) heterocycloalkyl;
      (vi) hydroxy;
      (vii) C₁-C₆-alkoxy;
      (viii) halogen consisting of Br, Cl, F or I; and
      (ix) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-,-N(substituted-heteroaryl-C₁-C₆-alkyl-)-, -S- or -S(O)ₙ-, wherein n is 1 or 2;
   (c) C₃-C7-cycloalkyl;
   (d) aryl;
   (e) substituted-aryl;
   (f) heteroaryl;
   (g) substituted-heteroaryl;
   (h) heterocycloalkyl;
      and
   (i) a group selected from option (b) above further substituted with -M-
R⁵, wherein M is selected from the group consisting of
   (aa) -C(O)-NH-;
   (bb) -NH-C(O)-;
   (cc) -NH-
   (dd) -N(CH₃)-
   (ee) -O-
   (ff) -S(O)ₙ-, wherein n is 0, 1 or 2; and
   (gg) -C(=NH)-NH-;
   and R⁵ is selected from the group consisting of:
   (aaa) C₁-C₆-alkyl, optionally substituted with a substituent selected from the group consisting of:
      (i) aryl;
      (ii) substituted-aryl;
      (iii) heteroaryl; and
      (iv) substituted-heteroaryl;
   (bbb) aryl;
   (ccc) substituted-aryl;
   (ddd) heteroaryl;
   (eee) substituted-heteroaryl; and
   (fff) heterocycloalkyl;
   or any one pair of substituents, consisting of AB, AD, AE, BD, BE or DE, is taken together with the atom or atoms to which they are attached to form a 3- to 7-membered ring optionally containing a hetero function consisting of:
   -O-,
   -NH-,
   -N(C₁-C₆-alkyl-)-,
   -N(aryl-C₁-C₆-alkyl-)-,
   -N(substituted-aryl-C₁-C6-alkyl-)-,
   -N(heteroaryl-C₁-C₆-alkyl-)-,
   -N(substituted-heteroaryl-C₁-C₆-alkyl-)-,
   -S- or -S(O)ₙ-, wherein n is 1 or 2;
   -C(O)-NH-;
   -C(O)-NR⁵-, wherein R⁵ is as defined above;
   -NH-C(O)-;
   -NR⁵-C(O)-, wherein R⁵ is as defined above; and
   -C(=NH)-NH-;
R¹ is selected from the group consisting of:
   (a) hydrogen;
   (b) hydroxy;
   (c) O-C₁-C₁₂-alkyl;
   (d) O-CO-C₁-C₆-alkyl;
   (e) O-CO-NH₂;
   (f) O-CO-NH-CO-C₁-C₁₂-alkyl; and
   (g) O-CO-NH-SO₂-C₁-C₁₂-alkyl; and
R² is hydrogen or a hydroxy-protecting group; the method comprising:
   (a) reacting a compound having the formula (II): 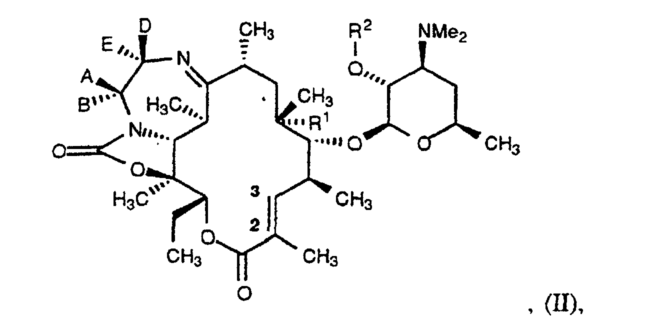 wherein R¹ is as above or is a hydroxy protecting group and R², A, B, D, and E are as defined above, with a suitable oxidizing agent to oxidize the imine nitrogen to the nitrone and the nitrogen atom on the desosamine moiety to the N-oxide to give an N-oxidized intermediate; and
   (b) treating the N-oxidized intermediate with a reducing agent to reduce the desosamine N-oxide, and extracting, optionally deprotecting, and isolating the desired compound.

A preferred embodiment of the process described immediately above is the one
wherein in step (a) the oxidizing agent is selected from the group consisting of hydrogen peroxide and a carboxylic peracid; and in step (b) the reducing agent is selected from the group consisting of triphenylphosphine and hydrogen in the presence of a catalyst.

The invention further provides a process for the preparation of a compound having the Formula (IV): 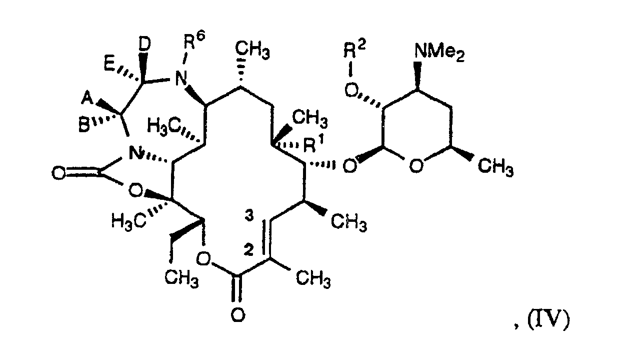 wherein
A, B, D and E are independently selected from the group consisting of:
   (a) hydrogen;
   (b) C₁-C₆-alkyl, optionally substituted with one or more substituents selected from the group consisting of:
      (i) aryl;
      (ii) substituted-aryl;
      (iii) heteroaryl;
      (iv) substituted-heteroaryl;
      (v) heterocycloalkyl;
      (vi) hydroxy;
      (vii) C₁-C₆-alkoxy;
      (viii) halogen consisting of Br, Cl, F or I; and
      (ix) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
   (c) C₃-C₇-cycloalkyl;
   (d) aryl;
   (e) substituted-aryl;
   (f) heteroaryl;
   (g) substituted-heteroaryl;
   (h) heterocycloalkyl;
      and
   (i) a group selected from option (b) above further substituted with -M-R⁵, wherein M is selected from the group consisting of:
      (aa) -C(O)-NH-;
      (bb) -NH-C(O)-;
      (cc) -NH-
      (dd) -N(CH₃)-
      (ee) -O-
      (ff) -S(O)ₙ-, wherein n is 0, 1 or 2; and
      (gg) -C(=NH)-NH-;
         and R⁵ is selected from the group consisting of:
         (aaa) C₁-C₆-alkyl, optionally substituted with a substituent selected from the group consisting of:
            (i) aryl;
            (ii) substituted-aryl;
            (iii) heteroaryl; and
            (iv) substituted-heteroaryl;
         (bbb) aryl;
         (ccc) substituted-aryl;
         (ddd) heteroaryl;
         (eee) substituted-heteroaryl; and
         (fff) heterocycloalkyl;
            or any one pair of substituents, consisting of AB, AD, AE, BD, BE or DE, is taken together with the atom or atoms to which they are attached to form a 3- to 7-membered ring optionally containing a hetero function consisting of
         -O-,
         -NH-,
         -N(C₁-C₆-alkyl-)-,
         -N(aryl-C₁-C₆-alkyl-)-,
         -N(substituted-aryl-C₁-C₆-alkyl-)-,
         -N(heteroaryl-C₁-C₆-alkyl-)-,
         -N(substituted-heteroaryl-C₁-C₆-alkyl-)-,
         -S- or -S(O)ₙ-, wherein n is 1 or 2;
         -C(O)-NH-;
         -C(O)-NR⁵-, wherein R⁵ is as defined above;
         -NH-C(O)-;
         -NR⁵-C(O)-, wherein R⁵ is as defined above; and
         -C(=NH)-NH-;
R¹ is selected from the group consisting of:
   (a) hydrogen;
   (b) hydroxy;
   (c) O-C₁-C₁₂-alkyl;
   (d) O-CO-C₁-C₆-alkyl;
   (e) O-CO-NH₂;
   (f) O-CO-NH-CO-C₁-C₁₂-alkyl; and
   (g) O-CO-NH-SO₂-C₁-C₁₂-alkyl; and
R² is hydrogen or a hydroxy-protecting group;
R⁶ is hydrogen or C₁-C₆-alkyl;
the method comprising:
(a) reacting a compound having the formula: 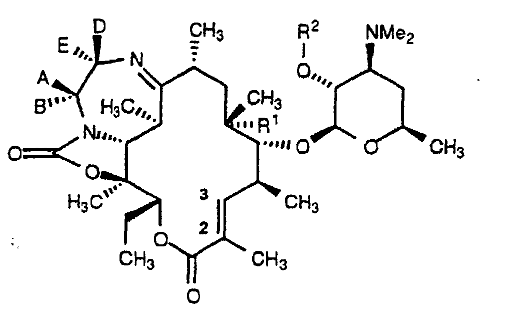 wherein R¹ is as above or is a hydroxy protecting group and R², A, B, D, and E are as defined above with a reducing agent in a suitable organic solvent to afford the desired compound wherein R⁶ is H;
(b) optionally reductively alkylating the amino the product of step (a) with a reducing reagent in the presence of a C₁-C₆-alkyl-group precursor to afford the desired compound wherein R⁶ is C₁-C₆-alkyl; and
(c) extracting, optionally deprotecting, and isolating the desired compound.

A preferred embodiment of the process described immediate above is the one
wherein in step (a) and in optional step (b) the reducing agent is selected from the group consisting of sodium cyanoborohydride, sodium borohydride, sodium triacetoxyborohydride, borane-tetrahydrofuran complex, and borane-piperidine complex.

The invention also provides for a novel intermediate compound having the formula: 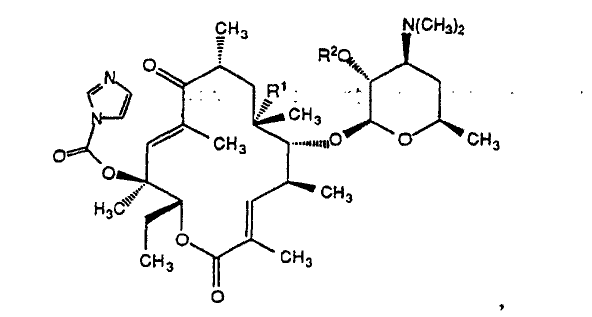 wherein R¹ is selected from the group consisting of:
(a) hydrogen;
(b) protected hydroxy;
(c) O-C₁-C₁₂-alkyl;
(d) O-CO-C₁-C₆-alkyl;
(e) O-CO-NH₂;
(f) O-CO-NH-CO-C₁-C₁₂-alkyl; and
(g) O-CO-NH-SO₂-C₁-C₁₂-alkyl; and R² is hydrogen or a hydroxy-protecting group.

A preferred embodiment of the intermediate compound is that wherein R¹ is O-C₁-C₁₂-alkyl.

A more preferred embodiment of the intermediate compound is that wherein R¹ is methoxy.

The present invention also provides a process for the preparation of a compound having the formula: 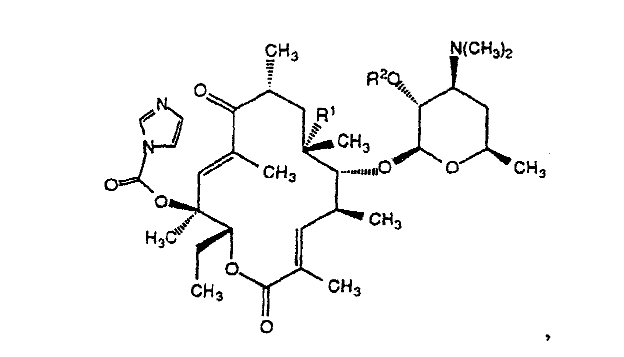 wherein
R¹ is selected from the group consisting of:
   (a) hydrogen;
   (b) protected hydroxy;
   (c) O-C₁-C₁₂-alkyl;
   (d) O-CO-C₁-C₆-alkyl;
   (e) O-CO-NH₂;
   (f) O-CO-NH-CO-C₁-C₁₂-alkyl; and
   (g) O-CO-NH-SO₂-C₁-C₁₂-alkyl; and
R² is hydrogen or a hydroxy-protecting group;
   the method comprising:
   (a) treating an erythromycin A compound having the formula: 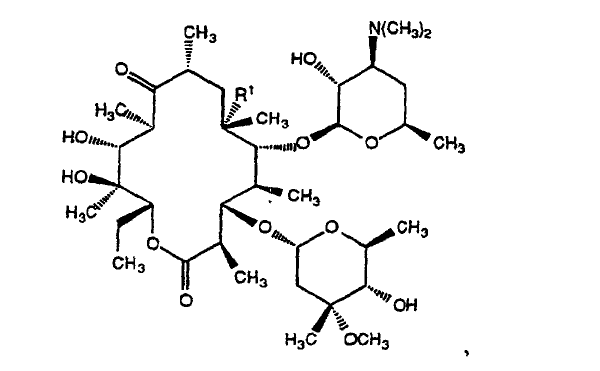 wherein R¹ is as defined above, with dehydrating reagents consisting of an organocarbonate in the presence of base at reflux temperature in an aprotic solvent to form an intermediate compound having the formula: 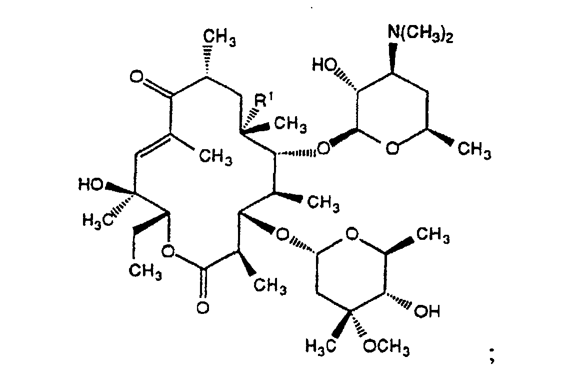
   (b) hydrolytically removing the cladinose moiety from the intermediate compound of step (a) by treatment in an aqueous alcohol suspension with a dilute concentration of a strong acid at ambient temperature for about 0.5 to about 24 hours, extracting and optionally isolating the compound having the formula: 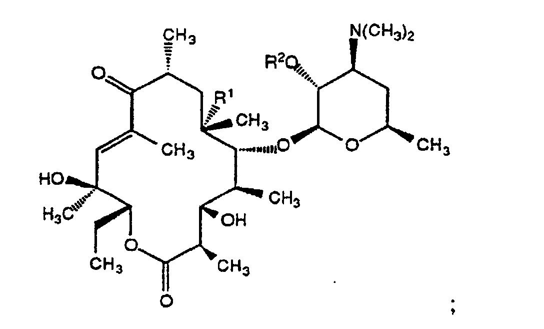
   (c) treating the compound of step (b) with a suitable hydroxy group protecting reagent in an aprotic solvent, and extractively isolating the compound wherein R² is a hydroxy protecting group;
   (d) treating a solution of the compound of step (c) with a sulfonylating agent at from about 0°C to ambient temperature for about 1 to about 24 hours, and extractively isolating the compound having the formula: 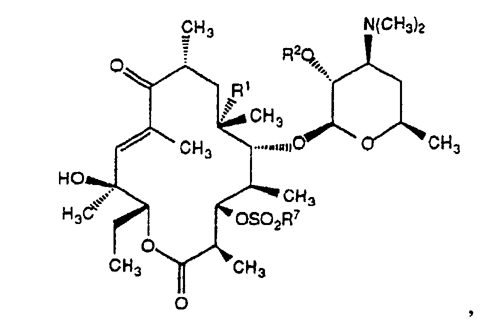 wherein R⁷ is alkyl or aryl;
   (e) dehydrating the compound of step (d) with a hydride base in the presence of carbonyldiimidazole in an aprotic solvent at a temperature from about -20°C to about 70°C for from about 0.5 hours to about 10 days, and extracting, optionally deprotecting, and isolating the desired compound.

In a preferred embodiment of the process immediately above, in step (a) the dehydrating reagents consist of an organocarbonate compound selected from the group consisting of ethylene carbonate, propylene carbonate, trimethylene carbonate, dipropyl carbonate, dibenzyl carbonate, isobutyl carbonate, dimethyl carbonate and diethyl carbonate, in the presence of a base selected from the group consisting of triethylamine, diisopropylethylamine, pyridine, 2,6-dimethylpyridne, 1,8-diazabicyclo[5.4.0]undec-7-ene, N-methylmorpholine, N-methylpyrrolidine, N-methylpiperidine, sodium carbonate, potassium carbonate, sodium bicarbonate and potassium carbonate; in step (b) the alcohol is chosen from the group consisting of methanol, ethanol, propanol, *iso*-propanol, butanol, *iso*-butanol and *t*-butanol, and the acid is selected from the group consisting of hydrochloric acid, sulfuric acid, dichloroacetic acid and trichloroacetic acid; in step (c) the hydroxy group protecting reagent is selected from the group consisting of acetyl chloride, acetic anhydride, benzoic anhydride, benzyl chloroformate, trimethylsilyl chloride and triethylsilyl chloride, and the aprotic solvent is selected from the group consisting of methylene chloride, chloroform, dimethylformamide, tetrahydrofuran, *N*-methylpyrrolidinone and mixtures thereof; in step (d) the sulfonylating agent is selected from the group consisting of methanesulfonyl anhydride, methanesulfonyl chloride, ethanesulfonyl chloride and p-toluenesulfonyl chloride, and the base is selected from the group defined in step (a) above; in step (e) the hydride base is selected from the group consisting of sodium hydride, potassium hydride and lithium hydride and the aprotic solvent is as defined for step (c).

In a more preferred embodiment of the process immediately above R¹ is H and steps (d) and (e) are replaced with a single step (d') consisting of:
(d') treatment of the compound from step (c) with sodium hexamethyldisilazane at from about -50 to about -28°C under an inert atmosphere followed by addition of carbonyldiimidazole at from about 0°C to about ambient temperature for about 15 minutes to about 6 hours, and extracting, optionally deprotecting, and isolating the desired compound.

The present invention also provides an alternate process for the preparation of a novel 3-descladinose-2,3-anhydroerythromycin intermediate compound having the formula: 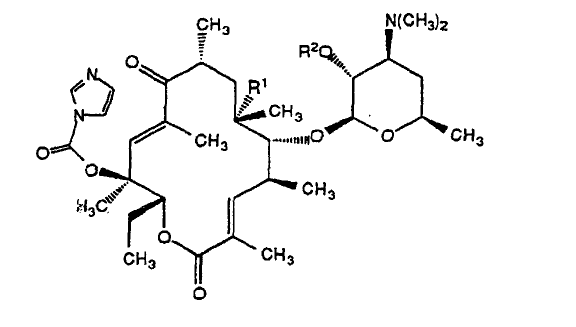 wherein
R¹ is selected from the group consisting of:
   (a) hydrogen;
   (b) protected hydroxy;
   (c) O-C₁-C₁₂-alkyl;
   (d) O-CO-C₁-C₆-alkyl;
   (e) O-CO-NH₂;
   (f) O-CO-NH-CO-C₁-C₁₂-alkyl; and
   (g) O-CO-NH-SO₂-C₁-C₁₂-alkyl; and
R² is hydrogen or a hydroxy-protecting group;
the method comprising:
(a) hydrolytically removing the cladinose moiety from an erythromycin A compound having the formula: 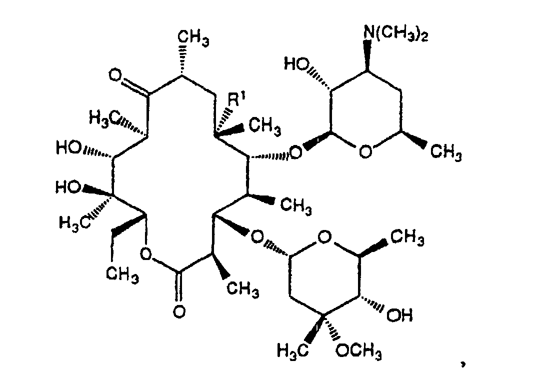 wherein R¹ is as described above by treatment in an aqueous alcohol suspension with a dilute concentration of a strong acid at ambient temperature for about 0.5 to about 24 hours, extracting and optionally isolating the first intermediate compound having the formula: 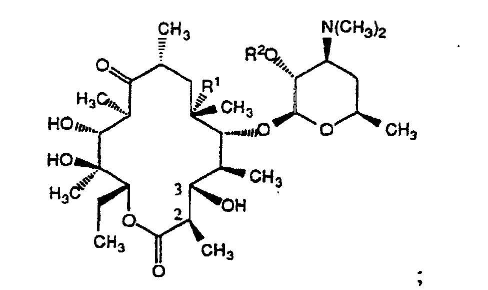
(b) optionally treating the first intermediate compound with a suitable hydroxy group protecting reagent, and extractively isolating the second intermediate compound having the formula of the compound of step (a) wherein R² is a hydroxy-protecting group;
(c) treating the second intermediate compound with an excess of a carbonylating reagent and isolating by aqueous work up the third intermediate compound having the formula: 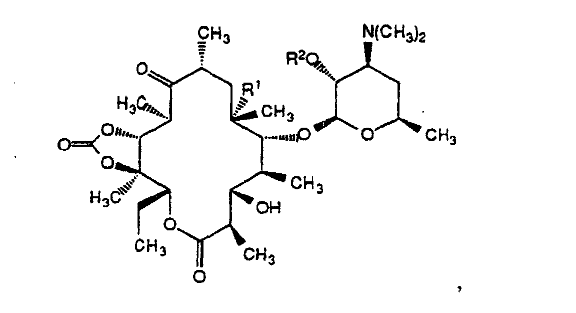 wherein R¹ may not be hydrogen but is otherwise as defined above;
(d) treating the third intermediate compound with a sulfonylating agent at from about 0°C to ambient temperature for about 1 to about 24 hours, and extractively isolating the fourth intermediate compound having the formula: 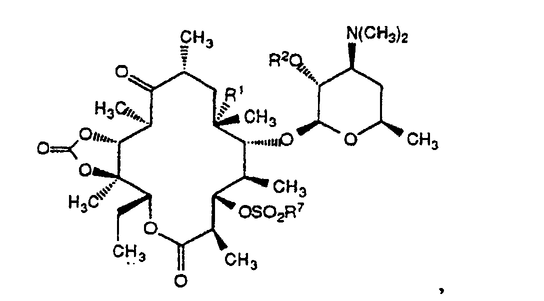 wherein R⁷ is alkyl or aryl;
(e) treating the fourth intermediate compound with a base, extracting and optionally isolating the to afford the fifth intermediate compound having the formula: 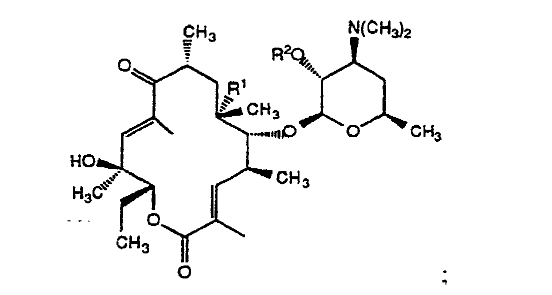
(f) treating the fifth intermediate compound with a hydride base and carbonyldiimidazole in an aprotic solvent at a temperature from about -20°C to about 70°C for from about 0.5 hours to about 10 days, and extracting, optionally deprotecting, and isolating the desired compound.

In a preferred embodiment of the process immediately above in step (a) the alcohol is chosen from the group consisting of methanol, ethanol, propanol, *iso*-propanol, butanol, *iso*-butanol and *t*-butanol, and the acid is selected from the group consisting of hydrochloric acid, sulfuric acid, dichloroacetic acid and trichloroacetic acid; in step (b) the hydroxy group protecting reagent is selected from the group consisting of acetyl chloride, acetic anhydride, benzoic anhydride, benzyl chloroformate, trimethylsilyl chloride and triethylsilyl chloride, and the aprotic solvent is selected from the group consisting of methylene chloride, chloroform, dimethylformamide, tetrahydrofuran, *N*-methylpyrrolidinone and mixtures thereof; in step (c) the carbonylating reagent is selected from the group consisting of phosgene, diphosgene and triphosgene; in step (d) the sulfonylaring agent is selected from the group consisting of methanesulfonyl anhydride, methanesulfonyl chloride, ethanesulfonyl chloride and p-toluenesulfonyl chloride; in step (e) the base is selected from the group consisting of triethylamine, diisopropylethylamine, pyridine, 2,6-dimethylpyridne. 1,8-diazabicyclo[5.4.0]undec-7-ene, N-methylmorpholine, N-methylpyrrolidine, N-methylpiperidine, sodium carbonate, potassium carbonate, sodium bicarbonate and potassium carbonate; in step (f) the hydride base is selected from the group consisting of sodium hydride, potassium hydride and lithium hydride.

In a more preferred embodiment of the process immediately above, in step (b) the hydroxy protecting reagent is benzoic anhydride and R² is benzoyl, and steps (c), (d) and (e) are replaced with a single step (c') consisting of:
(c') treatment of the compound from step (b) with sodium hexamethyldisilazane at from about -50 to about -28°C under an inert atmosphere followed by addition of carbonyldiimidazole at from about 0°C to about ambient temperature for about 15 minutes to about 6 hours, and extracting, optionally deprotecting, and isolating the desired compound.

### Definitions

The terms "C₁-C₃-alkyl", "C₁-C₆-alkyl", "C₁-C₁₂-alkyl" or "C₁-C₁₈-alkyl" as used herein refer to saturated, straight- or branched-chain hydrocarbon radicals containing between one and three, one and six, one and twelve, or one and eighteen carbon atoms, respectively. Examples of C₁-C₃ alkyl radicals include methyl, ethyl, propyl and isopropyl, examples of C₁-C₆-alkyl radicals include, but are not limited to, methyl, ethyl, propyl, isopropyl, *n*-butyl, *tert*-butyl, neopentyl and n-hexyl, examples of C₁-C₁₂-alkyl radicals include all of the preceding examples and n-heptyl, octyl, n-decyl, n-undecyl and n-dodecyl, for example, and examples of C₁-C₁₈-alkyl radicals include all of the preceding examples and n-triadecane, n-tetradecane, n-pentadecane, n-hexadecane, n-heptadecane, and n-octadecane. for example.

The term "C₁-C₆-alkoxy" as used herein refers to an C₁-C₆-alkyl group, as previously defined, attached to the parent molecular moiety through an oxygen atom. Examples of C₁-C₆-alkoxy, but are not limited to, methoxy, ethoxy, propoxy, isopropoxy, *n*-butoxy, *tert*-butoxy, neopentoxy and n-hexoxy.

The term "C₁-C₃-alkyl-amino" as used herein refers to one or two C₁-C₃-alkyl groups, as previously defined, attached to the parent molecular moiety through a nitrogen atom. Examples of C₁-C₃-alkyl-amino include, but are not limited to methylamino, dimethylamino, ethylamino, diethylamino, and propylamino.

The term "aprotic solvent" as used herein refers to a solvent that is relatively inert to proton activity, i.e., not acting as a proton-donor. Examples include, but are not limited to, hydrocarbons, such as hexane and toluene, for example, halogenated hydrocarbons, such as, for example, methylene chloride, ethylene chloride, chloroform, and the like, heterocyclic compounds, such as, for example, tetrahydrofuran and N-methyl-pyrrolidinone, ethers such as diethyl ether and bis-methoxymethyl ether, as well as various other compounds like dimethyl formamide, acetonitrile, acetone and ethyl acetate. Such compounds are well known to those skilled in the art, and it will be obvious to those skilled in the art that individual solvents or mixtures thereof may be preferred for specific compounds and reaction conditions, depending upon such factors as the solubility of reagents, reactivity of reagents and preferred temperature ranges, for example. Further discussions of aprotic solvents may be found in organic chemistry textbooks or in specialized monographs, for example: Organic Solvents Physical Properties and Methods of Purification, 4th ed., edited by John A. Riddick, *et al*., Vol. II, in the Techniques of Chemistry Series, John Wiley & Sons, NY, 1986.

The term "aryl" as used herein refers to unsubstituted carbocyclic aromatic groups including, but not limited to, phenyl, 1- or 2-naphthyl and the like.

The term "C₃-C₅-cycloalkyl- and C₃-C₇-cycloalkyl" as used herein refers to carbocyclic groups of 3 to 5 or 3 to 7 carbons, respectively, for example, cyclopropyl, cyclobutyl, cyclopentyl, cyclohexyl, and cycloheptyl.

The term "C₁-C₃-alkyl-C₃-C₅-cycloalkyl", as used herein refers to a C₃-C₅-cycloalkyl radical, as defined above, attached to a C₁-C₃-alkyl radical by replacement of a hydrogen atom on the latter.

The terms "halo" and "halogen" as used herein refer to an atom selected from fluorine, chlorine, bromine and iodine.

The term "halo-C₁-C₃-alkyl" as used herein refers to a C₁-C₃-alkyl group as defined above wherein 1, 2 or 3 hydrogen atoms thereon are independently replaced by a halogen atom.

The term "heteroaryl", as used herein, refers to a cyclic aromatic radical having from five to ten ring atoms of which one ring atom is selected from S, O and N; zero, one or two ring atoms are additional heteroatoms independently selected from S, O and N; and the remaining ring atoms are carbon, the radical being joined to the rest of the molecule via any of the ring atoms, such as, for example, pyridinyl, pyrazinyl, pyrimidinyl, pyrrolyl, pyrazolyl. imidazolyl. thiazolyl, oxazolyl, isoxazolyl, thiadiazolyl, oxadiazolyl, thienyl, furanyl, quinolinyl, isoquinolinyl, and the like.

The term "heterocycloalkyl" as used herein, refers to a non-aromatic 5-, 6- or 7-membered ring or a bi- or tri-cyclic group comprising fused six-membered rings having between one and three heteroatoms independently selected from oxygen, sulfur and nitrogen, wherein (i) each 5-membered ring has 0 to 1 double bonds and each 6-membered ring has 0 to 2 double bonds, (ii) the nitrogen and sulfur heteroatoms may optionally be oxidized, (iii) the nitrogen heteroatom may optionally be quaternized, and (iv) any of the above heterocyclic rings may be fused to a benzene ring. Representative heterocycles include, but are not limited to, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, and tetrahydrofuryl.

"Hydroxy-protecting group", as used herein, refers to an easily removable group to which are known in the art to protect a hydroxyl group against undesirable reaction during synthetic procedures and to be selectively removable. The use of hydroxy-protecting groups is well known in the art for protecting groups against undesirable reactions during a synthetic procedure and many such protecting groups are known, cf., for example, T.H. Greene and P.G.M. Wuts, Protective Groups in Organic Synthesis, 2nd edition, John Wiley & Sons, New York (1991). Examples of hydroxy-protecting groups include, but are not limited to, methylthiomethyl, *tert*-dimethylsilyl, *tert*-butyldiphenylsilyl, acyl substituted with an aromatic group and the like.

A the term "protected-hydroxy" refers to a hydroxy group protected with a hydroxy protecting group, as defined above, including benzoyl, acetyl, trimethylsilyl, triethylsilyl, methoxymethyl groups, for example.

The term "protogenic organic solvent" as used herein refers to a solvent that tends to provide protons, such as an alcohol, for example, methanol, ethanol, propanol, isopropanol, butanol, t-butanol, and the like. Such solvents are well known to those skilled in the art, and it will be obvious to those skilled in the art that individual solvents or mixtures thereof may be preferred for specific compounds and reaction conditions, depending upon such factors as the solubility of reagents, reactivity of reagents and preferred temperature ranges, for example. Further discussions of protogenic solvents may be found in organic chemistry textbooks or in specialized monographs, for example: Organic Solvents Physical Properties and Methods of Purification, 4th ed., edited by John A. Riddick, *et al*., Vol. II, in the Techniques of Chemistry Series, John Wiley & Sons, NY, 1986.

The term "substituted aryl" as used herein refers to an aryl group as defined herein substituted by independent replacement of one, two or three of the hydrogen atoms thereon with Cl, Br, F, I, OH, cyano, mercapto, nitro, C₁-C₃-alkyl, halo-C₁-C₃-alkyl, C₁-C₆-alkoxy, thio-C₁-C₆-alkoxy, methoxymethoxy, amino, C₁-C₃-alkyl-amino, di(C₁-C₃-alkyl-)amino, formyl, carboxy, alkoxycarbonyl, C₁-C₃-alkyl-CO-O-, C₁-C₃-alkyl-CO-NH-, or carboxamide; except that tetrafluorophenyl and pentafluorophenyl are also included within the definition of 'substituted aryl".

The term "substituted heteroaryl" as used herein refers to a heteroaryl group as defined herein substituted by independent replacement of one, two or three of the hydrogen atoms thereon with Cl, Br, F, I, OH, C₁-C₃-alkyl, C₁-C₆-alkoxy, methoxymethoxy, amino, or C₁-C₃-alkyl-amino, or may also refer to a mono-oxo substituted heteroaryl compound, such as 4-oxo-1H-quinoline, for example.

The term "substituted heterocycloalkyl" as used herein, refers to a heterocycloalkyl group, as defined above, substituted by independent replacement of one, two or three of the hydrogen atoms thereon with Cl, Br, F, I, OH, cyano, mercapto, nitro, C₁-C₃-alkyl, halo-C₁-C₃-alkyl, C₁-C₆-alkoxy, thio-C₁-C₆-alkoxy, methoxymethoxy, amino, C₁-C₃-alkyl-amino, di(C₁-C₃-alkyl-)amino, carboxaldehydo, carboxy, alkoxycarbonyl, C₁-C₃-alkyl-CO-O-, C₁-C₃-alkyl-CO-NH-, or carboxamide.

Numerous asymmetric centers may exist in the compounds of the present invention. Except where otherwise noted, the present invention contemplates the various stereoisomers and mixtures thereof. Accordingly, whenever a bond is represented by a wavy line, it is intended that a mixture of stereo-orientations or an individual isomer of assigned or unassigned orientation may be present.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, *et al.* describe pharmaceutically acceptable salts in detail in J. Pharmaceutical Sciences. 66: 1-19 (1977). The salts can be prepared *in situ* during the final isolation and purification of the compounds of the invention, or separately by reacting the free base function with a suitable organic acid. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate. alginate, ascorbate, aspartate, benzenesulfonate, benzoate, bisulfate, borate, butyrate, camphorate, camphorsulfonate. citrate, cyclopentanepropionate, digluconate, dodecylsulfate, ethanesulfonate. formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, *p*-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As used herein, the term "pharmaceutically acceptable ester" refers to esters which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters includes formates, acetates, propionates, butryates, acrylates and ethylsuccinates.

The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water, isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

Liquid dosage forms for oral administration include gharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol. and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets. dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type may also be employed as fillers in soft and hard-filled gelatin capsules using such excipients as lactose or milk sugar as well as high molecular weight polyethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to the use of pharmaceutical compositions according to the claimed invention, bacterial infections are treated or prevented in a patient such as a human or lower mammal by administering to the patient a therapeutically effective amount of a compound of the invention, in such amounts and for such time as is necessary to achieve the desired result. By a "therapeutically effective amount" of a compound of the invention is meant a sufficient amount of the compound to treat bacterial infections, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

The total daily dose of the compounds of this invention administered to a human or other mammal in single or in divided doses can be in amounts, for example, from 0.01 to 50 mg/kg body weight or more usually from 0.1 to 25 mg/kg body weight. Single dose compositions may contain such amounts or submultiples thereof to make up the daily dose. In general, treatment regimens according to the present invention comprise administration to a patient in need of such treatment from about 10 mg to about 1000 mg of the compound(s) of this invention per day in single or multiple doses.

### Abbreviations

Abbreviations which have been used in the descriptions of the scheme and the examples that follow are: 9-BBN for 9-borabicyclo[3.3.1]nonane; AIBN for azobisisobutyronitrile; Bu₃SnH for tributyltin hydride; CDI for carbonyldiimidazole; DBU for 1,8-diazabicyclo[5.4.0]undec-7-ene; DEAD for diethylazodicarboxylate; DMAP for 4-dimethylaminopyridine; DMF for dimethyl formamide; DPPA for diphenylphosphoryl azide; EtOAc for ethyl acetate; MeOH for methanol; NaHMDS for sodium hexamethyldisilazane; NaN(TMS)₂ for sodium bis(trimethylsilyl)amide; NMMO for N-methylmorpholine N-oxide; TEA for triethylamine; THF for tetrahydrofuran; TPP for triphenylphosphine.

### Synthetic Methods

The compounds and processes of the present invention will be better understood in connection with the following synthetic schemes which illustrate the methods by which the compounds of the invention may be prepared. The groups A, B, D, E, R¹ and R² are as defined above unless otherwise noted below.

### Scheme 1

### Preparation of Intermediate Compound (4)

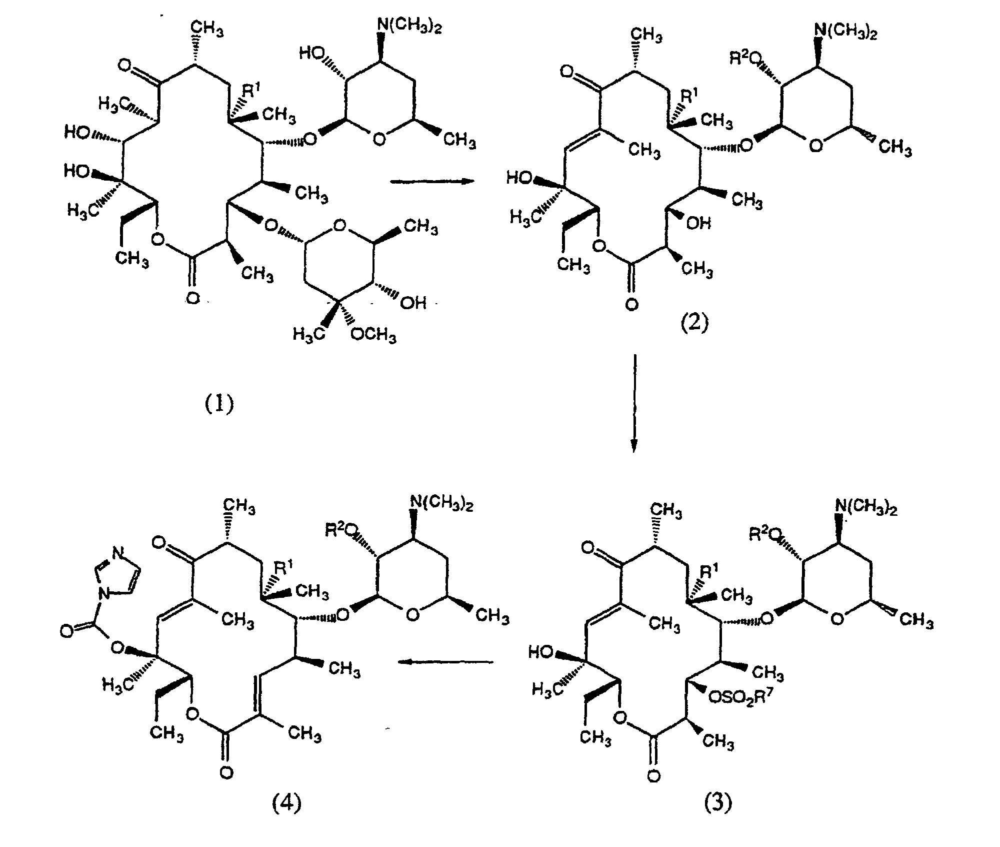

In accordance with Scheme 1 is prepared an intermediate compound 12*-O*-acylimidazolide-2,3 anhydroerythromycin compound (4) used as a starting material in Schemes 3 -5 below. An erythromycin A compound (1) (wherein R¹ is hydrogen, protected hydroxy, O-C₁-C₁₂-alkyl, O-CO-C₁-C₆-alkyl, O-CO-NH₂, O-CO-NH-CO-C₁-C₁₂-alkyl, or O-CO-NH-SO₂-C₁-C₁₂-alkyl, and R²=H or a hydroxy protecting group) is dehydrated at the 11-hydroxy position to form an intermediate compound (1a, not shown) having a C10-C11 double bond. The dehydration may be accomplished by treatment of compound (1) at reflux temperature in an aprotic solvent with an organocarbonate in the presence of base. Suitable organocarbonate compounds include, but are not limited to, ethylene carbonate, propylene carbonate, trimethylene carbonate, dipropyl carbonate, dibenzyl carbonate, isobutyl carbonate, dimethyl carbonate and diethyl carbonate. Suitable bases which may be utilized, include for example, triethylamine, diisopropylethylamine, pyridine, 2,6-dimethylpyridne, 1,8-diazabicyclo[5.4.0]undec-7-ene, N-methylmorpholine, N-methylpyrrolidine, N-methylpiperidine, sodium carbonate, potassium carbonate, sodium bicarbonate and potassium carbonate.

The cladinose sugar moiety is then removed from intermediate compound (1a) by the reaction with a dilute concentration of a strong acid at ambient temperature for about 0.5 to about 24 hours. Suitable strong acids include, but are not limited to, hydrochloric acid, sulfuric acid, dichloroacetic acid, trichloroacetic acid and the like. The reaction may be accomplished with a suspension of the reagents in aqueous alcohol, such as for example, methanol, ethanol, propanol, *iso*-propanol, butanol, *iso*-butanol and *t*-butanol, for example. The reaction mixture is then neutralized with an alkali metal base, the product is extracted with a suitable organic solvent, such as ether, ethyl acetate or methylene chloride, for example, and the organic layer washed and dried. The compound is optionally isolated, but preferably is carried forward in solution.

The 2'-hydroxyl group is then protected by reaction with a suitable hydroxy group protecting reagent (*cf*. T.W.Greene and P.G.M. Wuts, Protective Groups in Organic Synthesis, 2nd ed., John Wiley & Son, Inc., 1991) such as acetyl chloride, acetic anhydride, benzoic anhydride. benzyl chloroformate, trimethylsilyl chloride or triethylsilyl chloride in an aprotic solvent that does not adversely affect the reaction, preferably methylene chloride, chloroform, dimethylformamide, tetrahydrofuran, N-methylpyrrolidinone or a mixture thereof, in the presence of a base such as triethylamine, with stirring at ambient temperature for 0.5 to 24 hours, for example. Preferably, a trialkylsilyl chloride or acetic anhydride is the protecting reagent. Extractive workup as before affords the desired 2'-protected macrolide of the formula (2) wherein R¹ is as above and R² is a hydroxy protecting group. When R¹ is a protected hydroxy group. it is preferred that the protecting group portion of it be the same as the R² protecting group.

Compounds of formula (2) are then reacted with a sulfonylating agent, such as methanesulfonyl anhydride, methanesulfonyl chloride, ethanesulfonyl chloride, or p-toluenesulfonyl chloride, in a aprotic solvent with stirring at from about 0°C to ambient temperature for about Ito about 24 hours. The crude product is isolated via an extractive workup similar to that described above to afford the desired 3-0-methanesulfonylated macrolide of the formula (3). wherein R⁷ is an alkyl or aryl residue, such as methyl, ethyl or p-tolyl.

Treatment of compound (3) with a hydride base in the presence of carbonyldiimidazole in an aprotic solvent gives, after an extractive workup, the desired 12-*O*-acylimidazolide-2,3 anhydro macrolide (4). The hydride base may be, for example, sodium hydride, potassium hydride, or lithium hydride, and the aprotic solvent may be one as defined above. The reaction may require cooling or heating, depending on the conditions used. The reaction temperature may be from about -20°C to about 70°C, and preferably from about 0°C to about room temperature. The reaction may require about 0.5 hours to about 10 days, and preferably about 1-5 days, to complete.

In an alternate process for compounds wherein R¹ is H, it is possible replace the sulfonylating and dehydrating steps described above with two different steps: first treatment with NaHMDS at from about -50 to to about -28°C under an inert atmosphere followed by addition of carbonyldiimidazole at from about 0°C to about ambient temperature for about 15 minutes to about 6 hours, or until the reaction is complete. The compound (4) is obtained after quenching of the reaction and extraction of the product.

Scheme 2 provides for an alternate method of synthesis of the macrolide compound (4). Hydrolytic removal of the cladinose moiety from compound (1), wherein R¹ is as defined in Scheme 1 and R² is H is accomplished by the procedure described for Scheme 1, followed by protection of the 2'- hydroxyl group, also by the procedure of Scheme 1, affords the macrolide compound (5). When R¹ is a protected hydroxy group, it is preferred that the protecting group portion of it be the same as the R² protecting group.

Subsequent treatment of compound (5) with an excess of carbonylating reagent, such as phosgene, diphosgene, or triphosgene, for example, in an aprotic solvent followed by aqueous workup yields the 11,12-carbonate (5a, not shown) in which the 3-hydroxy group is unprotected.

Sulfonylation of the 3-hydroxy group of compound (5a) by procedures similar to those described above for macrolide compound (3) in Scheme 1 affords the desired compound (6) wherein R⁷ is as defined in Scheme 1.

Treatment of compound (6) with a base in an aprotic solvent affords the diene macrolide (7). Suitable bases which may be utilized, include for example, triethylamine, diisopropylethylamine, pyridine, 2,6-dimethylpyridne, 1,8-diazabicyclo[5.4.0]undec-7-ene, N-methylmorpholine, N-methylpyrrolidine, N-methylpiperidine, sodium carbonate, potassium carbonate, sodium bicarbonate and potassium carbonate. Compound (7) can be treated with a hydride base and carbonyldiimidazole in an aprotic solvent to give the key intermediate macrolide (4).

In an alternate procedure for Scheme 2 wherein compound (1) is the 6-deoxy-erythromycin A compound (R¹ is H), removal of the cladinose residue from compound (1) and protection of the 2'- hydroxyl group to give the macrolide compound (5), wherein R¹ is H and R² is benzoyl follows the methods described above. However, the compound (5), wherein R¹ is H and R² is benzoyl may then be treated directly with an excess of sodium hexamethyldisilazane at -28 to -50°C under an inert atmosphere, then with carbonyldiimidazole at 0°C or at ambient temperature stirring for 15 minutes to 6 hours or until the reaction is complete to obtain the compound (4).

### Scheme 2

### Alternate Preparation of Intermediate Compound (4)

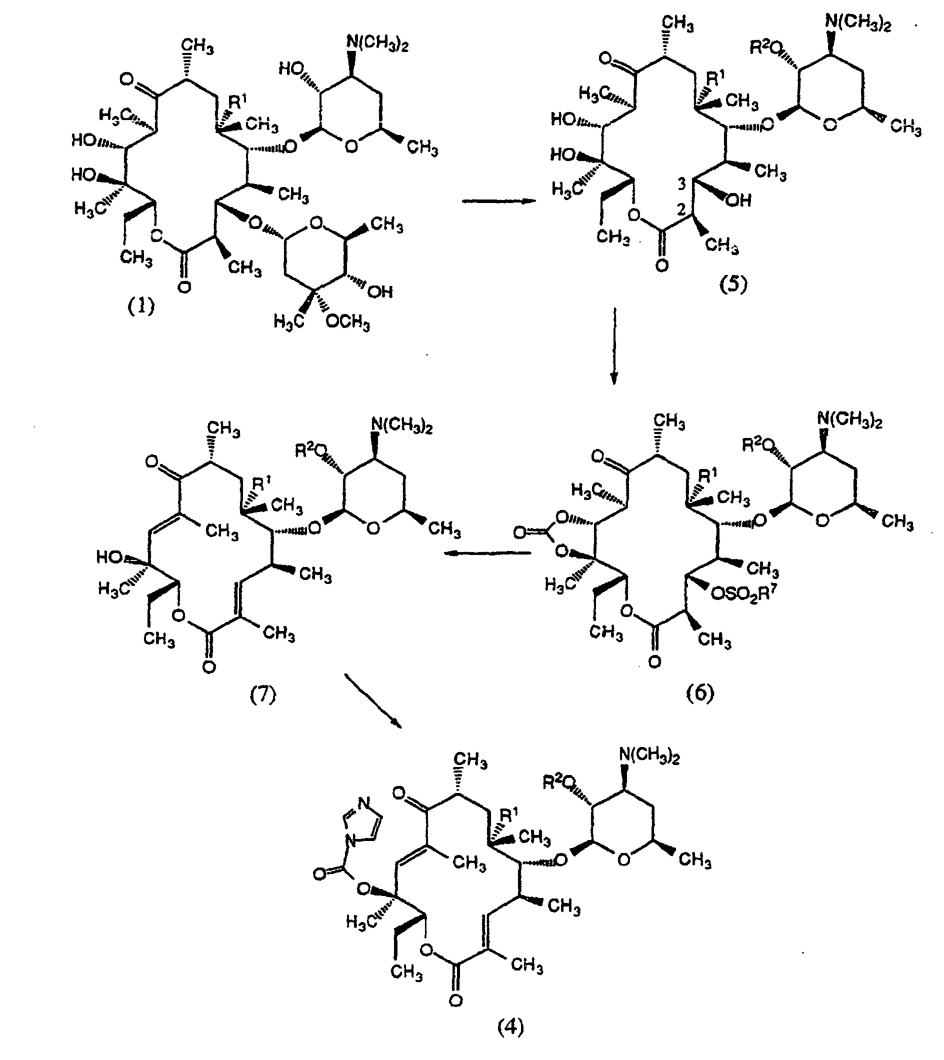

### Scheme 3

### Preparation of compounds of Formulas (I) and (II)

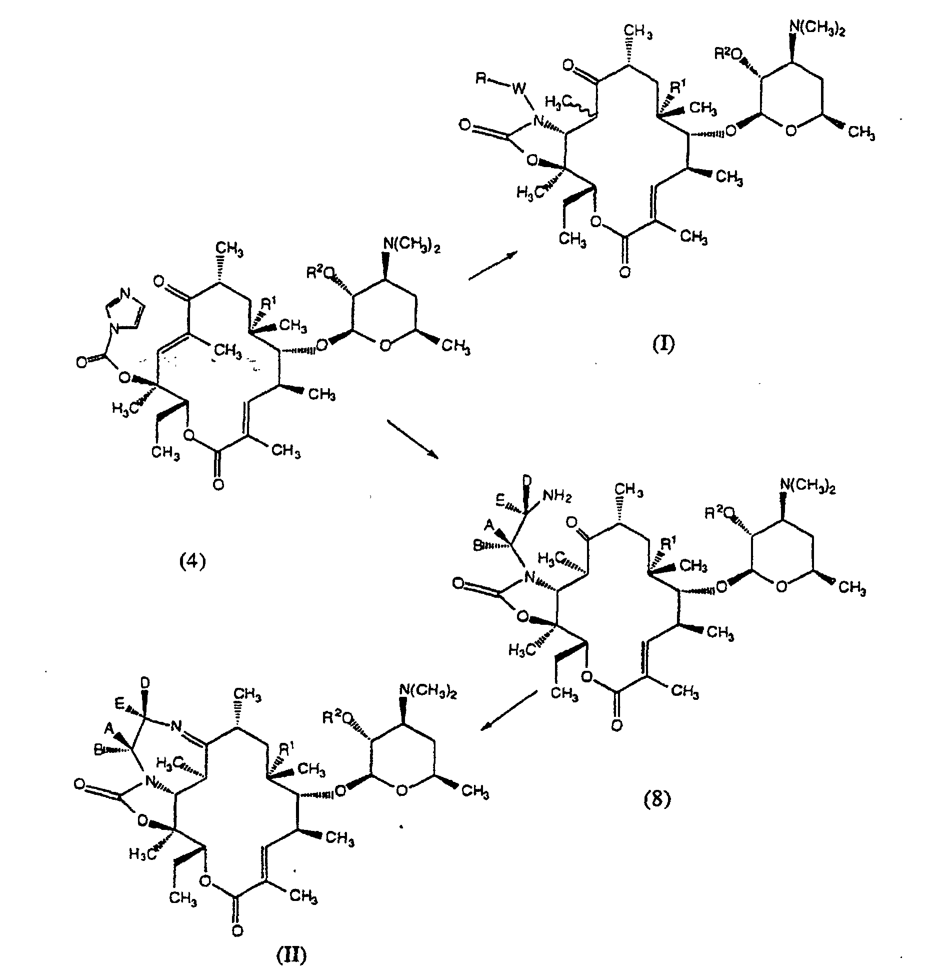

In accordance with Scheme 3 compound (4), wherein R¹ and R² are as defined in Scheme 1, is converted to desired compounds of the invention having Formulas (I) or (II).

To prepare a compound of Formula (I) wherein W is absent, compound (4) is reacted with a primary amine RNH₂ in a suitable solvent at room temperature to reflux temperature for about 4 to about 48 hours. Suitable solvents include alcohols such as methanol, ethanol, propanol, isopropanol, butanol, t-butanol, and the like, aprotic solvents such as methylene chloride, tetrahydrofuran, N-methyl-pyrrolidinone, diethyl ether, bis-methoxymethyl ether, dimethyl formamide, and acetone, for example, as well as aqueous mixtures thereof. Preferred solvents are aqueous acetonitrile. aqueous DMF, and aqueous acetone.

In the primary amine RNH₂ and in the resulting compound of Formula (I), R may be hydrogen, C₁-C₆-alkyl, C₃-C₇-cycloalkyl, aryl, substituted-aryl, heteroaryl or substituted-heteroaryl. When R is a C₁-C₆-alkyl substituent, the alkyl group may be optionally substituted with one or more substituents such as aryl, substituted-aryl, heteroaryl, substituted-heteroaryl, hydroxy, C₁-C₆-alkoxy, NR³R⁴, wherein R³ and R⁴ are independently selected from hydrogen and C₁-C₆-alkyl, or NR³R⁴, wherein R³ and R⁴ are taken with the nitrogen atom to which they are connected to form a 3- to 7-membered ring. In the instance wherein the NR³R⁴ substituent is a 5- to 7-membered ring, the ring may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2. Additionally, when R is C₁-C₆-alkyl it may bear an optional substituent of the formula -CH₂-M-R⁵, wherein M may be -C(O)-NH-, -NH-C(O)-, -NH-, -N=, -N(CH₃)-, -O-, -S(O)ₙ-, wherein n is 0, 1 or 2, -CO-O-, -O-CO-, or -CO-; and R⁵ may be aryl, substituted-aryl, heteroaryl, substituted-heteroaryl, heterocycloalkyl, or a C₁-C₆-alkyl optionally bearing one or more substituents such as aryl, substituted-aryl, heteroaryl, or substituted-heteroaryl. Chromatographic treatment of the crude reaction product affords both the natural and *epi* isomers at position C-10 of the molecule.

To prepare a compound wherein W is -NH-, compound (4) is reacted with a hydrazine reagent such as unsubstituted hydrazine or a substituted hydrazine in a solvent such as described immediately above to afford the desired compound of Formula (I). The natural and C-10 epimers of these compounds may be isolated from the reaction mixture.

Thus, treatment of compound (4) with unsubstituted hydrazine affords the compound of Formula (I) wherein W is -NH- and R is H.

Also, treatment of (4) with a substituted hydrazine RR⁴NNH₂, wherein R is as defined for Formula (I) and R⁴ is C₁-C₆-alkyl, gives the compounds of Formula (I) wherein W is -N(C₁-C₆-alkyl)-.

Optionally, the compound of Formula (I) wherein W is -NH- and R is H can be treated with an R-acyl acylating agent, wherein R is as defined for Formula (I), to afford a compound of Formula (I) wherein W is -NH-CO-. The acylating agents can be, for example, an acid chloride, an acid fluoride, an acid anhydride, or a carboxylic acid in the presence of a carbodiimide coupling reagent such as carbonyldiimidazole or 1-(3-dimethylaminopropyl)-3-ethylcarbodiimide hydrochloride, for example, wherein R is as defined above.

Optionally, the compound of Formula (I) wherein W is -NH- and R is H can be treated with an aldehyde R-CHO, wherein R is as defined for Formula (I), to afford a compound of Formula (I) wherein W is -N=CH-.

Optionally, the compounds of Formula (I) wherein W is -N=CH- can be reduced to yield additional compounds of Formula (I) above, wherein W is -NH- using reducing reagents such as sodium cyanoborohydride, sodium borohydride, sodium triacetoxyborohydride, borane-tetrahydrofuran complex, and borane-piperidine complex, for example.

Also shown in Scheme 3 is the procedure by which compounds of Formula (I) wherein W is -O- and R is H or *O*-C₁-C₆-alkyl may be prepared. Under reaction conditions similar to those shown above for hydrazine reagents, treatment of compound (4) with unsubstituted hydroxylamine or an *O*-C₁-C₆-alkylated hydroxylamine affords the desired compound.

For example, treatment of compound (4) with an excess of hydroxylamine affords the compound of formula (I) wherein W is -O- and R is H.

Treatment of compound (4) with an *O*-C₁-C₆-alkylated hydroxylamine affords the desired compound of Formula (I) wherein W is -O- and R is C₁-C₆-alkyl.

Optionally, it is possible to further treat the compound of Formula (I) wherein W is -O- and R is H with a suitable base and an appropriate electrophile to prepared a compound wherein W is -O- and R is C₁-C₆-alkyl, C₃-C₇-cycloalkyl, aryl, substituted-aryl, heteroaryl or a substituted-heteroaryl group, wherein these terms are as defined for compounds of Formula (I) above. The base may be an alkali metal hydride or an organo-alkali metal compound, including but not limited to sodium hydride, potassium hydride, lithium hydride, lithium diethylamide, and butyllithium. The electrophile is a compound having the formula R-L, wherein R is as defined immediately above, and L is halide or another suitable leaving group, such as a methanesulfonyl or p-toluenesulfonyl moiety.

Optional deprotection of any of the compounds wherein W is -O- may be accomplished by standard methods as described by Wuts and Greene (op. cit.).

Compounds of Formula (II) may also be synthesized as outlined in Scheme 3. Thus, a starting material compound of formula (4) is reacted with a 1,2-diamine compound with a compound having the formula: 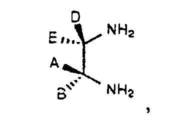 wherein A, B, D, and E are as defined above, in a suitable solvent at room temperature to reflux temperature for about 4 to about 48 hours to give the bicyclic compound of formula (8). The 1,2-diamine compound may have substituents A, B, D and E, as defined above for the compounds of Formula (II), but with C2 or Cs symmetry or A=B=H. Suitable solvents include alcohols such as methanol, ethanol, propanol, isopropanol, butanol, t-butanol, and the like, aprotic solvents such as methylene chloride, tetrahydrofuran, N-methyl-pyrrolidinone, diethyl ether, bis-methoxymethyl ether, dimethyl formamide, and acetone, for example, as well as aqueous mixtures thereof. Preferred solvents are aqueous acetonitrile, aqueous DMF, and aqueous acetone.

The 2'-hydroxy protecting group on compound (8) is then removed by standard methods as described by Wuts and Greene (*op. cit.*). When OR² is an ester, for example, such as acetate or benzoate, the compound is preferably deprotected by treatment with methanol or ethanol. When R² is a trialkylsilyl group, the compound may be deprotected by treatment with fluoride in THF or acetonitrile. The reaction time required may be from about 1 to about 24 hours.

The deprotected compound of formula (8) wherein R² is H is then cyclized to give compounds of Formula (II) by treatment with a dilute concentration of a strong acid at ambient temperature to reflux temperature for about 4 hours to about 10 days in a suitable organic solvent. Suitable acids include, but are not limited to, hydrochloric acid, sulfuric acid, dichloroacetic acid, trichloroacetic acid and the like. The reaction may be accomplished with a suspension of the reagents in aqueous alcohol, such as for example, methanol, ethanol, propanol, *iso*-propanol, butanol, *iso*-butanol and *t*-butanol, for example.

Optional deprotection may be accomplished by standard methods as described by Wuts and Greene (op. cit.).

Scheme 4 illustrates an alternate preparation for compounds of formula (II). Starting material (4) is reacted with a compound having the formula: 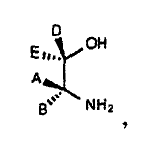 wherein A, B, D, and E are as defined above, in a suitable solvent at 0 - 70 °C for about 4 to about 48 hours to give compound (9) where Y=OH. Suitable solvents are those such as methanol. ethanol, propanol, isopropanol, butanol, t-butanol, methylene chloride, tetrahydrofuran, N-methyl-pyrrolidinone, diethyl ether, bis-methoxymethyl ether, dimethyl formamide, acetone, aqueous acetonitrile, aqueous DMF, and aqueous acetone, for example.

### Scheme 4

### Alternate Preparation of compounds of Formula (II)

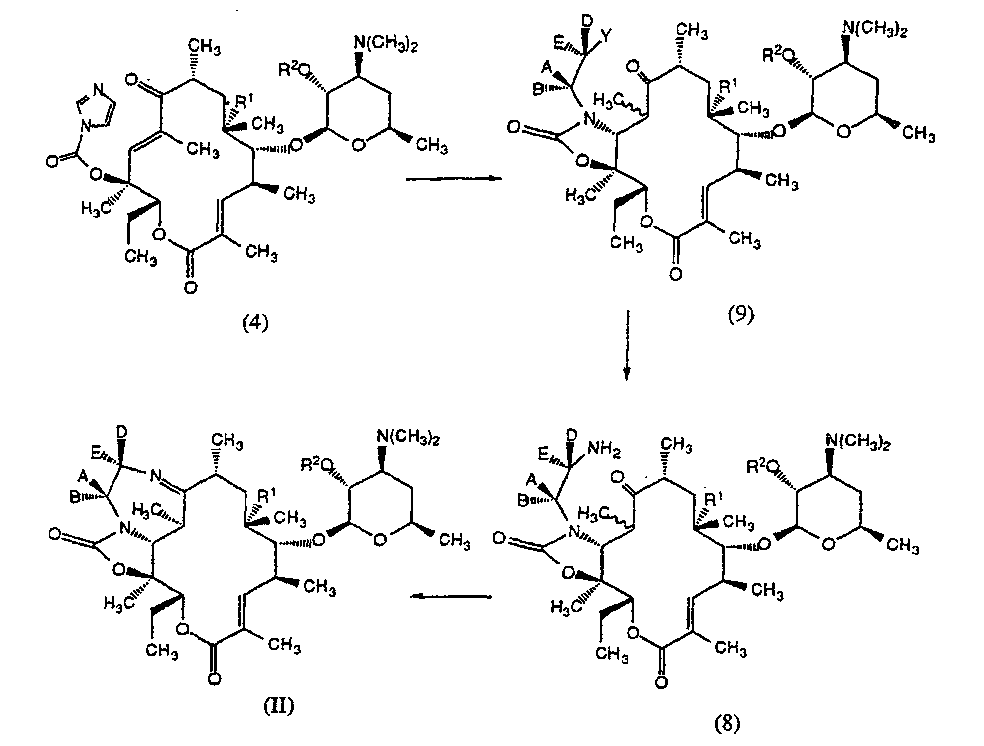

The azido intermediate, compound (9) Y=N₃, is prepared by Mitsunobu reaction by treating compound (9) wherein Y=OH with triphenylphosphine and diphenylphosphoryl azide-DEAD in tetrahydrofuran under Mitsunobu reaction conditions. Compound (9) wherein Y=N₃ is then deprotected by standard methods as described by Wuts and Greene *(op. cit*.). When OR² is an ester, for example, such as acetate or benzoate, the compound may be preferably deprotected by treatment with methanol or ethanol. When R² is a trialkylsilyl group, the compound may be deprotected by treatment with fluoride in THF or acetonitrile, for example.

The azido intermediate, compound (9) wherein Y=N₃, is then reduced to the amino compound (9) wherein Y=NH₂. Preferable reducing reagents are triphenylphosphine-water, hydrogen with a catalyst, sodium borohydride, or dialkylaluminum hydride.

Compound (9) wherein Y=NH₂ is then cyclized to prepare the compound of Formula (II) by treatment with a dilute concentration of a strong acid at ambient temperature to reflux temperature for about 4 hours to about 10 days in a suitable organic solvent. Suitable acids include, but are not limited to, hydrochloric acid, sulfuric acid, dichloroacetic acid, trichloroacetic acid and the like. The reaction may be accomplished with a suspension of the reagents in aqueous alcohol, such as for example, methanol, ethanol, propanol, *iso*-propanol, butanol, *iso*-butanol and *t*-butanol, for example. This treatment also removes protecting groups at positions R¹ and R².

Alternately, the hydroxy group (Y=OH) in compound (9) may be activated by treatment with a sulfonating agent, such as sulfonyl chloride, alkyl or aryl sulfonic anhydride or trifluoromethanesufonic anhydride, in an aprotic solvent (e.g., diethyl ether, dichloromethane, tetrahydrofuran, chloroform, pyridine or a mixture thereof) to give the compound (9) wherein Y is a sulfonate ester. The reaction requires cooling or heating, depending on the conditions used. The reaction temperature is preferably -100°C to 10 °C. The reaction may require 20 minutes to 24 hours to complete. The sulfonate ester activated hydroxy group in (9) (e.g. Y=-OSO₂CF₃) is then converted to an azide to give the second intermediate azide compound (9, Y=N₃) by reacting with an alkali metal azide, such as lithium azide or sodium azide, in the same solvent defined above. The reaction temperature is preferably about 0°C to about 100°C. The azido compound is then converted to compound (8) according to the procedures described above.

As outlined in Scheme 5 below, the tricyclic macrolides of formula (II), wherein substituents A, B, D and E are as defined above, can be further transformed into macrolides having the formulas (III) and (IV). Treatment of the imine nitrogen atom of compound (II) with a suitable oxidizing agent, such as hydrogen peroxide or a carboxylic peracid, oxidizes the imine nitrogen to the nitrone and the nitrogen atom on the desosamine moiety to the N-oxide, to give an N-oxidized intermediate which is directly treated with a reducing agent such as triphenylphosphine or hydrogen in the presence of a catalyst, for example, to reduce the desosarnine N-oxide, to give the desired compound of formula (III). Optional deprotection may be accomplished by standard methods as described by Wuts and Greene (*op. cit.*).

Macrolides of the formula (II) can also be treated with reducing agents such as sodium cyanoborohydride at pH 4-5 or sodium borohydride in a suitable organic solvent to yield the tricyclic amine of the formula (IVa), which is a compound of formula (IV) wherein R⁶ is H. Compounds of the formula (IVa) can be further transformed into compounds of type (IVb), which are compounds of formula (IV) wherein R⁶ is C₁-C₆-alkyl, via reductive alkylation of the amine with a reducing reagent, preferably sodium cyanoborohydride, sodium borohydride, sodium triacetoxyborohydride, borane-tetrahydrofuran complex, or borane-piperidine complex, in the presence of a C₁-C₆-alkyl-group precursor. Optional deprotection may be accomplished by standard methods as described by Wuts and Greene (*op. cit*.).

### Scheme 5

### Preparation of compounds (III) and (IV)

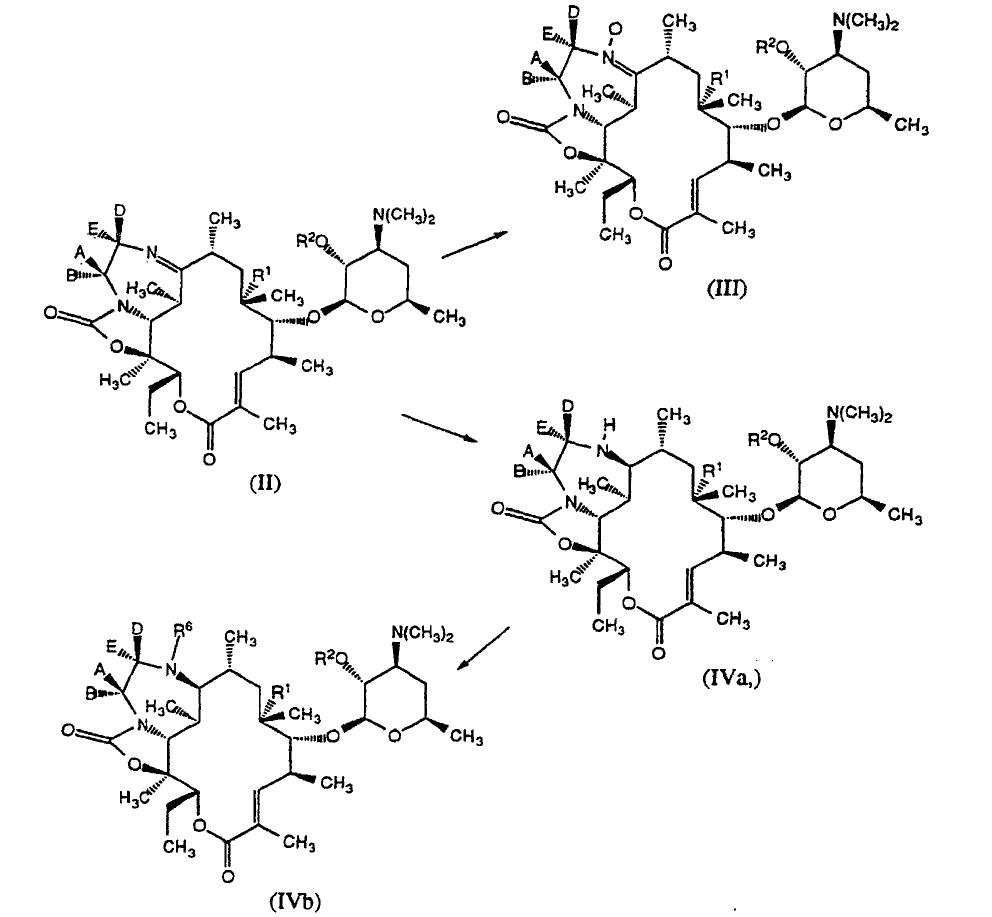

The compounds and processes of the present invention will be better understood in connection with the following examples, which are intended as an illustration of and not a limitation upon the scope of the invention.

### Examples

The procedures described above for preparing the compounds of the present invention will be better understood in connection with the following examples, which are intended as an illustration of and not a limitation upon the scope of the invention. Various changes and modifications to the disclosed embodiments will be apparent to those skilled in the art. Such changes and modifications, including without limitation those relating to the chemical structures, substituents, derivatives, intermediates, syntheses, formulations and/or methods of use of the invention, may be made without departing from the scope thereof.

The NMR data for the central portion of the erythromycin compounds exemplified below are given in Table 1, which is placed after Example 133.

### Example 1

### Preparation Of Intermediate Compound (3) (Scheme 1); R¹=OMe

### Step 1a. Compound of Formula (2) Scheme 1; R¹=OMe; R²=H

A suspension of Clarithromycin (MW = 747.97, 98.48g, 131.69 mmol, obtained from Abbott Laboratories), ethylene carbonate (50 mL) and triethylamine (200 mL) was refluxed for 29 hours, additional ethylene carbonate (-30 mL) was added, and the reaction mixture refluxed for an additional 18 hours. The triethylamine was removed *in vacuo*, 2% aqueous HCl (600 mL) and EtOH (50 mL) were added (pH=1-2), and the mixture was stirred at room temperature for 24 hours. Subsequent basification with 10% aqueous NaOH to pH ∼12-14 yielded a precipitate. The aqueous layer was decanted, and the precipitate was taken up in EtOAc (500 mL), which was washed with 200-mL portions of saturated aqueous NaHCO₃, H₂O and brine. The solution was dried (MgSO₄) and concentrated to afford the crude product as a light brown foam. Decolorization with charcoal and silica gave 65.94 g of the tide compound (87%). MS m/z 572 (M+H)⁺.

### Step 1b. Compound of Formula (3) (R¹=OMe; R²=CH₃CO)

A solution of the compound from step 1a (25.00 g, 43.72 mmol), acetic anhydride (8.25 mL, 87.45 mmol) and triethylamine (12.18 mL, 87.45 mmol) in CH₂C₁₂ (250 mL) was stirred at room temperature for 7 hours. Then the organic layer was washed with 100 mL portions of saturated aqueous NaHCO₃ (2 x), H₂O (1 x), brine (1 x), dried (MgSO₄), and concentrated to afford the crude product as a brown foam (26.88 g, quantitative crude yield). MS m/z 614 (M+H)⁺.

### Step 1c. Compound of Formula (3) (R¹=OMe; R²=CH₃CO)

A solution of the compound from step 1b (26.84 g, 43.72 mmol) and methanesulfonic anhydride (9.14 g, 52.47 mmol) in pyridine (40 mL) was stirred at room temperature for 24 hours. Then the pyridine was removed *in vacuo*, and the resulting solid was washed with 300-mL portions of saturated aqueous NaHCO₃ (2 x), H₂O (1 x), and hexane (1 x) to afford the crude product as a brown solid (27.65 g, 91% crude yield). MS m/z 692(M+H)⁺.

### Step 1d. Compound of Formula (4) (R²=CH₃CO; R¹=OMe)

A solution of the compound from step 1c (5.00 g, 7.22 mmol) in a mixture of DMF (45 mL) and THF (15 mL) was treated with CDI (5.86g, 36.1 mmoL) followed by NaH (1.15g, 60 wt %, 28.9 mmoL) at 0°C under N₂. The cooling bath was removed, and the reaction mixture was stirred at room temperature for 2 hours. The reaction mixture was then slowly quenched into a mixture of saturated aqueous NaHCO₃ (150 mL) and EtOAc (300 mL). The layers were separated, and the aqueous layer was washed with an additional portion of EtOAc (150 mL), then the combined organic layer was washed with 150-mL portions of H₂O (3 x) and brine (3x) and dried (MgSO₄). The solvent was removed to afford the crude product as a white solid (5.28 g, theory=4.98 g). MS m/z 590 (M-112)⁺.

### Example 2

### Compound of Formula (I); R¹=H; R2=H; W is absent; R=4-phenylbutyl

### Step 2a. Compound (5) (Scheme 2), R¹=H; R²=H

6-Deoxyerythromycin A (50 g, 72 mmol, obtained from by the procedures of McAlpine, *et al*., 30th Interscience Conference on Antimicrobial Agents, Atlanta, US, (1990), Abstract No. 810 and Webber, *et al.,* Science 252:114-117 (**1991**)) was suspended in 570 mL of water and 145 mL of 1N HCl was added. The compound went into solution within a few minutes, and the solution was stirred at room temperature for 16 hours. The reaction mixture was made basic by addition of 145 mL of 1N NaOH, and the resulting precipitate was removed by filtration. This material was resuspended in water and refiltered. CHCl₃ (400 mL) was added, and to the resulting emulsion was added 200 mL of saturated brine and 200 mL of NaHCO₃. The organic layer was separated, and the emulsion was re-extracted with three additional portions of CHCl₃. The extracts were combined and dried over Na₂SO₄. The solvent was removed and the residue dried under high vacuum to afford 24 g of crude product.

### Step 2b. Compound (5) (Scheme 1), R¹=H; R²=benzyoyl

The compound from step 2a (24 g, 44 mmol) was dissolved in dry methylene chloride (200 mL), triethylamine (122 mL, 88 mmol) and benzoyl anhydride (20 g, 88 mmol) were added, and the mixture was stirred under N₂ for 15 hours. The reaction was quenched by addition of 200 mL of saturated NaHCO₃ solution, and the resulting mixture was extracted with methylene chloride (2 x 100 mL). The extracts were combined, washed with water and brine, and dried over Na₂SO₄. The solvent was removed, and the residue was flash chromatographed on silica gel, eluting with 1:1 to 3:1 ethyl acetate:hexane to afford the title compound (4.06 g).

### Step 2c. Compound (4) (Scheme 2); R¹=H; R²=benzoyl

The compound from step 2b (541 mg, 0.816 mmol) was dissolved in 8 mL of THF under nitrogen, and the solution was cooled to -40°C in an acetone/dry ice bath. NaHMDS (1.6 mL, 1.6 mmol) was added dropwise with stirring over a 3 minute period. The reaction mixture was stirred for 10 minutes, then CDI (560 mg, 3.46 mmol, dissolved in 12 mL of THF) was added over a 15 minute interval. The reaction mixture was stirred for 5 hours at ambient temperature, then cooled to 0°C and quenched with 25 mL of 5% aqueous KH₂PO₄ solution. The mixture was extracted with ethyl acetate (2 x 40 mL), and the extract was washed with brine and dried over Na₂SO₄. The solvent was removed, and the residue was flash chromatographed on silica gel, eluting with 1:1 to 0:1 hexane/acetone to afford 230 mg of the title compound.

### Step 2d. Compound (I) (Scheme 3); R¹=H: R²=benzoyl; W is absent; R=4-phenylbutyl

The compound from step 2c (180 mg, 0.250 mmol) was dissolved in 0.9 mL of acetonitrile and 0.1 mL of water. To this solution was added 4-phenylbutylamine (0.300 mL, 1.90 mmoL. Aldrich), and the reaction mixture was stirred at 50°C under nitrogen for 5.5 hours. The reaction mixture was cooled to room temperature and diluted with 30 mL of methylene chloride. The solution was washed with 5% aqueous KH₂PO₄ solution and brine and dried over Na₂SO₄. The solvent was removed, and the residue was flash chromatographed on silica gel, eluting with 15 to 20 % acetone/hexane to afford 73 mg of the title compound.

### Step 2e. Compound of Formula (I); R¹=H; R²=H; W is absent; R=4-phenylbutyl

The compound from step 2d (70 mg) was dissolved in 5 mL of methanol, and the solution was stirred at room temperature for 14 hours. The solvent was removed, and the residue was flash chromatographed on silica gel, eluting with 5 % methanol in methylene chloride to afford the title compound. MS m/z 699 (M+H)⁺. Anal.Calcd. for C₄₀H₆₂N₂O₈: C, 68.74; H, 8.94; N, 4.01; Found: C, 68.57; H, 8.91; N, 3.88.

### Example 3

### Compound of Formula (I); R¹=methoxy; R²=H; W is absent; R=4-phenylbutyl

### Step 3a. Compound of Formula (I); R¹=methoxy; R²=acetyl; W is absent; R=4-phenylbutyl

The title compound of Example 1 (231 mg, 0.334 mmol) and 4-phenylbutylamine (0.177 mL, 1.105 mmol, Aldrich) were dissolved in 3 mL of DMF, and the solution was stirred at room temperature for 24 hours. The solution was diluted with ethyl acetate (40 mL), washed with 30 mL portions of water and brine, then dried over MgSO₄. The solvent was removed. The residue was flash chromatographed on silica gel, eluting with hexane - 25% acetone/hexane, to afford the title compound (147 mg, 57% yield). MS m/z 771 (M+H)⁺.

### Step 3b. Compound of Formula (I); R¹=methoxy; R²=H; W is absent; R=4-phenylbutyl

The compound from step 3a (140 mg) was dissolved in 5 mL of methanol, and the solution was stirred at room temperature for 40 hours. The solvent was removed, and the residue was flash chromatographed on silica gel, eluting with 0-4 % methanol in methylene chloride to afford the title compound (50 mg, 37% yield). MS m/z 729 (M+H)⁺. Anal.Calcd. for C₄₁H₆₄N₂O₉: C, 67.55; H, 8.85; N, 3.84; Found: C, 67.17; H, 8.95; N, 3.66.

### Example 4

### Compound of Formula (I): R¹=methoxy; R²=H; W is absent; R=3-phenoxypropyl

### Step 4a. ompound of Formula (I); R¹=methoxy; R²=acetyl; W is absent; R=3-phenoxypropyl

The title compound of Example I (1.00g, 1.45 mmol) was dissolved in 3 mL of acetonitrile, 3-phenoxypropylamine (1.1 g, 7.28 mmol, prepared in a manner similar to that described by K. Smith, *et al*., *J. Chem. Soc. Perkin Trans*. I, (1988) 77-83) and 0.3 mL of water were added, and the reaction mixture was stirred for 21 hours. The solution was diluted with ethyl acetate (100 mL), washed with 5% aqueous KH₂PO₄, water and brine, then dried over Na₂SO₄. The solvent was removed. The residue was flash chromatographed on silica gel, eluting with 1:2 acetone/hexane, to afford the title compound (490 mg). MS m/z 773 (M+H)⁺.

### Step 4b. Compound of Formula (I); R¹=methoxy; R²=H; W is absent; R=3-phenoxypropyl

The compound from step 4a (400 mg) was dissolved in 20 mL of methanol, and the solution was stirred at room temperature for 27 hours. The solvent was removed, and the residue was flash chromatographed on silica gel, eluting with 4% methanol in methylene chloride containing 0.1% NH₄OH to afford the title compound (237 mg). MS m/z 731 (M+H)⁺. Anal.Calcd. for C₄₀H₆₂N₂O₁₀: C, 65.73; H, 8.55; N, 3.83; Found: C, 65.47; H, 8.71; N, 3.66.

### Example 5

### Compound of Formula (I); R¹=methoxy; R²=H: W is absent; R=2-((phenylmethyl)amino)ethyl

### Step 5a. Compound of Formula (I); R¹=methoxy; R²=acetyl; W is absent; R=2-((phenlmethyl)amino)ethyl

The title compound of Example 1 (500 mg, 0.724 mmol) was added to a solution of N-benzylethylenediamine (0.5 g, 3.33 mmol, Eastman) in 1.5 mL of acetonitrile and 0.15 mL of water, and the reaction mixture was stirred for 40 hours. The solution was diluted with methylene chloride, washed with 5% aqueous KH₂PO₄, water and brine, then dried over Na₂SO₄. The solvent was removed. The residue was flash chromatographed on silica gel, eluting with 3% methanol in methylene chloride containing 0.1% NH₄OH, to afford the title compound (151 mg). MS m/z 772 (M+H)⁺.

### Step 5b. Compound of Formula (I); R¹=methoxy; R²=H; W is absent; R=2-((phenylmethyl)amino)ethyl

The compound from step 5a (145 mg) was dissolved in 10 mL of methanol, and the solution was stirred at room temperature for 27 hours. The solvent was removed, and the residue was flash chromatographed on silica gel, eluting with 3.5-5% methanol in methylene chloride containing 0.1% NH₄OH to afford the title compound (118 mg). The NMR was consistent with the C-10 epimeric form of this compound. MS m/z 730 (M+H)⁺. Anal.Calcd. for C₄₀H₆₃N₃O₉: C. 65.82; H, 8.70; N, 5.76; Found: C, 65.58; H, 8.66; N, 5.76.

### Example 6

### Compound of Formula (I); R¹=methoxy; R²=H; W is absent; R=3-(N-methyl-N-phenylamino)propyl

### Step 6a. Compound of Formula (I); R¹=methoxy; R²=acetyl; W is absent; R=3-(N-methyl-N-phenylamino)propyl

The title compound of Example 1 (500 mg, 0.724 mmol) was dissolved in 1.5 mL of acetonitrile, N-(3-aminopropyl)-N-methylaniline (0.50 g, 3.05 mmol, TCI) and 0.15 mL of water were added, and the reaction mixture was stirred for 16 hours. The solution was diluted with ethyl acetate (50 mL). This solution was then washed with 5% aqueous KH₂PO₄, water and brine, and dried over Na₂SO₄. The solvent was removed, and the residue was flash chromatographed on silica gel, eluting with 2.5 % methanol/methylene chloride, to afford the title compound (410 mg). MS m/z 786 (M+H)⁺.

### Step 6b. Compound of Formula (I); R¹=methoxy; R²=H; W is absent; R=3-(N-methyl-N-phenylamino)propyl

The compound from step 6a (398 mg) was dissolved in 10 mL of methanol, and the solution was stirred at room temperature for 16 hours. The solvent was removed, and the residue was flash chromatographed on silica gel, eluting with 4 % methanol in methylene chloride containing 0.2% NH₄OH to afford the title compound (205 mg), which was recrystallized from hexane/ethyl acetate (120 mg). mp 155-156°C. MS m/z 744 (M+H)⁺. Anal.Calcd. for C₄₁H₆₅N₃O₉: C, 66.19; H, 8.81; N, 5.65; Found: C, 66.38; H, 8.87; N, 5.49.

### Example 7

### Compound of Formula (I); R¹=methoxy; R²H; W is absent; R=3-(4-chlorophenoxy)propyl

### Step 7a. Compound of Formula (I); R¹=methoxy; R²=acetyl; W is absent; R=3-(4-chlorophenoxy)propyl

The title compound of Example 1 (500 mg, 0.726 mmol) was dissolved in 3 mL of acetonitrile, 3-(4-chlorophenoxy)propylamine (470 mg, 2.53 mmol, prepared in a manner similar to that described by K. Smith, *et al*., J. Chem. Soc. Perkin Trans. I, (1988) 77-83) and 0.3 mL of water were added, and the reaction mixture was stirred for 19 hours. The solution was diluted with ethyl acetate (100 mL). This solution was washed with 5% aqueous KH₂PO₄, water and brine, then dried over Na₂SO₄. The solvent was removed. The residue was flash chromatographed on silica gel, eluting with 3% methanol in methylene chloride containing 0.1% NH₄OH, to afford the title compound (328 mg). MS m/z 807 (M+H)⁺.

### Step 7b. Compound of Formula (I); R¹=methoxy; R²=H; W is absent; R=3-(4-chlorophenoxy)propyl

The compound from step 7a (322 mg) was dissolved in 10 mL of methanol, and the solution was stirred at room temperature for 21 hours. The solvent was removed, and the residue was flash chromatographed on silica gel, eluting with 4 % methanol in methylene chloride containing 0.1% NH₄OH to afford the title compound (162 mg), which was recrystallized from hexane/ethyl acetate. mp 183.5-185°C. MS m/z 765 (M+H)⁺. Anal.Calcd. for C₄₀H₆₁ClN₂O₁₀: C, 62.77; H, 8.03; N, 3.66; Found: C, 62.78; H, 8.17; N, 3.53.

### Example 8

### Compound of Formula (II); R¹=methoxy; R²=H; A=B=C=D=H

### Step 8a. Compound of Formula (I); W absent; R¹=methoxy; R²=acetyl; A=B=C=D=H

The title compound of Example 1 (2.0 g, 7.899 mmol) was dissolved in 5 mL of DMF, ethylenediamine (2 mL, Aldrich) was added, and the reaction mixture was stirred for 22.5 hours. The solution was diluted with ethyl acetate (60 mL), and this solution was washed with water and brine, then dried over MgSO₄. The solvent was removed to afford the title compound, which was used without further purification. MS m/z 682.

### Step 8b. Compound of Formula (II); R¹=methoxy; R²=H; A=B=C=D=H

A solution of the compound from step 8a, dissolved in 6 mL of methanol, was stirred at room temperature for 19 hours. This residue was purified by chromatography on silica gel, eluting with 5% methanol/l % triethylamine/CHCl₃. The material was rechromatographed eluting with 5% methanol/CHCl₃ to afford the title compound (138 mg). mp 183.5-185°C. MS m/z 622 (M+H)⁺. Anal.Calcd. for C₃₃H₅₅N₃O₈·H₂O: C, 61.95; H, 8.98; N, 6.57; Found: C, 62.22; H, 8.83; N, 6.50.

### Example 9

### Compound of Formula (I); R¹=methoxy; R²=H; W is absent; R=3-(8-quinoyloxy)propyl

### Step 9a. Compound of Formula (I); R¹=methoxy; R²=acetyl; W is absent; R=3-(8-quinoyloxy)propyl

The title compound of Example 1 (508 mg, 0.737 mmol) was dissolved in 3 mL of 10% aqueous acetonitrile containing 3-(1-naphthyloxy)propylamine (500 mg, 2.5 mmol, prepared in a manner similar to that described by K. Smith, *et al., J. Chem. Soc. Perkin Trans.* I, (1988) 77-83). The reaction mixture was stirred for 21.5 hours, then diluted with ethyl acetate (100 mL), washed with 5% aqueous KH₂PO₄, water and brine, then dried over Na₂SO₄. After removal of solvent the residue was flash chromatographed on silica gel, eluting with 3% methanol in methylene chloride containing 0.1% NH₄OH, to afford the title compound (495 mg). MS m/z 824 (M+H)⁺.

### Step 9b. Compound of Formula (I); R¹=methoxy; R²=H; W is absent; R=3-(8-quinoyloxy)propyl

The compound from step 9a (485 mg) was dissolved in 20 mL of methanol, and the solution was stirred at room temperature for 18 hours. The solvent was removed, and the residue was flash chromatographed on silica gel, eluting with 5% methanol in methylene chloride containing 0.1% NH₄OH to afford the title compound (218 mg), which was recrystallized from hexane/ethyl acetate. mp 168-170°C. MS m/z 782 (M+H)⁺. Anal.Calcd. for C₄₃H₆₃N₃O₁₀: C, 66.05; H, 8.12; N, 5.37; Found: C, 66.02; H, 8.21; N, 5.28.

### Example 10

### Compound of Formula (I); R¹=methoxy; R²=H; W is absent; R=4-(4-chlorophenyl)-3(Z)-butenyl

### Step 10a. Compound of Formula (I); R¹=methoxy; R²=acetyl; W is absent; R=4-(4-chlorophenyl)-3-butenyl

The title compound of Example 1 (750 mg) was dissolved in 4 mL of 10% aqueous acetonitrile containing 4-(4-chlorophenyl)-3-buteneamine (0.5 mL, prepared in a manner similar to that described by D. Olsen, *et al*., *J. Org. Chem*. (1980) 45, 4049-4052), and the reaction mixture was stirred for 12 hours. The solution was diluted with ethyl acetate (100 mL), washed with saturated aqueous NH₄Cl and brine, then dried over Na₂SO₄. The solvent was removed, and the residue was flash chromatographed on silica gel, eluting with 2.5% methanol in methylene chloride containing 0.1% NH₄OH, to afford the title compound (513 mg).

### Step 10b. Compound of Formula (I); R¹=methoxy; R²=H; W is absent; R=4-(4-chlorophenyl)-3-butenyl

The compound from step 10a (513 mg) was dissolved in 50 mL of methanol, and the solution was stirred at room temperature for 18 hours. The solvent was removed, and the residue was flash chromatographed on silica gel, eluting with 3.5% methanol in methylene chloride containing 0.1% NH₄OH to afford the tide compound (200 mg), which was recrystallized from hexane/ethyl acetate (120 mg). mp 180-182.5°C. MS m/z 761 (M+H)⁺. Anal.Calcd. for C₄₁H₆₁ClN₂O₉: C, 64.68; H, 8.08; N, 3.68; Found: C, 64.75; H, 8.14; N, 3.45.

### Example 11

### Compound of Formula (I); R¹=methoxy; R²=H; W is absent; R=2-phenylethyl

### Step 11a. Compound of Formula (I); R¹=methoxy; R²=acetyl; W is absent; R=2-phenylethyl

The title compound of Example 1 (500 mg, 0.724 mmol) was dissolved in 3 mL of 10% aqueous acetonitrile containing 2-phenylethylamine (0:5 mL, 3.62 mmol, Aldrich), and the reaction mixture was stirred for 16 hours. The solution was diluted with ethyl acetate (100 mL), and this solution was washed with saturated aqueous 5% aqueous KH₂PO₄, water and brine, then dried over Na₂SO₄. The solvent was removed, and the residue was taken directly to the next step.

### Step 11b. Compound of Formula (I); R¹=methoxy; R²=H; W is absent; R=2-phenylethyl

The compound from step 11a was dissolved in 10 mL of methanol, and the solution was stirred at room temperature for 18 hours. The solvent was removed, and the residue was flash chromatographed on silica gel, eluting with 4% methanol in methylene chloride containing 0.1% NH₄OH to afford the title compound. MS m/z 701 (M+H)⁺. Anal.Calcd. C₃₉H₆₀N₂O₉: C, 66.83; H, 8.62; N, 3.99; Found: C, 66.80; H, 8.58; N, 3.97.

### Example 12

### Compound of Formula (I); R¹=methoxy; R²=H; W is absent; R=2-(3,4-dichlorophenyl)ethyl

### Step 12a. Compound of Formula (I); R¹=methoxy; R²=acetyl; W is absent; R=2-(3.4-dichlorophenvl)ethyl

The title compound of Example 1 (500 mg, 0.724 mmol) was dissolved in 5 mL of 10% aqueous acetonitrile containing 2-(3,4-dichlorophenyl)ethylamine (0.7 g, 3.62 mmol, Aldrich), and the reaction mixture was stirred for 72 hours. The solution was diluted with ethyl acetate (100 mL), and this solution was washed with saturated aqueous 5% aqueous KH₂PO₄, water and brine, then dried over Na₂SO₄. The solvent was removed, and the residue was taken directly to the next step.

### Step 12b. Compound of Formula (I); R¹=methoxy; R²=H; W is absent: R=2-(3,4-dichlorophenyl)ethyl

The compound from step 12a was dissolved in 10 mL of methanol, and the solution was stirred at room temperature for 18 hours. The solvent was removed, and the residue was flash chromatographed on silica gel, eluting with 4% methanol in methylene chloride containing 0.1% NH₄OH to afford the title compound. MS m/z 769 (M+H)⁺. Anal.Calcd. for C₃₉H₅₈Cl₂N₂O₉: C, 60.85; H,.7.59; N, 3.63; Found: C, 60.22; H, 7.19; N, 3.63.

### Example 13

### Compound of Formula (I); R¹=methoxy; R²=H; W is absent: R=phenylmethyl

### Step 13a. Compound of Formula (I); R¹=methoxy; R²=acetyl; W is absent; R=phenylmethyl

The title compound of Example 1 (500 mg, 0.724 mmol) was dissolved in 5 mL of 10% aqueous acetonitrile containing benzylamine (0.4 g, 3.62 mmol, Aldrich), and the reaction mixture was stirred for 16 hours. The solution was diluted with ethyl acetate (100 mL), and this solution was washed with saturated aqueous 5% aqueous KH₂PO₄, water and brine, then dried over Na₂SO₄. The solvent was removed, and the residue was taken directly to the next step.

### Step 13b. Compound of Formula (I); R¹=methoxy; R²=H; W is absent; R=phenylmethyl

The compound from step 13a was dissolved in 10 mL of methanol, and the solution was stirred at room temperature for 18 hours. The solvent was removed, and the residue was flash chromatographed on silica gel, eluting with 4% methanol in methylene chloride containing 0.1% NH₄OH to afford the title compound (83 mg). MS m/z 687 (M+H)⁺. Anal.Calcd. for C₃₈H₅₈N₂O₉: C, 66.44; H, 8.51; N, 4.07; Found: C, 66.23; H, 8.19; N, 4.21.

### Example 14

### Compound of Formula (I): R¹=methoxy; R²=H: W is -NH-; R=3-phenylpropyl

### Step 14a. Compound (I) of Scheme 1; R¹=methoxy; R²=H; W is -NH-; R=H

The title compound of Example 1 (2.0 g, 2.90 mmol) was dissolved in 10 mL of DMF, hydrazine (0.225 mL, 7.18 mmol, Aldrich) was added, and the reaction mixture was stirred for 0.5 hours. The solution was diluted with methylene chloride (125 mL), and this solution was washed with saturated aqueous 5% aqueous KH₂PO₄, water and brine, then dried over Na₂SO₄. The solvent was removed, and the residue was dissolved in methanol and allowed to stand for 16 hours. The solvent was removed, and the residue was flash chromatographed on silica gel, eluting with 2.5% methanol in t-butyl methyl ether containing 0.5% NH₄OH, to afford the mixture of compounds. The solvent was removed, and the residue was again flash chromatographed on silica gel, however eluting with 5% methanol in methylene chloride containing 0.1% NH₄OH to 10% methanol in methylene chloride containing 0.2% NH₄OH to give the title compound (0.19 g). MS m/z 612 (M+H)⁺.

### Step 14b. Compound of Formula (I); R¹=methoxy; R²=H; W is -N=- R=phenylethyl-CH=

The compound from step 14a was dissolved in toluene (5 mL), 3-phenylpropanal (0.200 mL, Aldrich) and 4Å molecular sieves were added, and the reaction mixture was stirred for 48 hours. An additional portion of 3-phenylpropanal was added, and the reaction mixture was stirred for an additional 6 hours. The mixture was filtered, the solvent was removed, and the residue was flash chromatographed on silica gel, eluting with 4% methanol/methylene chloride containing 0.1% NH₄OH to give the title compound (181 mg). MS m/z 728 (M+H)⁺.

### Step 14c. Compound of Formula (I); R¹=methoxy; R²=H; W is -NH-; R=3-phenylpropyl

The compound from step 14b (170 mg, 0.234 mmol) was dissolved in 5 mL of methanol, NaBH₃CN (30 mg, 0.47 mmol) was added, and the solution was stirred at room temperature for 4.5 hours. Saturated aqueous NaHCO₃ was added, and the mixture was extracted with methylene chloride. The extract was washed with saturated aqueous NaHCO₃, water and brine, then dried over Na₂SO₄. The solvent was removed, and the residue was flash chromatographed on silica gel, eluting with 5% methanol in methylene chloride containing 0.1% NH₄OH to afford the title compound (135 mg). MS m/z 730 (M+H)⁺. Anal.Calcd. for C₄₀H₆₃N₃O₉: C, 65.82; H, 8.70; N, 5.76; Found: C, 65.97; H, 8.79; N, 5.64.

### Example 15

### Compound of Formula (I); R¹=methoxy; R²=H; W is absent; R=3-(4-phenoxyphenyl)ethyl

### Step 15a. Compound of Formula (I); R¹=methoxy; R²=acetyl; W is absent; R=3-(4-phenoxyphenyl)propyl

The title compound of Example 1 (0.4 mg, 0.0.58 mmol) was dissolved in 5 mL of 10% aqueous acetonitrile containing 2-(4-(phenoxy)phenyl)ethylamine (0.618 g, 2.89 mmol, Trans World Chemicals), and the reaction mixture was stirred for 20 hours. The solution was diluted with ethyl acetate (100 mL), and this solution was washed with 5% NaH₂PO₄, water and brine, then dried over Na₂SO₄. The solvent was removed, and the residue was flash chromatographed on silica gel, eluting with 1-2% ethanol in chloroform to afford the title compound (270 mg), mixture of natural and *epi* isomers.

### Step 15b. Compound of Formula (I); R¹=methoxy; R²=H; W is absent; R=3-(4-phenoxyphenyl)ethyl

The compound from step 15a (80 mg) was dissolved in 5 mL of methanol, and the solution was stirred at room temperature for 20 hours. The solvent was removed, and the residue was flash chromatographed on silica gel, eluting with 4% methanol in methylene chloride to afford the title compound (19 mg) as a mixture of natural and *epi* isomers. MS m/z 793 (M+H)⁺. Anal. Calcd. for C₄₅H₆₄N₂O₁₀: C, 68.15; H, 8.13; N, 3.53; Found: C, 68.23; H, 8.15; N, 3.62.

### Example 16

### Compound of Formula (I); R¹=methoxy; R²=H; W is absent; R=3-phenylpropyl

### Step 16a. Compound of Formula (I); R¹=methoxy; R²=acetyl; W is absent; R=3-phenylpropyl

The title compound of Example 1 (500 mg, 0.724 mmol) was dissolved in 5 mL of 10% aqueous acetonitrile containing 3-phenyl-1-propylamine (0.5 mL, 3.62 mmol, Aldrich). and the reaction mixture was stirred for 16 hours. The solution was diluted with methylene chloride, and this solution was washed with 5% aqueous NaH₂PO₄, water and brine, then dried over Na₂SO₄. The solvent was removed, and the residue was taken directly to the next step.

### Step 16b. Compound of Formula (I); R¹=methoxy; R²=H; W is absent; R=3-phenylpropyl

The compound from step 16a was dissolved in 5 mL of methanol, and the solution was stirred at room temperature for 16 hours. The solvent was removed, and the residue was flash chromatographed on silica gel, eluting with 4% methanol in methylene chloride to afford the tide compound.(mixture of natural and *epi* isomers). MS m/z 715 (M+H)⁺. Anal. Calcd. for C₄₀H₆₂N₂O₉: C, 67.19; H, 8.74; N, 3.91; Found: C, 67.23; H, 8.70; N, 3.90.

### Example 17

### Compound of Formula (I); R¹=methoxy; R²=H; W is absent; R=2,2-diphenylethyl

### Step 17a. Compound of Formula (I); R¹=methoxy; R²=acetyl; W is absent; R=2,2-diphenylethyl

The title compound of Example 1 (300 mg, 0.434 mmol) was dissolved in 5 mL of 10% aqueous acetonitrile containing 2,2-diphenylethylamine (420 mg 2.174 mmol, Aldrich), and the reaction mixture was stirred for 16 hours. The solution was diluted with methylene chloride, and this solution was washed with 5% aqueous NaH₂PO₄, water and brine, then dried over Na₂SO₄. The solvent was removed, and the residue was taken directly to the next step.

### Step 17b. Compound of Formula (I); R¹=methoxy; R²=H; W is absent; R=2,2-diphenylethyl

The compound from step 17a was dissolved in 5 mL of methanol, and the solution was stirred at room temperature for 16 hours. The solvent was removed, and the residue was flash chromatographed on silica gel, eluting with 4% methanol in methylene chloride to afford the title compound (as a mixture of natural and *epi* isomers). MS m/z 778 (M+H)⁺.

### Example 18

### Compound of Formula (I); R¹=methoxy;R²=H; W is absent; R=H

### Step 18a. Compound of Formula (I); R¹=methoxy; R²=acetyl; W is absent; R=H

The title compound of Example 1 (300 mg, 0.434 mmol) was dissolved in acetonitrile, placed in a pressure bottle and NH₃ was introduced. The reaction mixture was stirred for 17 hours. The solution was diluted with methylene chloride, and this solution was washed with 5% aqueous NaH₂PO₄, water and brine, then dried over Na₂SO₄. The solvent was removed, and the residue was taken directly to the next step.

### Step 18b. Compound of Formula (I); R¹=methoxy; R²=H; W is absent; R=H

The compound from step 17a was dissolved in 5 mL of methanol, and the solution was stirred at room temperature for 16 hours. The solvent was removed, and the residue was flash chromatographed on silica gel, eluting with 4% methanol in methylene chloride to afford the title compound. MS m/z 597 (M+H)⁺. Anal. Calcd. for C₃₁H₅₂N₂O₉: C, 62.39; H, 8.78; N, 4.69; Found: C, 62.19; H, 8.77; N, 4.72.

### Example 19

### Compound of Formula (IV): R¹=methoxy; R²=H; A=B=C=D=H; R=H

A sample of the compound of Example 8 (166 mg, 0.207 mmol) (Formula (II); R¹=methoxy; R²=H; A=B=C=D=H) was dissolved in 6 mL of methanol and 0.5 mL of acetic acid. To this solution was added NaBH₃CN (126 mg,2.0 mmol), and the mixture was stirred at room temperature for 5 hours. The reaction was diluted with methylene chloride, washed with saturated aqueous NaHCO₃, dried and concentrated. The residue was chromatographed on silica gel, eluting with 3-10 % methanol/methylene chloride to give 63 mg of the title compound. MS m/z 624 (M+H)⁺.

### Example 20

### Compound of Formula (I); R¹=methoxy; R²=H; W is -NH-; R=H; C10 methyl is epi-isomer

A sample of the compound from Example 14a (300 mg) was further purified by flash chromatography on silica gel, eluting with 10% methanol/t-butyl methyl ether, to separate the mixture into two fractions. Fraction A had the C10-methyl group of the opposite epimeric configuration compared to the orientation of the C10-methyl group in natural erythromycins and is characterized herein, and Fraction B is characterized in the following Example. Fraction A: MS m/z 612 (M+H)⁺. Anal.Calcd. for C₃₁H5₃N₃O₉: C, 60.86; H, 8.73; N, 6.87; Found: C, 60.94; H, 8.85; N, 6.50.

### Example 21

### Compound of Formula (I); R¹=methoxy; R²=H; W is -NH-; R=H; C10 methyl is natural isomer

Fraction B from Example 20, possessing the C10 methyl group in the orientation of the C10-methyl group in natural erythromycins, was characterized as follows. MS m/z 612 (M+H)⁺. Fraction B: Anal.Calcd. for C₃₁H₅₃N₃O₉: C, 60.86; H, 8.73; N, 6.87; Found: C, 60.97; H, 8.74; N, 6.54.

### Example 22

### Compound of Formula (I); R¹=methoxy; R²=H; W is -NH-; R=3-(4-quinolinyl)propyl: C10 methyl is natural isomer

### Step 22a. Ethyl 3-(4-quinolinyl)propenoate

LiCl (972 mg, 22.9 mmol) and 60 mL of CH₃CN were placed in a dry flask, and triethyl phosphonoacetate (4.55 mL, 22.9 mmol, Aldrich) and DBU (3.05 mL, 20.4 mmol) were added. The mixture was stirred until the reagents were dissolved, and quinoline-4-carboxyaldehyde (3.00 g, 19.1 mmol) was added. The reaction mixture was stirred under nitrogen for 6 hours, and the reaction was quenched by addition of 5% KH₂PO₄. The mixture was extracted with ether, and the organic extract was washed with water and brine then dried over MgSO₄. The solvent was removed, and the residue was flash chromatographed on silica gel, eluting with t-butyl methyl ether, to afford the title compound.

### Step 22b. Ethyl 3-(4-quinolinyl)propionate

The compound from step 22a (2.60 g) was dissolved in 20 mL of methanol, and hydrogenated at 1 atm while stirring over Pd/C catalyst for 17 hours. The mixture was filtered, and the solvent was removed. The residue was flash chromatographed on silica gel, eluting with 50% ethyl acetate/hexane, to afford the title compound.

### Step 22c. 3-(4-Ouinolinyl)propanal

The compound from step 22b (1.51 g, 6.59 mmol) was dissolved in 60 mL of toluene, and the solution was cooled to -78°C. DIBAL-H (13.2 mL, 13.2 mmol) was added, and the reaction mixture was stirred under nitrogen for 2 hours. The reaction was quenched by addition of water (0.25 mL) and acetic acid (1 mL) dissolved in 3 mL of ether. The mixture was allowed to warm to room temperature then filtered. The solvent was removed, and the residue (1.2 g) was flash chromatographed on silica gel, eluting with 75-100% ethyl acetate/hexane, to afford the title compound (0.79 g, oil).

### Step 22d. Compound of Formula (I): R¹=methoxy; R²=H; W is -N=CH-; R=(4-quinolinyl)-CH₂-CH₂-; C₁₀ methyl is natural isomer

A sample of the compound of Example 21 (270 mg, 0.442 mmol) was added to a solution of the aldehyde compound of step 22c (0.50 g, 2.7 mmol) in 10 mL of toluene. Molecular sieves (4Å) were added, and the mixture was stirred under nitrogen for 16 hours. A small amount of p-toluenesulfonate•H₂O (98 mg) was added, and the mixture was stirred for 26 hours. The mixture was filtered, the solvent was removed, and the residue was flash chromatographed on silica gel, eluting with 4-5% methanol/methylene chloride containing 0.1% NH₄OH, to afford the title compound (242 mg). MS m/z 779 (M+H)⁺.

### Step 22e. Compound of Formula (I); R¹=methoxy; R²=H; W is -NH-; R=3-(4-quinolinyl)propyl; C₁₀ methyl is natural isomer

A sample of the compound of step 22d (235 mg, 0.302 mmol) was dissolved in 10 mL of methanol and NaBH₃CN (40 mg) was added as well as enough acetic acid to turn bromocresol green indicator yellow, and the reaction mixture was stirred for 5 hours. The reaction was quenched with saturated NaHCO₃, and the mixture was extracted with methylene chloride. The solution was washed with saturated NaHCO₃, water and brine, then dried over Na₂SO₄. The solvent was removed, and the residue was flash chromatographed on silica gel, eluting with 5% methanol/methylene chloride containing 0.2% NH₄OH, to afford the title compound (128 mg). Flash chromatography was repeated, eluting with 10% methanol/t-butyl methyl ether to give 108 mg of title compound MS m/z 781 (M+H)⁺.

### Example 23

### Compound of Formula (I); R¹=methoxy; R²=H; W is absent; R=3-(2-naphthyloxy)propyl

### Step 23a. N-(3-(2-naphthyloxy)propyl)phthalimide

A mixture of N-(3-bromopropyl)phthalimide (8 g, 29.83 mmol, Aldrich), 2-naphthol (4.30 g, 29.83 mmol) and K₂CO₃ (20.61 g, 149 mmol) in acetone (150 mL) was heated at reflux for 16 hours. The resulting suspension was filtered, and the filtrate was concentrated to give the title compound (10.28 g).

### tep 23b. 3-(2-naphthyloxy)-1-propylamine

A sample of the compound from step 23a (10.28 g, 31.06 mmol) was suspended in ethanol, hydrazine (1.07 mL, 34.16 mmol) was added, and the mixture was heated at reflux for 21 hours. The mixture was cooled to room temperature, and the ethanol was removed. The residue was dissolved in 1N HCl, and an attempt was made to extract the solution with ethyl acetate. A stubborn emulsion formed, which was broken by adding K₂CO₃ to pH 10. The layers separated, the ethyl acetate fraction was discarded, and the aqueous layer was extracted with methylene chloride. The solvent was dried and removed to afford the title compound.

### Step 23c. Compound of Formula (I); R¹=methoxy; R²=acetyl; W is absent; R=3-(2-naphthyloxy)propyl

The title compound of Example 1 (500 mg, 0.724 mmol) and 3-(2-naphthyloxy)-1-propylamine from step 23b (720 mg, 3.62 mmol) were dissolved in 10 mL of 10% aqueous acetonitrile, and the reaction mixture was stirred for 16 hours. The solution was diluted with methylene chloride, and this solution was washed with 5% aqueous NaH₂PO₄, water and brine, then dried over Na₂SO₄. The solvent was removed, and the residue was taken directly to the next step.

### Step 23d. Compound of Formula (I); R¹=methoxy; R²=H; W is absent; R=3-(2-naphthyloxy)propyl

The compound from step 23c was dissolved in 5 mL of methanol, and the solution was stirred at room temperature for 16 hours. The solvent was removed, and the residue (640 mg) was flash chromatographed on silica gel, eluting with 4% methanol in methylene chloride containing 0.1% NH₄OH to afford the title compound (180 mg). MS m/z 781 (M+H)⁺. Anal. Calcd. for C₄₄H₆₄N₂O₁₀: C, 67.66; H, 8.25; N, 3.58; Found: C, 67.70; H, 8.25; N, 3.61.

### Example 24

### Compound of Formula (I); R¹=methoxy; R²=H; W is absent; R=3-(3-pyridyloxy)propyl

### Step 24a. N-(3-(3-pyridyloxy)propyl)phthalimide

3-Hydroxypyridine(2.83 g, 29.83 mmol) was dissolved in DMF (70 mL), the solution was cooled in an ice bath, NaH (1.3 g, 32.81 mmol) and N-(3-bromopropyl)-phthalimide (8 g, 29.83 mmol, Aldrich) were added, the ice bath was removed, and the reaction mixture was stirred for 64 hours. The reaction was diluted with CH₂Cl₂ and filtered through celite to give a 1:1 mixture of N- and O-alkylated products. Recrystallization from EtOH afforded a 4:1 mixture of N- and O-alkylated products. The mother liquor was taken up in water and extracted with CH₂Cl₂, then the organic layer washed with water (5x) to afford the desired N-(3-(3-pyridyloxy)propyl)phthalimide (8.6 g).

### Step 24b. 3-(3-pyridyloxy)-1-propylamine

A sample of the compound from step 24a (8.6 g, 30.49 mmol) was suspended in ethanol, hydrazine (1.05 mL, 33.54 mmol) was added, and the mixture was heated at reflux for 16 hours. The mixture was cooled to room temperature, and the ethanol was removed. The residue was dissolved in IN HCl, and an attempt was made to extract the solution with ethyl acetate. A stubborn emulsion formed, which was broken by adding K₂CO₃ to pH 10. The layers separated, the ethyl acetate fraction was saved, and the aqueous layer was extracted with methylene chloride. The organic extracts were combined and concentrated to afford the title compound (0.42 g).

### Step 24c. Compound of Formula (I); R¹=methoxy; R²=acetyl; W is absent; R=3-(3-pyridyloxy)propyl

The title compound of Example 1 (420 mg, 0.57 mmol) and 3-(3-pyridyloxy)-1-propylamine from step 24b (420 mg, 2.78 mmol) were dissolved in 5 mL of 10% aqueous acetonitrile, and the reaction mixture was stirred for 16 hours. The solution was diluted with methylene chloride, and this solution was washed with 5% aqueous NaH₂PO₄, water and brine, then dried over Na₂SO₄. The solvent was removed, and the residue was taken directly to the next step.

### Step 24d. Compound of Formula (I); R¹=methoxy; R²=H; W is absent; R=3-(3-pyridyloxy)propyl

The compound from step 24c was dissolved in 5 mL of methanol, and the solution was stirred at room temperature for 16 hours. The solvent was removed, and the residue (510 mg) was flash chromatographed on silica gel, eluting with 4-6% methanol in methylene chloride containing 0.1% NH₄OH to afford the title compound (100 mg). MS m/z 774 (M+H)⁺.

### Example 25

### Compound of Formula (I); R¹=methoxy; R²=H: W is absent: R=3-(2-pyridyloxy)propyl

### Step 25a. N-(3-(2-pyridyloxy)propyl)phthalimide

A mixture of N-(3-hydroxypropyl)phthalimide (Aldrich, 8.00 g, 38.98 mmol), 2-chloropyridine (3.68 mL, 38.98 mmol) and NaH (60% dispersion, 2.33 g, 58.47 mmol) in DMF (100 mL) was heated at 75°C and stirred for 5 days. The reaction mixture was then diluted with CH₂Cl₂, filtered, and the filtrate concentrated to give the title compound (13 g).

### Step 25b. 3-(2-pyridyloxy)-1-propylamine

A sample of the compound from step 25a (11 g, 39 mmol) was suspended in ethanol, hydrazine (1.3 mL, 42.9 mmol) was added, and the mixture was heated at reflux for 16 hours. The mixture was cooled to room temperature, and then 8.7 mL of 6N HCl was added. The mixture was then heated at reflux for 2 hours. The mixture was cooled to room temperature and concentrated. The residue was taken up in IN NaOH, and the solution was extracted with ethyl acetate. The organic extracts were combined, washed with water and brine, dried over Na₂SO₄ and concentrated to afford the title compound.

### Step 25c. Compound of Formula (I); R¹=methoxy; R²=acetyl; W is absent; R=3-(3-pyridyloxy)propyl

The title compound of Example 1 (420 mg, 0.57 mmol) and 3-(2-pyridyloxy)-1-propylamine from step 25b were dissolved in 5 mL of 10% aqueous acetonitrile, and the reaction mixture was stirred for 16 hours. The solution was diluted with methylene chloride, and this solution was washed with 5% aqueous NaH₂PO₄, water and brine, then dried over Na₂SO₄. The solvent was removed, and the residue was taken directly to the next step.

### Step 25d. Compound of Formula (I); R¹=methoxy; R²=H; W is absent; R=3-(3-pyridyloxy)propyl

The compound from step 25c was dissolved in 5 mL of methanol, and the solution was stirred at room temperature for 16 hours. The solvent was removed, and the residue (51 mg) was flash chromatographed on silica gel, eluting with 4-6% methanol in methylene chloride containing 0.1% NH₄OH to afford the title compound (100 mg). MS m/z 732 (M+H)⁺.

### Example 26

### Compound of Formula (I); R¹=OH; R²=H; W is absent; R=4-phenylbutyl

Following the procedures of Example 1, except substituting erythromycin A for the 6-methoxyerythromycin A thereof and carrying the product forward according to the procedures of Example 3, the title compound is prepared.

### Example 27

### Compound of Formula (I): R¹=OCONH2: R²=H; W is absent; R=4-phenylbutyl

Following the procedures of Example 1, except substituting 6-O-carbamoyl-erythromycin A (prepared according to procedures described by E.G. Brain in European Patent Application EP 212169, published April 1, 1987) for the 6-methoxyerythromycin A thereof and carrying the product forward according to the procedures of Example 3, the title compound is prepared.

### Example 28

### Compound of Formula (I); R¹=OCONHCO-methyl; R²=HW is absent; R=4-phenylbutyl

Following the procedures of Example 1, except substituting 6-O-(N-acetyl)carbamoyl erythromycin A (prepared according to procedures described by E.G. Brain in European Patent Application EP 212169, published April 1, 1987) for the 6-methoxyerythromycin A thereof and carrying the product forward according to the procedures of Example 3, the title compound is prepared.

### Example 29

### Compound of Formula (I); R¹=OCONHSO2-methyl; R²=H; W is absent; R=4-phenylbutyl

Following the procedures of Example 1, except substituting 6-O-(N-methane-sulfonyl)carbamoylerythromycin A (prepared according to procedures described by E.G. Brain in European Patent Application EP 212169, published April 1, 1987) for the 6-methoxyerythromycin A thereof and carrying the product forward according to the procedures of Example 3, the title compound is prepared.

### Example 30

### Compound of Formula (I); R¹=OMe; R²=H; W is absent; R=phenyl

Following the procedures of Example 3, except substituting aniline for the 4-phenylbutylamine thereof, the title compound is prepared.

### Example 31

### Compound of Formula (I); R¹=OMe; R²=H; W is absent; R=3-pyridyl

Following the procedures of Example 3, except substituting 3-aminopyridine for the 4-phenylbutylamine thereof, the title compound is prepared.

### Example 32

### Compound of Formula (I); R¹=OMe; R²=H; W is -O-: R=H

Following the procedure of Example 14a, except substituting hydroxylamine for the hydrazine of step 14a, the title compound is prepared.

### Example 33

### Compound of Formula (I); R¹=OMe; R²=H; W is -O-; R=methyl

Following the procedure of Example 14a, except substituting methoxylamine for the hydrazine of step 14a, the title compound is prepared.

### Example 34

### Compound of Formula (I); R¹=OMe; R²=H; W is -NH-CO-; R=phenyl

Treatment of the compound described in example 14a with benzoyl chloride in the presence of a suitable base such as triethylamine or pyridine followed by chromatographic separation of the product and removal of the 2'-protecting group as described in step 2e affords the title compound.

### Example 35

### Compound of Formula (II); R¹=OMe; R²=H; A=benzyl; B=D=E=H

Following the procedures of Example 8, except substituting 2-amino-3-phenyl-1-propanol for the ethylenediamine thereof, to give an intermediate compound (Compound 11 of Scheme 4, wherein Y is OH and A is benzyl), then the intermediate thus prepared is reacted with triphenylphosphine, DEAD and DPPA under Mitsunobu conditions to replace the hydroxyl group with an azido group, then the azido group is reduced to an amino group with triphenylphosphine and water, then the amino compound is refluxed with acetic acid and water to close the ring to give the title compound.

### Example 36

### Compound of Formula (II); R¹=OMe: R²=H; A=D=3,4-pyrrolidinyl; B=E=H

Following the procedures of Example 8, except substituting cis-3,4-diaminopyrrolidine for the ethylenediamine thereof, the title compound is prepared.

### Example 37

### Compound of Formula (III); R¹=OMe; R²=H; A=B=D=E=H

The title compound of Example 8 (Compound of Formula (II); R¹=methoxy; R²=H; A=B=C=D=H) is treated with H₂O₂ to oxidize the imine nitrogen, and the intermediate compound is treated with triphenyl phosphine in water to reduce any of the byproduct (the dimethylamine N-oxide on the desosamine moiety) to afford the title compound.

### Example 38

### Compound of Formula (IV); R¹=OMe; R²=H; A=benzyl; B=D=E=H; R=H

The title compound of Example 35 is treated with sodium cyanoborohydride at pH 4-5, under conditions similar to that described for example 19, to afford the title compound.

### Example 39

### Compound of Formula (IV); R¹=OMe; R²=H; A=D=3,4-pyrrolidinyl; B=E=H; R=H

The title compound of Example 36 is treated with sodium cyanoborohydride at pH 4-5, under conditions similar to that described for example 19, to afford the title compound.

### Example 40

### Compound of Formula (IV); R¹=OMe; R²=H; A=B=D=E=H, R=CH₂CH₂CH₂C₆H₅

The title compound of Example 19 is reductively alkylated by treatment with 3-phenylpropanal and sodium cyanoborohydride to afford the title compound.

### Example 41

### Compound of Formula (IV): R¹=OMe; R²=H; A=B=D=E=H, R=2,4-dinitrobenzene

The title compound of Example 19 (wherein R²=acetyl) is treated with R=2,4-dinitrofluorobenzene followed by removal of the 2'-protecting group as described in step 2e affords the title compound.

### Example 42

### Compound of Formula (IV); R¹=OMe; R²=H; A=B=D=E=H, R=4-quinolyl

The title compound of Example 19 (wherein R²=acetyl) is treated with 4-bromoquinine, in the presence of a suitable catalyst such as CuBr, followed by removal of the 2'-protecting group as described in step 2e affords the title compound.

### Example 43

### Compound of Formula (I); R¹=methoxy; R²=H; W is absent; R=3-(4H-4-oxo-1-quinolyl)propyl

### Step 43a. N-(1-(3-aminopropyl)-1H-4-oxoquinolin-1-yl)phthalimide

4-Hydroxyquinoline (4.04 g, 27.9 mmol) and N-(3-bromopropyl)phthalimide (7.48 g, 27.9 mmol) were suspended in acetone (140 mL), and K₂CO₃ (20g) was added. The mixture was stirred at reflux under N₂ for 51 hours, then cooled, diluted with methylene chloride and filtered. The solvents were removed, and the residue was crystallized from ethanol (5.8 g). Chromatographic separation on silica gel eluting with 5% methanol in chloroform gave the title compound (2.8 g), which was taken directly to the next step.

### Step 43b. 1-(3-aminopropyl)-1H-4-oxoquinoline

The compound from step 43a was dissolved in ethanol (80 mL) and heated at reflux in the presence of 0.5 mL of hydrazine for 16 hours. The solution was cooled and concentrated. The residue was taken up in 80 mL of ethanol containing 1.5 mL of conc. HCl, and the solution was heated at reflux for 3 hours. The mixture was filtered, and the filter cake was extracted with methylene chloride and 1N NaOH. The layers were separated, and the organic layer was washed with brine, dried and concentrated to afford the title compound (280 mg).

### Step 43c. Compound of Formula (I); R¹=methoxy; R²=acetyl; W is absent; R=(4H-4-oxo-1-quinolyl)propyl

The title compound of Example 1 (330 mg, 0.479 mmol) and 1-(3-aminopropyl)-1H-4-oxoquinoline from step 43b (330 mg) were dissolved in 4 mL of 10% aqueous acetonitrile, and the reaction mixture was stirred for 40 hours. The solution was diluted with methylene chloride, and this solution was washed with 5% aqueous NaH₂PO₄, water and brine, then dried over Na₂SO₄. The solvent was removed, and the residue was purified by flash chromatography on silica gel, eluting with 4% methanol in methylene chloride containing 0.1% NH₄OH.

### Step 43d. Compound of Formula (I): R¹=methoxy; R²=H; W is absent; R=(4H-4-oxo-1-quinolyl)propyl

The compound from step 43c was dissolved in 10 mL of methanol, and the solution was held at room temperature for 16 hours. The solvent was removed, and the residue was flash chromatographed on silica gel, eluting with 10% methanol in methylene chloride containing 0.1% NH₄OH to afford the title compound (98 mg). mp 214.5-215.5°C. MS m/z 782 (M+H)⁺. Anal.Calcd. for C₄₃H₆₃N₃O₁₀: C, 65.82; H, 8.70; N, 5.76; Found: C, 65.97; H, 8.79; N, 5.64.

### Examples 44-66

Following the procedures of Example 11, except substituting the R²-reagent compound shown in the table below for the phenylethylamine of Example 11a, the compounds of Examples 44-66 were prepared.

**Table 1**

| **Examples 44-66** | | |
|---|---|---|
| **Ex. No.** | **R**^{**2**}**-reagent compound** | **Title Compound** |
| 44 | 2-(4-nitrophenyl)ethylamine | Compound of Formula (I); R¹=methoxy; R²=H; W is absent; R=2-(4-nitrophenyl)ethyl |
| 45 | 2-(4-aminophenyl)ethylamine | Compound of Formula (I); R¹=methoxy; R²=H; W is absent; R=2-(4-aminophenyl)ethyl |
| 46 | 3-ethoxypropylamine | Compound of Formula (I); R¹=methoxy; R²=H; W is absent; R=3-ethoxypropyl |
| 47 | isopropylamine | Compound of Formula (I); R¹=methoxy; R²=H; W is absent; R=isopropyl |
| 48 | 2-(4-bromophenyl)ethylamine | Compound of Formula (I); R¹=methoxy; R²=H; W is absent; R=2-(4-bromophenyl)ethyl |
| 49 | 2-(4-hydroxylphenyl)ethylamine | Compound of Formula (I); R¹=methoxy; R²=H; W is absent; R=2-(4-hydroxylphenyl)ethyl |
| 50 | 2-(4-fluorophenyl)ethylamine | Compound of Formula (I); R¹=methoxy; R²=H; W is absent; R=2-(4-fluorophenyl)ethyl |
| 51 | 2-(3-methoxyphenyl)ethylamine | Compound of Formula (I); R¹=methoxy; R²=H; W is absent; R=2-(3-methoxyphenyl)ethyl |
| 52 | 3-vinyloxypropylamine | Compound of Formula (I); R¹=methoxy; R²=H; W is absent; R=3-vinyloxypropyl |
| 53 | 2-(3-trifluoromethyl)-phenylethylamine | Compound of Formula (I); R¹=methoxy; R²=H; W is absent; R=2-(3-trifluoromethyl)phenylethyl |
| 54 | 2-thienylethylamine | Compound of Formula (I); R¹=methoxy; R²=H; W is absent; R=2-thienylethyl |
| 55 | 2-(3,4-dibenzyloxyphenyl)ethylamine | Compound of Formula (I); R¹=methoxy; R²=H; W is absent; R=2-(3,4-benzyloxyphenyl)ethyl |
| 56 | 2-(4-methylphenyl)ethylamine | Compound of Formula (I); R¹=methoxy; R²=H; W is absent; R=2-(4-methylphenyl)ethyl |
| 57 | N-allylamine | Compound of Formula (I); R¹=methoxy; R²=H; W is absent; R=allyl |
| 58 | 1,3-dihydroxypropylamine | Compound of Formula (I); R¹=methoxy; R²=H; W is absent; R=1,3-dihydroxypropyl |
| 59 | 1,3-dihydroxypropylamine (10-*epi*) | Compound of Formula (I); R¹=methoxy; R²=H; W is absent; R=1,3-dihydroxypropyl (10*-epi*) |
| 60 | 3-hydroxypropylamine | Compound of Formula (I); R¹=methoxy; R²=H; W is absent; R=3-hydroxypropyl |
| 61 | 3-hydroxypropylamine (10-*epi*) | Compound of Formula (I); R¹=methoxy; R²=H; W is absent; R=3-hydroxypropyl (10-*epi*) |
| 62 | propylamine | Compound of Formula (I); R¹=methoxy; R²=H; W is absent; R=propyl |
| 63 | isobutylamine | Compound of Formula (I); R¹=methoxy; R²=H; W is absent; R=isobutyl |
| 64 | 2-(benzoylamino)ethylamine | Compound of Formula (I); R¹=methoxy; R²=H; W is absent; R=2-(benzoylamino)ethyl |
| 65 | 3-(benzoylamino)propylamine | Compound of Formula (I); R¹=methoxy; R²=H; W is absent; R=3-(benzoylamino)propyl |
| 66 | 3-(acetylamino)propylamine | Compound of Formula (I); R¹=methoxy; R²=H; W is absent; R=3-(acetylamino)-propyl |

### Example 67

### Compound of Formula (I); R¹=methoxy; R²=H; W is absent; R=H (10-epi)

This compound was separated by flash chromatography from the mixture of products generated in Example 18, step b. MS m/z 597 (M+H)⁺.

### Example 68

### Compound of Formula (I); R¹=methoxy; R²=H; W is absent: R=3-phenylpropyl (10-epi)

This compound was separated by flash chromatography from the mixture of products generated in Example 16, step b. MS m/z 715 (M+H)⁺.

### Example 69

### Compound of Formula (I); R¹=methoxy; R²=H; W is absent: R=3-(4-phenoxyphenyl)ethyl (10-epi)

This compound was separated by flash chromatography from the mixture of products generated in Example 15, step b. MS m/z 794 (M+H)⁺.

### Example 70

### Compound of Formula (I); R¹=methoxy; R²=H; W is absent; R=3-(4-chlorophenyl)propyl

To a sample of the title compound of Example 21 (225 mg, 0.368 mmol) dissolved in methanol (4 mL) were added 3-(4-chlorophenyl)propionaldehyde (400 mg, 2.4 mmol, prepared according to the procedure of T. Jeffery, *J. Chem. Soc. Chem. Commun*., **1984:**1287) and sufficient acetic acid to change bromocresol green indicator from blue to yellow. To this solution was added sodium cyanoborohydride (160 mg, 2.5 mmol), and the mixture was stirred under nitrogen for about 20 hours, adjusting the pH with acetic acid as necessary. The reaction was quenched by addition of aqueous sodium bicarbonate, then the mixture was extracted with methylene chloride. The organic layer was washed with aqueous sodium bicarbonate and water, then dried over Na₂SO₄. The solvent was removed, and the residue was flash chromatographed on silica gel, eluting with 10% methanol in methylene chloride containing 0.1% NH₄OH to afford the title compound (159 mg). MS m/z 764 (M+H)⁺.

### Examples 71-109

Following the procedure of Example 70, except replacing the 3-(4-chlorophenyl)-propionaldehyde with the reagent aldehyde as indicated, the compounds of Examples 71-109 were prepared as shown in Table 2 below. The aldehydes of Examples 71-81 were prepared from precursor aryl iodides (commercially available) and allyl alcohol via a Heck-type reaction (T. Jeffrey, *J. Chem. Soc. Chem. Commun.,* **1984**:1287). The aldehyde reagents of Examples 82-109 were obtained commercially.

**Table 2**

| **Examples 71-109** | | |
|---|---|---|
| **Ex. No.** | **Aldehyde Reagent** | **Title Compound** |
| 71 | 3-(3-chlorophenyl)-propionaldehyde | Compound of Formula (I); R¹=methoxy; R²=H; W is -NH-; R=3-(3-chlorophenyl)-propyl |
| 72 | 3-(2-chlorophenyl)-propionaldehyde | Compound of Formula (I); R¹=methoxy; R²=H; W is -NH-; R=3-(2-chlorophenyl)-propyl |
| 73 | 3-(2,4-dichlorophenyl)-propionaldehyde | Compound of Formula (I); R¹=methoxy; R²=H; W is -NH-; R=3-(2,4-dichloro-phenyl)propyl |
| 74 | 3-(4-hydroxyphenyl)-propionaldehyde | Compound of Formula (I); R¹=methoxy; R²=H; W is -NH-; R=3-(4-hydroxy-phenyl)propyl |
| 75 | 3-(3-hydroxyphenyl)-propionaldehyde | Compound of Formula (I); R¹=methoxy; R²=H; W is -NH-; R=3-(3-hydroxy-phenyl)propyl |
| 76 | 3-(2-hydroxyphenyl)-propionaldehyde | Compound of Formula (I); R¹=methoxy; R²=H; W is -NH-; R=3-(2-hydroxy-phenyl)propyl |
| 77 | 3-(4-methoxyphenyl)-propionaldehyde | Compound of Formula (I); R¹=methoxy; R²=H; W is -NH-; R=3-(4-methoxy-phenyl)propyl |
| 78 | 3-(4-nitrophenyl)-propionaldehyde | Compound of Formula (I); R¹=methoxy; R²=H; W is -NH-; R=3-(4-nitrophenyl)propyl |
| 79 | 3-(3-nitrophenyl)-propionaldehyde | Compound of Formula (I); R¹=methoxy; R² =H; W is -NH-; R=3-(3-nitrophenyl)-propyl |
| 80 | 3-(2-nitrophenyl)-propionaldehyde | Compound of Formula (I); R¹=methoxy; R²=H; W is -NH-; R=3-(2-nitrophenyl)-propyl |
| 81 | 3-(4-(acetylamino)-phenyl)-propionaldehyde | Compound of Formula (I); R¹=methoxy; R²=H; W is -NH-; R=3-((4-(acetyl-amino)phenyl)propyl |
| 82 | trans-cinnamaldehyde | Compound of Formula (I); R¹=methoxy; R²=H: W is -NH-: R=*trans*-3-phenylprop-2-enyl |
| 83 | phenylacetaldehyde | Compound of Formula (I); R¹=methoxy; R²=H; W is -NH-; R=2-phenylethyl |
| 84 | benzaldehyde | Compound of Formula (I); R¹=methoxy; R²=H; W is -NH-; R=phenylmethyl |
| 85 | indole-3-carboxaldehyde | Compound of Formula (I); R¹=methoxy; R²=H; W is -NH-; R=(3-indolyl)methyl |
| 86 | 4-methoxybenzaldehyde | Compound of Formula (I); R¹=methoxy; R²=H; W is -NH-; R=(4-methoxy-phenyl)methyl |
| 87 | 4-acetylaminobenzaldehyde | Compound of Formula (I); R¹=methoxy; R²=H; W is -NH-; R=(4-acetylamino-phenyl)methyl |
| 88 | 4-chlorobenzaldehyde | Compound of Formula (I); R¹=methoxy; R²=H; W is -NH-; R=(4-chlorophenyl)-methyl |
| 89 | 4-(dimethylamino)benzaldehyde | Compound of Formula (I); R¹=methoxy; R²=H; W is -NH-; R=(4-dimethylamino-phenyl)methyl |
| 90 | trans-4-nitro-cinnamaldehyde | Compound of Formula (I); R¹=methoxy; R²=H; W is -NH-; R=*trans*-3-(4-nitrophenyl)prop-2-enyl |
| 91 | 4-nitrobenzaldehyde | Compound of Formula (I); R¹=methoxy; R²=H; W is -NH-; R=(4-nitrophenyl)methyl |
| 92 | 3,4-dihydroxy-benzaldehyde | Compound of Formula (I); R¹=methoxy; R²=H; W is -NH-; R=(3,4-dihydroxyphenyl)methyl |
| 93 | 2,5-dihydroxy-benzaldehyde | Compound of Formula (I); R¹=methoxy; R²=H; W is absent; R=(2,5-dihydroxy-phenyl)methyl |
| 94 | 2-hydroxy-5-nitrobenzaldehyde | Compound of Formula (I); R¹=methoxy; R²=H; W is -NH-; R=(2-hydroxy-5-nitro-phenyl)methyl |
| 95 | terephthaldicarboxaldehyde | Compound of Formula (I); R¹=methoxy; R²=H; W is -NH-; R=(4-hydroxymethyl-phenyl)methyl |
| 96 | 5-nitrofuranacrolein | Compound of Formula (I); R¹=methoxy; R²=H; W is -NH-; R=*trans*-3-(5-nitro-2-furanyl)prop-2-enyl |
| 97 | phthalicdicarboxaldehyde | Compound of Formula (I); R¹=methoxy; R²=H; W is N; R=(-CH₂-(1,2-phenylene)-CH₂- |
| 98 | 4-hydroxybenzaldehyde | Compound of Formula (I); R¹=methoxy; R²=H; W is -NH-; R=(4-hydroxyphenyl)-methyl |
| 99 | 3-hydroxybenzaldehyde | Compound of Formula (I); R¹=methoxy; R²=H; W is -NH-; R=(3-hydroxyphenyl)-methyl |
| 100 | salicylaldehyde | Compound of Formula (I); R¹=methoxy; R²=H; W is -NH-; R=(2-hydroxyphenyl)-methyl |
| 101 | trifluoro-p-tolualdehyde | Compound of Formula (I); R¹=methoxy; R²=H; W is -NH-; R=(4-trifluoromethyl- |
| 102 | 4-cyanobenzaldehyde | Compound of Formula (I); R¹=methoxy; R²=H; W is -NH-; R=(4-cyanophenyl)-methyl |
| 103 | 2-pyridinecarboxaldehyde | Compound of Formula (I); R¹=methoxy; R²=H; W is -NH-; R=(2-pyridyl)methyl |
| 104 | 3-pyridinecarboxaldehyde | Compound of Formula (I); R¹=methoxy; R²=H; W is -NH-; R=(3-pyridyl)methyl |
| 105 | 4-pyridinecarboxaldehyde | Compound of Formula (I); R¹=methoxy; R²=H; W is -NH-; R=(4-pyridyl)methyl |
| 106 | 2-hydroxy-1-naphthaldehyde | Compound of Formula (I); R¹=methoxy; R²=H; W is -NH-; R=(2-hydroxy-1-naphthyl)methyl |
| 107 | 4-dimethylamino-1-naphthaldehyde | Compound of Formula (I); R¹=methoxy; R²=H; W is -NH-; R=(4-dimethylamino-1-naphthyl)methyl |
| 108 | 4-(methylthio)-benzaldehyde | Compound of Formula (I); R¹=methoxy; R²=H; W is -NH-; R=(4-(methylthio)-phenyl)methyl |
| 109 | 4-Phenoxybenzaldehyde | Compound of Formula (I); R¹=methoxy; R²=H; W is -NH-; R=(4-phenoxyphenyl)-methyl |

### Example 110

### Compound of Formula (I); R¹=methoxy; R²=H; W is -NH-; R=3-(4-fluorophenyl)propyl

Following the procedures of Example 22, except substituting 4-fluorobenzaldehyde for the quinoline-4-carboxyaldehyde of Example 22a, and carrying the product forward as in Example 22 steps b-d, the title compound was prepared. Anal.Calcd. for C₄₀H₆₂FN₃O₉: C, 64.23; H, 8.35; N, 5.62; Found: C, 64.27; H, 8.60; N, 5.51.

### Example 111

### Compound of Formula (I); R¹=methoxy; R²=H; W is -NH-; R=(trans-3-(4-nitrophenyl)prop-2-enyl

A sample of the title compound from Example 90 (R=(*trans*-3-(4-nitrophenyl)prop-2-enyl, 144 mg, 0.193 mmol) in 10 mL of methanol was added to a solution of acetyl chloride (0.300 mL, 4.2 mmol) in methanol (5 mL), then Zn dust (380 mg, 5.81 mmol) was added and the mixture was stirred from 16 hours. Saturated aqueous sodium carbonate and ethyl acetate were added, and the mixture was stirred for 15 minutes. The organic layer was separated, washed dried (NaSO4) and concentrated. The residue was purified by flash chromatography on silica gel, eluting with 5% methanol/chloroform containing 0.1% ammonium hydroxide to give the title compound (85 mg): MS m/z 717 (M+H)⁺; Anal.Calcd. for C₄₀H₆₂N₄O₉: C, 63.66; H, 8.44; N, 7.86; Found: C, 63.62; H, 8.66; N, 7.68.

### Example 112

### Compound of Formula (I); R¹=methoxy; R²=H; W is -NH-; R=4-aminophenylmethyl

Following the procedure of Example 111, except substituting the compound of Example 91 for the starting material of Example 111, the title compound was prepared. Anal.Calcd. for C₃₈H₆₀N₄O₉: C, 63.66; H, 8.44; N, 7.81; Found: C, 63.62; H, 8.66; N, 7.66.

### Example 113

### Compound of Formula (I); R¹=methoxy; R²=H; W is -NH-; R=3-(4-aminophenyl)propyl

Following the procedure of Example 111, except substituting the compound of Example 78 for the starting material of Example 111, the title compound was prepared. Anal.Calcd. for C₄₀H₆₄N₄O₉: C, 64.49; H, 8.66; N, 7.52; Found: C, 64.35; H, 8.86; N, 7.31.

### Example 114

### Compound of Formula (I): R¹=methoxy; R²=H; W is -NH-; R=3-(3-aminophenyl)propyl

Following the procedure of Example 111, except substituting the compound of Example 79 for the starting material of Example 111, the title compound was prepared. Anal.Calcd. for C₄₀H₆₄N₄O₉: C, 64.49; H, 8.66; N, 7.52; Found: C, 64.57; H, 8.87; N, 7.31.

### Example 115

### Compound of Formula (I); R¹=methoxy; R²=H; W is -NH-; R=3-(2-aminophenyl)propyl

Following the procedure of Example 111, except substituting the compound of Example 80 for the starting material of Example 111, the title compound was prepared. Anal.Calcd. for C₄₀H₆₄N₄O₉: C, 61.99; H, 8.06; N, 7.23; Found: C, 62.25; H, 7.87; N, 7.08.

### Example 116

### Compound of Formula (I); R¹=methoxy; R²=H; W is -NH-; R=trans-3-(4-acetylaminophenyl)prop-2-enyl

A sample of the compound of Example 111 was treated with acetyl chloride in methylene chloride at 0°C for 3 hours to give the title compound. Anal.Calcd. for C₄₂H₆₄N₄O₁₀: C, 64.26; H, 8.22; N, 7.14; Found: C, 64.21; H, 8.36; N, 6.93.

### Example 117

### Compound of Formula (I); R¹=methoxy; R²=H; W is -NH-; R=trans-3-(4-(4-nitrobenzoylamino)phenyl)prop-2-enyl

A sample of the compound of Example 111 was treated with 4-nitrobenzoyl chloride in methylene chloride at 0°C for 3 hours to give the title compound. Anal.Calcd. for C₄₇H₆₅N₅O₁₂: C, 63.23; H, 7.34; N, 7.85; Found: C, 63.35; H, 7.59; N, 7.60.

### Example 118

### Compound of Formula (I); R¹=methoxy; R²=H; W is -NH-; R=3-(2-benztriazolyl)propyl

### Step 118a. 1-(2-(1,3-dioxolan-2-yl)ethyl)benztriazole and 2-(2-(1,3-dioxolan-2-yl)ethyl)benztriazole

Benztriazole (2.02 g, 16.97 mmol) was added to a suspension of NaH (1 g, 20 mmol) in dry DMF (25 mL) at 0°C. To this mixture was added in portions, 2-(2-bromoethyl)-1,3-dioxolane, and the mixture was warmed to room temperature and stirred for 16 hours. Brine was added, and the mixture was extracted with ether. The ether extract was washed with brine, dried (Na₂SO₄) and concentrated. The residue was purified by flash chromatography on silica gel, eluting with 25% ethyl acetate/hexane to give the two isomeric products. MS m/z 220 (M+H)⁺.

### Step 118b. 3-(2-benztriazolyl)propanaldehyde

The 2-(2-(1.3-dioxolan-2-yl)ethyl)benztriazole isomer from step 118a (550 mg) was dissolved in acetone (25 mL), and 2 N HCl (10 mL) was added. The mixture was heated at 40-50°C for 23 hours, then diluted with methylene chloride. The solution was washed with brine. then dried and concentrated to give the title compound: MS m/z 176 (M+H)⁺.

### Step 118c. Compound of Formula (I); R¹=methoxy; R²=H; W is -NH-; R=3-(2-benztriazolyl)propyl

Following the procedure of Example 70, except replacing the 3-(4-chlorophenyl)propionaldehyde thereof with the aldehyde from step 118b, the title compound was prepared. MS m/z 771 (M+H)⁺.

### Example 119

### Compound of Formula (I); R¹=methoxy; R²=H; W is -NH-; R=3-(1-benztriazolyl)propyl

### Step 119a. 3-(1-benztriazolyl)propanaldehyde

The 2-(2-(1,3-dioxolan-2-yl)ethyl)benztriazole isomer from Example 118a (630 mg) was dissolved in acetone (25 mL), and 2 N HCl (10 mL) was added. The mixture was heated at 40-50°C for 23 hours, then diluted with methylene chloride. The solution was washed with brine, then dried and concentrated to give the title compound: MS m/z 176 (M+H)⁺.

### Step 119b. Compound of Formula (I); R¹=methoxy; R²=H; W is -NH-; R=3-(1-benztriazolyl)propyl

Following the procedure of Example 70, except replacing the 3-(4-chlorophenyl)-propionaldehyde thereof with the aldehyde from step 119a, the title compound was prepared. Anal.Calcd. for C₄₀H₆₂N₆O₉: C, 62.32; H, 8.11; N, 10.90; Found: C, 62.27; H, 8.21; N, 10.61.

### Example 120

### Compound of Formula (I); R¹=methoxy; R²=H; W is -NH-; R=3-(4-phenylimidazolyl)propyl

### Step 120a. 1-(2-(1,3-dioxan-2-yl)ethyl)-4-phenylimidazole

Following the procedure of Example 118 step a, except substituting 4-phenyl-imidazole (2.01 g, 13.96 mmol) for the benztriazole thereof, the title compound (2.15 g) was prepared. MS m/z 245 (M+H)⁺.

### Step 120b. 3-(4-phenylimidazolyl)propanaldehyde

Following the procedure of Example 118 step b, except substituting the compound of step 120a for the compound of I 18a thereof, the title compound was prepared. MS m/z 201 (M+H)⁺.

### Step 120c. Compound of Formula (I); R¹=methoxy; R²=H; W is -NH-; R=3-(4-phenylimidazolyl)propyl

Following the procedure of Example 70, except replacing the 3-(4-chlorophenyl)-propionaldehyde thereof with the aldehyde from step 120b, the title compound was prepared. Anal.Calcd. for C₄₃H₆₅N₅O₉: C, 64.88; H, 8.23; N, 8.80; Found: C, 65.04; H, 8.35; N, 8.60.

### Example 121

### Compound of Formula (I); R¹=methoxy; R²=H; W is absent; R=3-(1-anhydro-1-cladinosyl)propyl

### Step 121a. 1-O-methyl-cladinose

To a solution of cladinose (5.0 g) in methanol (200 mL) was added acetyl chloride (3 mL), and the mixture was stirred for 3 hours. The reaction was quenched with 5% aqueous sodium bicarbonate solution, then the mixture was extracted with methylene chloride. The organic layer was dried (Na₂SO₄) and concentrated to give the title compound.

### Step 121b. 1-O-methyl-4-O-acetylcladinose

To a sample of 1-O-methyl-cladinose (2.85 g, 15.0 mmol) in methylene chloride (75 mL) cooled to 0°C were added acetic anhydride (1.6 mL, 16.9 mmol), triethylamine (4.2 mL, 30.1 mmol) and DMAP (100 mg, 0.82 mmol), and the mixture was stirred at room temperature for 19 hours. The reaction was quenched with saturated aqueous sodium bicarbonate solution, then the mixture was extracted with methylene chloride. The organic layer was washed with water and brine, then dried (Na₂SO₄) and concentrated. The residue was flash chromatographed on silica gel, eluting with 25% ethyl acetate/hexane to give 3.28 g of the title compound.

### Step 121c. 1-allyl-1-anhydro-4-O-acetylcladinose

To a solution of 1-O-methyl-4-O-acetylcladinose (2.68 g, 11.6 mmol) and allyltrimethylsilane (5.50 mL, Aldrich) in methylene chloride at -16°C flushed with nitrogen was added boron trifluoride etherate (4.26 mL) over an 8 minute period. The mixture was stirred at -15°C for 2 hours and at -5°C for 2 hours, then the reaction was quenched with saturated aqueous sodium bicarbonate solution. The mixture was extracted with methylene chloride, and the organic layer was washed with water and brine. then dried (Na₂SO₄) and concentrated. The residue was flash chromatographed on silica gel, eluting with 20% ethyl acetate/hexane to give 1.17 g of the title compound.

### Step 121d. 3-(1-dehydroxycladinosyl)propanol

To a solution of 1-allyl-1-anhydro-4-O-acetylcladinose (465 mg, 1.92 mmol) in dry THF was added 9-BBN (265 mg, 2.17 mmol), and the mixture was heated at reflux for 16 hours. An additional portion of 9-BBN (54 mg) was added, and the heating was continued for 2 hour hours. The mixture was cooled to room temperature, and 15 % NaOH (0.7 mL) and 30 % H₂O₂ (0.9 mL) were added while cooling in an ice bath. The mixture was extracted with ether, and the organic layer was washed with water and brine, then dried (Na₂SO₄) and concentrated (0.53 mg). The residue was stirred in methanol with 450 mg of potassium carbonate for 4 hours. Methylene chloride was added, and the layers were separated. The organic layer was washed with water and brine, then dried (Na₂SO₄) and concentrated. The residue was flash chromatographed on silica gel, eluting with ethyl acetate to give 196 mg of the title compound. MS m/z 219 (M+H)⁺.

### Step 121e. 3-(1-anhydro-1-cladinosyl)propanaldehyde

To a solution of 3-(1-anhydrocladinosyl)propanol (190 mg, 0.872 mmol) in methylene chloride (3 mL) was added 0.5 M aqueous KBr (0.18 mL) and 2,2,6,6-tetramethylpiperidine-N-oxide (3 mg), and the mixture was cooled to 0°C. Aqueous 0.35 M NaOCl (3.0 mL) buffered with sodium bicarbonate was added, and the mixture was stirred rapidly for 45 minutes. Saturated aqueous sodium bicarbonate was added, and the mixture was extracted with methylene chloride. The organic layer was washed with water and brine, then dried (Na₂SO₄) and concentrated. The residue was flash chromatographed on silica gel, eluting with 1:1 ethyl acetate/hexane to give 96 mg of the title compound. MS m/z 234 (M+H)⁺.

### Step 121f. Compound of Formula (I); R¹=methoxy; R²=H; W is -NH-; R=3-(1-anhydro-1-cladinosyl)propyl

Following the procedure of Example 70, except replacing the 3-(4-chlorophenyl)-propionaldehyde thereof with the 3-(1-dehydroxy-1-cladinosyl)propanaldehyde from step 121e, the title compound was prepared.

### Example 122

### Compound of Formula (I); R¹=methoxy; R²=H; W is -NH-; R=3-phenylpropyl (10-epi)

This compound was prepared as in Example 14, except using the 10-*epi* isomer. MS m/z 730 (M+H)⁺.

### Example 123

### Compound of Formula (I); R¹=methoxy; R²=H; W is -NH-; R=isopropyl

This compound was made in a manner similar to example 14b and 14c; using the product compound of step 14a and substituting acetone (Aldrich) for 3-phenylpropanal and substituting refluxing acetone instead of toluene for step 14b; and using MeOH/AcOH as solvent for the reduction step 14c. MS m/z 654 (M+H)⁺.

### Example 124

### Compound of Formula (I); R¹=methoxy; R²=H; W is -NH-; R=1,3-diphenyl-2-propyl

This compound was made in a manner similar to example 14b and 14c; using the product compound from step 14a and substituting 1,3-diphenylacetone (Aldrich) for 3-phenylpropanal and substituting refluxing methanol instead of toluene for step 14b; and using MeOH/AcOH as solvent for the reduction step 14c. MS m/z 806 (M+H)⁺.

### Example 125

### Compound of Formula (I); R¹=methoxy; R²=H; W is -NH-; R=3-pentyl

This compound was made in a manner similar to example 14b and 14c; using the product compound from step 14a and substituting 3-pentanone (Aldrich) for 3-phenylpropanal and substituting refluxing methanol instead of toluene for step 14b; and using MeOH/AcOH as solvent for the reduction step 14c. MS m/z 682 (M+H)⁺.

### Example 126

### Compound of Formula (I); R¹=methoxy; R²=H: W is absent; R=2-(benzoylamino)ethyl

The compound was prepared from the title compound of Example 1 and N-benzoyl ethylenediamine according to the procedures of Example 3. MS m/z 744 (M+H)⁺.

### Example 127

### Compound of Formula (I); R¹=methoxy; R²=H; W is absent; R=2-((4-methoxybenzoyl)amino)ethyl

The compound was prepared from the title compound of Example 1 and N-(4-methoxybenzoyl)ethylene diamine according to the procedures of Example 3. MS m/z 774 (M+H)⁺.

### Example 128

### Compound of Formula (I); R¹=methoxy; R²=H; W is absent; R=4-hydroxybutyl

The compound was prepared from the title compound of Example 1 and 4-amino-1-butanol according to the procedures of Example 3. MS m/z 669 (M+H)⁺.

### Example 129

### Compound of Formula (I); R¹=methoxy; R²=H; W is absent; R=2-(piperidinyl)ethyl

The compound was prepared from the title compound of Example 1 and 1-(2-amino-ethyl)piperidine according to the procedures of Example 3. MS m/z 708 (M+H)⁺.

### Example 130

### Compound of Formula (I); R¹=methoxy; R²=H; W is absent; R=2-3-((4-methylbenzoyl)amino)propyl

The compound was prepared from the title compound of Example 1 and N-(4-methylbenzoyl)propylene diamine according to the procedures of Example 3. MS m/z 772 (M+H)⁺.

### Example 131

### Compound of Formula (I); R¹=methoxy; R²=H; W is absent; R=2-3-((4-chlorobenzoyl)amino)propyl

The compound was prepared from the title compound of Example 1 and N-(4-chlorobenzoyl)propylene diamine according to the procedures of Example 3. MS m/z 792 (M+H)⁺.

### Example 132

### Compound of Formula (I); R¹=methoxy; R²=H; W is absent; R=R=2-(pyrrolidinyl)ethyl

The compound was prepared from the title compound of Example I and 1-(2-amino-ethyl)pyrrolidine according to the procedures of Example 3. MS m/z 694 (M+H)⁺.

### Example 133

### Compound of Formula (I); R¹=methoxy; R²=H; W is absent; R=2-methoxyethyl

The compound was prepared from the title compound of Example 1 and 2-methoxyethylamine according to the procedures of Example 3. MS m/z 655 (M+H)⁺.

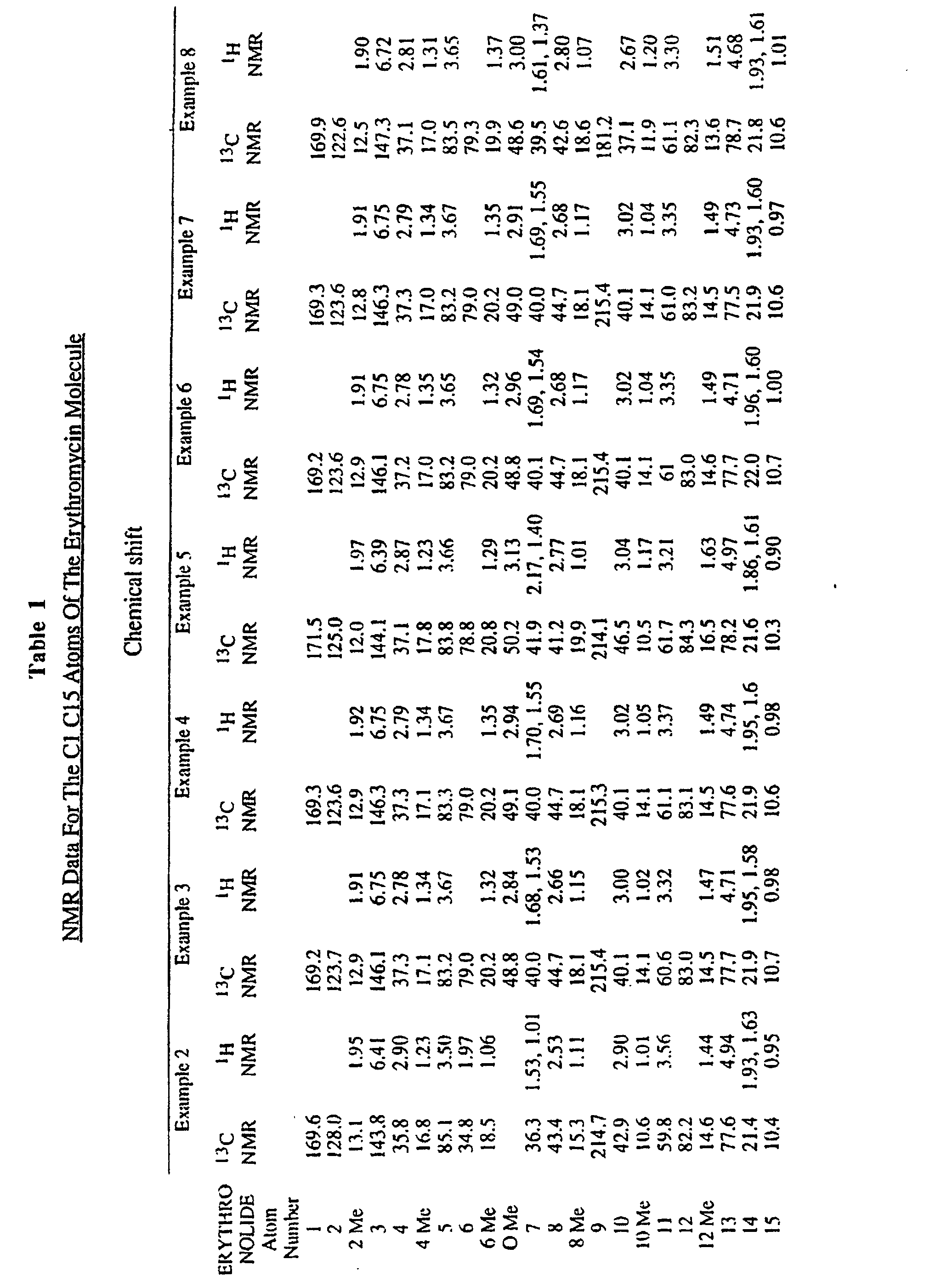

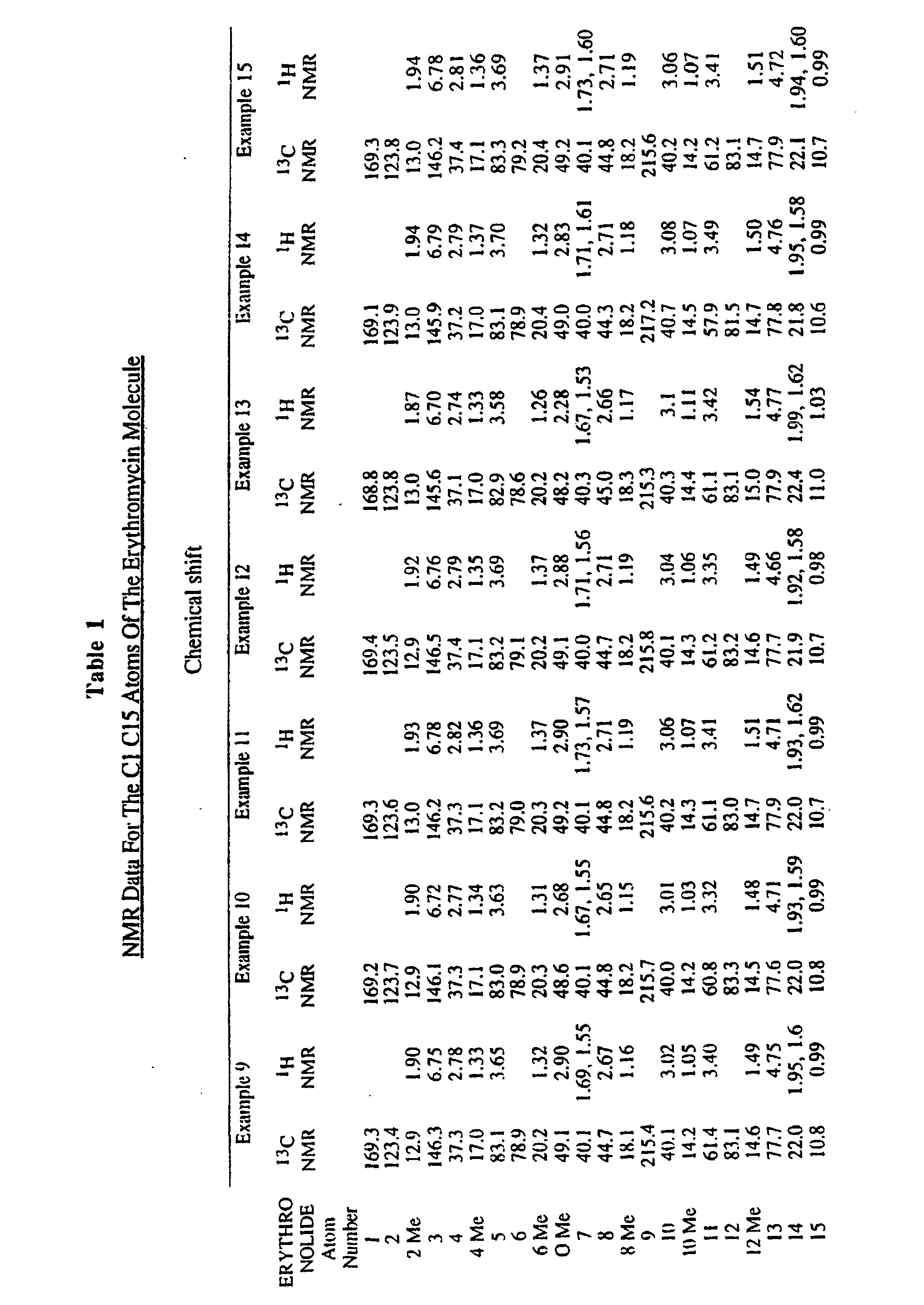

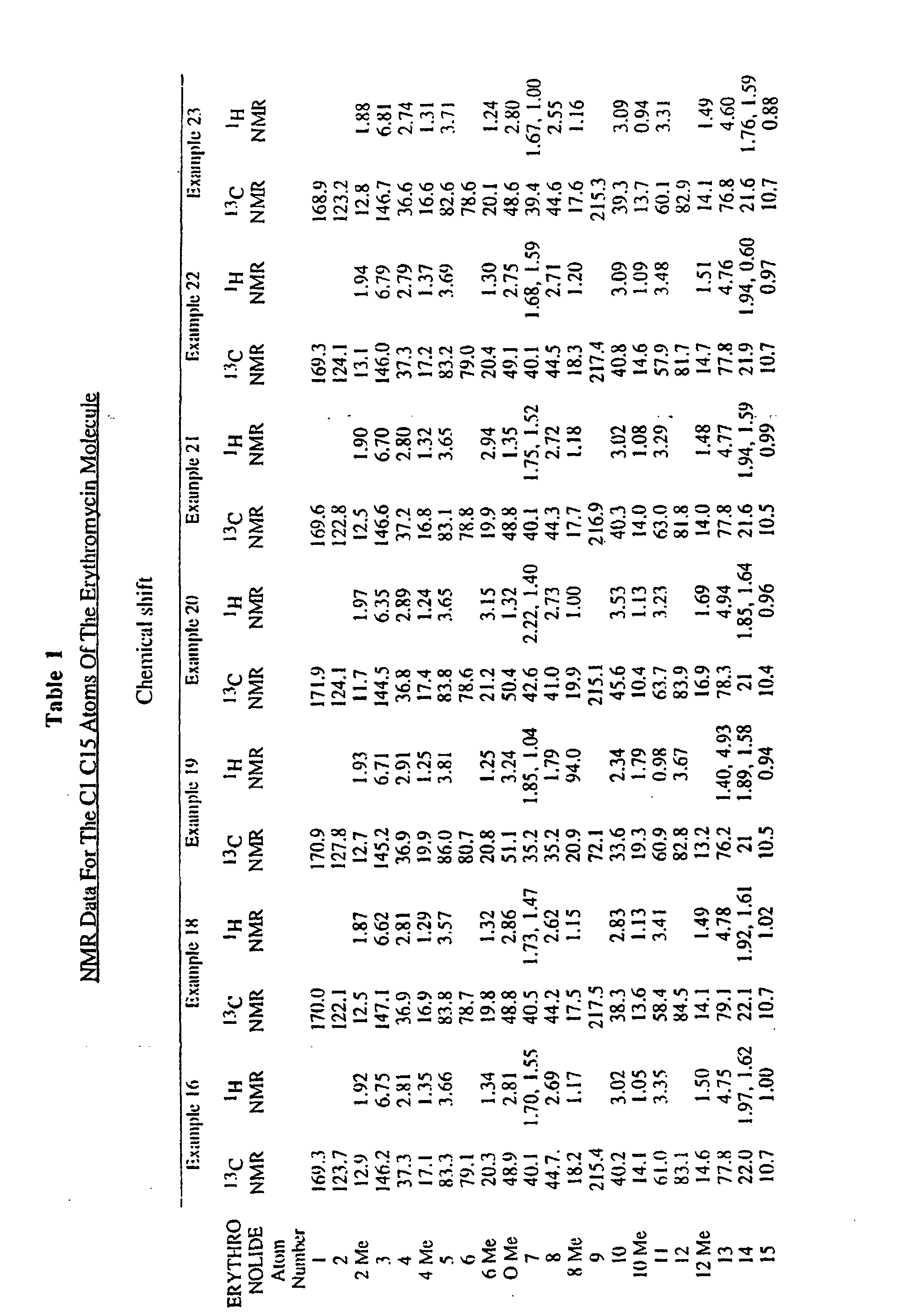

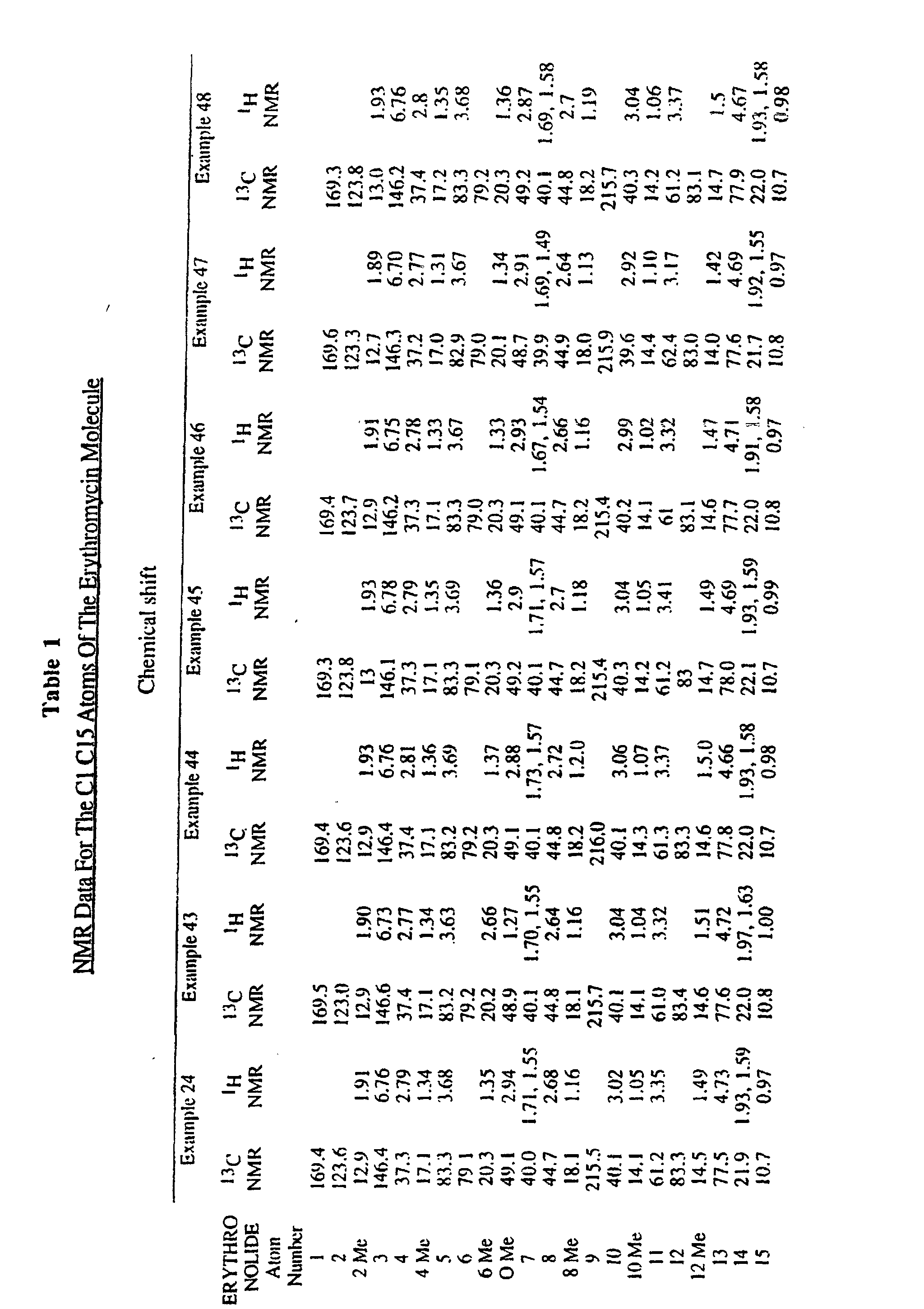

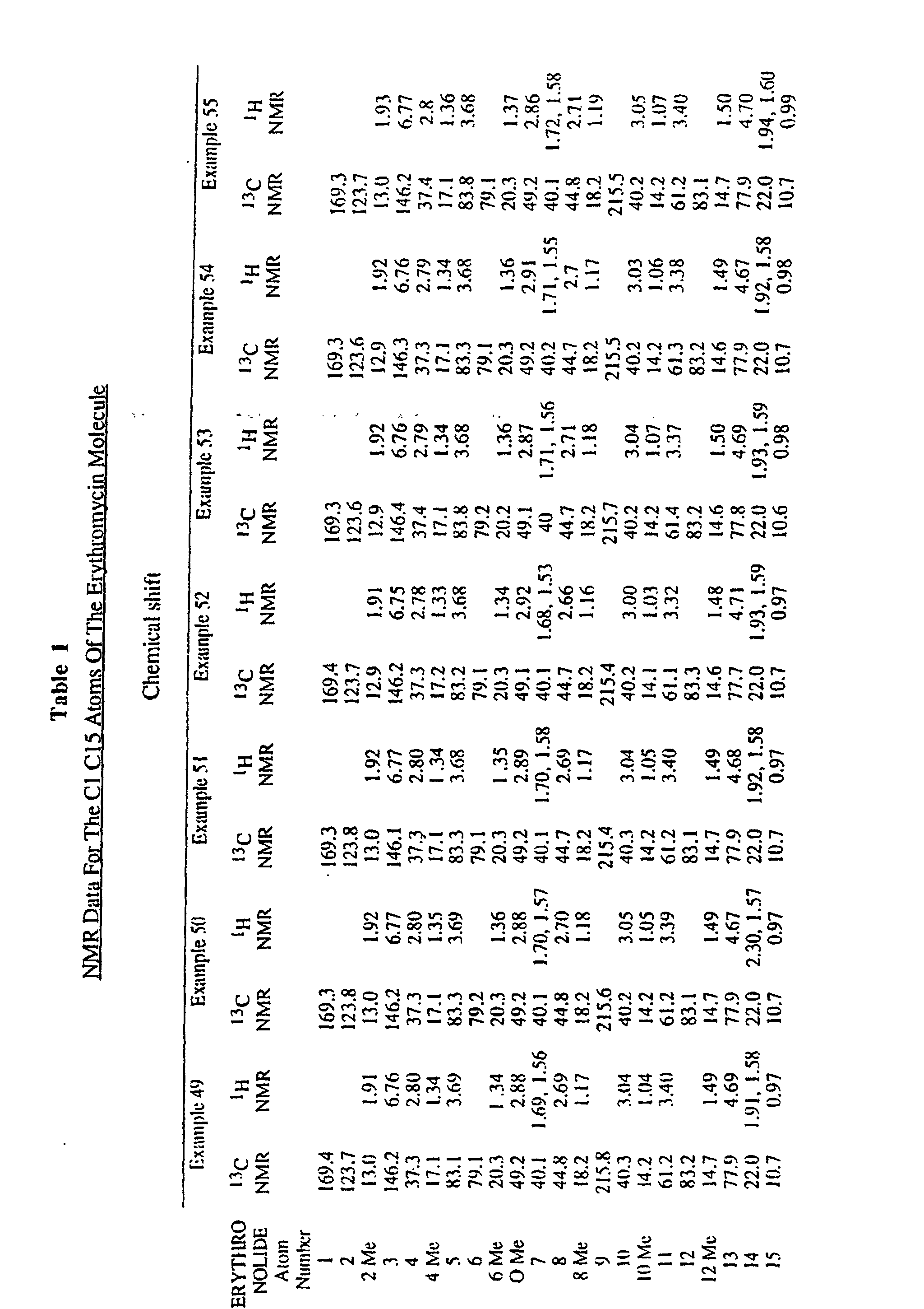

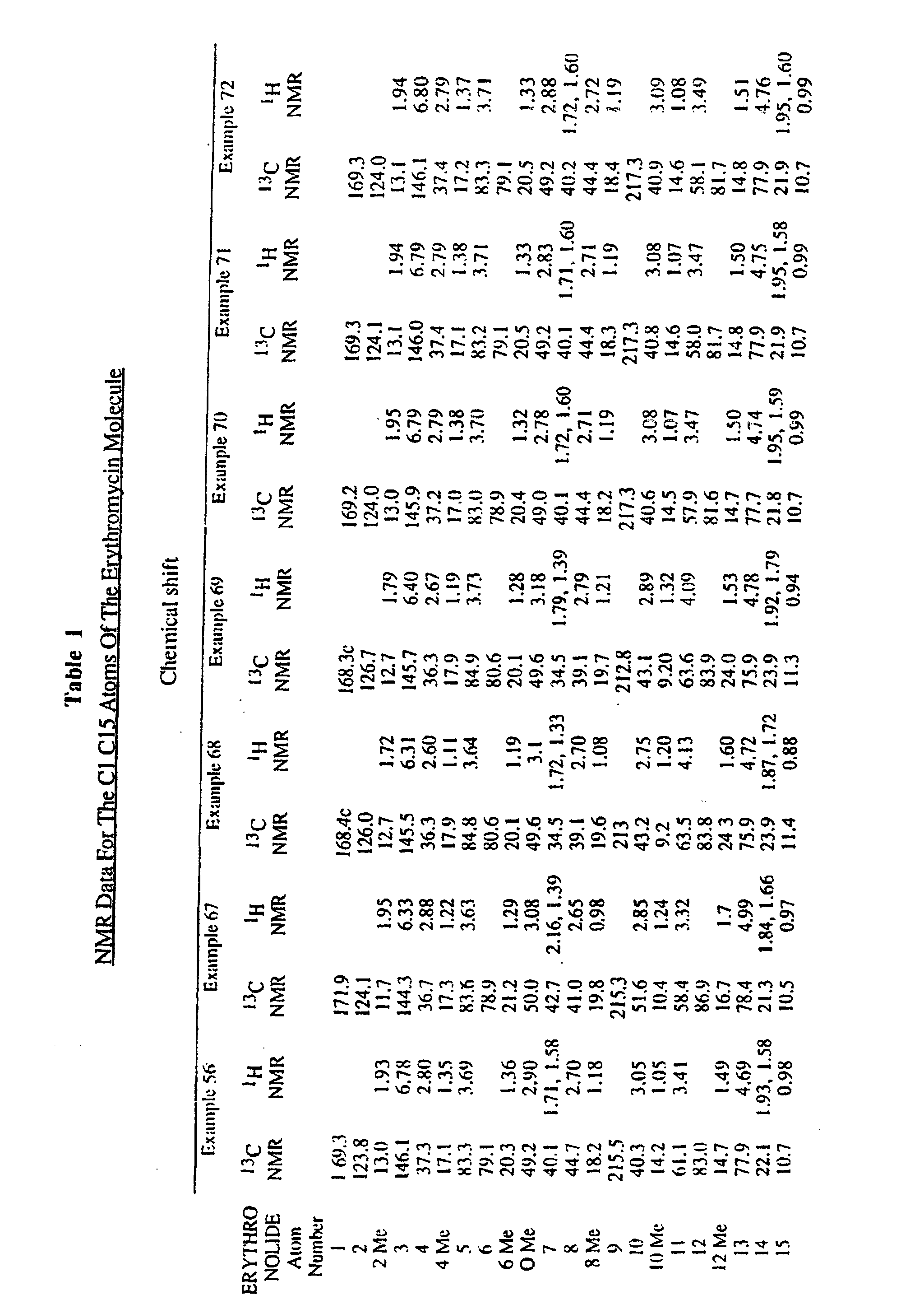

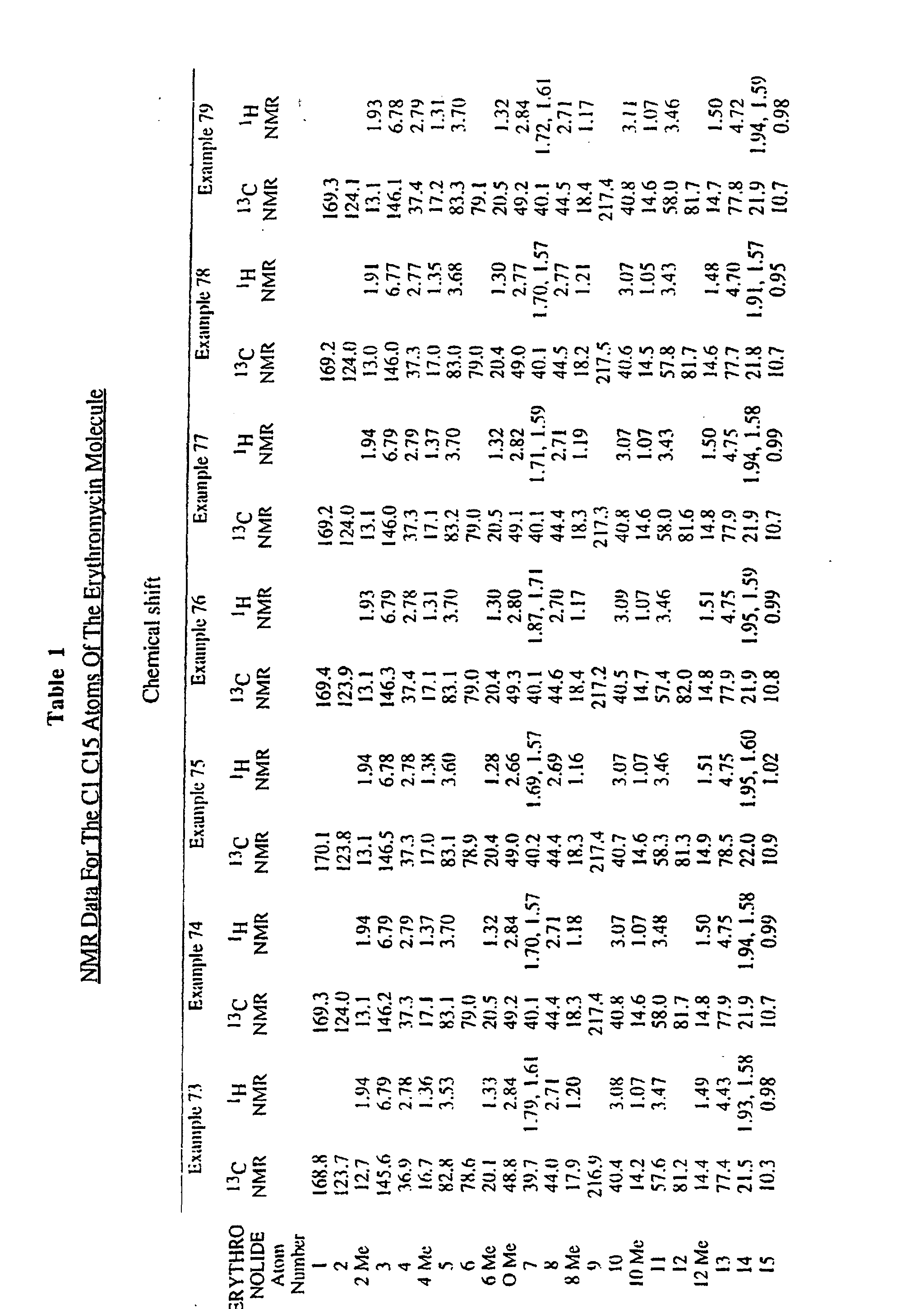

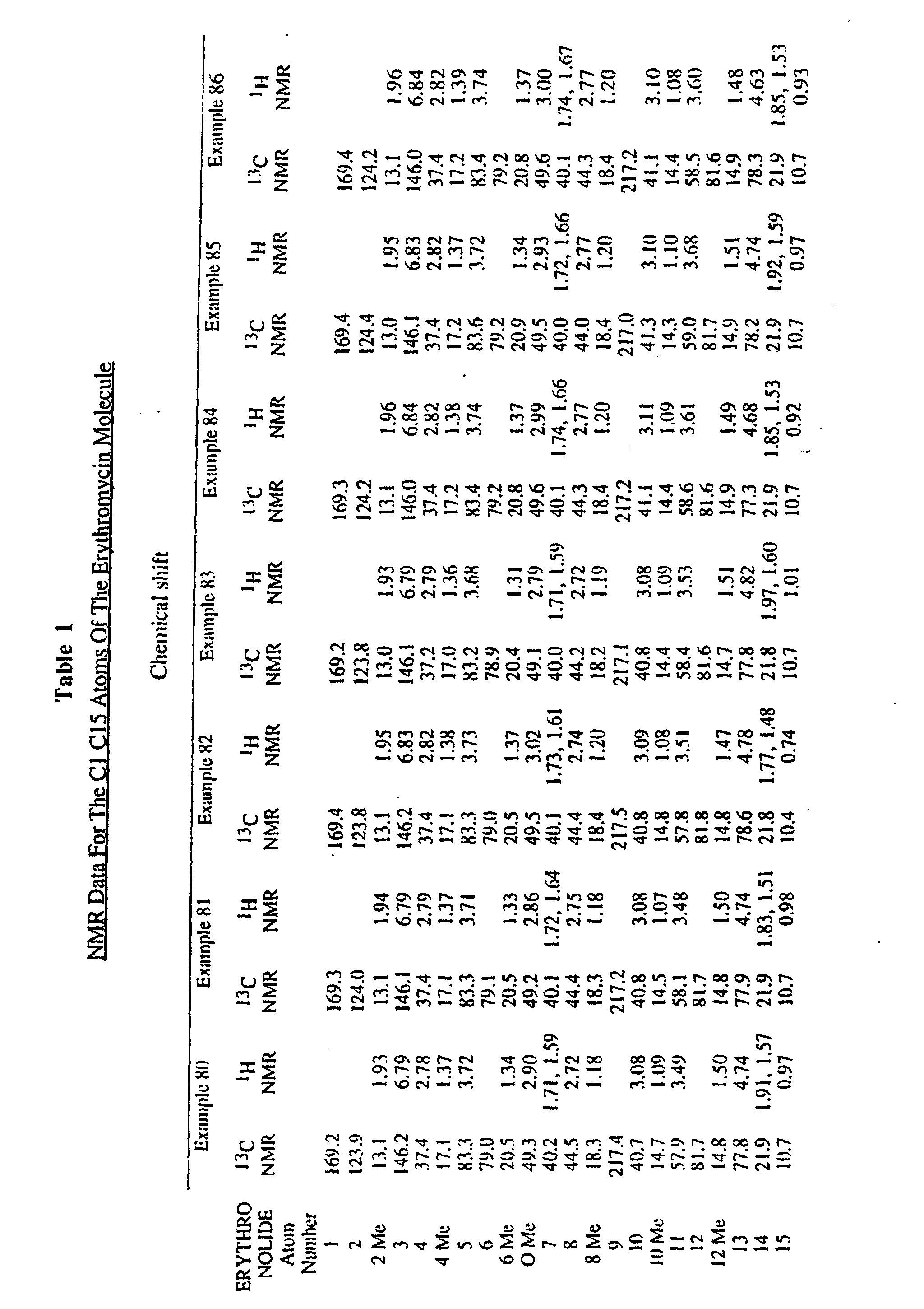

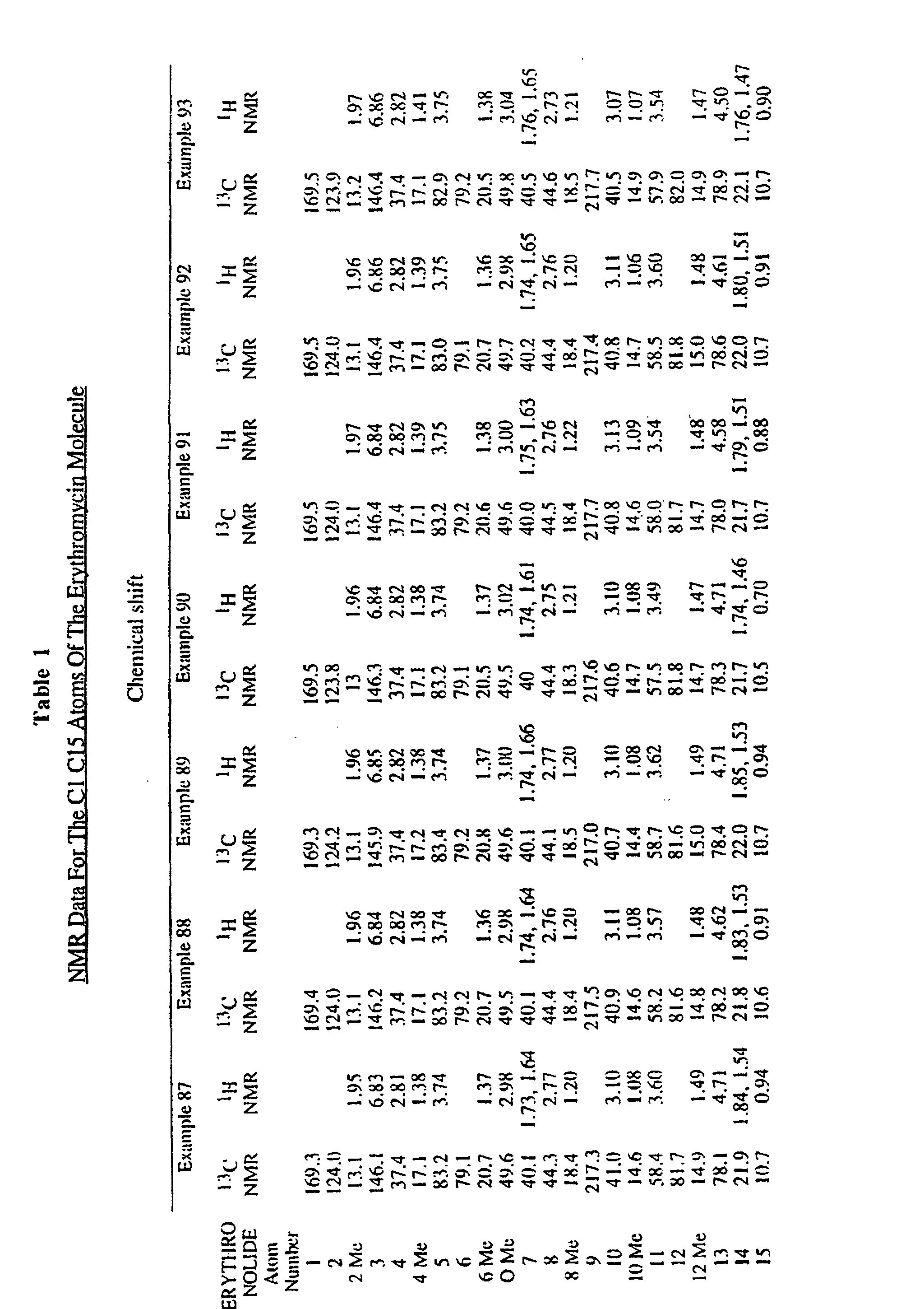

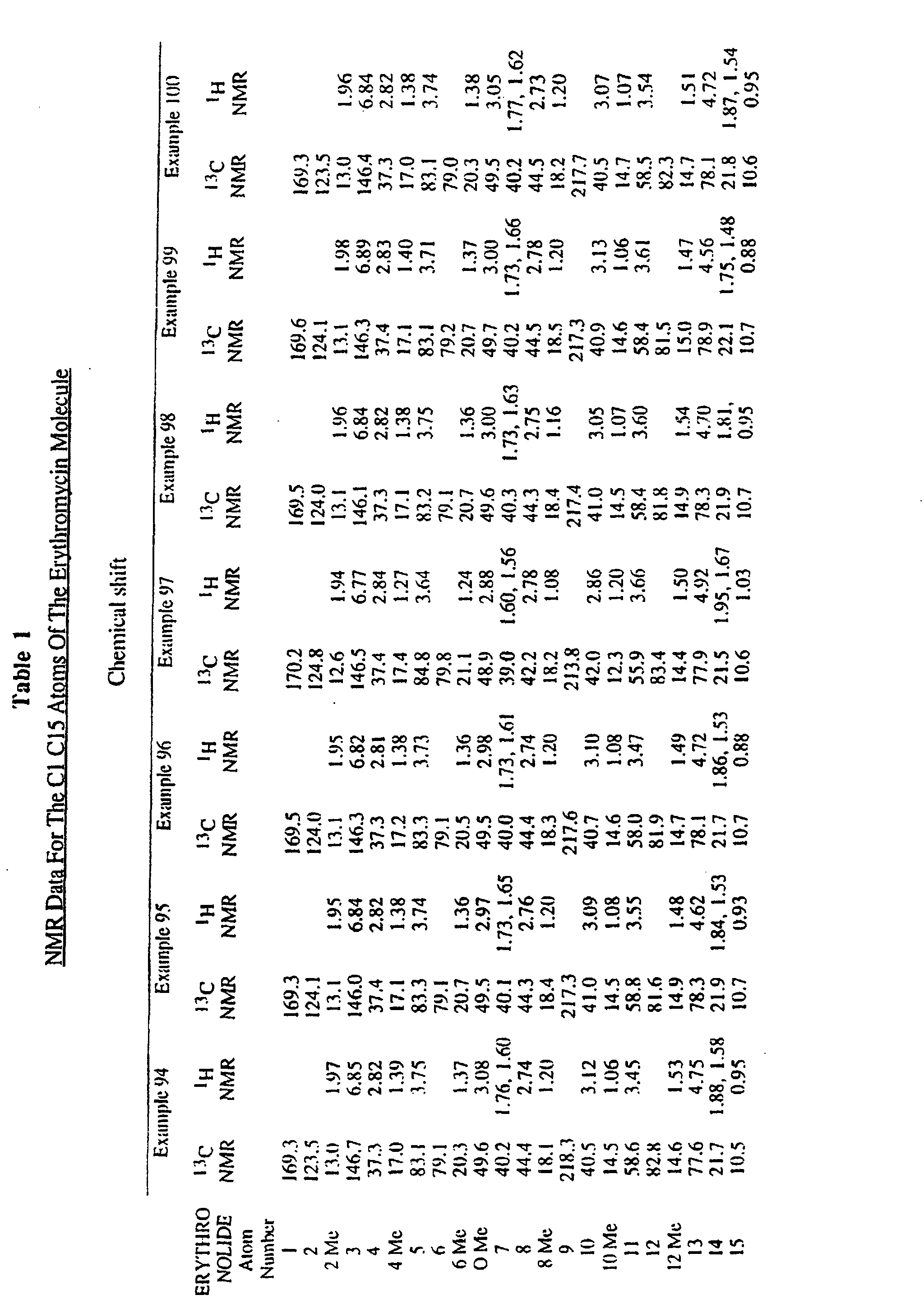

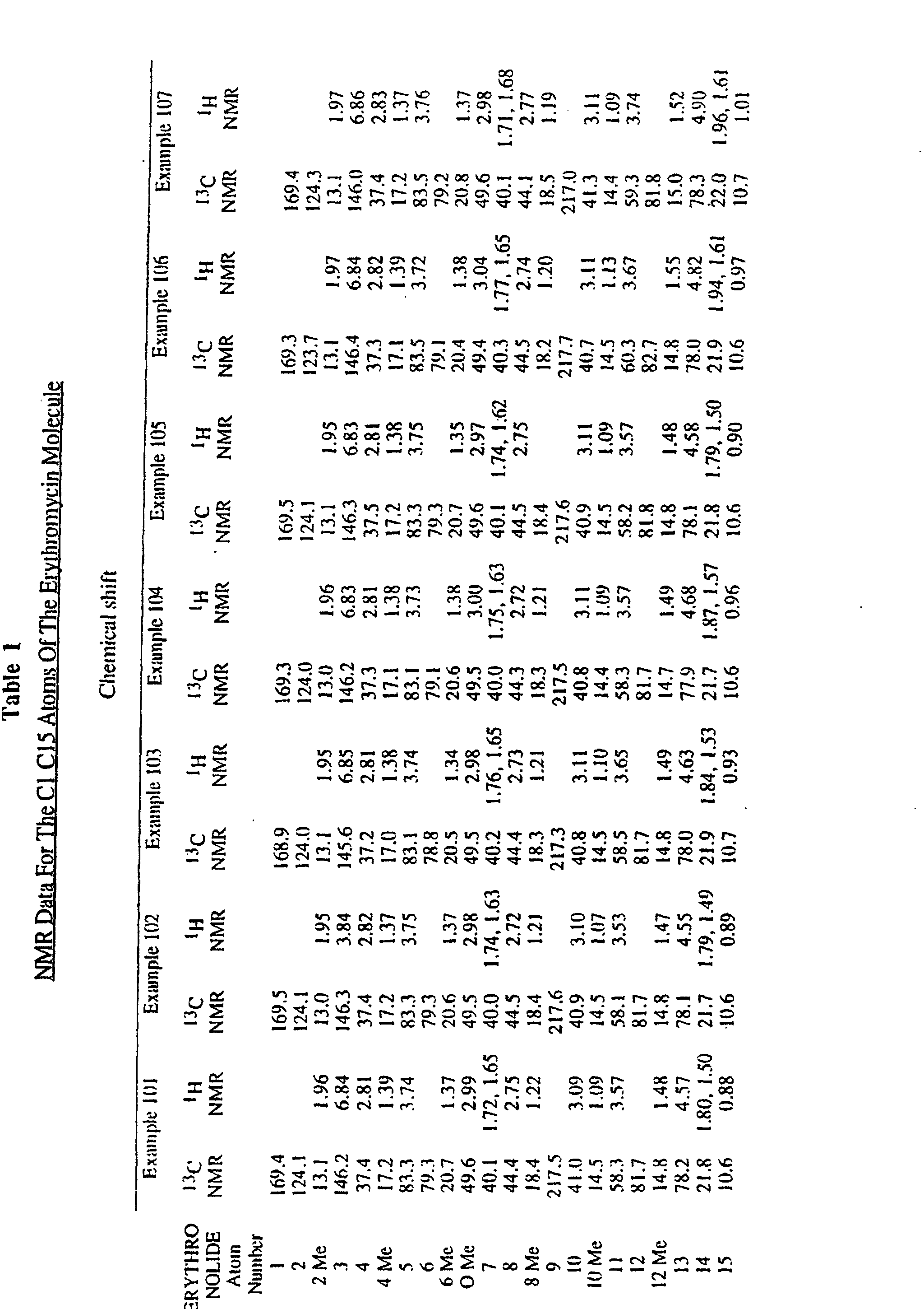

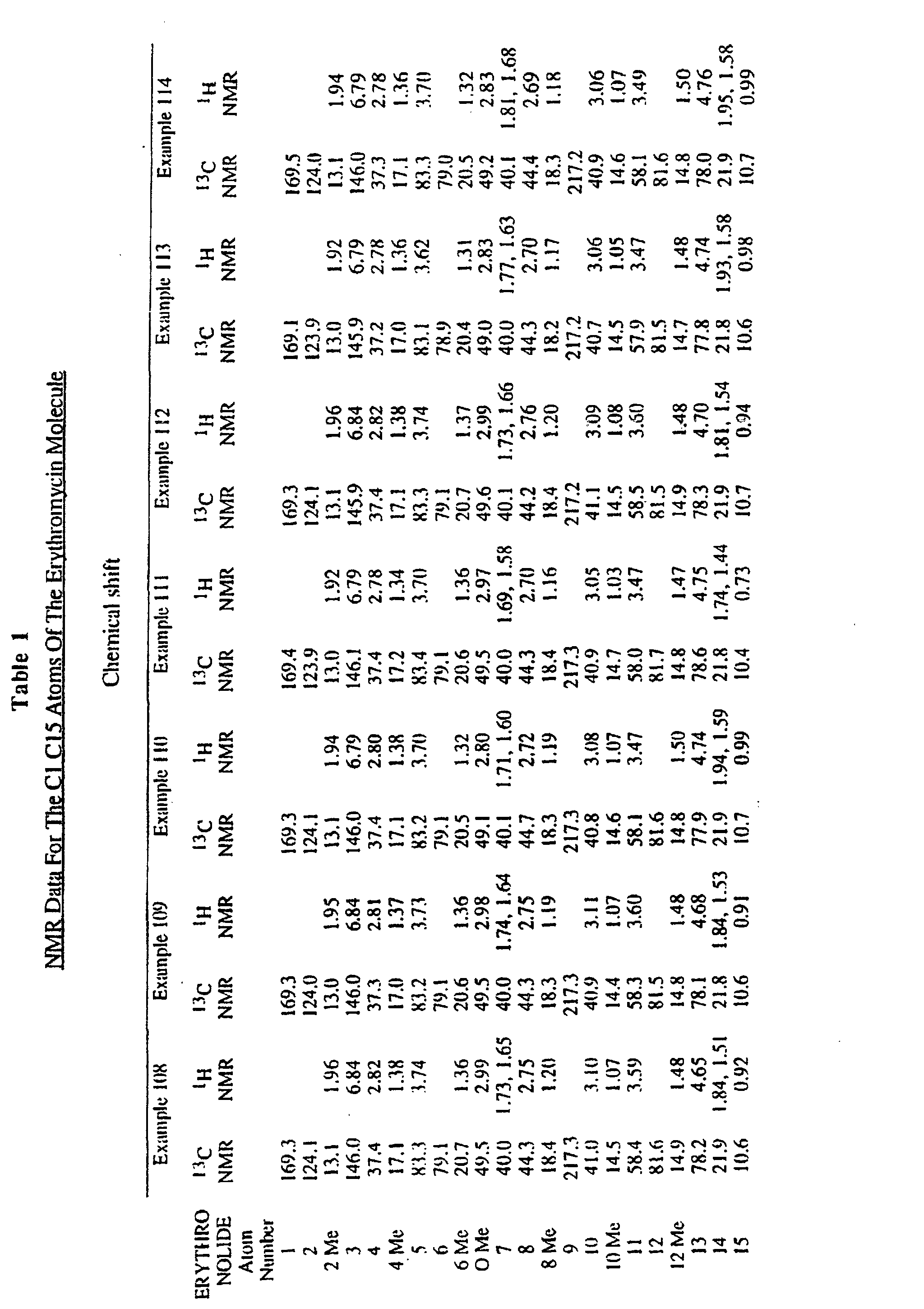

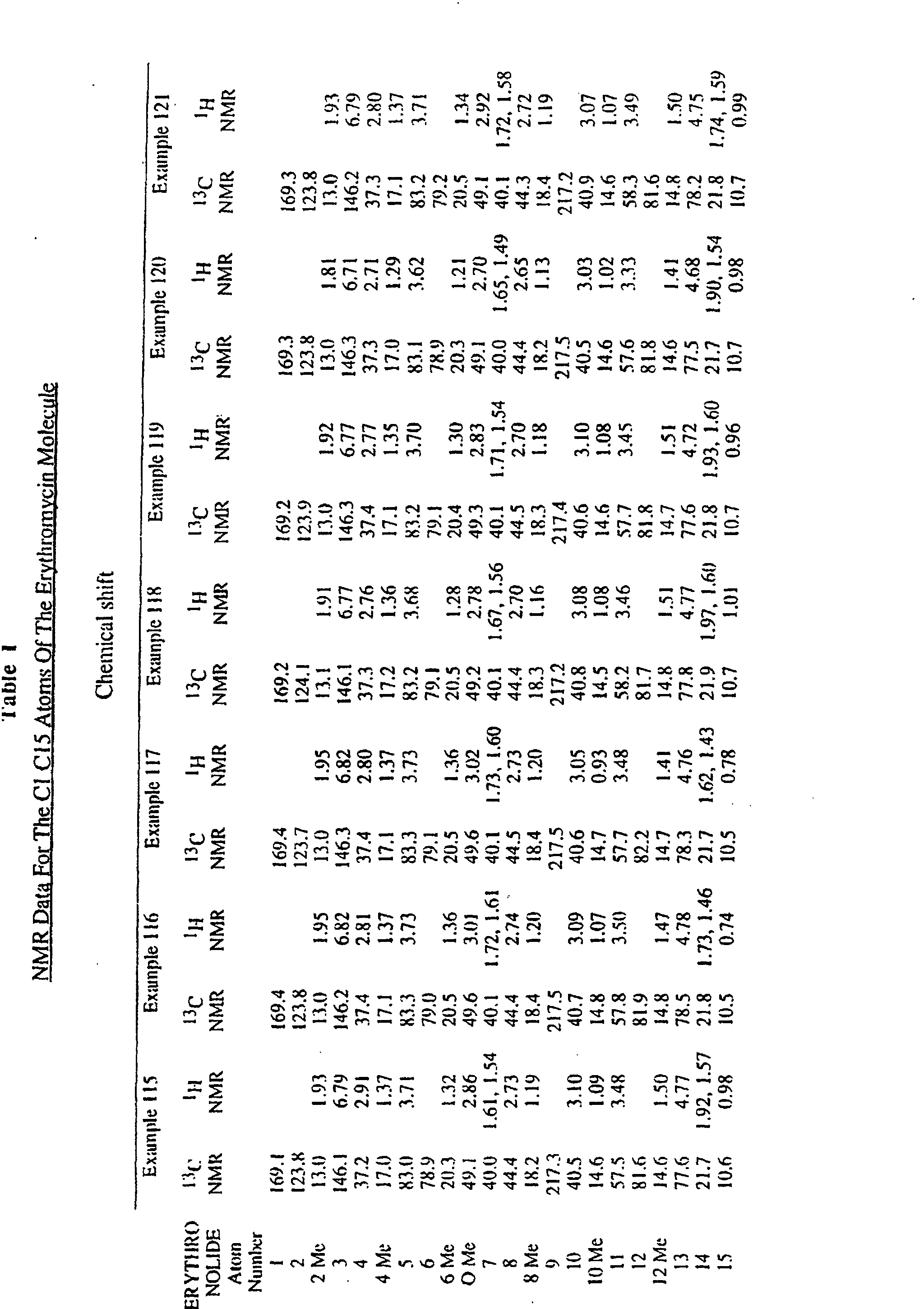

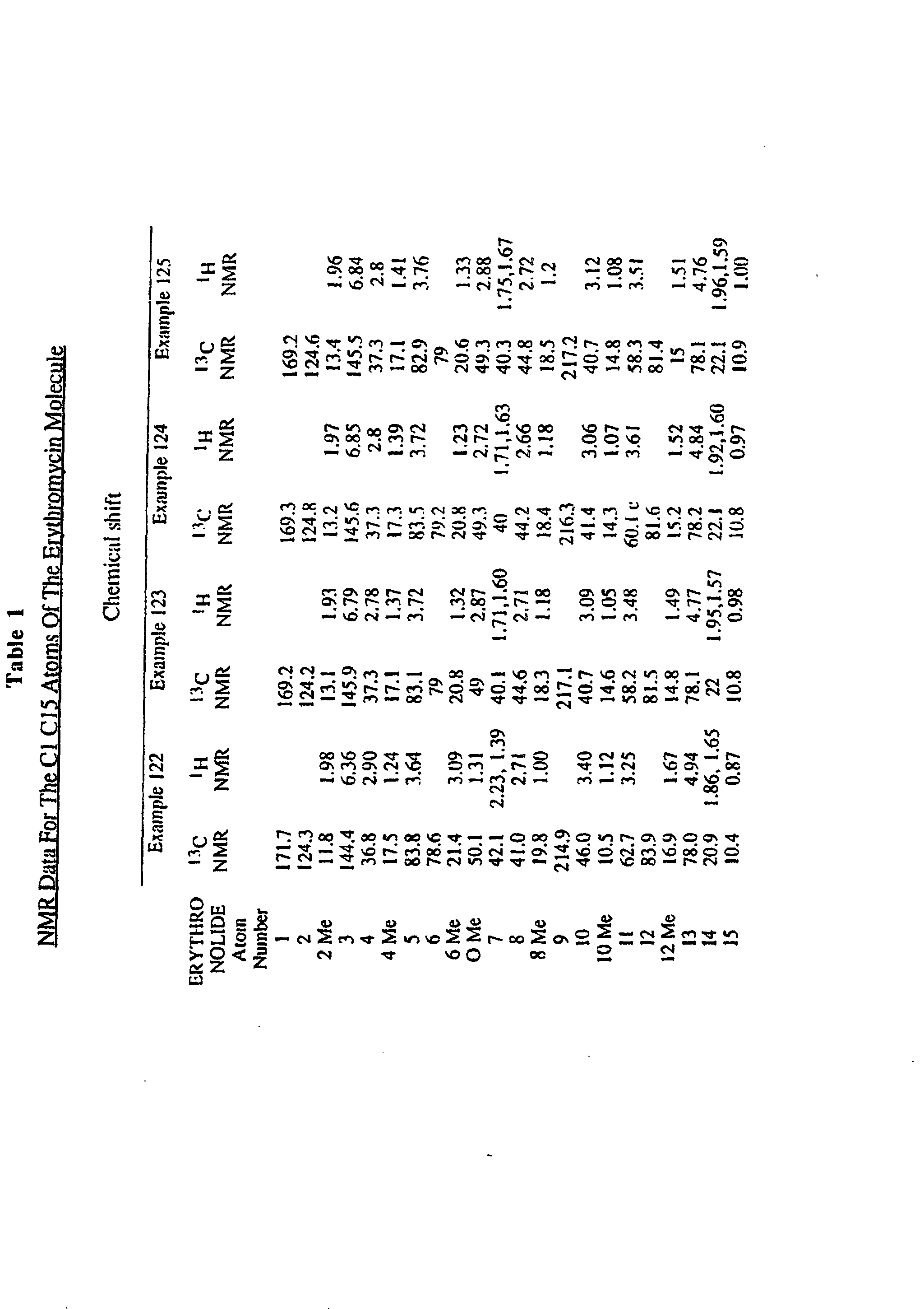

### Biological Data

### Example 44

### In Vitro Assay of Antibacterial Activity

Representative compounds of the present invention were assayed *in vitro* for antibacterial activity as follows: Twelve petri dishes containing successive aqueous dilutions of the test compound mixed with 10 mL of sterilized Brain Heart Infusion (BHI) agar (Difco 0418-01-5) were prepared. Each plate was inoculated with 1:100 (or 1:10 for slow-growing strains, such as *Micrococcus* and *Streptococcus)* dilutions of up to 32 different microorganisms, using a Steers replicator block. The inoculated plates were incubated at 35-37 °C for 20 to 24 hours. In addition, a control plate, using BHI agar containing no test compound, was prepared and incubated at the beginning and end of each test.

An additional plate containing a compound having known susceptibility patterns for the organisms being tested and belonging to the same antibiotic class as the test compound was also prepared and incubated as a further control, as well as to provide test-to-test comparability. Erythromycin A was used for this purpose.

After incubation, each plate was visually inspected. The minimum inhibitory concentration (MIC) was defined as the lowest concentration of drug yielding no growth, a slight haze, or sparsely isolated colonies on the inoculum spot as compared to the growth control. The results of this assay with selected compounds, shown in Table 2 below, demonstrate the antibacterial activity of the compounds of the invention.

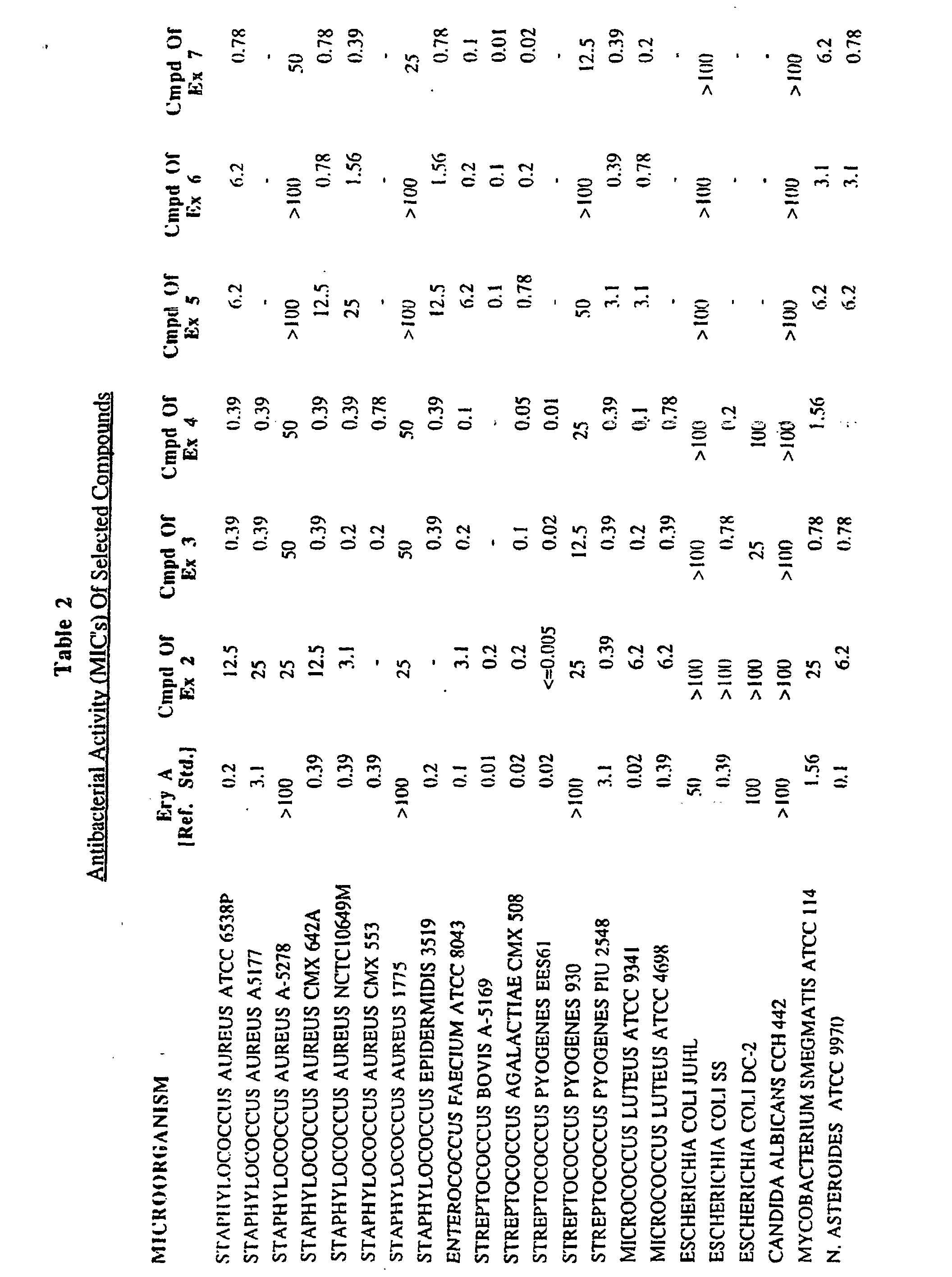

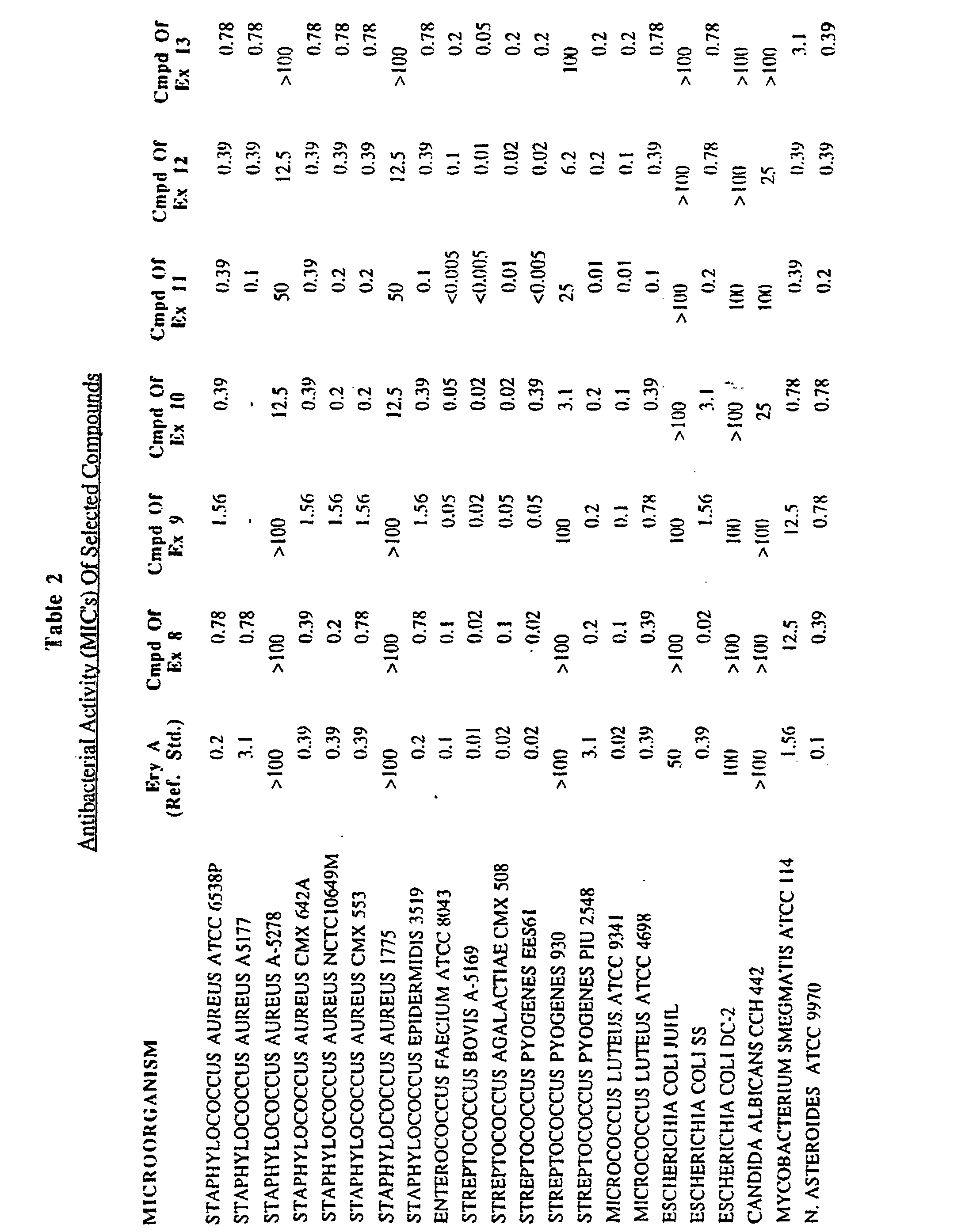

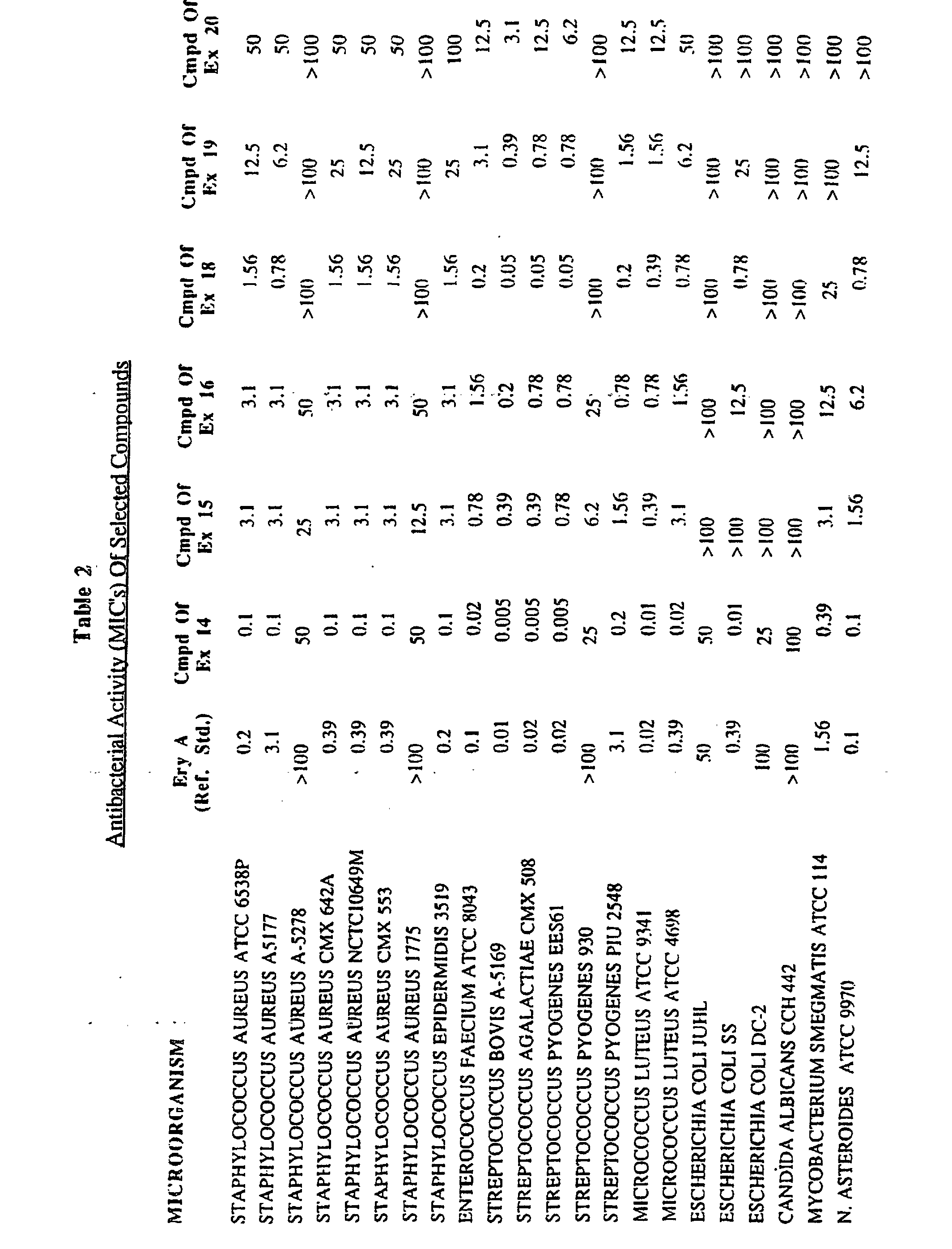

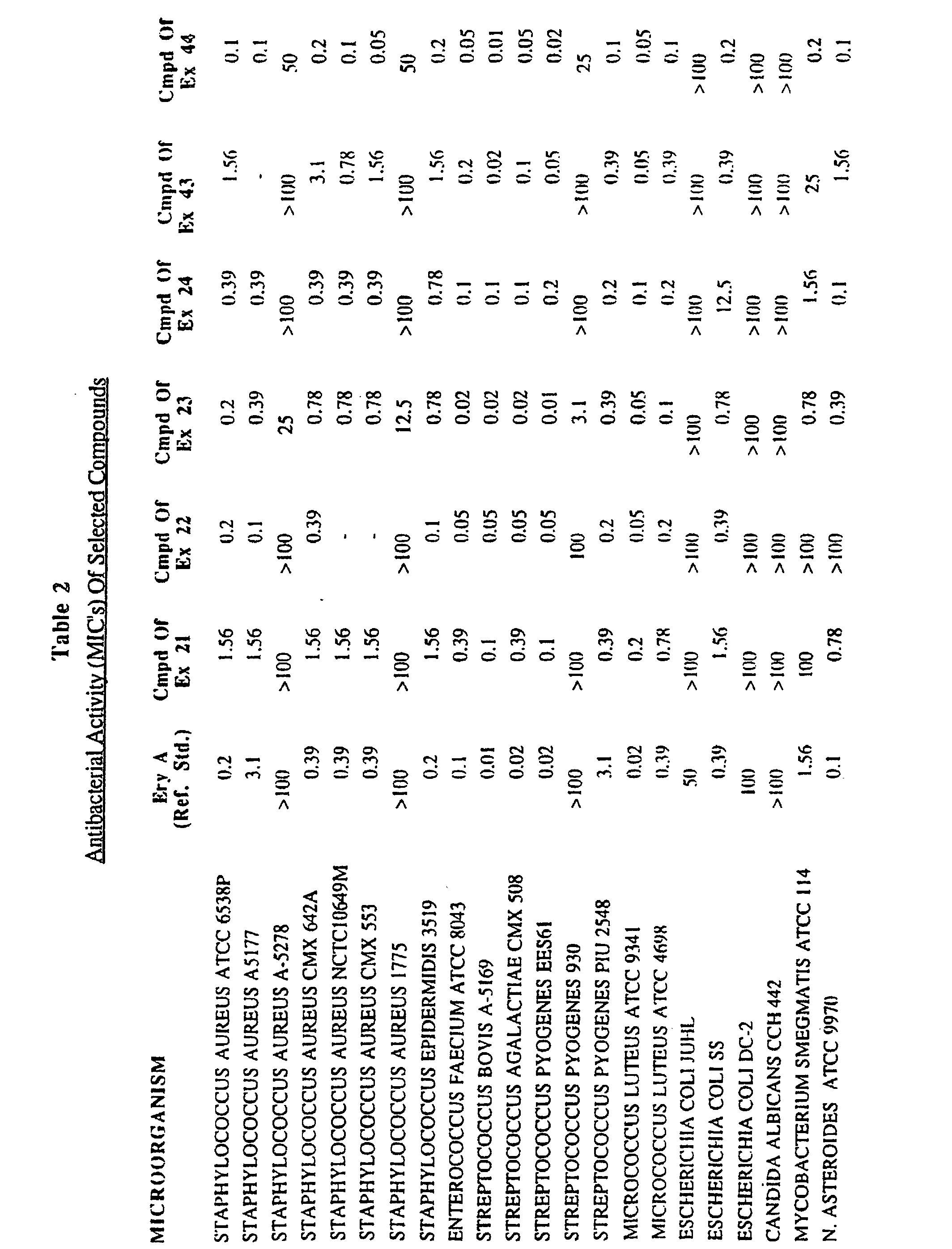

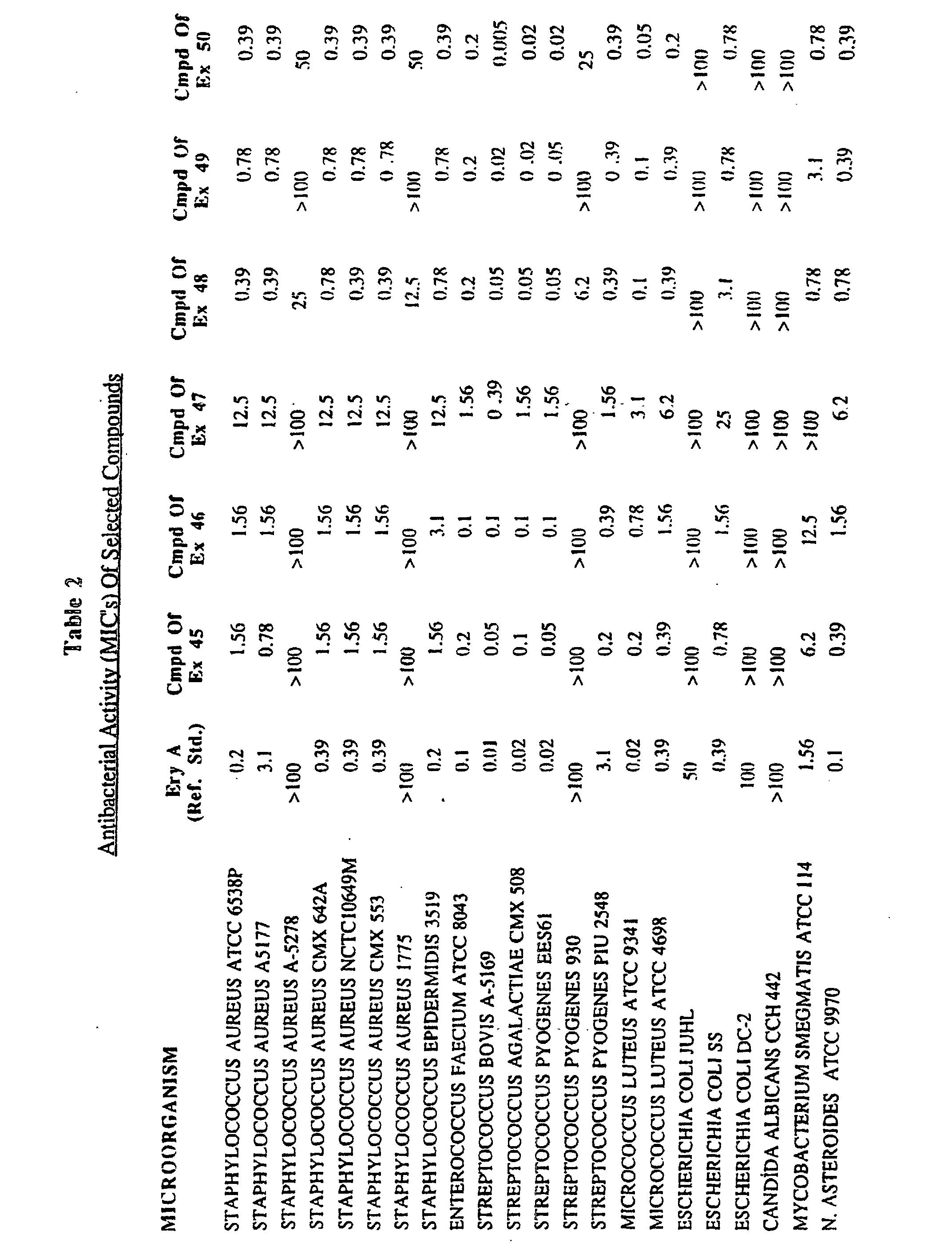

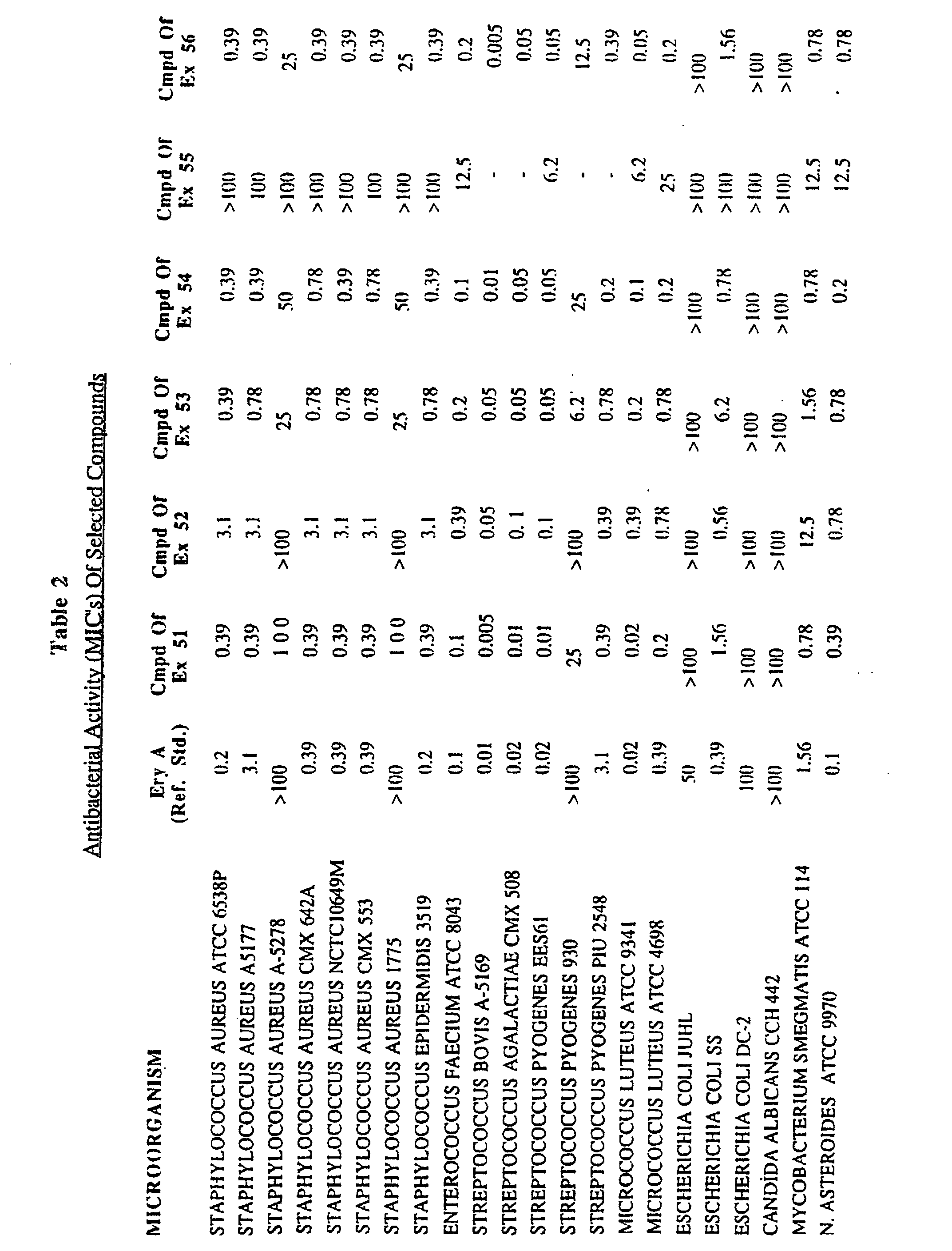

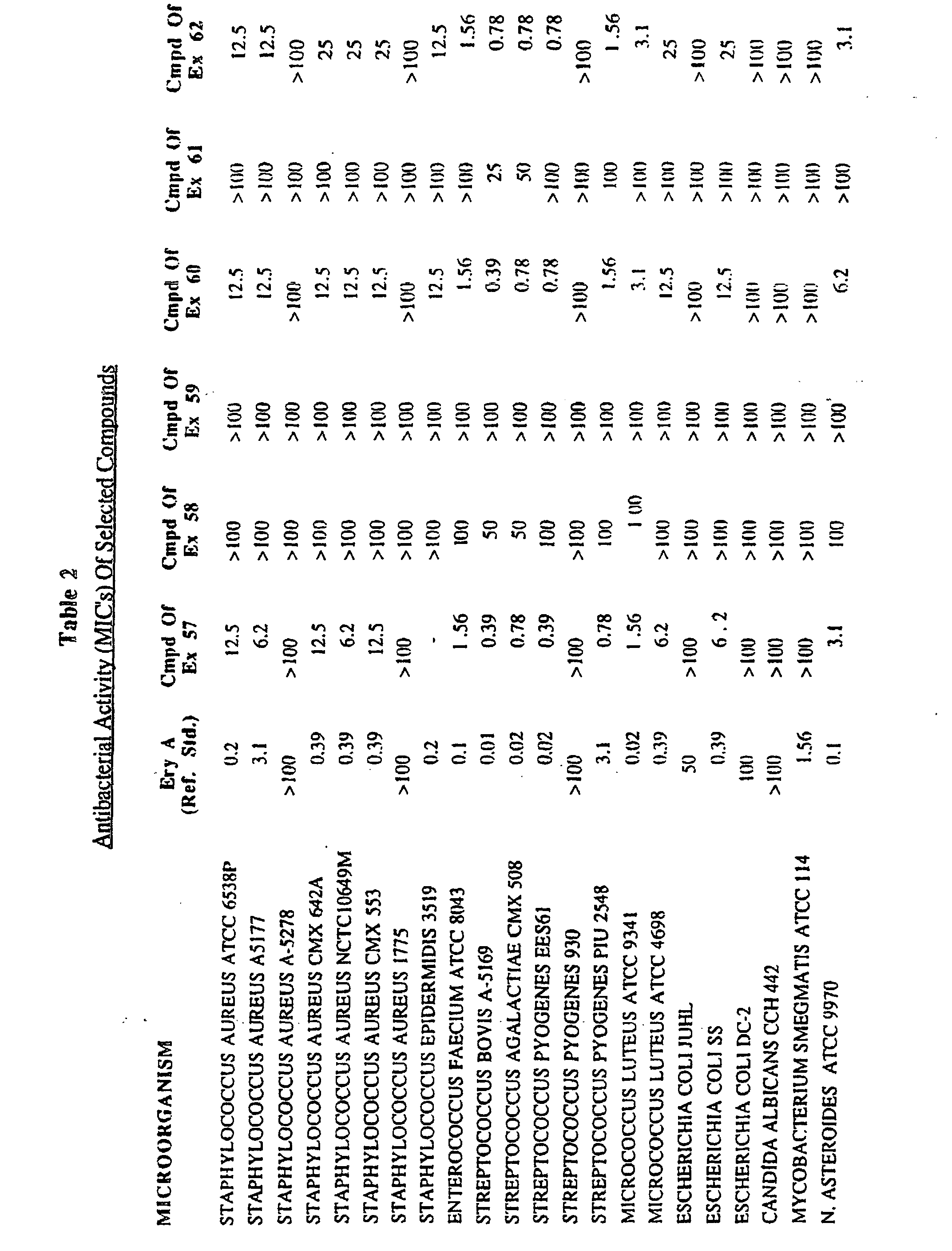

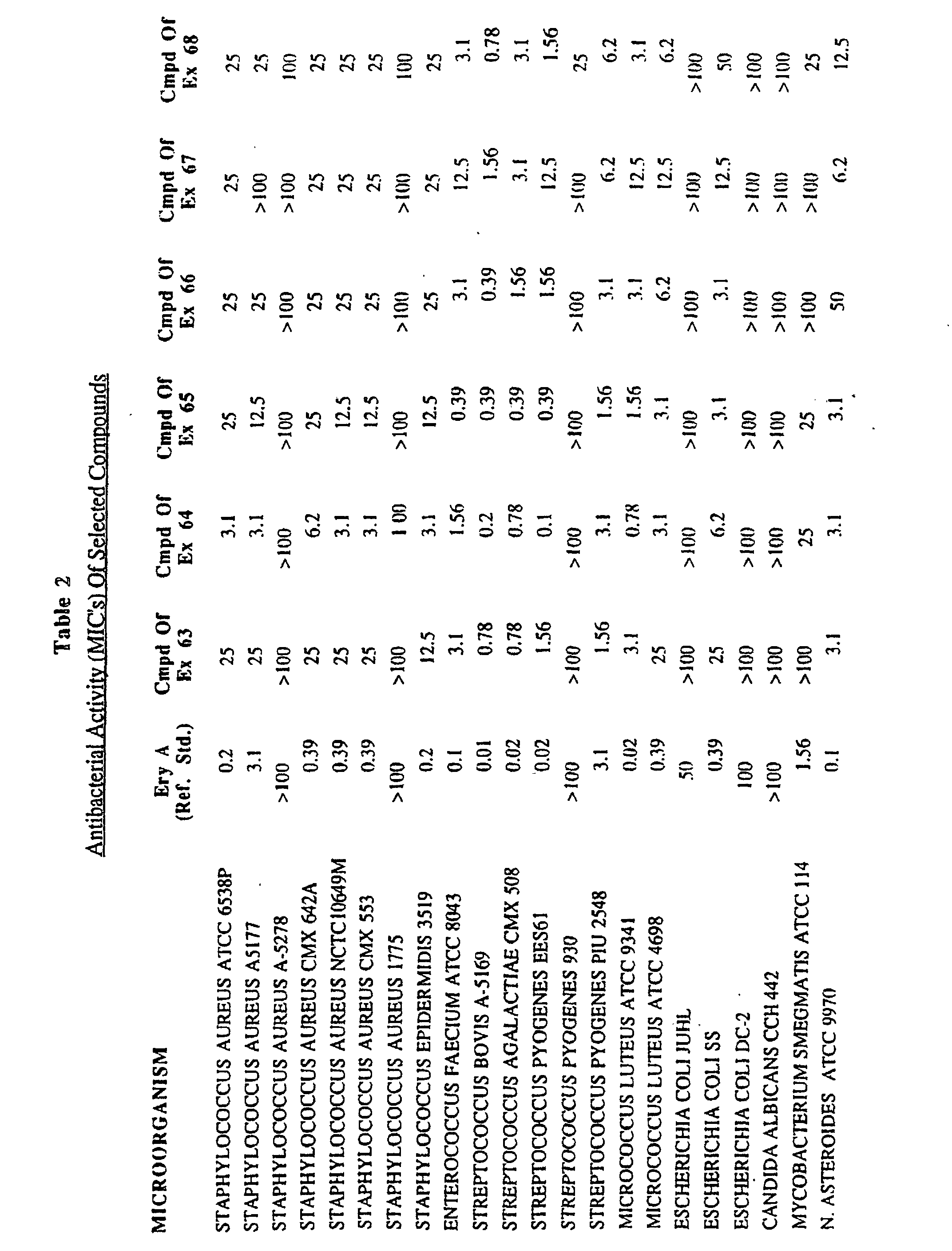

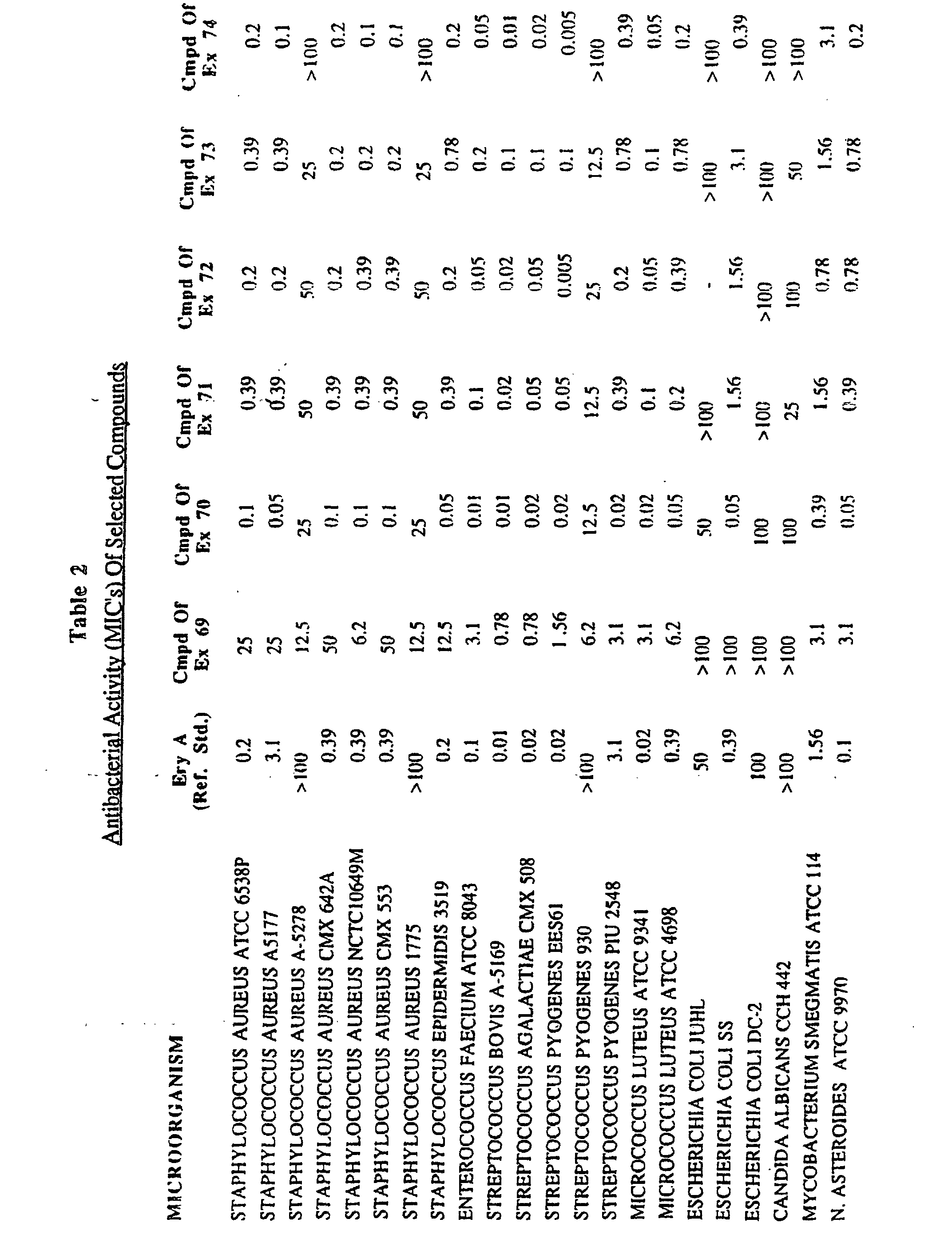

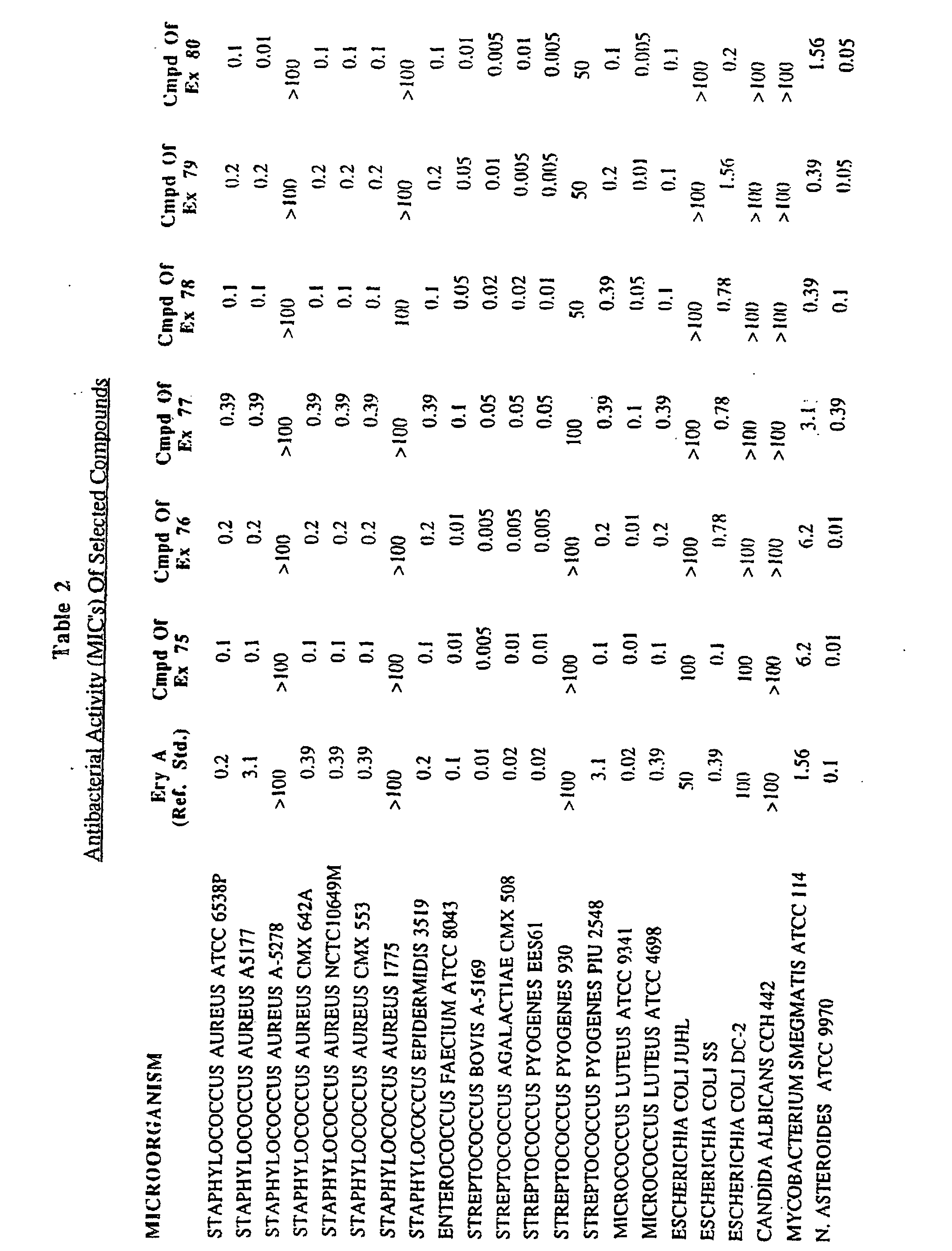

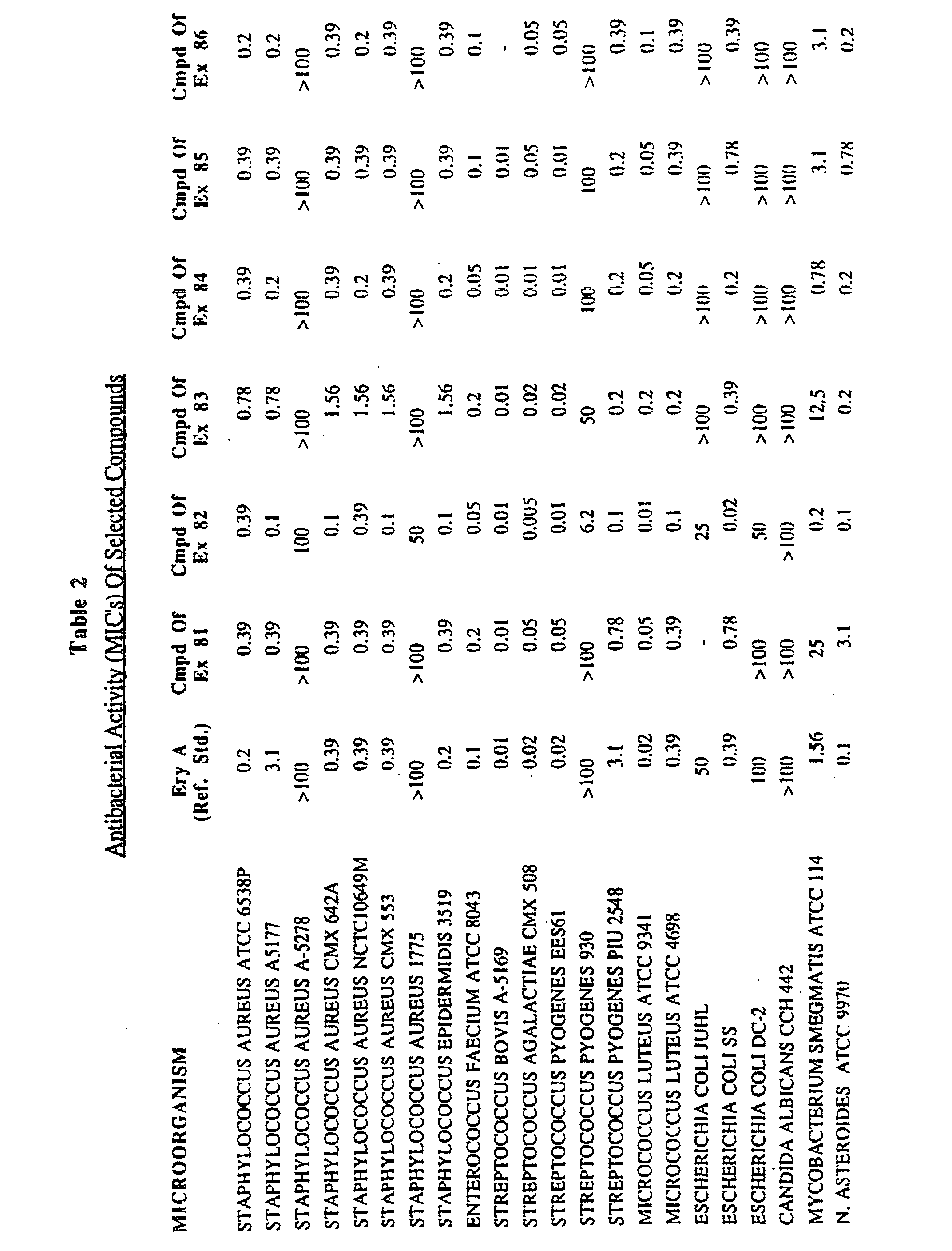

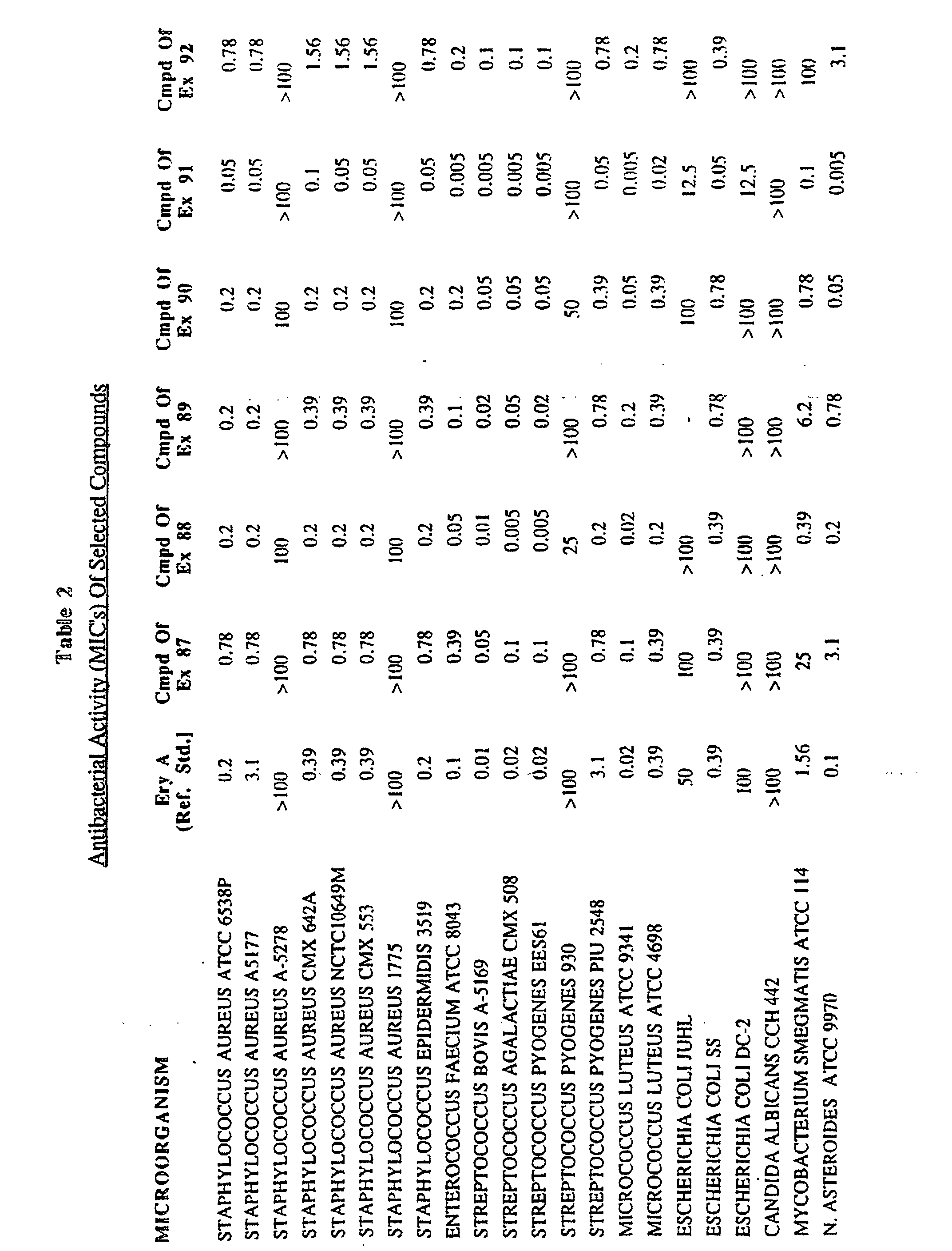

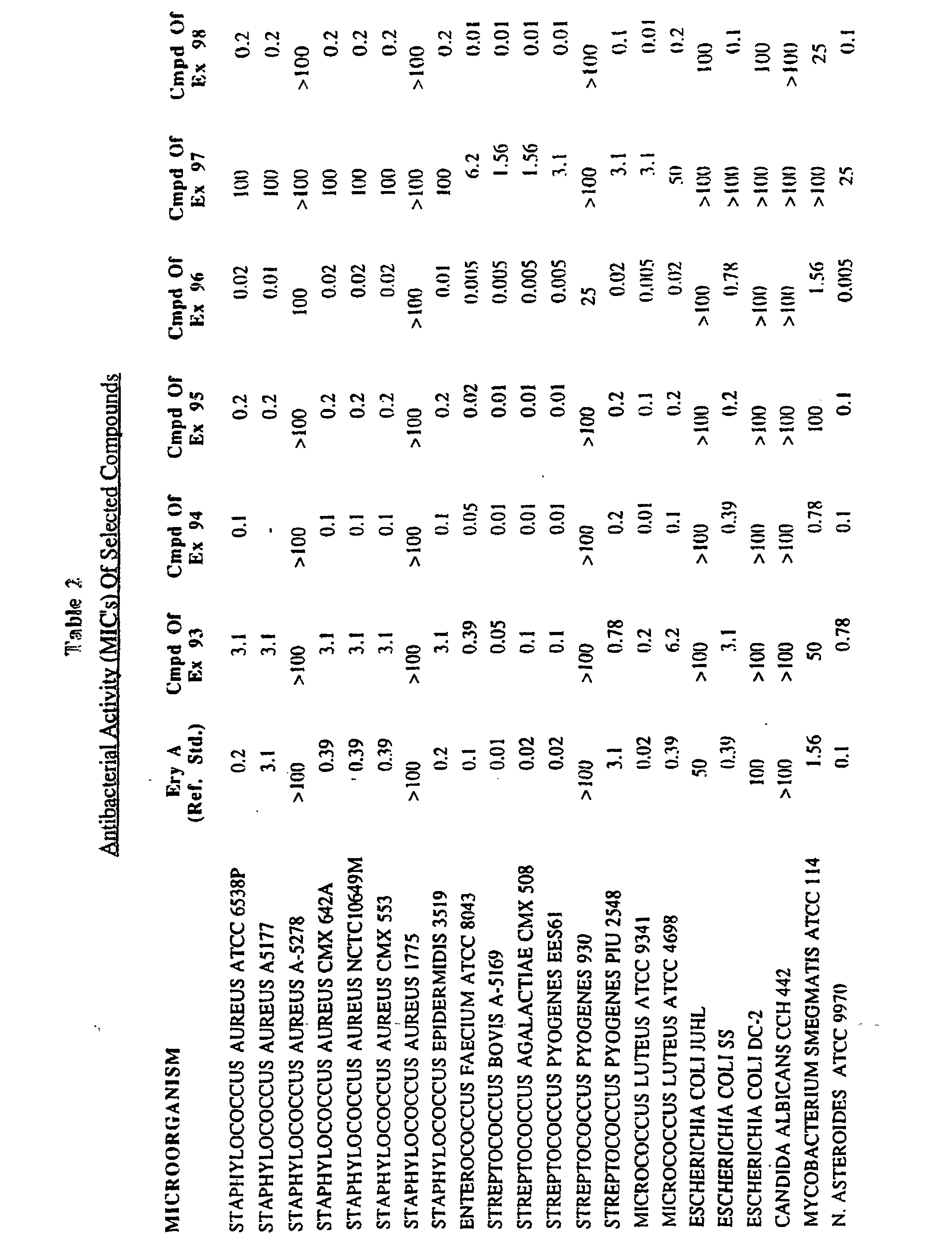

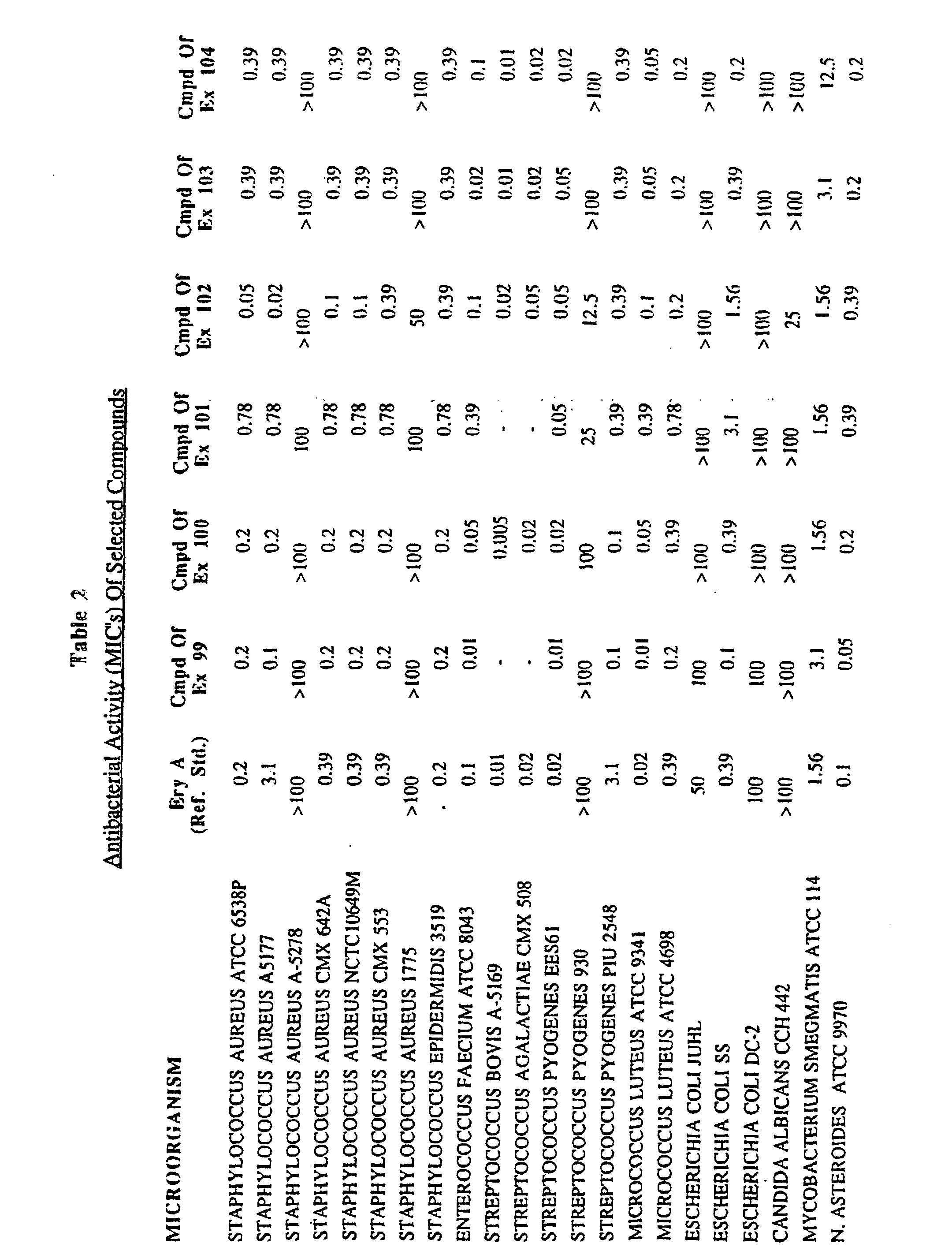

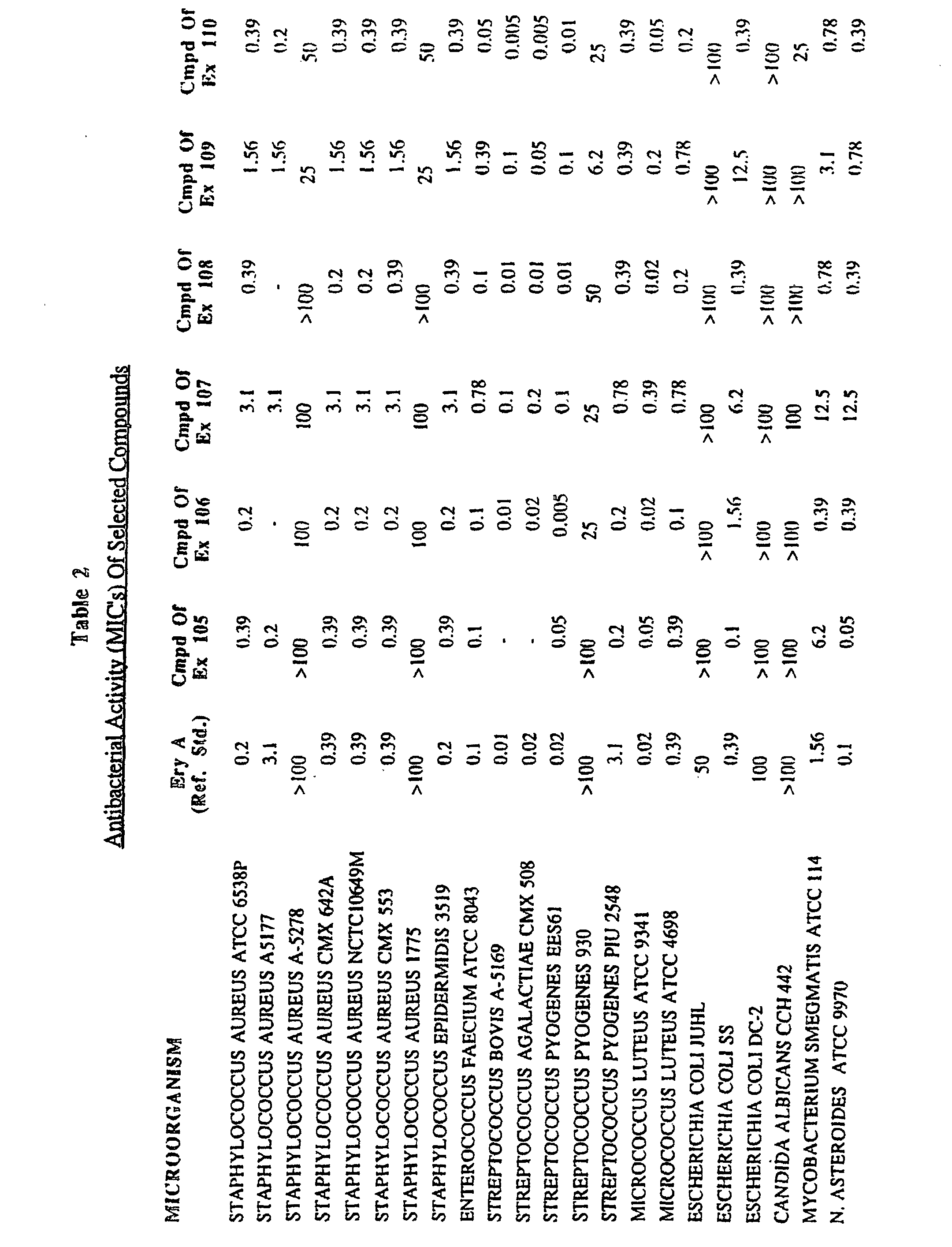

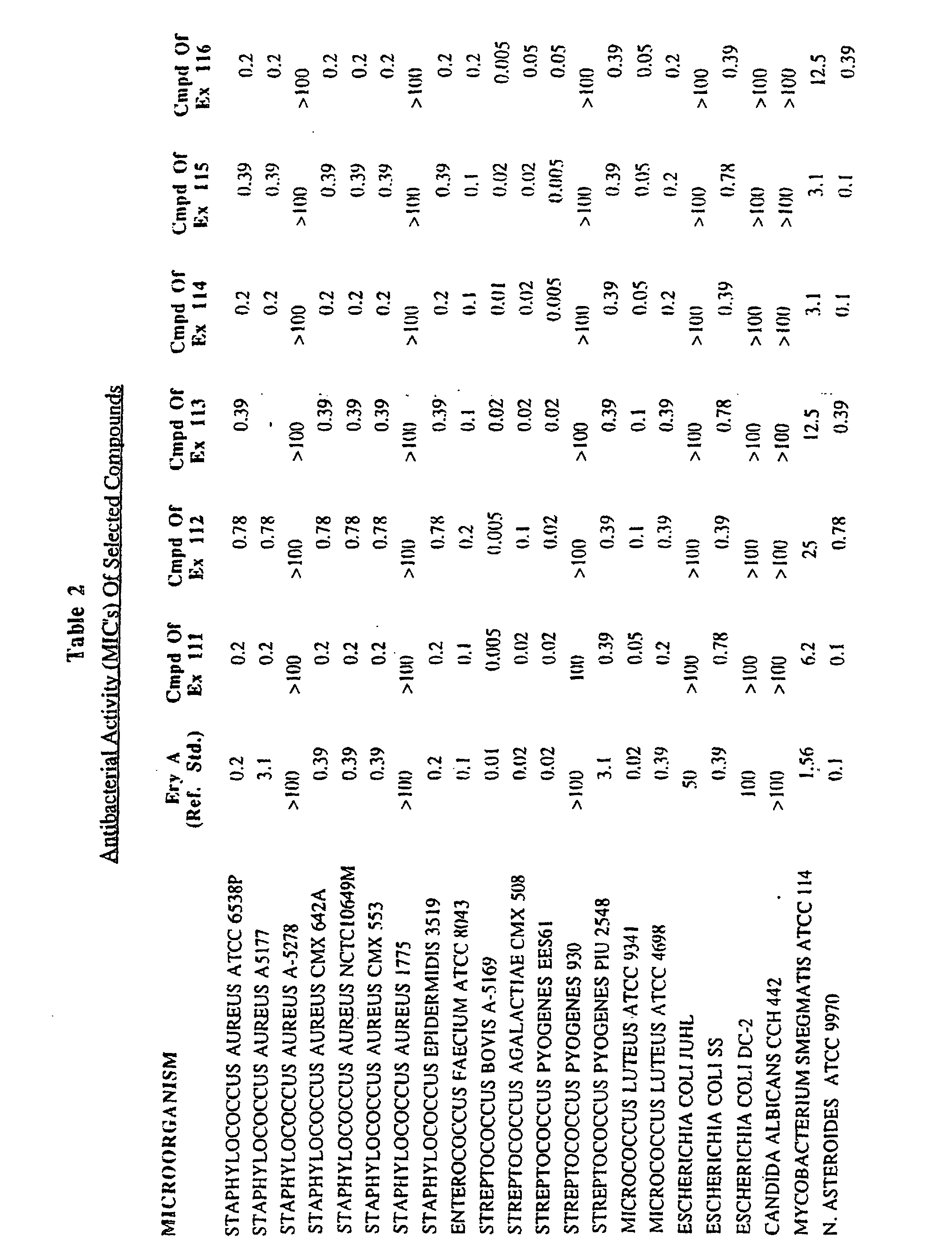

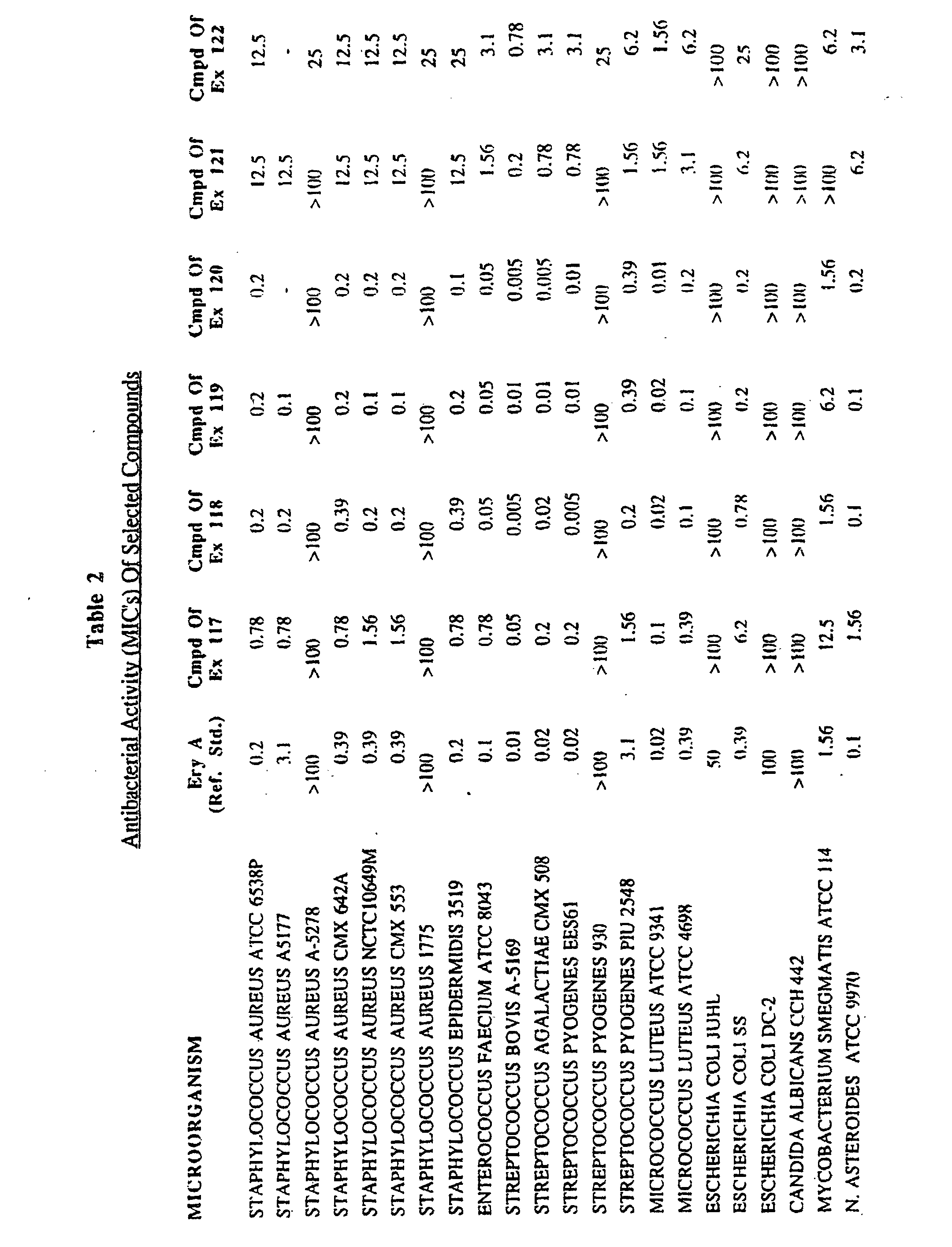

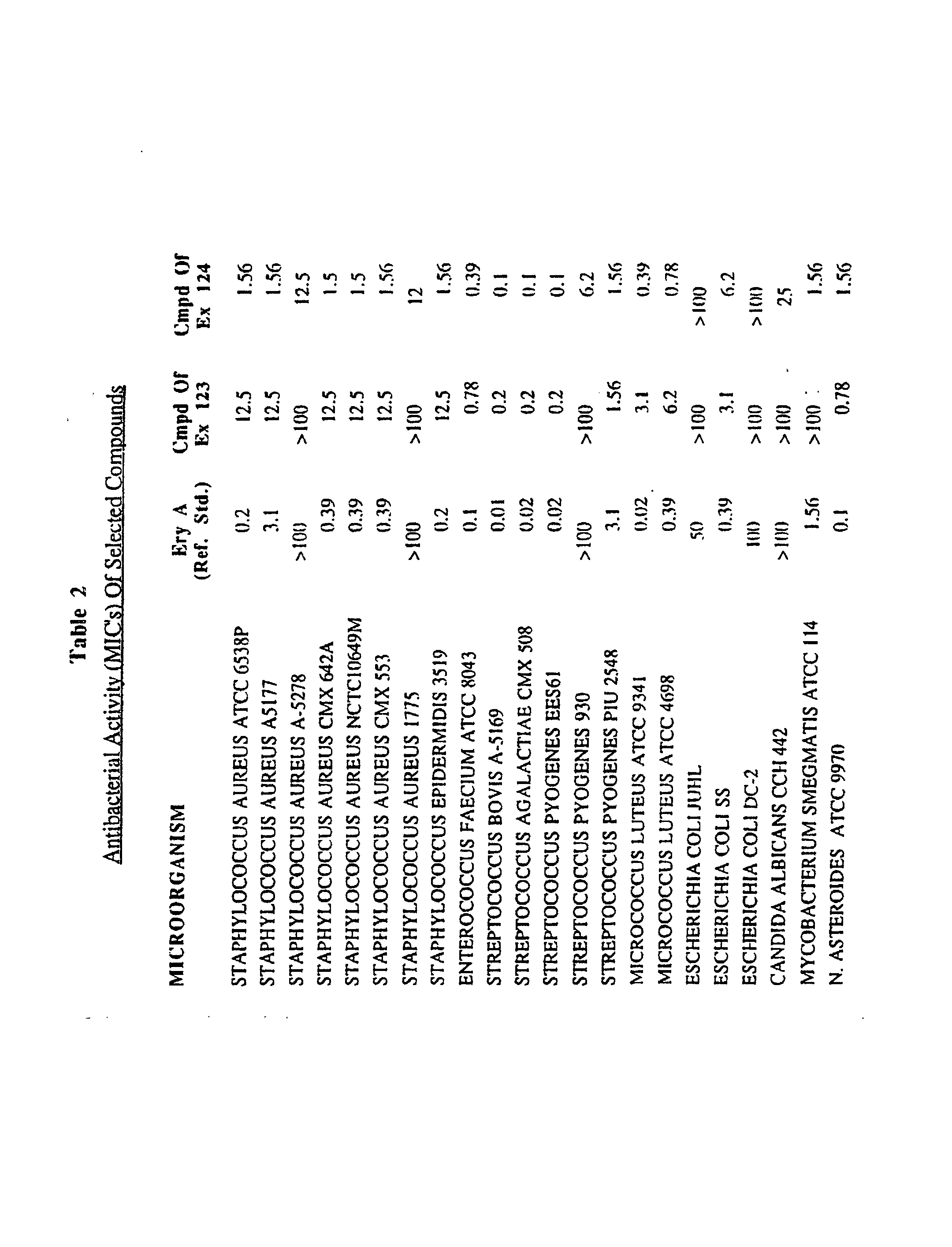

## Claims

1. A compound, or a pharmaceutically acceptable salt or ester thereof, selected from the group consisting of: 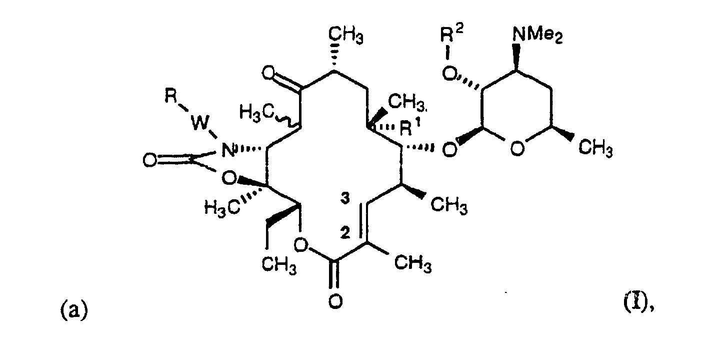 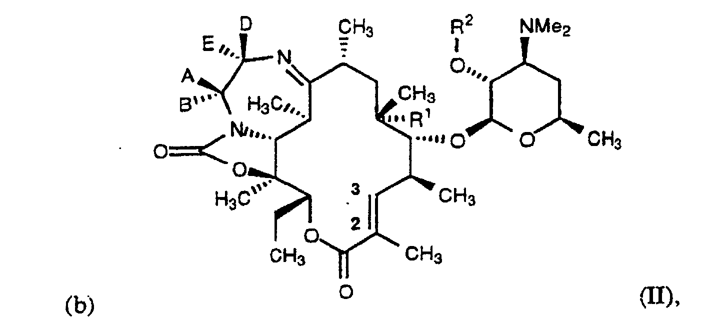 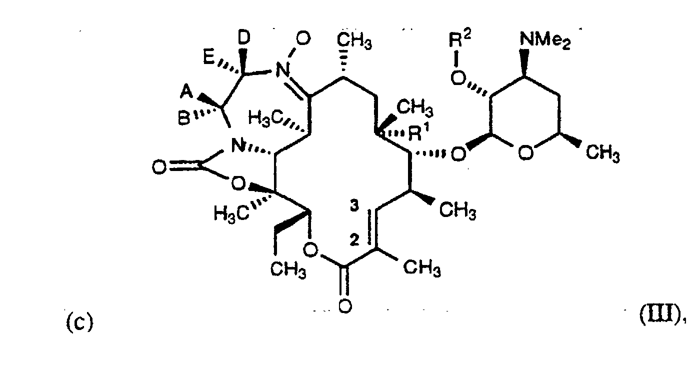 , and 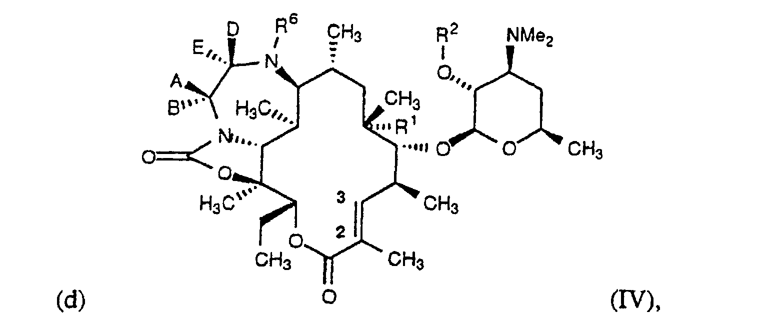 wherein,
R¹ is selected from the group consisting of:
(a) hydrogen;
(b) hydroxy;
(c) O-C₁-C₁₂-alkyl;
(d) O-CO-C₁-C₆-alkyl;
(e) O-CO-NH₂;
(f) O-CO-NH-CO-C₁-C₁₂-alkyl; and
(g) O-CO-NH-SO₂-C₁-C₁₂-alkyl;
R² is hydrogen or a hydroxy protecting group;
R⁶ is hydrogen or C₁-C₆-alkyl;
R is selected from the group consisting of:
(a) hydrogen;
(b) C₁-C₆-alkyl optionally substituted with one or more substituents selected from the group consisting of:
(i) aryl;
(ii) substituted-aryl;
(iii) heteroaryl;
(iv) substituted-heteroaryl;
(v) hydroxy;
(vi) C₁-C₆-alkoxy;
(vii) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
(viii) -CH₂-M-R⁵,
wherein M is selected from the group consisting of:
(aa) -C(O)-NH-;
(bb) -NH-C(O)-;
(cc) -NH-
(dd) -N=;
(ee) -N(CH₃)-
(ff) -O-
(gg) -S(O)ₙ-, wherein n is 0, 1 or 2;
(hh) -CO-O-
(ii) -O-CO-
(jj) -CO-; and
R⁵ is selected from the group consisting of:
(aaa) C₁-C₆-alkyl, optionally substituted with a substituent selected from the group consisting of:
(i) aryl;
(ii) substituted-aryl;
(iii) heteroaryl; and
(iv) substituted-heteroaryl;
(bbb) aryl;
(ccc) substituted-aryl;
(ddd) heteroaryl;
(eee) substituted-heteroaryl; and
(fff) heterocycloalkyl; and
(c) C₃-C₇-cycloalkyl;
(d) aryl;
(e) substituted-aryl;
(f) heteroaryl;
(g) substituted-heteroaryl;
W is absent or selected from the group consisting of -O-, -NH-CO-, -N=CH-, -NH-and -N(C₁-C₆-alkyl)-;
A, B, D and E are independently selected from the group consisting of:
(a) hydrogen;
(b) C₁-C₆-alkyl, optionally substituted with one or more substituents selected from the group consisting of:
(i) aryl;
(ii) substituted-aryl;
(iii) heteroaryl;
(iv) substituted-heteroaryl;
(v) heterocycloalkyl;
(vi) hydroxy;
(vii) C₁-C₆-alkoxy;
(viii) halogen consisting of Br, Cl, F or I; and
(ix) NR³R⁴, where R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, -N(aryl-C₁-C₆-alkyl-)-, -N(substituted-aryl-C₁-C₆-alkyl-)-, -N(heteroaryl-C₁-C₆-alkyl-)-, -N(substituted-heteroaryl-C₁-C₆-alkyl-)-, -S-or -S(O)ₙ-, wherein n is 1 or 2;
(c) C₃-C₇-cycloalkyl;
(d) aryl;
(e) substituted-aryl;
(f) heteroaryl;
(g) substituted-heteroaryl;
(h) heterocycloalkyl; and
(i) a group selected from option (b) above further substituted with -M-R⁵, wherein M and R⁵ are as defined above;
or any one pair of substituents, consisting of AB, AD, AE, BD, BE or DE, is taken together with the atom or atoms to which they are attached to form a 3- to 7-membered ring optionally containing a hetero function consisting of:
-O-,
-NH-,
-N(C₁-C₆-alkyl-)-,
-N(aryl-C₁-C₆-alkyl-)-,
-N(substituted-aryl-C₁-C₆-alkyl-)-,
-N(heteroaryl-C₁-C₆-alkyl-)-,
-N(substituted-heteroaryl-C₁-C₆-alkyl-)-,
-S- or -S(O)ₙ-, wherein n is 1 or 2;
-C(O)-NH-;
-C(O)-NR⁵-, wherein R⁵ is as defined above;
-NH-C(O)-;
-NR⁵-C(O)-, wherein R⁵ is as defined above; and
-C(=NH)-NH-.

2. A compound according to Claim 1 having the formula (I).

3. A compound according to Claim 2, wherein W is absent or -NH-.

4. A compound according to Claim 1 having the formula (II).

5. A compound according to Claim 1 having the formula (III).

6. A compound according to Claim 1 having the formula (IV).

7. A compound according to Claim 1, or a pharmaceutically acceptable salt or ester thereof, which is:
Compound of Formula (I); R¹=H; R²=H; W is absent; R=4-phenylbutyl;
Compound of Formula (I); R¹=methoxy; R²=H; W is absent; R=4-phenylbutyl;
Compound of Formula (I); R¹=methoxy; R²=H; W is absent; R=3-phenoxypropyl;
Compound of Formula (I); R¹=methoxy; R²=H; W is absent; R=2-((phenylmethyl)amino)ethyl;
Compound of Formula (I); R¹=methoxy; R²=H; W is absent; R=3-(N-methyl-N-phenylamino)propyl;
Compound of Formula (I); R¹=methoxy; R²=H; W is absent; R=3-(4-chlorophenoxy)propyl;
Compound of Formula (II); R¹=methoxy; R²=H; A=B=C=D=H;
Compound of Formula (I); R¹=methoxy; R²=H; W is absent; R=3-(1-quinoyloxy)propyl:
Compound of Formula (I); R¹=methoxy; R²=H; W is absent; R=4-(4-chlorophenyl)-3(Z)-butenyl;
Compound of Formula (I); R¹=methoxy; R²=H; W is absent; R=2-phenylethyl;
Compound of Formula (I); R¹=methoxy; R²=H; W is absent; R=2-(3,4-dichlorophenyl)ethyl;
Compound of Formula (I); R¹=methoxy; R²=H; W is absent; R=phenylmethyl;
Compound of Formula (I); R¹=methoxy; R²=H; W is -NH-; R=3-phenylpropyl;
Compound of Formula (I); R¹=methoxy; R²=H; W is absent; R=3-(4-phenoxyphenyl)ethyl;
Compound of Formula (I); R¹=methoxy; R²=H; W is absent; R=3-phenylpropyl;
Compound of Formula (I); R¹=methoxy; R²=H; W is absent; R=2,2-diphenylethyl;
Compound of Formula (I); R¹=methoxy; R²=H; W is absent; R=H;
Compound of Formula (IV); R¹=methoxy; R²=H; A=B=C=D=H; R=H;
Compound of Formula (I); R¹=methoxy; R²=H; W is -NH-; R=H; C10 methyl is *epi*-isomer;
Compound of Formula (I); R¹=methoxy; R²=H; W is -NH-; R=H; C10 methyl is natural isomer;
Compound of Formula (I); R¹=methoxy; R²=H; W is -NH-; R=(4-quinolinyl)propyl; C10 methyl is natural isomer,
Compound of Formula (I); R¹=methoxy; R²=H; W is absent; R=3-(2-naphthyloxy)propyl;
Compound of Formula (I); R¹=methoxy; R²=H; W is absent; R=3-(3-pyridyloxy)propyl;
Compound of Formula (I); R¹=methoxy; R²=H; W is absent; R=3-(2-pyridyloxy)propyl;
Compound of Formula (I); R¹=OH; R²=H; W is absent; R=4-phenylbutyl;
Compound of Formula (I); R¹=OCONH2; R²=H; W is absent; R=4-phenylbutyl;
Compound of Formula (I); R¹=OCONHCO-methyl; R²=H; W is absent; R=4-phenylbutyl;
Compound of Formula (I); R¹=OCONHSO2-methyl; R²=H; W is absent; R=4-phenylbutyl:
Compound of Formula (I); R¹=OMe; R²=H; W is absent; R=phenyl;
Compound of Formula (I); R¹=OMe; R²=H; W is absent; R=3-pyridyl;
Compound of Formula (I); R¹=OMe; R²=H; W is -O-; R=H;
Compound of Formula (I); R¹=OMe; R²=H; W is -O-; R=Me;
Compound of Formula (I); R¹=OMe; R²=H; W is -NH--CO-; R=phenyl;
Compound of Formula (II); R¹=OMe; R²=H; A=benzyl; B,D,E=H;
Compound of Formula (II); R¹=OMe; R²=H; A,D=3,4-pyrrolidinyl; B,E=H;
Compound of Formula (III); R¹=OMe; R²=H; A,B,D,E=H;
Compound of Formula (IV); R¹=OMe; R²=H; A=benzyl; B,D,E=H; R=H;
Compound of Formula (IV); R¹=OMe; R²=H; A,D=3,4-pyrrolidinyl; B,E=H; R=H;
Compound of Formula (IV); R¹=OMe; R²=H; A,B,D,E=H, R=CH₂CH₂CH₂C₆H₅;
Compound of Formula (IV); R¹=OMe; R²=H; A,B,D,E=H, R=2,4-dinitrobenzene;
Compound of Formula (IV); R¹=OMe; R²=H; A,B,D,E=H, R=4-quinolyl;
Compound of Formula (I); R¹=methoxy; R²=H; W is absent; R=(4H-4-oxo-1-quinolyl)propyl;
Compound of Formula (I); R¹=methoxy; R²=H; W is absent; R=2-(4-nitrophenyl)ethyl;
Compound of Formula (I); R¹=methoxy; R²=H; W is absent; R=2-(4-aminophenyl)ethyl:
Compound of Formula (I); R¹=methoxy; R²=H; W is absent; R=3-ethoxypropyl;
Compound of Formula (I); R¹=methoxy; R²=H; W is absent; R=isopropyl;
Compound of Formula (I); R¹=methoxy, R²=H; W is absent; R=2-(4-bromophenyl)ethyl;
Compound of Formula (I); R¹=methoxy; R²=H; W is absent; R=2-(4-hydroxylphenyl)ethyl;
Compound of Formula (I); R¹=methoxy; R²=H; W is absent; R=2-(4-fluorophenyl)ethyl;
Compound of Formula (I); R¹=methoxy; R²=H; W is absent; R=2-(3-methoxyphenyl)ethyl;
Compound of Formula (I); R¹=methoxy; R²=H; W is absent; R=3-vinyloxypropyl;
Compound of Formula (I); R¹=methoxy; R²=H; W is absent; R=2-(3-trifluoromethyl)phenylethyl;
Compound of Formula (I); R¹=methoxy; R²=H; W is absent; R=2-thienylethyl;
Compound of Formula (I); R¹=methoxy; R²=H; W is absent; R=2-(3,4-di-benzyloxyphenyl)ethyl;
Compound of Formula (I); R¹=methoxy; R²=H; W is absent; R=2-(4-methylphenyl)ethyl;
Compound of Formula (I); R¹=methoxy; R²=H; W is absent; R=allyl;
Compound of Formula (I); R¹=methoxy; R²=H; W is absent; R=1,3-dihydroxypropyl;
Compound of Formula (I); R¹=methoxy; R²=H; W is absent; R=1,3-dihydroxypropyl (10-*epi*);
Compound of Formula (I); R¹=methoxy; R²=H; W is absent; R=3-hydroxypropyl;
Compound of Formula (I); R¹=methoxy; R²=H; W is absent; R=3-hydroxypropyl (10-*epi*);
Compound of Formula (I); R¹=methoxy; R²=H; W is absent; R=propyl;
Compound of Formula (I); R¹=methoxy; R²=H; W is absent; R=isobutyl;
Compound of Formula (I); R¹=methoxy; R²=H; W is absent; R=2-(benzoylamino)ethyl;
Compound of Formula (I); R¹=methoxy; R²=H; W is absent; R=3-(benzoylamino)propyl;
Compound of Formula (I); R¹=methoxy; R²=H; W is absent; R=3-(acetylamino)propyl;
Compound of Formula (I); R¹=methoxy; R²=H; W is absent; R=H (10-*epi*);
Compound of Formula (I); R¹=methoxy; R²=H; W is absent; R=3-phenylpropyl (10-*epi*);
Compound of Formula (I); R¹=methoxy; R²=H; W is absent; R=3-(4-phenoxyphenyl)ethyl (10-*epi*);
Compound of Formula (I); R¹=methoxy; R²=H; W is -NH-; R=3-(4-chlorophenyl)propyl;
Compound of Formula (I); R¹=methoxy; R²=H; W is -NH-; R=3-(3-chlorophenyl)propyl;
Compound of Formula (I); R¹=methoxy; R²=H; W is -NH-; R=3-(2-chlorophenyl)propyl;
Compound of Formula (I); R¹=methoxy; R²=H; W is -NH-; R=3-(2,4-dichlorophenyl)propyl;
Compound of Formula (I); R¹=methoxy; R²=H; W is -NH-; R=3-(4-hydroxyphenyl)propyl;
Compound of Formula (I); R¹=methoxy; R²=H; W is -NH-; R=3-(3-hydroxyphenyl)propyl;
Compound of Formula (I); R¹=methoxy; R²=H; W is -NH-; R=3-(2-hydroxyphenyl)propyl;
Compound of Formula (I); R¹=methoxy; R²=H; W is -NH-; R=3-(4-methoxyphenyl)propyl;
Compound of Formula (I); R¹=methoxy; R²=H; W is -NH-; R=3-(4-nitrophenyl)propyl;
Compound of Formula (I); R¹=methoxy; R²=H; W is -NH-; R=3-(3-nitrophenyl)propyl;
Compound of Formula (I); R¹=methoxy; R²=H; W is -NH-; R=3-(2-nitrophenyl)propyl;
Compound of Formula (I); R¹=methoxy; R²=H; W is -NH-; R=3-((4-(acetylamino)phenyl)propyl;
Compound of Formula (I); R¹=methoxy; R²=H; W is -NH-; R=*trans*-3-phenylprop-2-enyl;
Compound of Formula (I); R¹=methoxy; R²=H; W is -NH-; R=2-phenylethyl;
Compound of Formula (I); R¹=methoxy; R²=H; W is -NH-; R=phenylmethyl;
Compound of Formula (I); R¹=methoxy; R²=H; W is -NH-; R=(3-indolyl)methyl;
Compound of Formula (I); R¹=methoxy; R²=H; W is -NH-; R=(4-methoxyphenyl)methyl;
Compound of Formula (I); R¹=methoxy; R²=H; W is -NH-; R=(4-acetylaminophenyl)methyl;
Compound of Formula (I); R¹=methoxy; R²=H; W is -NH-; R=(4-chlorophenyl)methyl;
Compound of Formula (I); R¹=methoxy; R²=H; W is -NH-; R=(4-dimethylaminophenyl)methyl;
Compound of Formula (I); R¹=methoxy; R²=H; W is -NH-; R=*trans*-3-(4-nitrophenyl)prop-2-enyl;
Compound of Formula (I): R¹=methoxy; R²=H; W is -NH-; R=(4-nitrophenyl)methyl;
Compound of Formula (I); R¹=methoxy; R²=H; W is -NH-; R=(3,4-dihydroxyphenyl)methyl;
Compound of Formula (I); R¹=methoxy; R²=H; W is -NH-; R=(2,5-dihydroxyphenyl)methyl;
Compound of Formula (I); R¹=methoxy; R²=H; W is -NH-; R=(2-hydroxy-5-nitrophenyl)methyl;
Compound of Formula (I); R¹=methoxy; R²=H; W is -NH-; R=(4-hydroxymethylphenyl)methyl;
Compound of Formula (I); R¹=methoxy; R²=H; W is -NH-; R=*trans*-3-(5-nitro-2-furanyl)prop-2-enyl;
R¹R²
Compound of Formula (I); R¹=methoxy; R²=H; W is -NH-; R=(4-hydroxyphenyl)methyl;
Compound of Formula (I); R¹=methoxy; R²=H; W is -NH-; R=(3-hydroxyphenyl)methyl;
Compound of Formula (I); R¹=methoxy; R²=H; W is -NH-; R=(2-hydroxyphenyl)methyl;
Compound of Formula (I); R¹=methoxy; R²=H; W is -NH-; R=(4-trifluoromethylphenyl)methyl;
Compound of Formula (I); R¹=methoxy; R²=H; W is -NH-; R=(4-cyanophenyl)methyl;
Compound of Formula (I); R¹=methoxy; R²=H; W is -NH-; R=(2-pyridyl)methyl;
Compound of Formula (I); R¹=methoxy; R²=H; W is -NH-; R=(3-pyridyl)methyl;
Compound of Formula (I); R¹=methoxy; R²=H; W is -NH-; R=(4-pyridyl)methyl;
Compound of Formula (I); R¹=methoxy; R²=H; W is -NH-; R=(2-hydroxy-1-naphthyl)methyl;
Compound of Formula (I); R¹=methoxy; R²=H; W is -NH-; R=(4-dimethylamino-1-naphthyl)methyl;
Compound of Formula (I); R¹=methoxy; R²=H; W is -NH-; R=(4-(methylthio)phenyl)methyl;
Compound of Formula (I); R¹=methoxy; R²=H; W is -NH-; R=(4-phenoxyphenyl)methyl;
Compound of Formula (I); R¹=methoxy; R²=H; W is -NH-; R=3-(4-fluorophenyl)propyl;
Compound of Formula (I); R¹=methoxy; R²=H; W is -NH-; R=(*trans*-3-(4-nitrophenyl)prop-2-enyl;
Compound of Formula (I); R¹=methoxy; R²=H; W is -NH-; R=(4-amino-phenyl)methyl;
Compound of Formula (I); R¹=methoxy; R²=H; W is -NH-; R=3-(4-amino-phenyl)propyl;
Compound of Formula (I); R¹=methoxy; R²=H; W is -NH-; R=3-(3-amino-phenyl)propyl;
Compound of Formula (I); R¹=methoxy; R²=H; W is -NH-; R=3-(2-amino-phenyl)propyl;
Compound of Formula (1); R¹=methoxy; R²=H; W is -NH-; R=*trans*-3-(4-acetylaminophenyl)prop-2-enyl;
Compound of Formula (I); R¹=methoxy; R²=H; W is -NH-; R=*trans*-3-(4-(4-nitrobenzoylamino)phenyl)prop-2-enyl;
Compound of Formula (I); R¹=methoxy; R²=H; W is -NH-; R=3-(2-benztriazolyl)propyl;
Compound of Formula (I); R¹=methoxy; R²=H; W is -NH-; R=3-(1-benztriazolyl)propyl;
Compound of Formula (I); R¹=methoxy; R²=H; W is -NH-; R=3-(4-phenylimidazolyl)propyl;
Compound of Formula (I); R¹=methoxy; R²=H; W is -NH-; R=3-(1-anhydro-1-cladinosyl)propyl;
Compound of Formula (I); R¹=methoxy; R²=H; W is -NH-; R=3-phenylpropyl (10-*epi*);
Compound of Formula (I); R¹=methoxy; R²=H; W is -NH-; R=isopropyl;
Compound of Formula (I); R¹=methoxy; R²=H; W is -NH-; R=1,3-diphenyl-2-propyl; or
Compound of Formula (I); R¹=methoxy; R²=H; W is -NH-; R=3-pentyl.

8. A process for the preparation of a compound having the Formula (I): 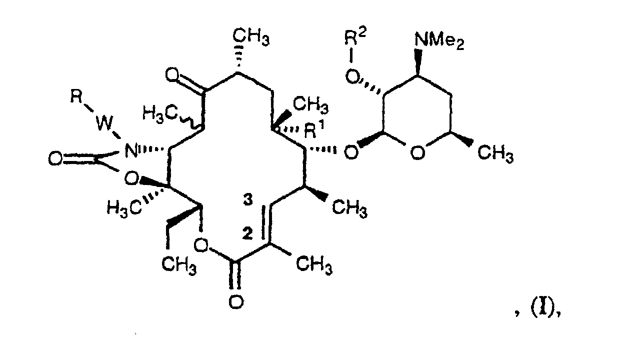 wherein
R¹ is selected from the group consisting of:
(a) hydrogen;
(b) hydroxy;
(c) O-C₁-C₁₂-alkyl;
(d) O-CO-C₁-C₆-alkyl;
(e) O-CO-NH₂;
(f) O-CO-NH-CO-C₁-C₁₂-alkyl; and
(g) O-CO-NH-SO₂-C₁-C₁₂-alkyl;
R² is hydrogen or a hydroxy protecting group;
R is selected from the group consisting of:
(a) hydrogen;
(b) C₁-C₆-alkyl optionally substituted with one or more substituents selected from the group consisting of:
(i) aryl;
(ii) substituted-aryl;
(iii) heteroaryl;
(iv) substituted-heteroaryl;
(v) hydroxy;
(vi) C₁-C₆-alkoxy;
(vii) NR³R⁴, where R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, -N(aryl-C₁-C₆-alkyl-)-, -N(substituted-aryl-C₁-C₆-alkyl-)-, -N(heteroaryl-C₁-C₆-alkyl-)-, -N(substituted-heteroaryl-C₁-C₆-alkyl-)-, -S-or -S(O)ₙ-, wherein n is 1 or 2;
(viii) -CH₂-M-R⁵,
wherein M is selected from the group consisting of:
(aa) -C(O)-NH-;
(bb) -NH-C(O)-;
(cc) -NH-
(dd) -N=;
(ee) -N(CH₃)-
(ff) -O-
(gg) -S(O)ₙ-, wherein n is 0, 1 or 2;
(hh) -CO-O-
(ii) -O-CO-
(jj) -CO- ; and
R⁵ is selected from the group consisting of:
(aaa) C₁-C₆-alkyl, optionally substituted with a substituent selected from the group consisting of:
(i) aryl;
(ii) substituted-aryl;
(iii) heteroaryl; and
(iv) substituted-heteroaryl;
(bbb) aryl;
(ccc) substituted-aryl;
(ddd) heteroaryl;
(eee) substituted-heteroaryl; and
(fff) heterocycloalkyl; and
(c) C₃-C₇-cycloalkyl;
(d) aryl;
(e) substituted-aryl;
(f) heteroaryl;
(g) substituted-heteroaryl; and
W is absent;
the method comprising;
(a) treating a compound having the formula: 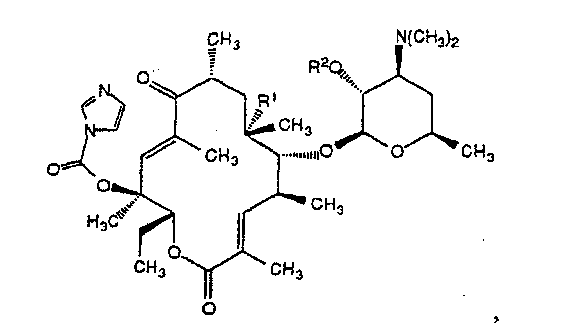
wherein R¹ is selected from the group consisting of hydrogen, protected hydroxy, O-CO-C₁-C₆-alkyl, O-C₁-C₁₂-alkyl, O-CO-NH₂, O-CO-NH-CO-C₁-C₁₂-alkyl, and O-CO-NH-SO₂-C₁-C₁₂-alkyl; and R² is a hydroxy protecting group, with a primary amine RNH₂, wherein R is as defined above, in a suitable organic solvent at room temperature to reflux temperature for about 4 to about 48 hours, extracting, optionally deprotecting, and isolating the desired compound.

9. A process according to Claim 9 wherein R is hydrogen, C₁-C₆-alkyl, C₃-C₇-cycloalkyl, aryl, substituted-aryl, heteroaryl or substituted-heteroaryl, and the solvent is selected from the group consisting of methylene chloride, tetrahydrofuran, N-methyl-pyrrolidinone, diethyl ether, bis-methoxymethyl ether, dimethyl formamide, acetonitrile, acetone and aqueous mixtures thereof.

10. A process for the preparation of a compound having the Formula (I): 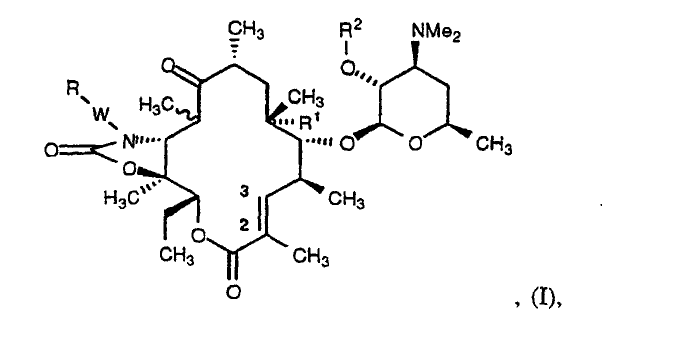 wherein
R¹ is selected from the group consisting of:
(a) hydrogen;
(b) hydroxy;
(c) O-C₁-C₁₂-alkyl;
(d) O-CO-C₁-C₆-alkyl;
(e) O-CO-NH₂;
(f) O-CO-NH-CO-C₁-C₁₂-alkyl; and
(g) O-CO-NH-SO₂-C₁-C₁₂-alkyl;
R² is hydrogen or a hydroxy protecting group;
R is selected from the group consisting of:
(a) hydrogen;
(b) C₁-C₆-alkyl optionally substituted with one or more substituents selected from the group consisting of:
(i) aryl;
(ii) substituted-aryl;
(iii) heteroaryl;
(iv) substituted-heteroaryl;
(v) hydroxy;
(vi) C₁-C₆-alkoxy;
(vii) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
(viii) -CH₂-M-R⁵,
wherein M is selected from the group consisting of:
(aa) -C(O)-NH-;
(bb) -NH-C(O)-;
(cc) -NH-
(dd) -N=;
(ee) -N(CH₃)-
(ff) -O-
(gg) -S(O)ₙ-, wherein n is 0, 1 or 2;
(hh) -CO-O-
(ii) -O-CO-
(jj) -CO- ; and
R⁵ is selected from the group consisting of:
(aaa) C₁-C₆-alkyl, optionally substituted with a substituent selected from the group consisting of:
(i) aryl;
(ii) substituted-aryl;
(iii) heteroaryl; and
(iv) substituted-heteroaryl;
(bbb) aryl;
(ccc) substituted-aryl;
(ddd) heteroaryl;
(eee) substituted-heteroaryl; and
(fff) heterocycloalkyl; and
(c) C₃-C₇-cycloalkyl;
(d) aryl;
(e) substituted-aryl;
(f) heteroaryl;
(g) substituted-heteroaryl; and
W is selected from the group consisting of -NH-CO-, -N=CH-, -NH- and -N(C₁-C₆-alkyl)-;
the method comprising:
(a) treating a compound having the formula: 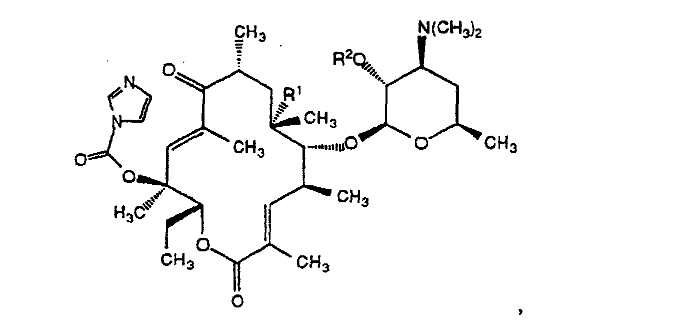 wherein R¹ is selected from the group consisting of hydrogen, protected hydroxy, O-CO-C₁-C₆-alkyl, O-C₁-C₁₂-alkyl, O-CO-NH₂, O-CO-NH-CO-C₁-C₁₂-alkyl, or O-CO-NH-SO₂-C₁-C₁₂-alkyl; and R² is a hydroxy protecting group, with a reagent selected from the group consisting of hydrazine, a substituted hydrazine, hydroxylamine and *O*-substituted hydroxylamine in a suitable organic solvent at room temperature to reflux for about 4 to about 48 hours to afford the desired compound;
(b) optionally acylating the compound of Formula (I) obtained in step (a) wherein W is -NH- and R is H with an acylating agent to afford a compound of Formula (I) wherein W is -NH-CO-;
(c) optionally condensing the compound of Formula (I) obtained in step (a) wherein W is -NH- and R is H with an aldehyde to afford a compound of Formula (I) wherein W is -N=CH-;
(d) optionally reducing the compound of Formula (I) obtained in step (c) wherein W is -N=CH- with a reducing agent to afford a compound of Formula (I) wherein W is -NH-;
(e) and extracting, optionally deprotecting, and isolating the desired compound.

11. A process according to Claim 10 wherein the solvent is selected from the group consisting of methanol, ethanol, propanol, isopropanol, butanol, t-butanol, methylene chloride, tetrahydrofuran, N-methyl-pyrrolidinone, diethyl ether, bis-methoxymethyl ether, dimethyl formamide, acetone, aqueous acetonitrile, aqueous DMF, and aqueous acetone.

12. A process according to Claim 10 wherein the product is a compound of Formula (I) wherein W is -NH- and R is H, and the hydrazine reagent is hydrazine.

13. A process according to Claim 10 wherein the product is a compound of Formula (I) wherein W is -N(C₁-C₆-alkyl)-, the hydrazine reagent is a substituted hydrazine RR⁴NNH₂, wherein R is as defined for Formula (I) and R⁴ is C₁-C₆-alkyl.

14. A process according to Claim 10 wherein the product is a compound of Formula (I) wherein W is -NH-CO-, the hydrazine reagent is hydrazine, and the product obtained in step (a) having the Formula (I) wherein W is -NH- and R is H is treated with an R-acyl acylating agent, wherein R is as defined for Formula (I).

15. A process according to Claim 10 wherein the acylating agent is selected from the group consisting of an acid chloride, an acid fluoride, an acid anhydride, a carboxylic acid in the presence of carbonyldiimidazole, and a carboxylic acid in the presence of 1-(3-dimethylaminopropyl)-3-ethylcarbodiimide hydrochloride.

16. A process according to Claim 10 wherein the product is a compound of Formula (I) wherein W is -N=CH-, the hydrazine reagent is hydrazine, and the product obtained in step (a) having the Formula (I) wherein W is -NH- and R is H is treated with an aldehyde having the formula R-CHO, wherein R is as defined for Formula (I).

17. A process according to Claim 10 wherein the product is a compound of Formula (I) wherein W is -NH- and R is not H, the hydrazine reagent is hydrazine, the product obtained in step (a) having the Formula (I) wherein W is -NH- and R is H is treated with an aldehyde having the formula R-CHO, wherein R is as defined for Formula (I), and the product obtained in step (c) having the Formula (I) wherein W is -N=CH- is treated with a reducing agent.

18. A process according to Claim 17 wherein the reducing agent is selected from the group consisting of sodium cyanoborohydride, sodium borohydride, sodium triacetoxyborohydride, borane-tetrahydrofuran complex, and borane-piperidine complex.

19. A process for the preparation of a compound having the Formula (I): 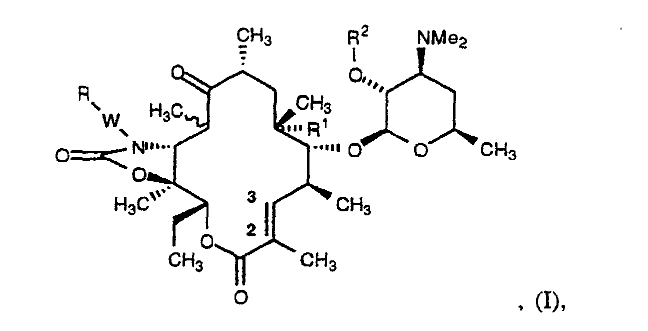 wherein
R¹ is selected from the group consisting of:
(a) hydrogen;
(b) hydroxy;
(c) O-C₁-C₁₂-alkyl;
(d) O-CO-C₁-C₆-alkyl;
(e) O-CO-NH₂;
(f) O-CO-NH-CO-C₁-C₁₂-alkyl; and
(g) O-CO-NH-SO₂-C₁-C₁₂-alkyl;
R² is hydrogen or a hydroxy protecting group;
R is selected from the group consisting of:
(a) hydrogen;
(b) C₁-C₆-alkyl optionally substituted with one or more substituents selected from the group consisting of:
(i) aryl;
(ii) substituted-aryl;
(iii) heteroaryl;
(iv) substituted-heteroaryl;
(v) hydroxy;
(vi) C₁-C₆-alkoxy;
(vii) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
(viii) -CH₂-M-R⁵,
wherein M is selected from the group consisting of:
(aa) -C(O)-NH-;
(bb) -NH-C(O)-;
(cc) -NH-
(dd) -N=;
(ee) -N(CH₃)-
(ff) -O-
(gg) -S(O)ₙ-, wherein n is 0, 1 or 2;
(hh) -CO-O-
(ii) -O-CO-
(jj) -CO- ; and
R⁵ is selected from the group consisting of:
(aaa) C₁-C₆-alkyl, optionally substituted with a substituent selected from the group consisting of:
(i) aryl;
(ii) substituted-aryl;
(iii) heteroaryl; and
(iv) substituted-heteroaryl;
(bbb) aryl;
(ccc) substituted-aryl;
(ddd) heteroaryl;
(eee) substituted-heteroaryl; and
(fff) heterocycloalkyl; and
(c) C₃-C₇-cycloalkyl;
(d) aryl;
(e) substituted-aryl;
(f) heteroaryl;
(g) substituted-heteroaryl; and
W is -O-;
the method comprising:
(a) treating a compound having the formula: 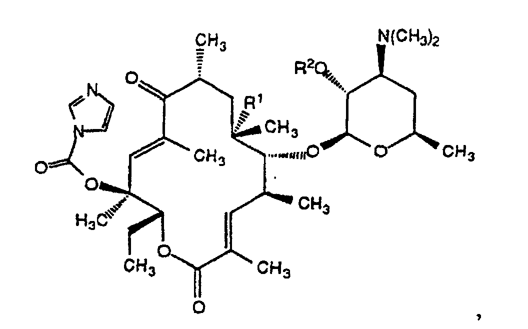 wherein R¹ is selected from the group consisting of hydrogen, protected hydroxy, O-C₁-C₁₂-alkyl, O-CO-C₁-C₆-alkyl, O-CO-NH₂, O-CO-NH-CO-C₁-C₁₂-alkyl, or O-CO-NH-SO₂-C₁-C₁₂-alkyl; and R² is a hydroxy protecting group, with a hydroxylamine reagent selected from the group consisting of unsubstituted hydroxylamine and an *O*-C₁-C₆-alkylated hydroxylamine in a suitable organic solvent at room temperature to reflux for about 4 to about 48 hours, to give the desired compound;
(b) optionally treating the product obtained in step (a) having the Formula (I) wherein W is -O- and R is H with base and an appropriate electrophile having the formula R-L, wherein R is selected from the group consisting of C₁-C₆-alkyl, C₃-C₇-cycloalkyl, aryl, substituted-aryl, heteroaryl and a substituted-heteroaryl group, as defined for compounds of Formula (I) above, and L is suitable leaving group, to give the desired compound of formula (I) wherein W is -O- and R is selected from the group consisting of C₁-C₆-alkyl, C₃-C₇-cycloalkyl, aryl, substituted-aryl, heteroaryl and a substituted-heteroaryl group; and
(c) extracting, optionally deprotecting, and isolating the desired compound.

20. A process according to Claim 19 wherein the solvent is selected from the group consisting of methanol, ethanol, propanol, isopropanol, butanol, t-butanol, methylene chloride, tetrahydrofuran, N-methyl-pyrrolidinone, diethyl ether, bis-methoxymethyl ether, dimethyl formamide, acetone, aqueous acetonitrile, aqueous DMF, and aqueous acetone.

21. A process according to Claim 20 wherein the product is a compound of Formula (I) wherein W is -O- and R is H and the hydroxylamine reagent is unsubstituted hydroxylamine.

22. A process according to Claim 20 wherein the product is a compound of Formula (I) wherein W is -O- and R is *O*-C₁-C₆-alkyl and the hydroxylamine reagent is an *O*-C₁-C₆-alkylated hydroxylamine.

23. A process according to Claim 20 wherein the final product is a compound of Formula (I) wherein W is -O- and R is selected from the group consisting of C₁-C₆-alkyl, C₃-C₇-cycloalkyl, aryl, substituted-aryl, heteroaryl or a substituted-heteroaryl group, and the product obtained in step (a) having the Formula (I) wherein W is -O- and R is H is treated with a suitable base and an electrophile having the formula R-L, wherein R is as defined above and L is a suitable leaving group.

24. A process according to Claim 22 wherein in the base is selected from the group consisting of sodium hydride, potassium hydride, lithium hydride, lithium diethylamide, and butyllithium, and L is selected from the group consisting of halide, methanesulfonyl and p-toluenesulfonyl.

25. A process for the preparation of a compound having the Formula (II): 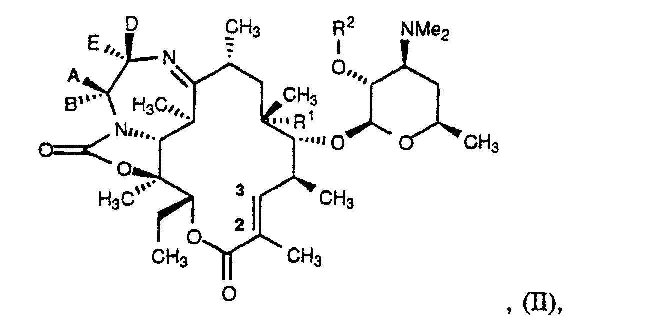 wherein
R¹ is selected from the group consisting of:
(a) hydrogen;
(b) hydroxy;
(c) O-C₁-C₁₂-alkyl;
(d) O-CO-C₁-C₆-alkyl;
(e) O-CO-NH₂;
(f) O-CO-NH-CO-C₁-C₁₂-alkyl; and
(g) O-CO-NH-SO₂-C₁-C₁₂-alkyl;
R² is hydrogen or a hydroxy protecting group;
A, B, D and E are independently selected from the group consisting of:
(a) hydrogen;
(b) C₁-C₆-alkyl, optionally substituted with one or more substituents selected from the group consisting of:
(i) aryl;
(ii) substituted-aryl;
(iii) heteroaryl;
(iv) substituted-heteroaryl;
(v) heterocycloalkyl;
(vi) hydroxy;
(vii) C₁-C₆-alkoxy;
(viii) halogen consisting of Br, Cl, F or I; and
(ix) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
(c) C₃-C₇-cycloalkyl;
(d) aryl;
(e) substituted-aryl;
(f) heteroaryl;
(g) substituted-heteroaryl;
(h) heterocycloalkyl;
and
(i) a group selected from option (b) above further substituted with -M-
R⁵, wherein M is selected from the group consisting of:
(aa) -C(O)-NH-;
(bb) -NH-C(O)-;
(cc) -NH-
(dd) -N(CH₃)-
(ee) -O-
(ff) -S(O)ₙ-, wherein n is 0, 1 or 2;
(gg) -C(=NH)-NH-;
(hh) -CO-O-
(ii) -O-CO-
(jj) -CO- ;
and R⁵ is selected from the group consisting of:
(aaa) C₁-C₆-alkyl, optionally substituted with a substituent selected from the group consisting of:
(i) aryl;
(ii) substituted-aryl;
(iii) heteroaryl; and
(iv) substituted-heteroaryl;
(bbb) aryl;
(ccc) substituted-aryl;
(ddd) heteroaryl;
(eee) substituted-heteroaryl; and
(fff) heterocycloalkyl;
or any one pair of substituents, consisting of AB, AD, AE, BD, BE or DE, is taken together with the atom or atoms to which they are attached to form a 3- to 7-membered ring optionally containing a hetero function consisting of:
-O-,
-NH-,
-N(C₁-C₆-alkyl-)-,
-N(aryl-C₁-C₆-alkyl-)-,
-N(substituted-aryl-C₁-C₆-alkyl-)-,
-N(heteroaryl-C₁-C₆-alkyl-)-,
-N(substituted-heteroaryl-C₁-C₆-alkyl-)-,
-S- or -S(O)ₙ-, wherein n is 1 or 2;
-C(O)-NH-;
-C(O)-NR⁵-, wherein R⁵ is as defined above;
-NH-C(O)-;
-NR⁵-C(O)-, wherein R⁵ is as defined above; and
-C(=NH)-NH-;
the method comprising:
(a) treating a compound having the formula: 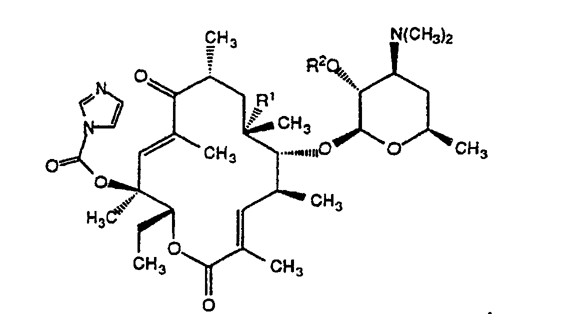 wherein R¹ is selected from the group consisting of hydrogen, protected hydroxy, O-C₁-C₁₂-alkyl, O-CO-C₁-C₆-alkyl, O-CO-NH₂, O-CO-NH-CO-C₁-C₁₂-alkyl, or O-CO-NH-SO₂-C₁-C₁₂-alkyl; and R² is a hydroxy protecting group, with a compound having the formula: 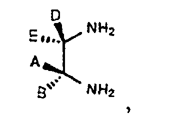 wherein A, B, D, and E are as defined for compounds of Formula (I) above, in a suitable solvent at room temperature to reflux temperature for about 4 to about 48 hours to give the bicyclic intermediate compound having the formula: 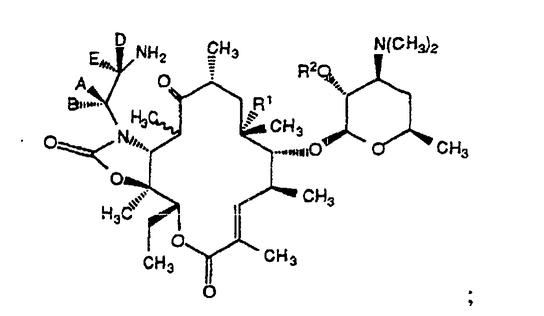
(b) deprotecting said bicyclic intermediate compounds to give the second intermediate compounds having the formula: 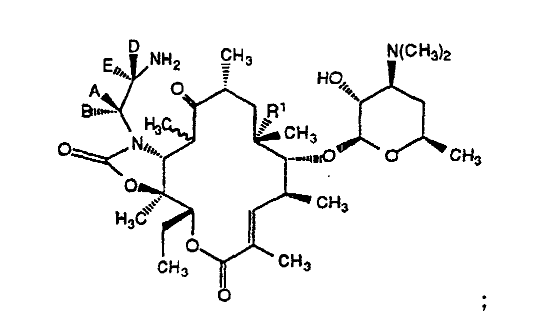
(c) cyclizing said second intermediate compounds by treatment with dilute concentration of a strong acid in a suitable organic solvent for a period of from about 4 hours to about 10 days at a temperature from ambient to reflux temperature of the solvent to give the desired compounds; and
(d) extracting, optionally deprotecting, and isolating the desired compound.

26. A process according to Claim 26 wherein in step (a) the solvent is selected from the group consisting of methanol, ethanol, propanol, isopropanol, butanol, t-butanol, methylene chloride, tetrahydrofuran, N-methyl-pyrrolidinone, diethyl ether, bis-methoxymethyl ether, dimethyl formamide, acetone, aqueous acetonitrile, aqueous DMF, and aqueous acetone; and in step (c) the solvent is selected the group consisting of methanol, ethanol, propanol, *iso*-propanol, butanol, *iso*-butanol and *t*-butanol.

27. A process for the preparation of a compound having the Formula (II): 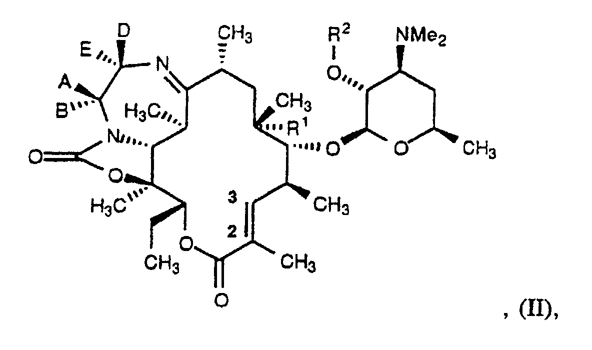 wherein
R¹ is selected from the group consisting of:
(a) hydrogen;
(b) hydroxy;
(c) O-C₁-C₁₂-alkyl;
(d) O-CO-C₁-C₆-alkyl;
(e) O-CO-NH₂;
(f) O-CO-NH-CO-C₁-C₁₂-alkyl; and
(g) O-CO-NH-SO₂-C₁-C₁₂-alkyl;
R² is hydrogen or a hydroxy protecting group;
A, B, D and E are independently selected from the group consisting of:
(a) hydrogen;
(b) C₁-C₆-alkyl, optionally substituted with one or more substituents selected from the group consisting of:
(i) aryl;
(ii) substituted-aryl;
(iii) heteroaryl;
(iv) substituted-heteroaryl;
(v) heterocycloalkyl;
(vi) hydroxy;
(viij C₁-C₆-alkoxy;
(viii) halogen consisting of Br, Cl, F or I: and
(ix) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
(c) C₃-C₇-cycloalkyl;
(d) aryl;
(e) substituted-aryl;
(f) heteroaryl;
(g) substituted-heteroaryl;
(h) heterocycloalkyl;
and
(i) a group selected from option (b) above further substituted with -M-
R⁵, wherein M is selected from the group consisting of:
(aa) -C(O)-NH-;
(bb) -NH-C(O)-;
(cc) -NH-
(dd) -N(CH₃)-
(ee) -O-
(ff) -S(O)ₙ-, wherein n is 0, 1 or 2;
(gg) -C(=NH)-NH-;
(hh) -CO-O-
(ii) -O-CO-
(jj) -CO-;
and R⁵ is selected from the group consisting of:
(aaa) C₁-C₆-alkyl, optionally substituted with a substituent selected from the group consisting of:
(i) aryl;
(ii) substituted-aryl;
(iii) heteroaryl; and
(iv) substituted-heteroaryl;
(bbb) aryl;
(ccc) substituted-aryl;
(ddd) heteroaryl;
(eee) substituted-heteroaryl; and
(fff) heterocycloalkyl;
or any one pair of substituents, consisting of AB, AD, AE, BD, BE or DE, is taken together with the atom or atoms to which they are attached to form a 3- to 7-membered ring optionally containing a hetero function consisting of:
-O-,
-NH-,
-N(C₁-C₆-alkyl-)-,
-N(aryl-C₁-C₆-alkyl-)-,
-N(substituted-aryl-C₁-C₆-alkyl-)-,
-N(heteroaryl-C₁-C₆-alkyl-)-,
-N(substituted-heteroaryl-C₁-C₆-alkyl-)-,
-S- or -S(O)ₙ-, wherein n is 1 or 2;
-C(O)-NH-;
-C(O)-NR⁵-, wherein R⁵ is as defined above;
-NH-C(O)-;
-NR⁵-C(O)-, wherein R⁵ is as defined above; and
-C(=NH)-NH-;
the method comprising:
(a) treating a compound having the formula: 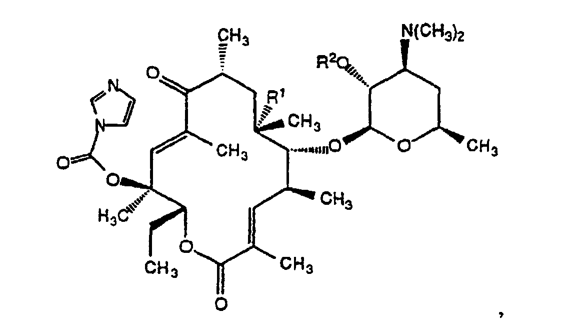 wherein R¹ is selected from the group consisting of hydrogen, protected hydroxy, O-C₁-C₁₂-alkyl, O-CO-C₁-C₆-alkyl, O-CO-NH₂, O-CO-NH-CO-C₁-C₁₂-alkyl, or O-CO-NH-SO₂-C₁-C₁₂-alkyl; and R² is a hydroxy protecting group, with a compound having the formula: 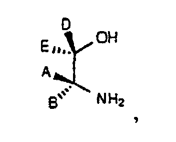 wherein A, B, D, and E are as defined above, in a suitable solvent at 0 - 70 °C for about 4 to about 48 hours to give a bicyclic intermediate compound having the formula: 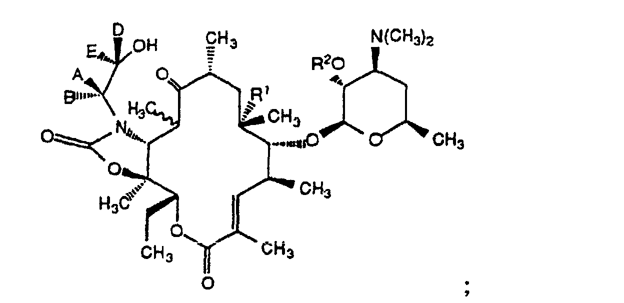
(b) treating the bicyclic intermediate compound from step (a) with triphenylphosphine and diphenylphosphoryl azide-diethylazodicarboxylate in tetrahydrofuran under Mitsunobu reaction conditions to prepare the second intermediate azide compound having the formula: 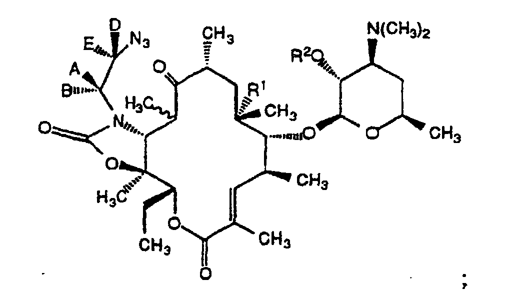
(c) reducing the second intermediate azide compound to prepare the third intermediate compound having the formula: 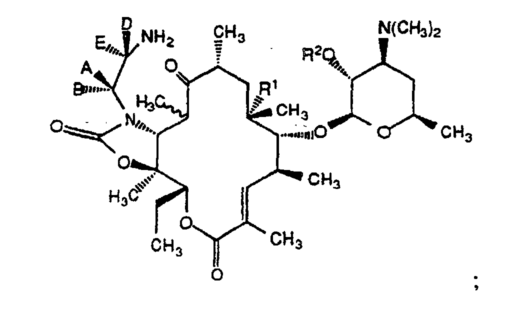
(d) cyclizing said third intermediate compound by treatment with a dilute concentration of a strong acid at ambient temperature to reflux temperature for about 4 hours to about 10 days in a aqueous alcohol solvent to give the desired compounds; and
(e) extracting, optionally deprotecting, and isolating the desired compound.

28. A process as in Claim 27 wherein in step (a) the solvent is selected from the group consisting of methanol, ethanol, propanol, isopropanol, butanol, t-butanol, methylene chloride, tetrahydrofuran, N-methyl-pyrrolidinone, diethyl ether, bis-methoxymethyl ether, dimethyl formamide, acetone, aqueous acetonitrile, aqueous DMF and aqueous acetone; in step (c) the reducing agent is selected from the group consisting of triphenylphosphine-water, hydrogen with a catalyst, sodium borohydride, and dialkylaluminum hydride; and in step (d) the solvent is selected the group consisting of methanol, ethanol, propanol, *iso*-propanol, butanol, *iso*-butanol and *t*-butanol.

29. A process as in Claim 27 wherein step (b) thereof is replaced with two steps consisting of:
(b') reacting the hydroxy group of the bicyclic intermediate compound with a sulfonating agent selected from the group consisting of sulfonyl chloride, alkyl sulfonic anhydride, aryl sulfonic anhydride, and trifluoromethanesulfonic anhydride, in an aprotic solvent at -78°C to room temperature to give an intermediate compound wherein the hydroxyl group has been replaced with a sulfonate ester moiety; and
(b") reacting the sulfonate ester of step (b') with an alkali metal azide in an aprotic solvent at from about 0°C to about 100°C to give the second intermediate azide compound.

30. A process for the preparation of a compound having the Formula (III): 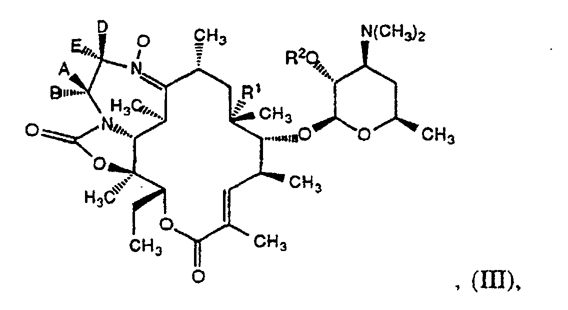 wherein
A, B, D and E are independently selected from the group consisting of:
(a) hydrogen;
(b) C₁-C₆-alkyl, optionally substituted with one or more substituents selected from the group consisting of:
(i) aryl;
(ii) substituted-aryl;
(iii) heteroaryl;
(iv) substituted-heteroaryl;
(v) heterocycloalkyl;
(vi) hydroxy;
(vii) C₁-C₆-alkoxy;
(viii) halogen consisting of Br, Cl, F or I; and
(ix) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
(c) C₃-C₇-cycloalkyl;
(d) aryl;
(e) substituted-aryl;
(f) heteroaryl;
(g) substituted-heteroaryl;
(h) heterocycloalkyl;
and
(i) a group selected from option (b) above further substituted with -M-R⁵, wherein M is selected from the group consisting of
(aa) -C(O)-NH-;
(bb) -NH-C(O)-;
(cc) -NH-
(dd) -N(CH₃)-
(ee) -O-
(ff) -S(O)ₙ-, wherein n is 0, 1 or 2; and
(gg) -C(=NH)-NH-;
and R⁵ is selected from the group consisting of:
(aaa) C₁-C₆-alkyl, optionally substituted with a substituent selected from the group consisting of:
(i) aryl;
(ii) substituted-aryl;
(iii) heteroaryl; and
(iv) substituted-heteroaryl;
(bbb) aryl;
(ccc) substituted-aryl;
(ddd) heteroaryl;
(eee) substituted-heteroaryl; and
(fff) heterocycloalkyl;
or any one pair of substituents, consisting of AB, AD, AE, BD, BE or DE, is taken together with the atom or atoms to which they are attached to form a 3- to 7-membered ring optionally containing a hetero function consisting of
-O-,
-NH-,
-N(C₁-C₆-alkyl-)-,
-N(aryl-C₁-C₆-alkyl-)-,
-N(substituted-aryl-C₁-C₆-alkyl-)-,
-N(heteroaryl-C₁-C₆-alkyl-)-,
-N(substituted-heteroaryl-C₁-C₆-alkyl-)-,
-S- or -S(O)ₙ-, wherein n is 1 or 2;
-C(O)-NH-;
-C(O)-NR⁵-, wherein R⁵ is as defined above;
-NH-C(O)-;
-NR⁵-C(O)-, wherein R⁵ is as defined above; and
-C(=NH)-NH-;
R¹ is selected from the group consisting of:
(a) hydrogen;
(b) hydroxy;
(c) O-C₁-C₁₂-alkyl;
(d) O-CO-C₁-C₆-alkyl;
(e) O-CO-NH₂;
(f) O-CO-NH-CO-C₁-C₁₂-alkyl; and
(g) O-CO-NH-SO₂-C₁-C₁₂-alkyl; and
R² is hydrogen or a hydroxy-protecting group; the method comprising:
(a) reacting a compound having the formula (II): 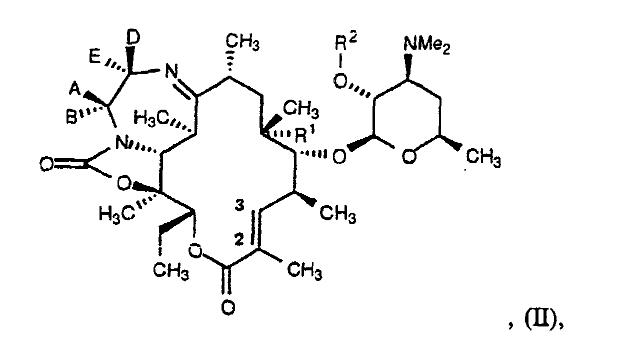 wherein R¹ is as above or is a hydroxy protecting group and R², A, B, D, and E are as defined above, with a suitable oxidizing agent to oxidize the imine nitrogen to the nitrone and the nitrogen atom on the desosamine moiety to the N-oxide to give an N-oxidized intermediate; and
(b) treating the N-oxidized intermediate with a reducing agent to reduce the desosamine N-oxide, and extracting, optionally deprotecting, and isolating the desired compound.

31. A process according to Claim 30 wherein in step (a) the oxidizing agent is selected from the group consisting of hydrogen peroxide and a carboxylic peracid; and in step (b) the reducing agent is selected from the group consisting of triphenylphosphine and hydrogen in the presence of a catalyst.

32. A process for the preparation of a compound having the Formula (IV): 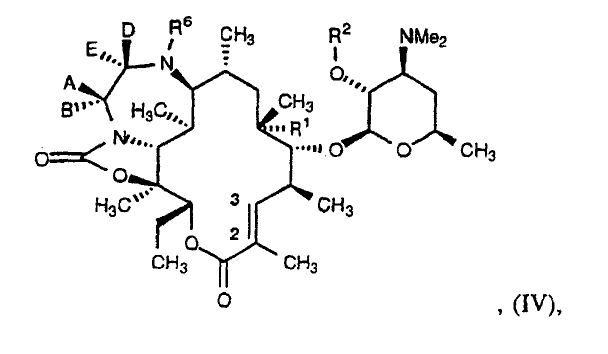 wherein
A, B, D and E are independently selected from the group consisting of:
(a) hydrogen;
(b) C₁-C₆-alkyl, optionally substituted with one or more substituents selected from the group consisting of:
(i) aryl;
(ii) substituted-aryl;
(iii) heteroaryl;
(iv) substituted-heteroaryl;
(v) heterocycloalkyl;
(vi) hydroxy;
(vii) C₁-C₆-alkoxy;
(viii) halogen consisting of Br, Cl, F or I; and
(ix) NR³R⁴, where R³ and R⁴ are independently selected from hydrogen and C₁-C₆-alkyl, or R³ and R⁴ are taken with the nitrogen atom to which they are connected to form a 3- to 7-membered ring which, when the ring is a 5- to 7-membered ring, may optionally contain a hetero function consisting of -O-, -NH-, -N(C₁-C₆-alkyl-)-, -N(aryl)-, -N(aryl-C₁-C₆-alkyl-)-, -N(substituted-aryl-C₁-C₆-alkyl-)-, -N(heteroaryl)-, -N(heteroaryl-C₁-C₆-alkyl-)-, -N(substituted-heteroaryl-C₁-C₆-alkyl-)-, -S- or -S(O)ₙ-, wherein n is 1 or 2;
(c) C₃-C₇-cycloalkyl;
(d) aryl;
(e) substituted-aryl;
(f) heteroaryl;
(g) substituted-heteroaryl;
(h) heterocycloalkyl;
and
(i) a group selected from option (b) above further substituted with -M-
R⁵, wherein M is selected from the group consisting of
(aa) -C(O)-NH-;
(bb) -NH-C(O)-;
(cc) -NH-
(dd) -N(CH₃)-
(ee) -O-
(ff) -S(O)ₙ-, wherein n is 0, 1 or 2; and
(gg) -C(=NH)-NH-;
and R⁵ is selected from the group consisting of:
(aaa) C₁-C₆-alkyl, optionally substituted with a substituent selected from the group consisting of:
(i) aryl;
(ii) substituted-aryl;
(iii) heteroaryl; and
(iv) substituted-heteroaryl;
(bbb) aryl;
(ccc) substituted-aryl;
(ddd) heteroaryl;
(eee) substituted-heteroaryl; and
(fff) heterocycloalkyl;
or any one pair of substituents, consisting of AB, AD, AE, BD, BE or DE, is taken together with the atom or atoms to which they are attached to form a 3- to 7-membered ring optionally containing a hetero function consisting of:
-O-,
-N(C₁-C₆-alkyl-)-,
-N(aryl-C₁-C₆-alkyl-)-,
-N(substituted-ayyl-C₁-C₆-alkyl-)-,
-N(heteroaryl-C₁-C₆-alkyl-)-,
-N(substituted-heteroaryl-C₁-C₆-alkyl-)-,
-S- or -S(O)ₙ-, wherein n is 1 or 2;
-C(O)-NH-;
-C(O)-NR⁵-, wherein R⁵ is as defined above;
-NH-C(O)-;
-NR⁵-C(O)-, wherein R⁵ is as defined above; and
-C(=NH)-NH-;
R¹ is selected from the group consisting of:
(a) hydrogen;
(b) hydroxy;
(c) O-C₁-C₁₂-alkyl;
(d) O-CO-C₁-C₆-alkyl;
(e) O-CO-NH₂;
(f) O-CO-NH-CO-C₁-C₁₂-alkyl; and
(g) O-CO-NH-SO₂-C₁-C₁₂-alkyl; and
R² is hydrogen or a hydroxy-protecting group;
R⁶ is hydrogen or C₁-C₆-alkyl;
the method comprising:
(a) reacting a compound having the formula: 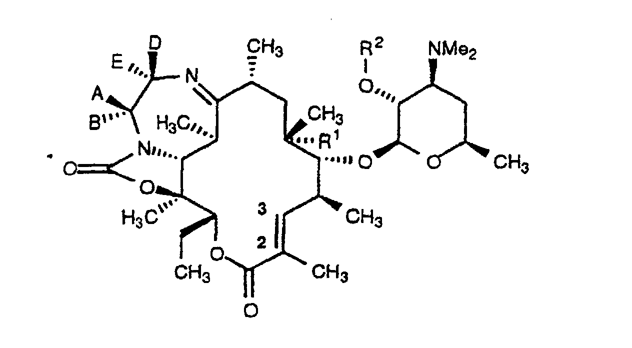 wherein R¹ is as above or is a hydroxy protecting group and R², A, B, D, and E are as defined above with a reducing agent in a suitable organic solvent to afford the desired compound wherein R⁶ is H;
(b) optionally reductively alkylating the amino the product of step (a) with a reducing reagent in the presence of a C₁-C₆-alkyl-group precursor to afford the desired compound wherein R⁶ is C₁-C₆-alkyl; and
(c) extracting, optionally deprotecting, and isolating the desired compound.

33. A process according to Claim 31 wherein in step (a) and in optional step (b) the reducing agent is selected from the group consisting of sodium cyanoborohydride, sodium borohydride, sodium triacetoxyborohydride, borane-tetrahydrofuran complex, and borane-piperidine complex.

34. A compound having the formula: 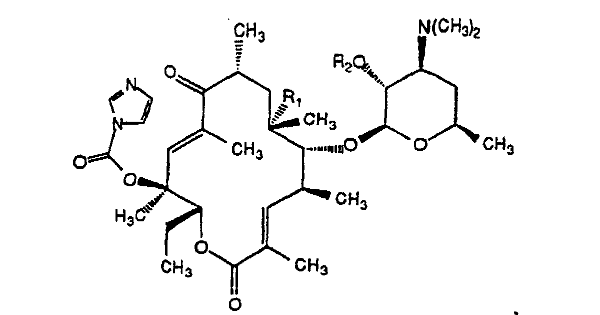 wherein
R¹ is selected from the group consisting of hydrogen, protected hydroxy, O-C₁-C₁₂-alkyl, O-CO-C₁-C₆-alkyl, O-CO-NH₂, O-CO-NH-CO-C₁-C₁₂-alkyl, or O-CO-NH-SO₂-C₁-C₁₂-alkyl; and
R² is hydrogen or a hydroxy protecting group.

35. The compound according to Claim 33 wherein R¹ is O-C₁-C₁₂-alkyl.

36. The compound according to Claim 34 wherein R¹ is methoxy.

37. A process for the preparation of a compound having the formula: 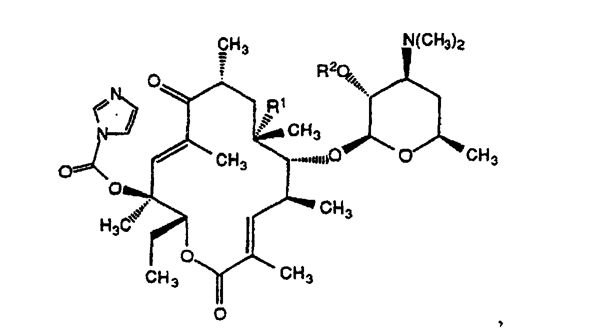 wherein
R¹ is selected from the group consisting of:
(a) hydrogen;
(b) protected hydroxy;
(c) O-C₁-C₁₂-alkyl;
(d) O-CO-C₁-C₆-alkyl;
(e) O-CO-NH₂;
(f) O-CO-NH-CO-C₁-C₁₂-alkyl; and
(g) O-CO-NH-SO₂-C₁-C₁₂-alkyl; and
R² is hydrogen or a hydroxy-protecting group;
the method comprising:
(a) treating an erythromycin A compound having the formula: 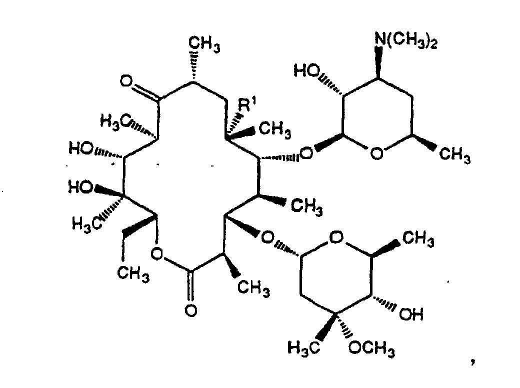 wherein R¹ is as defined above, with dehydrating reagents consisting of an organocarbonate in the presence of base at reflux temperature in an aprotic solvent to form an intermediate compound having the formula: 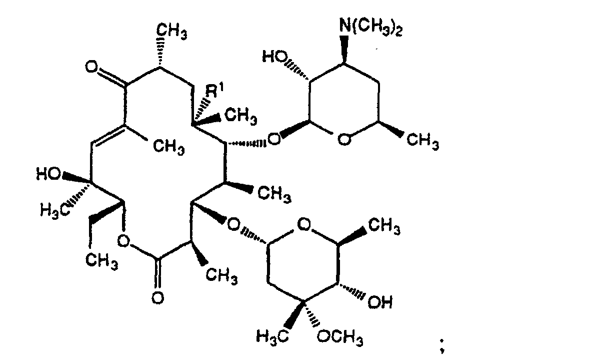
(b) hydrolytically removing the cladinose moiety from the intermediate compound of step (a) by treatment in an aqueous alcohol suspension with a dilute concentration of a strong acid at ambient temperature for about 0.5 to about 24 hours, extracting and optionally isolating the compound having the formula: 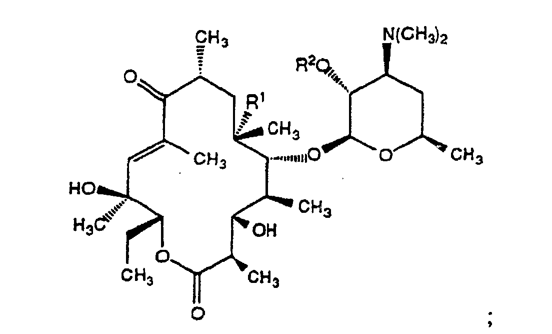
(c) treating the compound of step (b) with a suitable hydroxy group protecting reagent in an aprotic solvent, and extractively isolating the compound wherein R² is a hydroxy protecting group;
(d) treating a solution of the compound of step (c) with a sulfonylating agent at from about 0°C to ambient temperature for about 1 to about 24 hours, and extractively isolating the compound having the formula: 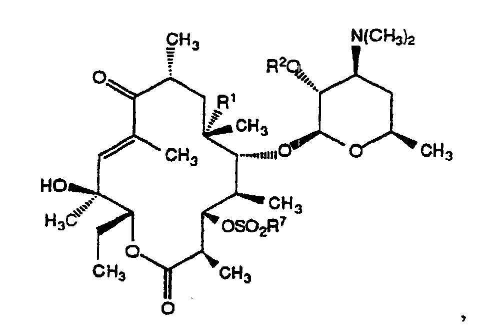 wherein R⁷ is alkyl or aryl;
(e) dehydrating the compound of step (d) with a hydride base in the presence of carbonyldiimidazole in an aprotic solvent at a temperature from about -20°C to about 70°C for from about 0.5 hours to about 10 days, and extracting, optionally deprotecting, and isolating the desired compound.

38. A process according to Claim 36 wherein in step (a) the dehydrating reagents consist of an organocarbonate compound selected from the group consisting of ethylene carbonate, propylene carbonate, trimethylene carbonate, dipropyl carbonate, dibenzyl carbonate, isobutyl carbonate, dimethyl carbonate and diethyl carbonate, in the presence of a base selected from the group consisting of triethylamine, diisopropylethylamine, pyridine, 2,6-dimethylpyridne, 1,8-diazabicyclo[5.4.0]undec-7-ene, N-methylmorpholine, N-methylpyrrolidine, N-methylpiperidine, sodium carbonate, potassium carbonate, sodium bicarbonate and potassium carbonate; in step (b) the alcohol is chosen from the group consisting of methanol, ethanol, propanol, iso-propanol, butanol, *iso*-butanol and t-butanol, and the acid is selected from the group consisting of hydrochloric acid, sulfuric acid, dichloroacetic acid and trichloroacetic acid; in step (c) the hydroxy group protecting reagent is selected from the group consisting of acetyl chloride, acetic anhydride, benzoic anhydride, benzyl chloroformate, trimethylsilyl chloride and triethylsilyl chloride, and the aprotic solvent is selected from the group consisting of methylene chloride, chloroform, dimethylformamide, tetrahydrofuran, *N*-methylpyrrolidinone and mixtures thereof; in step (d) the sulfonylating agent is selected from the group consisting of methanesulfonyl anhydride, methanesulfonyl chloride, ethanesulfonyl chloride and p-toluenesulfonyl chloride, and the base is selected from the group defined in step (a) above; in step (e) the hydride base is selected from the group consisting of sodium hydride, potassium hydride and lithium hydride and the aprotic solvent is as defined for step (c).

39. A process according to Claim 37 wherein R¹ is H and steps (d) and (e) are replaced with a single step (d') consisting of:
(d') treatment of the compound from step (c) with sodium hexamethyldisilazane at from about -50 to about -28°C under an inert atmosphere followed by addition of carbonyldiimidazole at from about 0°C to about ambient temperature for about 15 minutes to about 6 hours, and extracting, optionally deprotecting, and isolating the desired compound.

40. A process for the preparation of a compound having the formula: 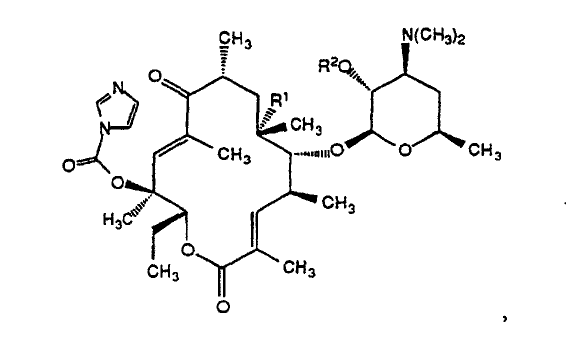 wherein
R¹ is selected from the group consisting of:
(a) hydrogen;
(b) protected hydroxy;
(c) O-C₁-C₁₂-alkyl;
(d) O-CO-C₁-C₆-alkyl;
(e) O-CO-NH₂;
(f) O-CO-NH-CO-C₁-C₁₂-alkyl; and
(g) O-CO-NH-SO₂-C₁-C₁₂-alkyl; and
R² is hydrogen or a hydroxy-protecting group;
the method comprising:
(a) hydrolytically removing the cladinose moiety from an erythromycin A compound having the formula: 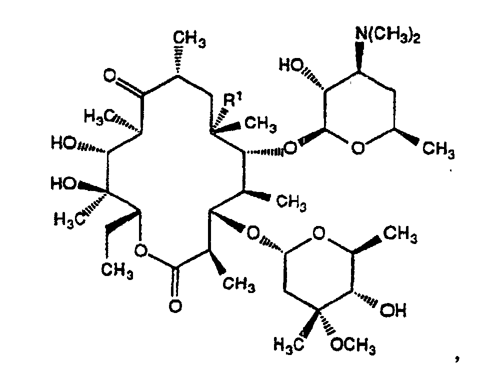 wherein R¹ is as described above by treatment in an aqueous alcohol suspension with a dilute concentration of a strong acid at ambient temperature for about 0.5 to about 24 hours, extracting and optionally isolating the first intermediate compound having the formula: 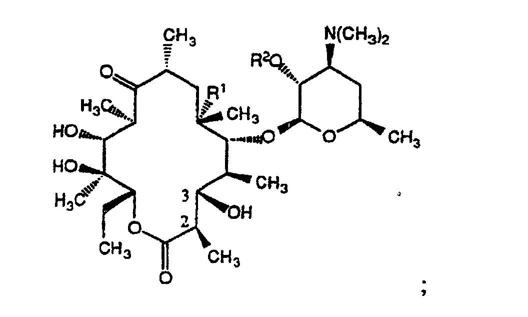
(b) optionally treating the first intermediate compound with a suitable hydroxy group protecting reagent and extractively isolating the second intermediate compound having the formula of the compound of step (a) wherein R² is a hydroxy-protecting group;
(c) treating the second intermediate compound with an excess of a carbonylating reagent and isolating by aqueous work up the third intermediate compound having the formula: 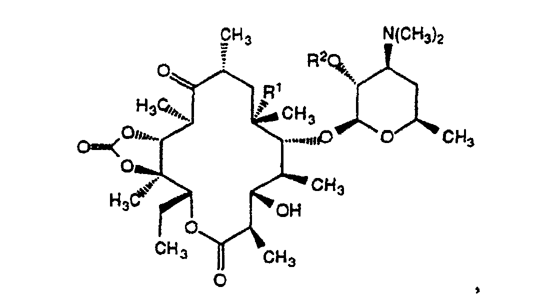 wherein R¹ may not be hydrogen but is otherwise as defined above;
(d) treating the third intermediate compound with a sulfonylating agent at from about 0°C to ambient temperature for about 1 to about 24 hours, and extractively isolating the fourth intermediate compound having the formula: 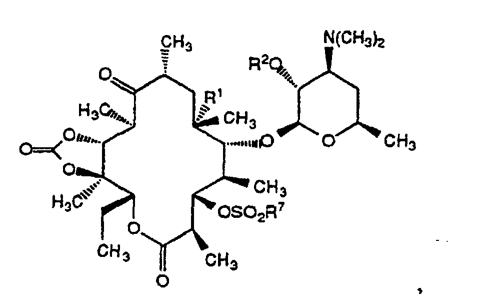 wherein R⁷ is alkyl or aryl;
(e) treating the fourth intermediate compound with a strong base, extracting and optionally isolating the to afford the fifth intermediate compound having the formula: 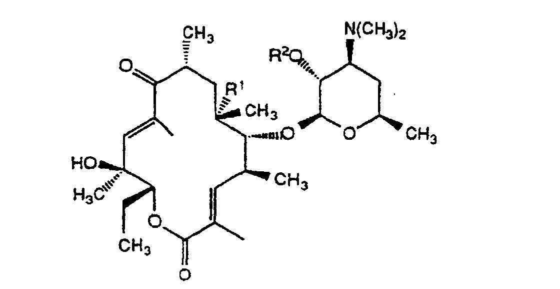
(f) treating the fifth intermediate compound with a hydride base and carbonyldiimidazole in an aprotic solvent at a temperature from about -20°C to about 70°C for from about 0.5 hours to about 10 days, and extracting, optionally deprotecting, and isolating the desired compound.

41. A process according to Claim 40 wherein in step (a) the alcohol is chosen from the group consisting of methanol, ethanol, propanol, *iso*-propanol, butanol, *iso*-butanol and *t*-butanol, and the acid is selected from the group consisting of hydrochloric acid, sulfuric acid, dichloroacetic acid and trichloroacetic acid; in step (b) the hydroxy group protecting reagent is selected from the group consisting of acetyl chloride, acetic anhydride, benzoic anhydride, benzyl chloroformate, trimethylsilyl chloride and triethylsilyl chloride, and the aprotic solvent is selected from the group consisting of methylene chloride, chloroform, dimethylformamide, tetrahydrofuran, *N*-methylpyrrolidinone and mixtures thereof; in step (c) the carbonylating reagent is selected from the group consisting of phosgene, diphosgene and triphosgene; in step (d) the sulfonylating agent is selected from the group consisting of methanesulfonyl anhydride, methanesulfonyl chloride, ethanesulfonyl chloride and p-toluenesulfonyl chloride; in step (e) the base is selected from the group consisting of triethylamine, diisopropylethylamine, pyridine, 2,6-dimethylpyridne, 1,8-diazabicyclo[5.4.0]undec-7-ene, N-methylmorpholine, N-methylpyrrolidine, N-methylpiperidine, sodium carbonate, potassium carbonate, sodium bicarbonate and potassium carbonate; in step (f) the hydride base is selected from the group consisting of sodium hydride, potassium hydride and lithium hydride.

42. A process according to Claim 40 wherein in step (b) the hydroxy protecting reagent is benzoic anhydride and R² is benzoyl, and steps (c), (d) and (e) are replaced with a single step (c') consisting of:
(c') treatment of the compound from step (b) with sodium hexamethyldisilazane at from about -50 to about -28°C under an inert atmosphere followed by addition of carbonyldiimidazole at from about 0°C to about ambient temperature for about 15 minutes to about 6 hours, and extracting, optionally deprotecting, and isolating the desired compound.

43. A pharmaceutical composition for treating bacterial infections comprising a therapeutically effective amount of a compound of Claim 1 in combination with a pharmaceutically acceptable carrier.

44. Use of a pharmaceutical composition containing a therapeutically-effective amount of a compound of Claim 1 for manufacturing a medicament for treating bacterial infections by administration to a mammal in need of such treatment.

## Patentansprüche

1. Eine Verbindung oder ein pharmazeutisch verträgliches Salz oder Ester davon, gewählt aus der Gruppe bestehend aus: 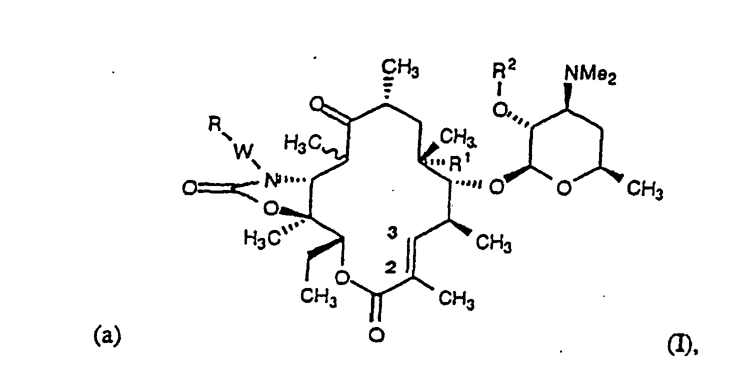 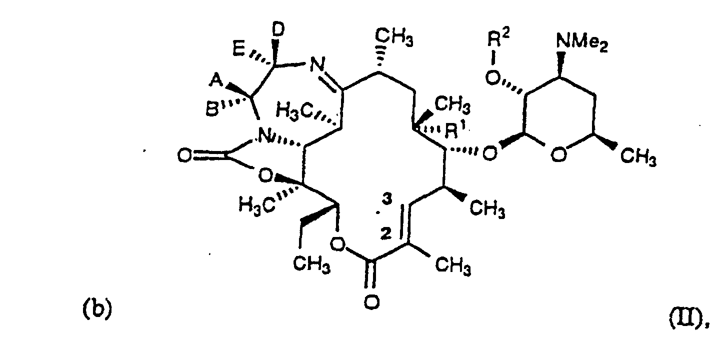 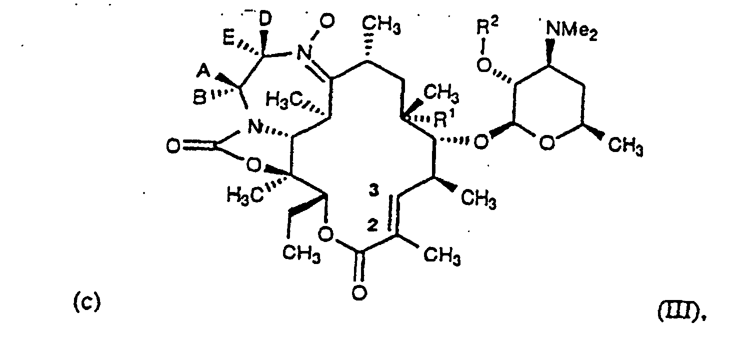 und 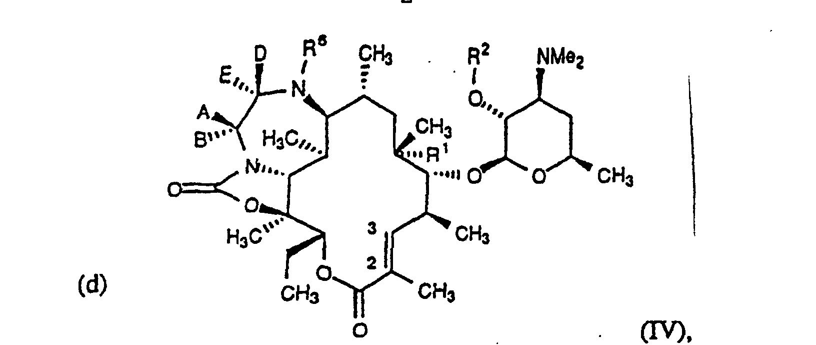 worin
R¹ gewählt ist aus der Gruppe bestehend aus
(a) Wasserstoff,
(b) Hydroxy,
(c) O-C₁-C₁₂-Alkyl;
(d) O-CO-C₁-C₆-Alkyl;
(e) O-CO-NH₂;
(f) O-CO-NH-CO-C₁-C₁₂-Alkyl; und
(g) O-CO-NH-SO₂-C₁-C₁₂-Alkyl;
R² ist Wasserstoff oder eine Hydroxyschutzgruppe;
R⁶ ist Wasserstoff oder C₁-C₆-Alkyl;
R ist gewählt aus der Gruppe bestehend aus:
(a) Wasserstoff;
(b) C₁-C₆-Alkyl, wahlweise substituiert mit einem oder mehreren Substituenten gewählt aus der Gruppe bestehend aus:
(i) Aryl,
(ii) substituiertem Aryl;
(iii) Heteroaryl;
(iv) substituiertem Heteroaryl;
(v) Hydroxy;
(vi) C₁-C₆-Alkoxy;
(vii) NR³R⁴, worin R³ und R⁴ unabhängig gewählt sind aus Wasserstoff und C₁-C₆-Alkyl, oder R³ und R⁴ sind zusammengenommen mit dem Wasserstoffatom, an welches sie angeschlossen sind, um einen 3- bis 7-gliedrigen Ring zu bilden, welcher, falls der Ring ein 5- bis 7-gliedriger Ring ist, wahlweise eine Heterofunktion enthalten kann bestehend aus -O-, -NH-, -N(C₁-C₆-Alkyl-)-, -N(Aryl) -, -N(Aryl-C₁-C₆-alkyl-)-, -N(substituiertes-Aryl-C₁-C₆-alkyl-)-, -N(Heteroaryl)-, -N(Heteroaryl-C₁-C₆-alkyl-)-, -N(substituiertes-Heteroaryl-C₁-C₆-alkyl-)-, -S- oder -S(O)ₙ-, worin n 1 oder 2 ist;
(viii) -CH₂-M-R⁵,
worin M gewählt ist aus der Gruppe bestehend aus:
(aa) -C(O)-NH-;
(bb) -NH-C(O)-;
(cc) -NH-;
(dd) -N=;
(ee) -N(CH₃)-;
(ff) -O-;
(gg) -S(O)ₙ-, worin n 0, 1 oder 2 ist;
(hh) -CO-O-;
(ii) -O-CO-;
(jj) -CO-; und
R⁵ ist gewählt aus der Gruppe bestehend aus:
(aaa) C₁-C₆-Alkyl, wahlweise substituiert mit einem Substituenten gewählt aus der Gruppe bestehend aus:
(i) Aryl;
(ii) substituiertem Aryl;
(iii) Heteroaryl; und
(iv) substituiertem Heteroaryl;
(bbb) Aryl;
(ccc) substituiertem Aryl;
(ddd) Heteroaryl;
(eee) substituiertem Heteroaryl; und
(fff) Heterocycloalkyl; und
(c) C₃-C₇-Cycloalkyl;
(d) Aryl;
(e) substituiertem Aryl;
(f) Heteroaryl;
(g) substituiertem Heteroaryl;
W ist fehlend oder gewählt aus der Gruppe bestehend aus -O-, -NH-CO-, -N=CH-, -NH- und -N (C₁-C₆-Alkyl)-;
A, B, D und E sind unabhängig gewählt aus der Gruppe bestehend aus:
(a) Wasserstoff;
(b) C₁-C₆-Alkyl, wahlweise substituiert mit einem oder mehreren Substituenten gewählt aus der Gruppe bestehend aus:
(i) Aryl;
(ii) substituiertem Aryl;
(iii) Heteroaryl;
(iv) substituiertem Heteroaryl;
(v) Hetercycloalkyl;
(vi) Hydroxy;
(vii) C₁-C₆-Alkoxy;
(viii) Halogen bestehend aus Br, Cl, F oder I; und
(ix) NR³R⁴, worin R³ und R⁴ unabhängig gewählt sind aus Wasserstoff und C₁-C₆-Alkyl, oder R³ und R4 sind zusammengenommen mit dem Stickstoffatom, an welches sie gebunden sind ein 3- bis 7-gliedriger Ring wahlweise eine Heterofunktion enthaltend, bestehend aus -O-, -NH-, -N(C₁-C₆-Alkyl-)-, -N(Aryl-C₁-C₆-alkyl-)-, -N(substitiertes-Aryl-C₁-C₆-alkyl-)-, -N(Heteroaryl-C₁-C₆-alkyl-)-, -N(substituiertes-Heteraryl-C₁-C₆alkyl-)-, -S- oder -S(O)ₙ-, worin n 1 oder 2 ist;
(c) C₃-C₇-Cycloalkyl;
(d) Aryl;
(e) substituiertem Aryl;
(f) Heteraryl;
(g) substitiertem Heteraryl;
(h) Heterocycloalkyl; und
(i) eine Gruppe gewählt aus Option (b) wie oben, weiterhin substituiert mit -M-R⁵, worin M und R⁵ so sind wie oben definiert;
oder
ein beliebiges Paar Substituenten, bestehend aus AB, AD, AE, BD, BE oder DE, zusammengenommen mit dem Atom oder den Atomen an welche sie angeheftet sind, um einen 3- bis 7-gliedrigen Ring zu bilden, wahlweise mit einer Heterofunktion bestehend aus:
-O-,
-NH-,
-N(C₁-C₆-Alkyl-)-,
-N(Aryl-C₁-C₆-alkyl-)-,
-N(substituiertes-Aryl-C₁-C₆-alkyl-)-,
-N(Heteroaryl-C₁-C₆-alkyl-)-,
-N(substitiertes-Heteroaryl-C₁-C₆-alkyl-)-,
-S- oder -S(O)ₙ-, worin n 1 oder 2 ist,
-C(O)-NH-,
-C(O)-NR⁵-, worin R⁵ so ist wie oben definiert,
-NH-C(O)-,
-NR⁵-C(O)-, worin R⁵ so ist wie oben definiert, und
-C(=NH)-NH-.

2. Eine Verbindung gemäß Anspruch 1, mit der Strukturformel (I).

3. Eine Verbindung gemäß Anspruch 2, worin W fehlend ist oder -NH-.

4. Eine Verbindung gemäß Anspruch 1, mit der Strukturformel (II).

5. Eine Verbindung gemäß Anspruch 1, mit der Strukturformel (III).

6. Eine Verbindung gemäß Anspruch 1, mit der Strukturformel (IV).

7. Eine Verbindung gemäß Anspruch 1, oder einem pharmazeutisch verträglichem Salz oder Ester davon, welche folgendes ist:
Verbindung der Formel (I); R¹=H; R²=H, W ist fehlend; R=4-Phenylbutyl;
Verbindung der Formel (I); R¹=Methoxy; R²=H; W ist fehlend; R=4-Phenylbutyl;
Verbindung der Formel (I); R¹=Methoxy, R²=H, W ist fehlend, R=3-Phenoxypropyl;
Verbindung der Formel (I); R¹=Methoxy; R²=H; W ist fehlend, R=2-((Phenylmethyl)amino)ethyl;
Verbindung der Formel (I); R¹=Methoxy, R²=H; W ist fehlend, R=3-(N-Methyl-N-phenylamino)propyl;
Verbindung der Formel (I); R¹=Methoxy, R²=H; W ist fehlend; R=3-(4-Chlorphenoxy)propyl;
Verbindung der Formel (II): R¹=Methoxy; R²=H; A=B=C=D=H;
Verbindung der Formel (I); R¹=Methoxy; R²=H; W ist fehlend; R=3-(1-Chinoyloxy)propyl;
Verbindung der Formel (I); R¹=Methoxy; R²=H; W ist fehlend; R=4-(4-Chlorphenyl)-3(Z)-butenyl;
Verbindung der Formel (I); R¹=Methoxy; R²=H; W ist fehlend; R=2-Phenylethyl;
Verbindung der Formel (I); R¹=Methoxy; R²=H; W ist fehlend; R=2-(3,4-Dichlorphenyl)ethyl;
Verbindung der Formel (I); R¹=Methoxy; R²=H; W ist fehlend; R=Phenylmethyl;
Verbindung der Formel (I); R¹=Methoxy; R²=H; W ist -NH-; R=3-Phenylpropyl;
Verbindung der Formel (I); R¹=Methoxy; R²=H; W ist fehlend; R=3-(4-Phenoxyphenyl)ethyl;
Verbindung der Formel (I); R¹=Methoxy; R²=H; W ist fehlend; R=3-Phenylpropyl;
Verbindung der Formel (I) ; R¹=Methoxy; R²=H; W ist fehlend; R=2,2-Diphenylethyl;
Verbindung der Formel (I); R¹=Methoxy; R²=H; W ist fehlend; R=H;
Verbindung der Formel (IV); R¹=Methoxy; R²=H; A=B=C=D=H; R=H;
Verbindung der Formel (I); R¹=Methoxy; R²=H; W ist -NH-; R¹=H; C10 Methyl ist *epi*-Isomer;
Verbindung der Formel (I); R¹=Methoxy; R²=H; W ist -NH-; R=H; C10 Methyl ist Naturisomer;
Verbindung der Formel (I); R¹=Methoxy; R²=H; W ist -NH-; R=(4-Chinolinyl)propyl; C10 Methyl ist Naturisomer;
Verbindung der Formel (I); R¹=Methoxy; R²=H; W ist fehlend; R=3-(2-Naphthyloxy)propyl;
Verbindung der Formel (I); R¹=Methoxy; R²=H; W ist fehlend; R=3-(3-Pyridyloxy)propyl;
Verbindung der Formel (I); R¹=Methoxy; R²=H; W ist fehlend; R=3-(2-Pyridyloxy)propyl;
Verbindung der Formel (I); R¹=OH; R²=H; W ist fehlend; R=4-Phenylbutyl;
Verbindung der Formel (I); R¹=OCONH2; R²=H; W ist fehlend; R=4-Phenylbutyl;
Verbindung der Formel (I); R¹=OCONHCO-Methyl; R²=H; W ist fehlend; R=4-Phenylbutyl;
Verbindung der Formel (I); R¹=OCONHSO2-Methyl; R²=H; W ist fehlend; R=4-Phenylbutyl;
Verbindung der Formel (I); R¹=OMe; R²=H; W ist fehlend; R=Phenyl;
Verbindung der Formel (I): R¹=OMe, R²=H; W ist fehlend; R=3-Pyridyl;
Verbindung der Formel (I); R¹=OMe, R²=H; W ist -O-; R=H;
Verbindung der Formel (I); R¹=OMe; R²=H; W ist -O-; R=Me;
Verbindung der Formel (I); R¹=OMe; R²=H; W ist -NH--CO-; R=Phenyl;
Verbindung der Formel (II); R¹=OMe; R²=H; A=Benzyl; B, D, E=H;
Verbindung der Formel (II); R¹=OMe; R²=H; A,D=3,4-Pyrrolidinyl; B, E=H;
Verbindung der Formel (III); R¹=OMe; R²=H; A,B,D,E=H;
Verbindung der Formel (IV); R¹=OMe; R²=H; A=Benzyl; B,D,E=H; R=H;
Verbindung der Formel (IV); R¹=OMe; R²=H; A,D=3,4-Pyrrolidinyl; B,E=H; R=H;
Verbindung der Formel (IV); R¹=OMe; R²=H; A, B, D, E=H, R=CH₂CH₂CH₂C₆H₅;
Verbindung der Formel (IV); R¹=OMe, R²=H; A,B,D,E=H, R=2,4-Dinitrobenzen;
Verbindung der Formel (IV); R¹=OMe, R²=H; A,B,D,E=H, R=4-Chinolyl;
Verbindung der Formel (I); R¹=Methoxy; R²=H; W ist fehlend; R=(4H-4-oxo-1-chinolyl)propyl;
Verbindung der Formel (I); R¹=Methoxy; R²=H; W ist fehlend; R=2-(4-Nitrophenyl)ethyl;
Verbindung der Formel (I); R¹=Methoxy; R²=H; W ist fehlend; R=2-(4-Aminophenyl)ethyl;
Verbindung der Formel (I); R¹=Methoxy; R²=H; W ist fehlend; R=3-Ethoxypropyl;
Verbindung der Formel (I); R¹=Methoxy; R²=H; W ist fehlend; R=Isopropyl;
Verbindung der Formel (I); R¹=Methoxy; R²=H; W ist fehlend; R=2-(4-Bromphenyl)ethyl;
Verbindung der Formel (I); R¹=Methoxy; R²=H; W ist fehlend; R=2-(4-Hydroxylphenyl)ethyl;
Verbindung der Formel (I); R¹=Methoxy; R²=H; W ist fehlend; R=2-(4-Fluorphenyl)ethyl;
Verbindung der Formel (I); R¹=Methoxy; R²=H; W ist fehlend; R=2-(3-Methoxyphenyl)ethyl;
Verbindung der Formel (I); R¹=Methoxy; R²=H; W ist fehlend; R=3-Vinyloxypropyl;
Verbindung der Formel (I); R¹=Methoxy; R²=H; W ist fehlend; R=2-(3-Trifluormethyl)phenylethyl;
Verbindung der Formel (I); R¹=Methoxy; R²=H; W ist fehlend; R=2-Thienylethyl;
Verbindung der Formel (I); R¹=Methoxy; R²=H; W ist fehlend; R=2-(3,4-di-Benzyloxyphenyl)ethyl;
Verbindung der Formel (I); R¹=Methoxy; R²=H; W ist fehlend; R=2-(4-Methylphenyl)ethyl;
Verbindung der Formel (I); R¹=Methoxy; R²=H; W ist fehlend; R=Allyl;
Verbindung der Formel (I); R¹=Methoxy; R²=H; W ist fehlend; R=1,3-Dihydroxypropyl;
Verbindung der Formel (I); R¹=Methoxy; R²=H; W ist fehlend; R=1,3-Dihydroxypropyl (10-*epi*);
Verbindung der Formel (I); R¹=Methoxy; R²=H; W ist fehlend; R=3-Hydroxypropyl;
Verbindung der Formel (I); R¹=Methoxy; R²=H; W ist fehlend; R=3-Hydroxypropyl (10-*epi*);
Verbindung der Formel (I); R¹=Methoxy; R²=H; W ist fehlend; R=Propyl;
Verbindung der Formel (I); R¹=Methoxy; R²=H; W ist fehlend; R=Isobutyl;
Verbindung der Formel (I); R¹=Methoxy; R²=H; W ist fehlend; R=2-(Benzoylamino)ethyl;
Verbindung der Formel (I); R¹=Methoxy; R²=H; W ist fehlend; R=3-(Benzoylamino)propyl;
Verbindung der Formel (I); R¹=Methoxy; R²=H; W ist fehlend; R=3-(Acetylamino)propyl;
Verbindung der Formel (I); R¹=Methoxy; R²=H; W ist fehlend; R=H (10-*epi*);
Verbindung der Formel (I); R¹=Methoxy; R²=H; W ist fehlend; R=3-Phenylpropyl (10-*epi*);
Verbindung der Formel (I); R¹=Methoxy; R²=H; W ist fehlend; R=3-(4-Phenoxyphenyl)ethyl (10-*epi*);
Verbindung der Formel (I); R¹=Methoxy; R²=H; W ist -NH-; R=3-(4-Chlorphenyl)propyl;
Verbindung der Formel (I); R¹=Methoxy; R²=H; W ist -NH-; R=3-(3-Chlorphenyl)propyl;
Verbindung der Formel (I); R¹=Methoxy; R²=H; W ist -NH-; R=3-(2-Chlorphenyl)propyl;
Verbindung der Formel (I); R¹=Methoxy; R²=H; W ist -NH-; R=3-(2,4-Dichlorphenyl)propyl;
Verbindung der Formel (I); R¹=Methoxy; R²=H; W ist -NH-; R=3-(4-Hydroxyphenyl)propyl;
Verbindung der Formel (I); R¹=Methoxy; R²=H; W ist -NH-; R=3-(3-Hydroxyphenyl)propyl;
Verbindung der Formel (I); R¹=Methoxy; R²=H; W ist -NH-; R=3-(2-Hydroxyphenyl)propyl;
Verbindung der Formel (I); R¹=Methoxy; R²=H; W ist -NH-;
Verbindung der Formel (I); R¹=Methoxy; R²=H; W ist fehlend; R=1,3-Dihydroxypropyl (10-*epi*);
Verbindung der Formel (I); R¹=Methoxy; R²=H; W ist fehlend; R=3-Hydroxypropyl;
Verbindung der Formel (I); R¹=Methoxy; R²=H; W ist fehlend; R=3-Hydroxypropyl (10-*epi*);
Verbindung der Formel (I); R¹=Methoxy; R²=H; W ist fehlend; R=Propyl;
Verbindung der Formel (I); R¹=Methoxy; R²=H; W ist fehlend; R=Isobutyl;
Verbindung der Formel (I); R¹=Methoxy; R²=H; W ist fehlend; R=2-(Benzoylamino)ethyl;
Verbindung der Formel (I); R¹=Methoxy; R²=H; W ist fehlend; R=3-(Benzoylamino)propyl;
Verbindung der Formel (I); R¹=Methoxy; R²=H; W ist fehlend; R=3-(Acetylamino)propyl;
Verbindung der Formel (I); R¹=Methoxy; R²=H; W ist fehlend; R=H (10-*epi*);
Verbindung der Formel (I); R¹=Methoxy; R²=H; W ist fehlend; R=3-Phenylpropyl (10-*epi*);
Verbindung der Formel (I); R¹=Methoxy; R²=H; W ist fehlend; R=3-(4-Phenoxyphenyl)ethyl (10-*epi*);
Verbindung der Formel (I); R¹=Methoxy; R²=H; W ist -NH-; R=3-(4-Chlorphenyl)propyl;
Verbindung der Formel (I); R¹=Methoxy; R²=H; W ist -NH-; R=3-(3-Chlorphenyl)propyl;
Verbindung der Formel (I); R¹=Methoxy; R²=H; W ist -NH-; R=3-(2-Chlorphenyl)propyl;
Verbindung der Formel (I); R¹=Methoxy; R²=H; W ist -NH-; R=3-(2,4-Dichlorphenyl)propyl;
Verbindung der Formel (I); R¹=Methoxy; R²=H; W ist -NH-; R=3-(4-Hydroxyphenyl)propyl;
Verbindung der Formel (I): R¹=Methoxy; R²=H; W ist -NH-; R=3-(3-Hydroxyphenyl)propyl;
Verbindung der Formel (I); R¹=Methoxy; R²=H; W ist -NH-; R=3-(2-Hydroxyphenyl)propyl;
Verbindung der Formel (I); R¹=Methoxy; R²=H; W ist -NH-; R=3-(4-Methoxyphenyl)propyl;
Verbindung der Formel (I); R¹=Methoxy; R²=H; W ist -NH-; R=3-(4-Nitrophenyl)propyl;
Verbindung der Formel (I); R¹=Methoxy; R²=H; W ist -NH-; R=3-(3-Nitrophenyl)propyl;
Verbindung der Formel (I); R¹=Methoxy; R²=H; W ist -NH-; R=3-(2-Nitrophenyl)propyl;
Verbindung der Formel (I); R¹=Methoxy; R²=H; W ist -NH-; R=3-((4-(Acetylamino)phenyl)propyl;
Verbindung der Formel (I); R¹=Methoxy; R²=H; W ist -NH-; R=*trans*-3-Phenylprop-2-enyl;
Verbindung der Formel (I); R¹=Methoxy; R²=H; W ist -NH-; R=2-Phenylethyl;
Verbindung der Formel (I); R¹=Methoxy; R²=H; W ist -NH-; R=Phenylmethyl;
Verbindung der Formel (I); R¹=Methoxy; R²=H; W ist -NH-; R=(3-Indolyl)methyl;
Verbindung der Formel (I); R¹=Methoxy; R²=H; W ist -NH-; R=(4-Methoxyphenyl)methyl;
Verbindung der Formel (I); R¹=Methoxy; R²=H; W ist -NH-; R=(4-Acetylaminophenyl)methyl;
Verbindung der Formel (I); R¹=Methoxy; R²=H; W ist -NH-; R=(4-Chlorphenyl)methyl;
Verbindung der Formel (I); R¹=Methoxy; R²=H; W ist -NH-; R=(4-Dimethylaminophenyl)methyl;
Verbindung der Formel (I); R¹=Methoxy; R²=H; W ist -NH-; R=*trans*-3-(4-Nitrophenyl)prop-2-enyl;
Verbindung der Formel (I); R¹=Methoxy; R²=H; W ist -NH-; R=(4-Nitrophenyl)methyl;
Verbindung der Formel (I); R¹=Methoxy; R²=H; W ist -NH-; R=(3,4-Dihydroxyphenyl)methyl;
Verbindung der Formel (I); R¹=Methoxy; R²=H; W ist -NH-; R=(2,5-Dihydroxyphenyl)methyl;
Verbindung der Formel (I); R¹=Methoxy; R²=H; W ist -NH-; R=(2-Hydroxy-5-nitrophenyl)methyl;
Verbindung der Formel (I); R¹=Methoxy; R²=H; W ist -NH-; R=(4-Hydroxymethylphenyl)methyl;
Verbindung der Formel (I); R¹=Methoxy; R²=H; W ist -NH-; R=*trans*-3-(5-nitro-2-furanyl)prop-2-enyl;
R¹R²
Verbindung der Formel (I); R¹=Methoxy; R²=H; W ist -NH-; R=(4-Hydroxyphenyl)methyl;
Verbindung der Formel (I); R¹=Methoxy; R²=H; W ist -NH-; R=(3-Hydroxyphenyl)methyl;
Verbindung der Formel (I); R¹=Methoxy; R²=H; W ist -NH-; R=(2-Hydroxyphenyl)methyl;
Verbindung der Formel (I); R¹=Methoxy; R²=H; W ist -NH-; R=(4-Trifluormethylphenyl)methyl;
Verbindung der Formel (I); R¹=Methoxy; R²=H; W ist -NH-; R=(4-Cyanophenyl)methyl;
Verbindung der Formel (I); R¹=Methoxy; R²=H; W ist -NH-; R=(2-Pyridyl)methyl;
Verbindung der Formel (I); R¹=Methoxy; R²=H; W ist -NH-; R=(3-Pyridyl)methyl;
Verbindung der Formel (I); R¹=Methoxy; R²=H; W ist -NH-; R=(4-Pyridyl)methyl;
Verbindung der Formel (I); R¹=Methoxy; R²=H; W ist -NH-; R=(2-Hydroxy-1-napthyl)methyl;
Verbindung der Formel (I); R¹=Methoxy; R²=H; W ist -NH-; R=(4-Dimethylamino-1-naphthyl)methyl;
Verbindung der Formel (I); R¹=Methoxy; R²=H; W ist -NH-; R=(4-(Methylthio)phenyl)methyl;
Verbindung der Formel (I); R¹=Methoxy; R²=H; W ist -NH-; R=(4-Phenoxyphenyl)methyl;
Verbindung der Formel (I); R¹=Methoxy; R²=H; W ist -NH-; R=3-(4-Fluorphenyl)propyl;
Verbindung der Formel (I); R¹=Methoxy; R²=H; W ist -NH-; R=(*trans*-3-(4-nitrophenyl)prop-2-enyl;
Verbindung der Formel (I); R¹=Methoxy; R²=H; W ist -NH-; R=(4-Amino-phenyl)methyl;
Verbindung der Formel (I); R¹=Methoxy; R²=H; W ist -NH-; R=3-(4-Amino-phenyl)propyl;
Verbindung der Formel (I); R¹=Methoxy; R²=H; W ist -NH-; R=3-(3-Amino-phenyl)propyl;
Verbindung der Formel (I); R¹=Methoxy; R²=H; W ist -NH-; R=3-(2-Amino-phenyl)propyl;
Verbindung der Formel (I); R¹=Methoxy; R²=H; W ist -NH-; R=*trans*-3-(4-Acetylaminophenyl)prop-2-enyl;
Verbindung der Formel (I); R¹=Methoxy; R²=H; W ist -NH-; R=*trans* -3-(4-(4-Nitrobenzoylamino)phenyl)prop-2-enyl;
Verbindung der Formel (I); R¹=Methoxy; R²=H; W ist -NH-; R=3-(2-Benztriazolyl)propyl;
Verbindung der Formel (I); R¹=Methoxy; R²=H; W ist -NH-; R=3-(1-Benztriazolyl)propyl;
Verbindung der Formel (I); R¹=Methoxy; R²=H; W ist -NH-; R=3-(4-Phenylimidazolyl)propyl;
Verbindung der Formel (I); R¹=Methoxy; R²=H; W ist -NH-; R=3-(1-Anhydro-1-cladinosyl)propyl;
Verbindung der Formel (I); R¹=Methoxy; R²=H; W ist -NH-; R=3-Phenylpropyl (10-*epi*);
Verbindung der Formel (I); R¹=Methoxy; R²=H; W ist -NH-; R=Isopropyl;
Verbindung der Formel (I); R¹=Methoxy; R²=H; W ist -NH-; R=1,3-Diphenyl-2-propyl; oder
Verbindung der Formel (I); R¹=Methoxy; R²=H; W ist -NH-; R=3-Pentyl.

8. Ein Verfahren zur Herstellung einer Verbindung mit der Strukturformel (I): 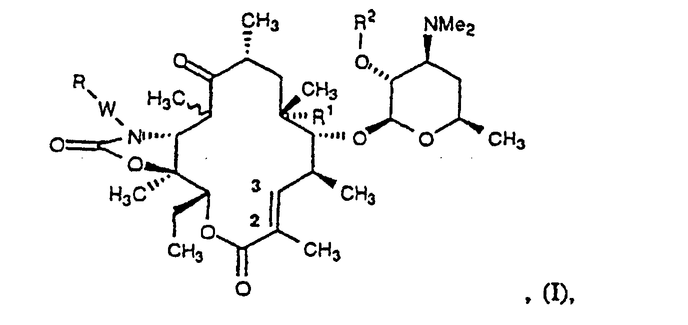 worin
R¹ gewählt ist aus der Gruppe bestehend aus:
(a) Wasserstoff;
(b) Hydroxy;
(c) O-C₁-C₁₂-Alkyl;
(d) O-CO-C₁-C₆-Alkyl;
(e) O-CO-NH₂;
(f) O-CO-NH-CO-C₁-C₁₂-Alkyl; und
(g) O-CO-NH-SO₂-C₁-C₁₂-Alkyl;
R² ist Wasserstoff oder eine Hydroxyschutzgruppe;
R ist gewählt aus der Gruppe bestehend aus:
(a) Wasserstoff;
(b) C₁-C₆-Alkyl wahlweise substituiert mit einem oder mehreren Substituenten gewählt aus der Gruppe bestehend aus:
(i) Aryl;
(ii) substituiertem Aryl;
(iii) Heteroaryl;
(iv) substituiertem Heteroaryl;
(v) Hydroxy;
(vi) C₁-C₆-Alkoxy;
(vii) NR³R⁴, worin R³ und R⁴ unabhängig gewählt sind aus Wasserstoff und C₁-C₆-Alkyl, oder R³ und R⁴ sind zusammen mit dem Stickstoffatom an welches sie gebunden sind, um einen 3- bis 7-gliedrigen Ring zu bilden wahlweise mit einer Heterofunktion bestehend aus -O-, -NH-, -N (C₁-C₆-Alkyl-) -, -N (Aryl-C₁-C₆alkyl-)-, -N(substituiertes-Aryl-C₁-C₆-alkyl-)-, -N(Heteroaryl-C₁-C₆alkyl-)-, -N(substituiertes-Heteroaryl-C₁-C₆-alkyl-)-, -S- oder -S(O)ₙ-, worin n 1 oder 2 ist;
(viii) -CH₂-M-R⁵,
worin M gewählt ist aus der Gruppe bestehend aus:
(aa) -C(O)-NH;
(bb) -NH-C(O)-;
(cc) -NH-;
(dd) -N=;
(ee) -N(CH₃)-;
(ff) -O-;
(gg) -S(O)ₙ-, worin n 0, 1 oder
2 ist;
(hh) -CO-O-;
(ii) -O-CO-;
(jj) -CO-; und
R⁵ ist gewählt aus der Gruppe bestehend aus:
(aaa) C₁-C₆-Alkyl, wahlweise substituiert mit einem Substituenten gewählt aus der Gruppe bestehend aus:
(i) Aryl;
(ii) substituiertem Aryl;
(iii) Heteroaryl; und
(iv) substituiertem Heteroaryl;
(bbb) Aryl;
(ccc) substituiertem Aryl;
(ddd) Heteroaryl;
(eee) substituiertem Heteroaryl; und
(fff) Heterocycloalkyl; und
(c) C₃-C₇-Cycloalkyl;
(d) Aryl;
(e) substitiertem Aryl;
(f) Heteroaryl;
(g) substitiertem Heteroaryl; und
W ist fehlend:
wobei das Verfahren folgendes umfaßt:
(a) Behandlung einer Verbindung mit der Strukturformel: 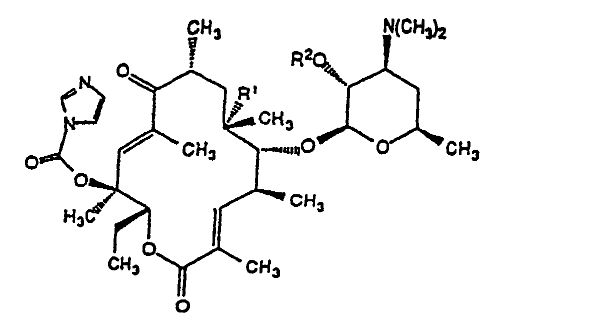
worin R¹ ist gewählt aus der Gruppe bestehend aus Wasserstoff, geschütztem Hydroxy, O-CO-C₁-C₆-Alkyl; O-C₁-C₁₂-Alkyl; O-CO-NH₂, O-CO-NH-CO-C₁-C₁₂-Alkyl, und O-CO-NH-SO₂-C₁-C₁₂-Alkyl; und R² ist eine Hydroxyschutzgruppe, mit einem primären Amin RNH₂, worin R wie oben defininiert ist, in einem geeigneten organischen Lösungsmittel bei Raumtemperatur bei Rückflusstemperatur für etwa 4 bis etwa 48 Stunden, Extraktion, wahlweise Schutzaufhebung, und Isolierung der gewünschten Verbindung.

9. Ein Verfahren gemäß Anspruch 8, worin R Wasserstoff ist, C₁-C₆-Alkyl, C₃-C₇-Cycloalkyl, Aryl, substituiertes Aryl, Heteroaryl oder substituiertes Heteroaryl und das Lösungsmittel ist gewählt aus der Gruppe bestehend aus Methylenchlorid, Tetrahydrofuran, N-Methyl-pyrrolidinon, Diethylether, bis-Methoxymethylether, Dimethylformamid, Acetonitril, Aceton und wässerigen Mischungen davon.

10. Ein Verfahren für die Herstellung einer Verbindung mit der Formel (I): 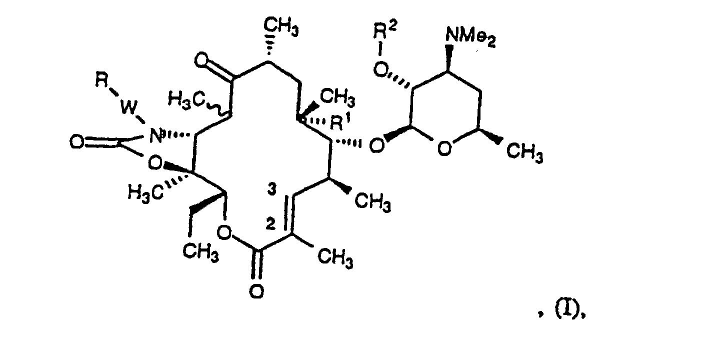 worin
R¹ gewählt ist aus der Gruppe bestehend aus:
(a) Wasserstoff;
(b) Hydroxy;
(c) O-C₁-C₁₂-Alkyl;
(d) O-CO-C₁-C₆-Alkyl;
(e) O-CO-NH₂;
(f) O-CO-NH-CO-C₁-C₁₂-Alkyl; und
(g) O-CO-NH-SO₂-C₁-C₁₂-Alkyl;
R² ist Wasserstoff oder eine Hydroxyschutzgruppe;
R ist gewählt aus der Gruppe bestehend aus:
(a) Wasserstoff;
(b) C₁-C₆-Alkyl wahlweise substituiert mit einem oder mehreren Substituenten gewählt aus der Gruppe bestehend aus:
(i) Aryl;
(ii) substituiertem Aryl;
(iii) Heteroaryl;
(iv) substituiertem Heteroaryl;
(v) Hydroxy;
(vi) C₁-C₆-Alkoxy;
(vii) NR³R⁴, worin R³ und R⁴ unabhängig gewählt sind aus Wasserstoff und C₁-C₆-Alkyl, oder R³ und R⁴ sind zusammengenommen mit dem Stickstoffatom an dem sie gebunden sind, um einen 3- bis 7-gliedrigen Ring zu bilden, welcher, falls der Ring ein 5- bis 7-gliedriger Ring ist, wahlweise eine Heterofunktion enthalten kann bestehend aus -O-, -NH-, -N(C₁-C₆-Alkyl-)-, -N (Aryl)-, -N (Aryl-C₁-C₆alkyl-)-, -N(substituiertes-Aryl-C₁-C₆-alkyl-)-, -N(Heteroaryl)-, -N(Heteroaryl-C₁-C₆-alkyl-)-, -N(substituiertes-Heteroaryl-C₁-C₆alkyl-)-, -S- oder -S(O)ₙ-, worin n 1 oder 2 ist;
(viii) -CH₂-M-R⁵,
worin M gewählt ist aus der Gruppe bestehend aus:
(aa) -C(O)-NH-;
(bb) -NH-C(O)-;
(cc) -NH-;
(dd) -N=;
(ee) -N(CH₃)-;
(ff) -O-;
(gg) -S(O)ₙ-, worin n 0, 1 oder 2 ist;
(hh) -CO-O-;
(ii) -O-CO-;
(jj) -CO-; und
R⁵ ist gewählt aus der Gruppe bestehend aus:
(aaa) C₁-C₆-Alkyl, wahlweise substituiert mit einem Substituenten gewählt aus der Gruppe bestehend aus:
(i) Aryl;
(ii) substituiertem Aryl;
(iii) Heteroaryl; und
(iv) substituiertem Heteroaryl;
(bbb) Aryl;
(ccc) substituiertem Aryl;
(ddd) Heteroaryl;
(eee) substituiertem Heteraryl;
und
(fff) Heterocycloalkyl; und
(c) C₃-C₇-Cycloalkyl;
(d) Aryl;
(e) substituiertem Aryl;
(f) Heteroaryl;
(g) substituiertem Heteroaryl; und
W ist gewählt aus der Gruppe bestehend aus -NH-CO-, -N=CH-, -NH- und -N(C₁-C₆-Alkyl)-;
wobei das Verfahren folgendes umfaßt:
(a) Behandlung einer Verbindung mit der Formel: 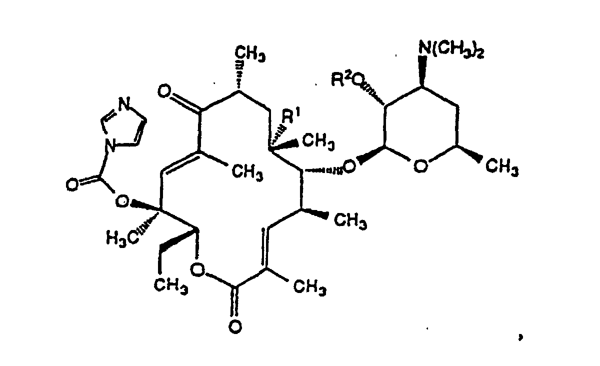 worin R¹ gewählt ist aus der Gruppe bestehend aus Wasserstoff, geschütztem Hydroxy, O-CO-C₁-C₆-Alkyl, O-C₁-C₁₂-Alkyl, O-CO-NH₂, O-CO-NH-CO-C₁-C₁₂-Alkyl oder O-CO-NH-SO₂-C₁-C₁₂-Alkyl; und R² ist eine Hydroxyschutzgruppe mit einem Reagenz gewählt aus der Gruppe bestehend aus Hydrazin, einem substituiertem Hydrazin, Hydroxylamin und O-substituiertem Hydroxylamin in einem geeigneten organischen Lösungsmittel bei Raumtemperatur bis Rückfluss für etwa 4 bis etwa 48 Stunden, um die gewünschte Verbindung zu liefern;
(b) wahlweise Acylieren der Verbindung aus Formel (I), die in Schritt (a) erzielt wurde, worin W -NH- ist und R ist H mit einem acylierenden Wirkstoff, um eine Verbindung gemäß Formel (I) zu ergeben, worin W -NH-CO- ist;
(c) wahlweise Kondensieren der Verbindung aus Formel (I), die in Schritt (a) erzielt wurde, worin W -NH- ist und R ist H mit einem Aldehyd, um eine Verbindung gemäß Formel (I) zu erzielen, worin W -N=CH- ist;
(d) wahlweise Reduzieren der Verbindung gemäß Formel (I), die in Schritt (c) erzielt wurde, worin W -N=CH- ist mit einem reduzierenden Wirkstoff, um eine Verbindung gemäß Formel (I) zu erzielen, worin W -NH- ist;
(e) und Extrahieren, wahlweise Aufheben des Schutzes, und Isolieren der gewünschten Verbindung.

11. Ein Verfahren gemäß Anspruch 10, worin das Lösungsmittel gewählt ist aus der Gruppe bestehend aus Methanol, Ethanol, Propanol, Isopropanol, Butanol, t-Butanol, Methylenchlorid, Tetrahydrofuran, N-Methyl-pyrrolidinon, Diethylether, bis-Methoxymethylether, Dimethylformamid, Aceton, wässeriges Acetonitril, wässeriges DMF und wässeriges Aceton.

12. Ein Verfahren gemäß Anspruch 10, worin das Produkt eine Verbindung von Formel (I) ist, worin W -NH- ist und R ist H und das Hydrazinreagenz ist Hydrazin.

13. Ein Verfahren gemäß Anspruch 10, worin das Produkt eine Verbindung von Formel (I) ist, worin W -N(C₁-C₆-Alkyl)- ist, das Hydrazinreagenz ist ein substitiertes Hydrazin RR⁴NNH₂, worin R wie definiert ist in Formel (I) und R⁴ ist C₁-C₆-Alkyl.

14. Ein Verfahren gemäß Anspruch 10, worin das Produkt eine Verbindung von Formel (I) ist, worin W -NH-CO- ist, das Hydrazinreagenz ist Hydrazin und das in Schritt (a) erhaltene Produkt mit der Formel (I), worin W -NH- ist und R ist H, wird behandelt mit einem R-Acyl acylierenden Mittel, worin R so ist wie für Formel (I) definiert.

15. Ein Verfahren gemäß Anspruch 10, worin das acylierende Mittel gewählt ist aus der Gruppe bestehend aus einem Säurechlorid, einem Säurefluorid, einem Säureanhydrid, einer Carbonsäure in Anwesenheit von Carbonyldiimidazol und einer Carbonsäure in Anwesenheit von 1-(3-Dimethylaminopropyl)-3-ethylcarbodiimidhydrochlorid.

16. Ein Verfahren gemäß Anspruch 10, worin das Produkt eine Verbindung von Formel (I) ist, worin W -N=CH- ist, die Hydrazinreagenz ist Hydrazin und das in Schritt (a) erhaltene Produkt mit der Strukturformel (I), worin W -NH- ist und R H ist, wird behandelt mit einem Aldehyd mit der Formel R-CHO,
worin R so ist wie für Formel (I) definiert.

17. Ein Verfahren gemäß Anspruch 10, worin das Produkt eine Verbindung gemäß Formel (I) ist, worin W -NH- ist und R ist nicht H, das Hydrazinreagenz ist Hydrazin, das in Schritt (a) erhaltene Produkt hat die Strukturformel (I), worin W -NH- ist und R ist H, und wird behandelt mit einem Aldehyd mit der Formel R-CHO, worin R so ist wie für Formel (I) definiert, und das in Schritt (c) erzielte Produkt mit der Strukturformel (I), worin W -N=CH- ist, wird behandelt mit einem reduzierenden Wirkstoff.

18. Ein Verfahren gemäß Anspruch 17, worin der reduzierende Wirkstoff gewählt ist aus der Gruppe bestehend aus Natriumcyanoborhydrid, Natriumborhydrid, Natriumtriacetoxyborhydrid, Boran-Tetrahydrofurankomplex und Boran-Piperidinkomplex.

19. Ein Verfahren für die Herstellung von einer Verbindung mit der Strukturformel (I): 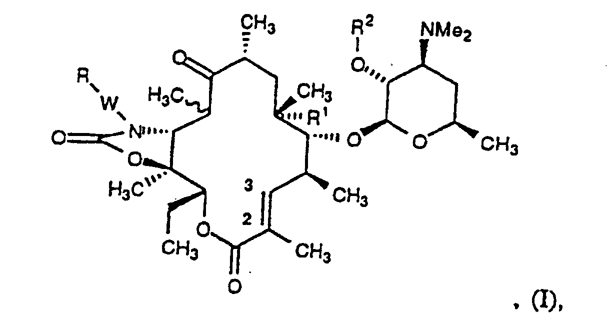 worin
R¹ gewählt ist aus der Gruppe bestehend aus:
(a) Wasserstoff;
(b) Hydroxy;
(c) O-C₁-C₁₂-Alkyl;
(d) O-CO-C₁-C₆-Alkyl;
(e) O-CO-NH₂;
(f) O-CO-NH-CO-C₁-C₁₂ -Alkyl; und
(g) O-CO-NH-SO₂-C₁-C₁₂-Alkyl;
R² ist Wasserstoff oder eine Hydroxyschutzgruppe;
R ist gewählt aus der Gruppe bestehend aus:
(a) Wasserstoff;
(b) C₁-C₆-Alkyl wahlweise substituiert mit einem oder mehreren Substituenten gewählt aus der Gruppe bestehend aus:
(i) Aryl;
(ii) substituiertem Aryl;
(iii) Heteroaryl;
(iv) substituiertem Heteroaryl;
(v) Hydroxy;
(vi) C₁-C₆-Alkoxy;
(vii) NR³R⁴, worin R³ und R⁴ unabhängig gewählt sind aus Wasserstoff und C₁-C₆-Alkyl, oder R³ und R⁴ sind zusammengenommen mit dem Stickstoffatom an dem sie gebunden sind, um einen 3- bis 7-gliedrigen Ring zu bilden, welcher, falls der Ring ein 5- bis 7-gliedriger Ring ist, wahlweise eine Heterofunktion enthalten kann bestehend aus -O-, -NH-, -N(C₁-C₆-Alkyl-) -, -N (Aryl) -, -N (Aryl-C₁-C₆alkyl-)-, -N(substituiertes-Aryl-C₁-C₆-alkyl-)-, -N(Heteroaryl)-, -N(Heteroaryl-C₁-C₆-alkyl-)-, -N(substituiertes-Heteroaryl-C₁-C₆alkyl-)-, -S- oder -S(O)ₙ-, worin n 1 oder 2 ist;
(viii) -CH₂-M-R⁵,
worin M gewählt ist aus der Gruppe bestehend aus:
(aa) -C(O)-NH-;
(bb) -NH-C(O)-;
(cc) -NH-;
(dd) -N=;
(ee) -N(CH₃)-;
(ff) -O-;
(gg) -S(O)ₙ-, worin n 0, 1 oder
2 ist;
(hh) -CO-O-;
(ii) -O-CO-;
(jj) -CO-; und
R⁵ ist gewählt aus der Gruppe bestehend aus:
(aaa) C₁-C₆-Alkyl, wahlweise substituiert mit einem Substituenten gewählt aus der Gruppe bestehend aus:
(i) Aryl;
(ii) substituiertem Aryl;
(iii) Heteroaryl; und
(iv) substituiertem Heteroaryl;
(bbb) Aryl;
(ccc) substituiertem Aryl;
(ddd) Heteroaryl;
(eee) substituiertem Heteraryl;
und
(fff) Heterocycloalkyl; und
(c) C₃-C₇-Cycloalkyl;
(d) Aryl;
(e) substituiertem Aryl;
(f) Heteroaryl;
(g) substituiertem Heteroaryl; und
W ist -O-;
wobei das Verfahren folgendes umfaßt:
(a) Behandlung einer Verbindung mit der Formel: 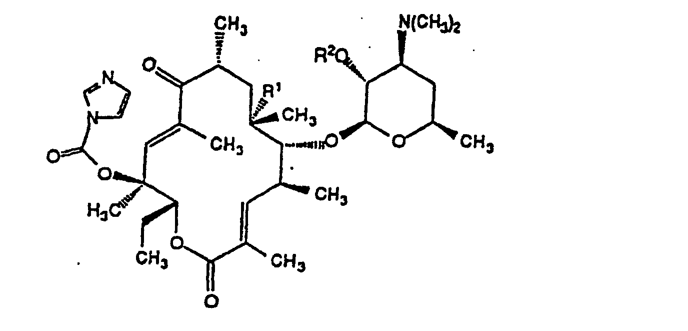 worin R¹ gewählt ist aus der Gruppe bestehend aus Wasserstoff, geschütztem Hydroxy, O-CO-C₁-C₆-Alkyl, O-C₁-C₁₂-Alkyl, O-CO-NH₂, O-CO-NH-CO-C₁-C₁₂-Alkyl oder O-CO-NH-SO₂-C₁-C₁₂-Alkyl; und R² ist eine Hydroxyschutzgruppe mit einem Hydroxylamin-Reagenz gewählt aus der Gruppe bestehend aus unsubstituiertem Hydroxylamin und einem O-C₁-C₆-alkylierten Hydroxylamin in einem geeigneten organischen Lösungsmittel bei Raumtemperatur bis Rückfluss für etwa 4 bis etwa 48 Stunden, um die gewünschte Verbindung zu liefern;
(b) wahlweise Behandlung des in Schritt (a) erzielten Produktes mit der Strukturformel (I) worin W -O- ist und R ist H mit einer Base und einem geeigneten Elektrophil mit der Formel R-L, worin R gewählt ist aus der Gruppe bestehend aus C₁-C₆-Alkyl, C₃-C₇-Cycloalkyl, Aryl, substituiertes Aryl, Heteroaryl und einer substituierten Heteroarylgruppe, wie für Verbindungen gemäß Formel (I) wie oben definiert und L ist eine geeignete Austrittsgruppe, um die gewünschte Verbindung gemäß Formel (I) zu ergeben, worin W -O- ist und R ist gewählt aus der Gruppe bestehend aus C₁-C₆-Alkyl, C₃-C₇-Cycloalkyl, Aryl, substituiertes Aryl, Heteroaryl und eine substituierte Heteroarylgruppe; und
(c) Extrahieren, wahlweise Schutzaufhebung und Isolieren der gewünschten Verbindung.

20. Ein Verfahren gemäß Anspruch 19, worin das Lösungsmittel gewählt ist aus der Gruppe bestehend aus Methanol, Ethanol, Propanol, Isopropanol, Butanol, t-Butanol, Methylenchlorid, Tetrahydrofuran, N-Methyl-pyrrolidinon, Diethylether, bis-Methoxymethylether, Dimethylformamid, Aceton, wässerigem Acetonitril, wässerigem DMF, und wässerigem Aceton.

21. Ein Verfahren gemäß Anspruch 20, worin das Produkt eine Verbindung von Formel (I) ist, worin W -O- ist und R ist H und das Hydroxylaminreagenz ist unsubstituiertes Hydroxylamin.

22. Ein Verfahren gemäß Anspruch 20, worin das Produkt eine Verbindung aus Formel (I) ist, worin W -O- ist und R ist O-C₁-C₆-Alkyl und das Hydroxylaminreagenz ist ein O-C₁-C₆-alkyliertes Hydroxylamin.

23. Ein Verfahren gemäß Anspruch 20, worin das endgültige Produkt eine Verbindung von Formel (I) ist, worin W -O- ist und R ist gewählt aus der Gruppe bestehend aus C₁-C₆-Alkyl, C₃-C₇-Cycloalkyl, Aryl, substituiertem Aryl, Heteroaryl oder einer substituierten Heteroarylgruppe, und das in Schritt (a) erhaltene Produkt mit der Formel (I), worin W -O- ist und R ist H, wird behandelt mit einer geeigneten Base und einem Elektrophil mit der Formel R-L, worin R wie oben definiert ist und L ist eine geeignete Austrittsgruppe.

24. Ein Verfahren gemäß Anspruch 22, worin die Base gewählt ist aus der Gruppe bestehend aus Natriumhydrid, Kaliumhydrid, Lithiumhydrid, Lithiumdiethylamid und Butyllithium und L ist gewählt aus der Gruppe bestehend aus Halid, Methansulfonyl und p-Toluensulfonyl.

25. Ein Verfahren für die Herstellung einer Verbindung mit der Strukturformel (II): 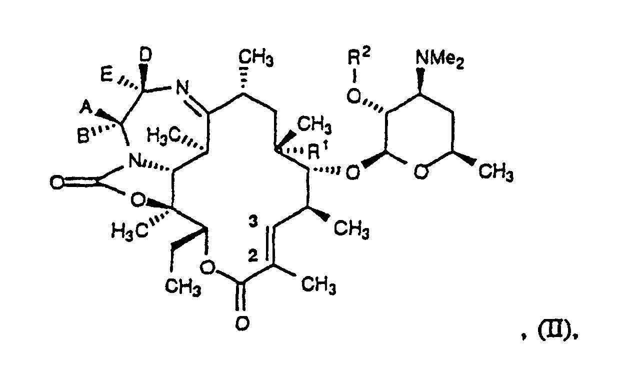 worin
R¹ ist gewählt aus der Gruppe bestehend aus:
(a) Wasserstoff;
(b) Hydroxy;
(c) O-C₁-C₁₂-Alkyl;
(d) O-CO-C₁-C₆-Alkyl;
(e) O-CO-NH₂;
(f) O-CO-NH-CO-C₁-C₁₂-Alkyl; und
(g) O-CO-NH-SO₂-C₁-C₁₂-Alkyl
R² ist Wasserstoff oder eine Hydroxyschutzgruppe;
A, B, D und E sind unabhängig gewählt aus der Gruppe bestehend aus:
(a) Wasserstoff;
(b) C₁-C₆-Alkyl wahlweise substituiert mit einem oder mehreren Substituenten gewählt aus der Gruppe bestehend aus:
(i) Aryl;
(ii) substituiertes Aryl;
(iii) Heteroaryl;
(iv) substituiertes Heteroaryl;
(v) Heterocycloalkyl; ;
(vi) Hydroxy;
(vii) C₁-C₆-Alkoxy;
(viii) Halogen bestehend aus Br, Cl, F oder
I; und
(ix) NR³R⁴, worin R³ und R⁴ unabhängig gewählt sind aus Wasserstoff und C₁-C₆-Alkyl, oder R³ und R⁴ sind zusammengenommen mit dem Stickstoffatom an dem sie gebunden sind, um einen 3- bis 7-gliedrigen Ring zu bilden, welcher, falls der Ring ein 5- bis 7-gliedriger Ring ist, wahlweise eine Heterofunktion enthalten kann bestehend aus -O-, -NH-, -N(C₁-C₆-Alkyl-)-, -N (Aryl) -, -N (Aryl-C₁-C₆alkyl-)-, -N(substituiertes-Aryl-C₁-C₆-alkyl-)-, -N(Heteroaryl)-, -N(Heteroaryl-C₁-C₆-alkyl-)-, -N(substituiertes-Heteroaryl-C₁-C₆alkyl-)-, -S- oder -S(O)ₙ-, worin n 1 oder 2 ist;
(c) C₃-C₇-Cycloalkyl;
(d) Aryl;
(e) substituiertem Aryl;
(f) Heteroaryl;
(g) substituiertem Heteroaryl;
(h) Heterocycloalkyl;
und
(i) eine Gruppe gewählt aus Option (b) wie oben, weiterhin substituiert mit -M-R⁵, worin M gewählt ist aus der Gruppe bestehend aus:
(aa) -C(O)-NH-;
(bb) -NH-C(O)-;
(cc) -NH-;
(dd) -N(CH₃)-;
(ee) -O-;
(ff) -S(O)ₙ-, worin n 0, 1 oder 2 ist;
(gg) -C(=NH)-NH-;
(hh) -CO-O-;
(ii) -O-CO-;
(jj) -CO-;
und R⁵ ist gewählt aus der Gruppe bestehend aus:
(aaa) C₁-C₆-Alkyl, wahlweise substituiert mit einem Substituenten gewählt aus der Gruppe bestehend aus:
(i) Aryl;
(ii) substituiertem Aryl;
(iii) Heteroaryl; und
(iv) substituiertem Heteroaryl;
(bbb) Aryl;
(ccc) substituiertem Aryl;
(ddd) Heteroaryl;
(eee) substituiertem Heteroaryl; und
(fff) Heterocycloalkyl;
oder ein beliebiges Paar Substituenten bestehend aus AB, AD, AE, BD, BE oder DE ist zusammengenommen mit dem Atom oder den Atomen, an welche sie angeheftet sind, um einen 3- bis 7-gliedrigen Ring zu bilden, welcher wahlweise eine Heterofunktion enthält, bestehend aus:
-O-,
-NH-,
-N(C₁-C₆-Alkyl-)-,
-N(Aryl-C₁-C₆-alkyl-)-,
-N(substituiertes-Aryl-C₁-C₆-alkyl-)-,
-N(Heteroaryl-C₁-C₆-alkyl-)-,
-N(substituiertes-Heteroaryl-C₁-C₆-alkyl-)-,
-S- oder -S(O)ₙ-, worin n 1 oder 2 ist,
-C(O)-NH-,
-C(O)-NR⁵-, worin R⁵ so ist wie oben definiert;
-NH-C(O)-,
-NR⁵-C(O)-, worin R⁵ so ist wie oben definiert; und
-C(=NH)-NH-;
wobei das Verfahren folgendes umfaßt:
(a) Behandlung einer Verbindung mit der Formel: 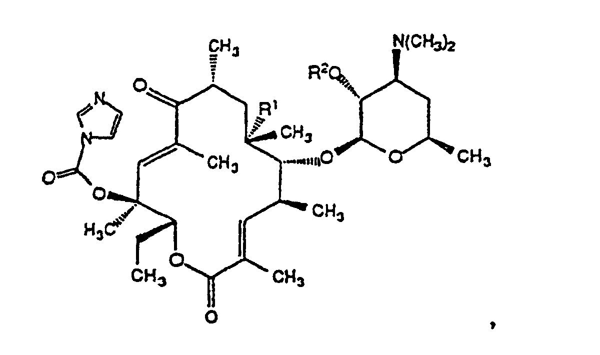 worin R¹ gewählt ist aus der Gruppe bestehend aus Wasserstoff, geschütztem Hydroxy, O-C₁-C₁₂-Alkyl, O-CO-C₁-C₆-Alkyl, O-CO-NH₂, O-CO-NH-CO-C₁-C₁₂-Alkyl, oder O-CO-NH-SO₂-C₁-C₁₂-Alkyl; und R² ist eine Hydroxyschutzgruppe, mit einer Verbindung mit der Formel: 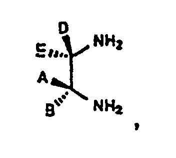 worin A, B, D und E so sind wie für die Verbindungen gemäß Formel (I) oben definiert, in einem geeigneten Lösungsmittel bei Raumtemperatur bis Rückflusstemperatur für etwa 4 bis etwa 48 Stunden, um die bicyclische intermediäre Verbindung mit der Formel zu ergeben: 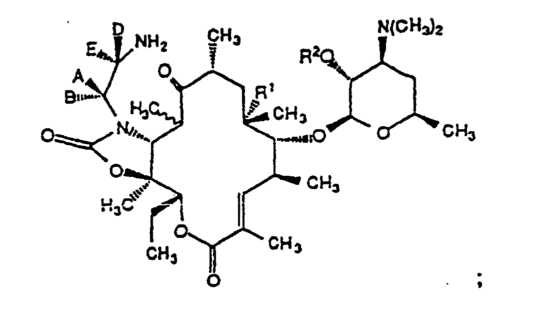
(b) Schutzaufhebung der bicyclischen intermediären Verbindungen, um die zweiten intermediären Verbindung zu ergeben, welche die Strukturformel besitzen; 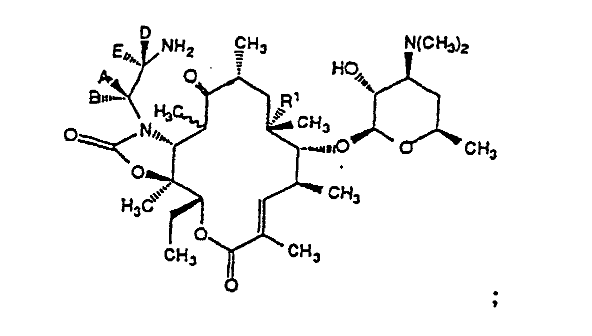
(c) Zyklisieren der zweiten intermediären Verbindungen durch Behandlung mit verdünnter Konzentration einer starken Säure in einem geeigneten organischen Lösungsmittel für einen Zeitraum von etwa 4 Stunden bis etwa 10 Tage bei einer Temperatur von Raumtemperatur bis Rückflusstemperatur des Lösungsmittels, um die gewünschten Verbindungen zu ergeben; und
(d) Extraktion, wahlweise Schutzaufhebung und Isolierung der gewünschten Verbindung.

26. Ein Verfahren gemäß Anspruch 25, worin in Schritt (a) das Lösungsmittel gewählt ist aus der Gruppe bestehend aus Methanol, Ethanol, Propanol, Isopropanol, Butanol, t-Butanol, Methylenchlorid, Tetrahydrofuran, N-Methyl-pyrrolidinon, Diethylether, bis-Methoxymethylether, Dimethylformamid, Aceton, wässerigem Acetonitril, wässerigem DMF, und wässerigem Aceton; und in Schritt (c) ist das Lösungsmittel gewählt aus der Gruppe bestehend aus Methanol, Ethanol, Propanol, iso-Propanol, Butanol, iso-Butanol und t-Butanol.

27. Ein Verfahren für die Herstellung einer Verbindung mit der Formel (II): 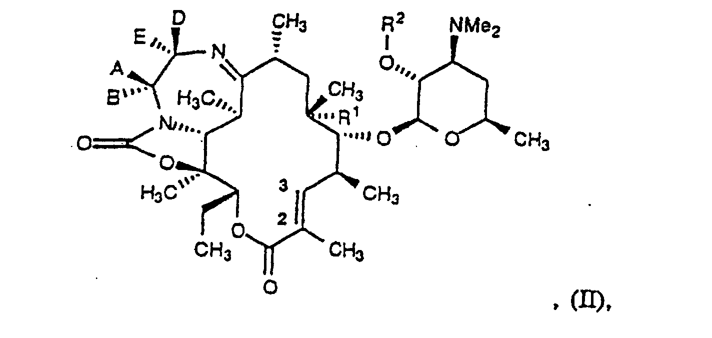 worin
R¹ gewählt ist aus der Gruppe bestehend aus:
(a) Wasserstoff;
(b) Hydroxy;
(c) O-C₁-C₁₂-Alkyl;
(d) O-CO-C₁-C₆-Alkyl;
(e) O-CO-NH₂;
(f) O-CO-NH-CO-C₁-C₁₂-Alkyl; und
(g) O-CO-NH-SO₂-C₁-C₁₂-Alkyl;
R² ist Wasserstoff oder eine Hydroxyschutzgruppe;
A, B, D und E sind unabhängig gewählt aus der Gruppe bestehend aus:
(a) Wasserstoff;
(b) C₁-C₆-Alkyl wahlweise substituiert mit einem oder mehreren Substituenten gewählt aus der Gruppe bestehend aus:
(i) Aryl;
(ii) substituiertem Aryl;
(iii) Heteroaryl;
(iv) substituiertem Heteroaryl;
(v) Heterocycloalkyl;
(vi) Hydroxy;
(vii) C₁-C₆-Alkoxy;
(viii) Halogen bestehend aus Br, Cl, F oder I; und
(ix) NR³R⁴, worin R³ und R⁴ unabhängig gewählt sind aus Wasserstoff und C₁-C₆-Alkyl, oder R³ und R⁴ sind zusammengenommen mit dem Stickstoffatom an dem sie gebunden sind, um einen 3- bis 7-gliedrigen Ring zu bilden, welcher, falls der Ring ein 5- bis 7-gliedriger Ring ist, wahlweise eine Heterofunktion enthalten kann bestehend aus -O-, -NH-, -N(C₁-C₆-Alkyl-)-, -N (Aryl) -, -N (Aryl-C₁-C₆alkyl-)-, -N(substituiertes-Aryl-C₁-C₆-alkyl-)-, -N (Heteroaryl) -, -N(Heteroaryl-C₁-C₆-alkyl-)-, -N(substituiertes-Heteroaryl-C₁-C₆alkyl-)-, -S- oder -S(O)ₙ-, worin n 1 oder 2 ist;
(c) C₃-C₇-Cycloalkyl;
(d) Aryl;
(e) substituiertem Aryl;
(f) Heteroaryl;
(g) substituiertem Heteroaryl;
(h) Heterocycloalkyl;
und
(i) eine Gruppe gewählt aus Option (b) wie oben, weiterhin substituiert mit -M-R⁵, worin M gewählt ist aus der Gruppe bestehend aus:
(aa) -C(O)-NH-;
(bb) -NH-C(O)-;
(cc) -NH-;
(dd) -N(CH₃)-;
(ee) -O-;
(ff) -S(O)ₙ-, worin n 0, 1 oder 2 ist;
(gg) -C(=NH)-NH-;
(hh) -CO-O-;
(ii) -O-CO-;
(jj) -CO-:
und R⁵ ist gewählt aus der Gruppe bestehend aus:
(aaa) C₁-C₆-Alkyl, wahlweise substituiert mit einem Substituenten gewählt aus der Gruppe bestehend aus:
(i) Aryl;
(ii) substituiertem Aryl;
(iii) Heteroaryl; und
(iv) substituiertem Heteroaryl;
(bbb) Aryl;
(ccc) substituiertem Aryl;
(ddd) Heteroaryl;
(eee) substituiertem Heteraryl; und
(fff) Heterocycloalkyl;
oder ein beliebiges Paar Substituenten bestehend aus AB, AD, AE, BD, BE oder DE ist zusammengenommen mit dem Atom oder den Atomen, an welche sie angeheftet sind, um einen 3- bis 7-gliedrigen Ring zu bilden, welcher wahlweise eine Heterofunktion enthält, bestehend aus:
-O-,
-NH-,
-N(C₁-C₆-Alkyl-)-,
-N(Aryl-C₁-C₆-alkyl-)-,
-N(substituiertes-Aryl-C₁-C₆-alkyl-)-,
-N(Heteroaryl-C₁-C₆-alkyl-)-,
-N(substituiertes-Heteroaryl-C₁-C₆-alkyl-)-,
-S- oder -S(O)ₙ-, worin n 1 oder 2 ist,
-C(O)-NH-,
-C(O)-NR⁵-, worin R⁵ so ist wie oben definiert;
-NH-C(O)-,
-NR⁵-C(O)-, worin R⁵ so ist wie oben definiert; und
-C(=NH)-NH-;
wobei das Verfahren folgendes umfaßt:
(a) Behandlung einer Verbindung mit der Formel: 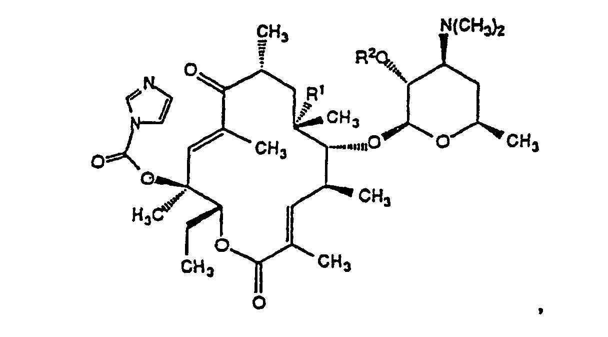 worin R¹ gewählt ist aus der Gruppe bestehend aus Wasserstoff, geschütztem Hydroxy, O-C₁-C₁₂-Alkyl, O-CO-C₁-C₆-Alkyl, O-CO-NH₂, O-CO-NH-CO-C₁-C₁₂-Alkyl, oder O-CO-NH-SO₂-C₁-C₁₂-Alkyl; und R² ist eine Hydroxyschutzgruppe mit einer Verbindung mit der Formel: 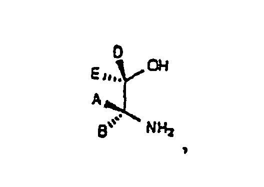 worin A, B, D und E so sind wie oben definiert, in einem geeigneten Lösungsmittel bei 0 - 70°C für etwa 4 bis etwa 48 Stunden, um die bicyclische intermediäre Verbindung mit der Formel: 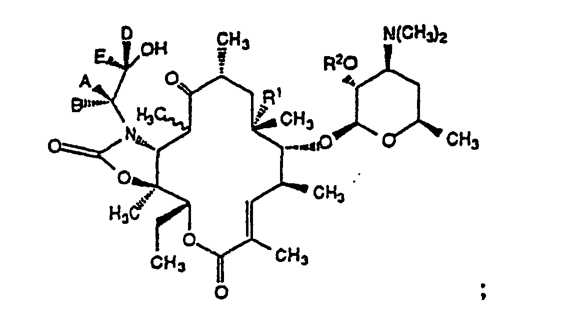
(b) Behandlung der bicyclischen intermediären Verbindung aus Schritt (a) mit Triphenylphosphin und Diphenylphosphoryl Azid-Diethylazodicarboxylat in Tetrahydrofuran unter Mitsunobu Reaktionsbedingungen, um die zweite intermediäre Azidverbindung mit der Strukturformel herzustellen: 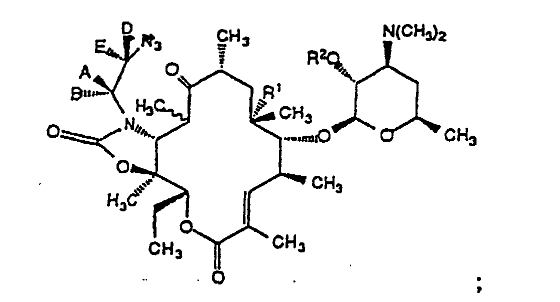
(c) Reduzieren der zweiten intermediären Azidverbindung, um eine dritte intermediäre Verbindung herzustellen mit der Formel: 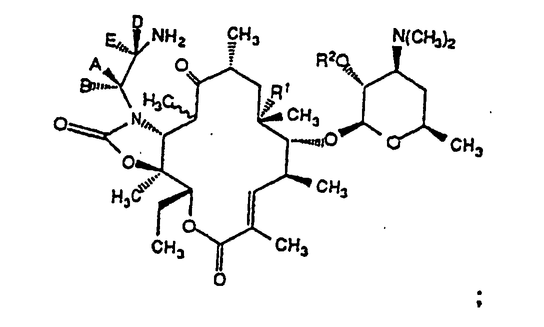
(d) Zyklisieren der dritten intermediären Verbindung durch Behandlung mit einer verdünnten Konzentration einer starken Säure bei Raumtemperatur bis Rückflusstemperatur für etwa 4 Stunden bis etwa 10 Tage in einem wässerigen Alkohollösungsmittel um die gewünschten Verbindungen zu ergeben; und
(e) Extraktion, wahlweise Schutzaufhebung, und Isolierung der gewünschten Verbindung.

28. Ein Verfahren gemäß Anspruch 27, worin in Schritt (a) das Lösungsmittel gewählt ist aus der Gruppe bestehend aus Methanol, Ethanol, Propanol, Isopropanol, Butanol, t-Butanol, Methylenchlorid, Tetrahydrofuran, N-Methyl-pyrrolidinon, Diethylether, bis-Methoxymethylether, Dimethylformamid, Aceton, wässeriges Acetonitril, wässeriges DMF, und wässeriges Aceton; in Schritt (c) ist das reduzierende Mittel gewählt aus der Gruppe bestehend aus Triphenylphosphin-Wasser, Wasserstoff mit einem Katalysator, Natriumborhydrid und Dialkylaluminiumhydrid; und in Schritt (d) ist das Lösungsmittel gewählt aus der Gruppe bestehend aus Methanol, Ethanol, Propanol, iso-Propanol, Butanol, iso-Butanol und t-Butanol.

29. Ein Verfahren wie in Anspruch 27, worin Schritt (b) davon ersetzt wird durch zwei Schritte bestehend aus:
(b') Umsetzen der Hydroxygruppe der bicyclischen intermediären Verbindung mit einem sulfonierenden Wirkstoff gewählt aus der Gruppe bestehend aus Sulfonylchlorid, Alkylsulfonanhydrid, Arylsulfonanhydrid und Trifluormethansulfonanhydrid, in einem aprotischen Lösungsmittel bei -78°C bis Raumtemperatur, um eine intermediäre Verbindung zu ergeben, worin die Hydroxylgruppe durch einen Sulfonatesteranteil ersetzt wurde; und
(b'') Umsetzen des Sulfonatesters aus Schritt (b') mit einem Alkalimetallazid in einem aprotischen Lösungsmittel bei von etwa 0°C bis etwa 100°C, um die zweite intermediäre Azidverbindung zu ergeben.

30. Ein Verfahren für die Herstellung einer Verbindung mit der Formel (III): 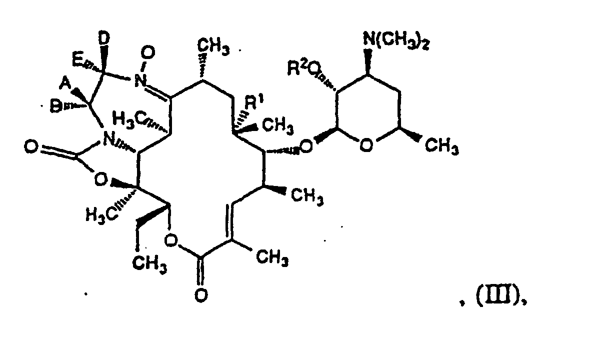 worin
A, B, D und E unabhängig gewählt sind aus der Gruppe bestehend aus:
(a) Wasserstoff;
(b) C₁-C₆-Alkyl wahlweise substituiert mit einem oder mehreren Substituenten gewählt aus der Gruppe bestehend aus:
(i) Aryl;
(ii) substituiertem Aryl;
(iii) Heteroaryl;
(iv) substituiertem Heteroaryl;
(v) Heterocycloalkyl;
(vi) Hydroxy;
(vii) C₁-C₆-Alkoxy;
(viii) Halogen bestehend aus Br, Cl, F oder I; und
(ix) NR³R⁴, worin R³ und R⁴ unabhängig gewählt sind aus Wasserstoff und C₁-C₆-Alkyl, oder R³ und R⁴ sind zusammengenommen mit dem Stickstoffatom an dem sie gebunden sind, um einen 3- bis 7-gliedrigen Ring zu bilden, welcher, falls der Ring ein 5- bis 7-gliedriger Ring ist, wahlweise eine Heterofunktion enthalten kann bestehend aus -O-, -NH-, -N(C₁-C₆-Alkyl-)-, -N (Aryl) -, -N (Aryl-C₁-C₆alkyl-)-, -N(substituiertes-Aryl-C₁-C₆-alkyl-) -, -N (Heteroaryl)-, -N(Heteroaryl-C₁-C₆-alkyl-)-, -N(substituiertes-Heteroaryl-C₁-C₆alkyl-)-, -S- oder -S(O)ₙ-, worin n 1 oder 2 ist;
(c) C₃-C₇-Cycloalkyl;
(d) Aryl;
(e) substituiertem Aryl;
(f) Heteroaryl;
(g) substituiertem Heteroaryl;
(h) Heterocycloalkyl;
und
(i) einer Gruppe gewählt aus Option (b) wie oben, weiterhin substituiert mit -M-R⁵, worin M gewählt ist aus der Gruppe bestehend aus:
(aa) -C(O)-NH-;
(bb) -NH-C(O)-;
(cc) -NH-;
(dd) -N(CH₃)-;
(ee) -O-;
(ff) -S(O)ₙ-, worin n 0, 1 oder 2 ist;
(gg) -C(=NH)-NH-;
und R⁵ ist gewählt aus der Gruppe bestehend aus:
(aaa) C₁-C₆-Alkyl, wahlweise substituiert mit einem Substituenten gewählt aus der Gruppe bestehend aus:
(i) Aryl;
(ii) substituiertem Aryl;
(iii) Heteroaryl; und
(iv) substituiertem Heteroaryl;
(bbb) Aryl;
(ccc) substituiertem Aryl;
(ddd) Heteroaryl;
(eee) substituiertem Heteroaryl; und
(fff) Heterocycloalkyl;
oder
ein beliebiges Paar Substituenten bestehend aus AB, AD, AE, BD, BE oder DE ist zusammengenommen mit dem Atom oder den Atomen, an welche sie angeheftet sind, um einen 3- bis 7-gliedrigen Ring zu bilden, welcher wahlweise eine Heterofunktion enthält, bestehend aus:
-O-,
-NH-,
-N(C₁-C₆-Alkyl-)-,
-N(Aryl-C₁-C₆-alkyl-)-,
-N(substituiertes-Aryl-C₁-C₆-alkyl-)-,
-N(Heteroaryl-C₁-C₆-alkyl-)-,
-N(substituiertes-Heteroaryl-C₁-C₆-alkyl-)-,
-S- oder -S(O)ₙ-, worin n 1 oder 2 ist,
-C(O)-NH-,
-C(O)-NR⁵-, worin R⁵ so ist wie oben definiert;
-NH-C(O)-,
-NR⁵-C(O)-, worin R⁵ so ist wie oben definiert; und
-C(=NH)-NH-;
R¹ ist gewählt aus der Gruppe bestehend aus:
(a) Wasserstoff;
(b) Hydroxy;
(c) O-C₁-C₁₂-Alkyl;
(d) O-CO-C₁-C₆-Alkyl;
(e) O-CO-NH₂;
(f) O-CO-NH-CO-C₁-C₁₂-Alkyl; und
(g) O-CO-NH-SO₂-C₁-C₁₂-Alkyl;
R² ist Wasserstoff oder eine Hydroxyschutzgruppe;
wobei das Verfahren folgendes umfaßt:
(a) Behandlung einer Verbindung mit der Formel: 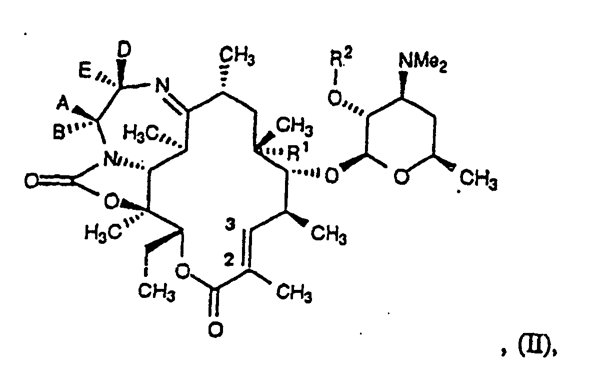 worin R¹ so ist wie oben definiert oder eine Hydroxyschutzgruppe ist und R², A, B, D und E sind so wie oben definiert, mit einem geeigneten oxidierenden Wirkstoff, um den Iminstickstoff an das Nitron und das Stickstoffatom an den Desosaminanteil des N-Oxids zu oxidieren, um einen N-oxidierten Zwischenstoff zu ergeben; und
(b) Behandlung des N-oxidierten Zwischenstoffes mit einem reduzierenden Wirkstoff, um das Desosamin N-Oxid zu reduzieren, und Extraktion, wahlweise Schutzaufhebung, und Isolierung der gewünschten Verbindung.

31. Ein Verfahren gemäß Anspruch 30, worin in Schritt (a) der oxidierende Wirkstoff gewählt ist aus der Gruppe bestehend aus Wasserstoffperoxid und einem Carbonsäureperacid; und in Schritt (b) ist der reduzierende Wirkstoff gewählt aus der Gruppe bestehend aus Triphenylphosphin und Wasserstoff in Anwesenheit eines Katalysators.

32. Ein Verfahren für die Herstellung einer Verbindung mit der Strukturformel (IV): 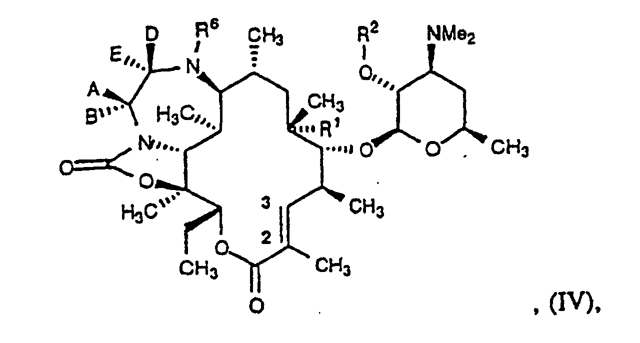 worin
A, B, D und E unabhängig gewählt sind aus der Gruppe bestehend aus:
(a) Wasserstoff;
(b) C₁-C₆-Alkyl wahlweise substituiert mit einem oder mehreren Substituenten gewählt aus der Gruppe bestehend aus:
(i) Aryl;
(ii) substituiertem Aryl;
(iii) Heteroaryl;
(iv) substituiertem Heteroaryl;
(v) Heterocycloalkyl;
(vi) Hydroxy;
(vii) C₁-C₆-Alkoxy;
(viii) Halogen bestehend aus Br, Cl, F oder I; und
(ix) NR³R⁴, worin R³ und R⁴ unabhängig gewählt sind aus Wasserstoff und C₁-C₆-Alkyl, oder R³ und R⁴ sind zusammengenommen mit dem Stickstoffatom an dem sie gebunden sind, um einen 3- bis 7-gliedrigen Ring zu bilden, welcher, falls der Ring ein 5- bis 7-gliedriger Ring ist, wahlweise eine Heterofunktion enthalten kann bestehend aus -O-, -NH-, -N(C₁-C₆-Alkyl-) -, -N (Aryl) -, -N (Aryl-C₁-C₆alkyl-)-, -N(substituiertes-Aryl-C₁-C₆-alkyl-)-, -N(Heteroaryl)-, -N(Heteroaryl-C₁-C₆-alkyl-)-, -N(substituiertes-Heteroaryl-C₁-C₆alkyl-)-, -S- oder -S(O)ₙ-, worin n 1 oder 2 ist;
(c) C₃-C₇-Cycloalkyl;
(d) Aryl;
(e) substituiertem Aryl;
(f) Heteroaryl;
(g) substituiertem Heteroaryl;
(h) Heterocycloalkyl;
und
(i) eine Gruppe gewählt aus Option (b) wie oben, weiterhin substituiert mit -M-R⁵, worin M gewählt ist aus der Gruppe bestehend aus:
(aa) -C(O)-NH-;
(bb) -NH-C(O)-;
(cc) -NH-;
(dd) -N(CH₃)-;
(ee) -O-;
(ff) -S(O)ₙ-, worin n 0, 1 oder 2 ist; und
(gg) -C(=NH)-NH-;
und R⁵ ist gewählt aus der Gruppe bestehend aus:
(aaa) C₁-C₆-Alkyl, wahlweise substituiert mit einem Substituenten gewählt aus der Gruppe bestehend aus:
(i) Aryl;
(ii) substituiertem Aryl;
(iii) Heteroaryl; und
(iv) substituiertem Heteroaryl;
(bbb) Aryl;
(ccc) substituiertem Aryl;
(ddd) Heteroaryl;
(eee) substituiertem Heteraryl; und
(fff) Heterocycloalkyl;
oder ein beliebiges Paar Substituenten bestehend aus AB, AD, AE, BD, BE oder DE ist zusammengenommen mit dem Atom oder den Atomen, an welche sie angeheftet sind, um einen 3- bis 7-gliedrigen Ring zu bilden, welcher wahlweise eine Heterofunktion enthält, bestehend aus:
-O-,
-NH-,
-N(C₁-C₆-Alkyl-)-,
-N(Aryl-C₁-C₆-alkyl-)-,
-N(substituiertes-Aryl-C₁-C₆-alkyl-)-,
-N(Heteroaryl-C₁-C₆-alkyl-)-,
-N(substituiertes-Heteroaryl-C₁-C₆-alkyl-)-,
-S- oder -S(O)ₙ-, worin n 1 oder 2 ist,
-C(O)-NH-,
-C(O)-NR⁵-, worin R⁵ so ist wie oben definiert;
-NH-C(O)-,
-NR⁵-C(O)-, worin R⁵ so ist wie oben definiert; und
-C(=NH)-NH-;
R¹ ist gewählt aus der Gruppe bestehend aus:
(a) Wasserstoff;
(b) Hydroxy;
(c) O-C₁-C₁₂-Alkyl;
(d) O-CO-C₁-C₆-Alkyl;
(e) O-CO-NH₂;
(f) O-CO-NH-CO-C₁-C₁₂-Alkyl; und
(g) O-CO-NH-SO₂-C₁-C₁₂-Alkyl;
R² ist Wasserstoff oder eine Hydroxyschutzgruppe;
R⁶ ist Wasserstoff oder C₁-C₆-Alkyl;
wobei das Verfahren folgendes umfaßt:
(a) Reagieren einer Verbindung mit der Formel: 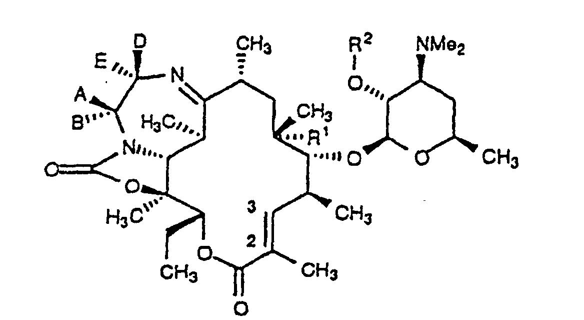 worin R¹ so ist wie oben oder eine Hydroxyschutzgruppe ist und R², A, B, D und E so sind wie oben definiert, mit einem reduzierenden Wirkstoff in einem geeigneten organischen Lösungsmittel, um die gewünschte Verbindung zu liefern, worin R⁶ H ist;
(b) wahlweise reduktives Alkylieren des Amino des Produktes aus Schritt (a) mit einem reduzierenden Reagenz in Anwesenheit eines C₁-C₆-Alkylgruppenvorläufers, um die gewünschte Verbindung zu ergeben, worin R⁶ C₁-C₆-Alkyl ist; und
(c) Extraktion, wahlweise Schutzaufhebung, und Isolierung der gewünschten Verbindung.

33. Ein Verfahren gemäß Anspruch 31, worin in Schritt (a) und in optionalen Schritt (b) der reduzierende Wirkstoff gewählt ist aus der Gruppe bestehend aus Natriumcyanoborhydrid, Natriumborhydrid, Natriumtriacetoxyborhydrid, Boran-Tetrahydrofurankomplex und Boran-Piperidinkomplex.

34. Eine Verbindung mit der Strukturformel: 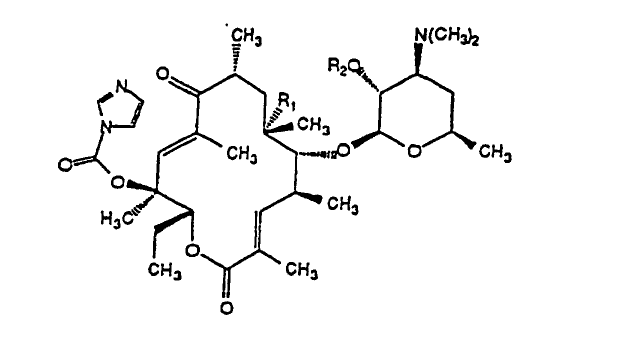 worin
R¹ gewählt ist aus der Gruppe bestehend aus Wasserstoff, geschütztem Hydroxy, O-C₁-C₁₂-Alkyl, O-CO-C₁-C₆-Alkyl, O-CO-NH₂, O-CO-NH-CO-C₁-C₁₂-Alkyl oder O-CO-NH-SO₂-C₁-C₁₂-Alkyl; und
R² ist Wasserstoff oder eine Hydroxyschutzgruppe.

35. Die Verbindung gemäß Anspruch 33, worin R¹ O-C₁-C₁₂-Alkyl ist.

36. Die Verbindung gemäß Anspruch 34, worin R¹ Methoxy ist.

37. Ein Verfahren für die Herstellung einer Verbindung mit der Strukturformel: 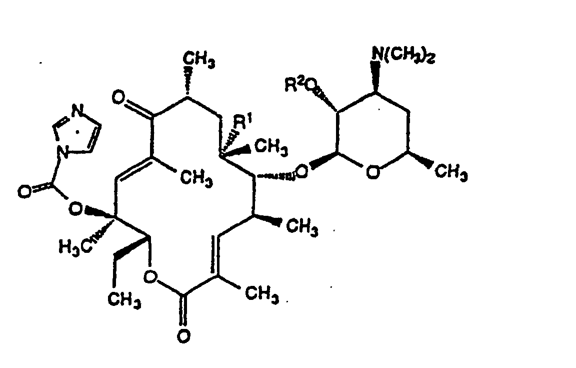 worin
R¹ gewählt ist aus der Gruppe bestehend aus:
(a) Wasserstoff;
(b) geschütztem Hydroxy;
(c) O-C₁-C₁₂-Alkyl;
(d) O-CO-C₁-C₆-Alkyl;
(e) O-CO-NH₂;
(f) O-CO-NH-CO-C₁-C₁₂-Alkyl; und
(g) O-CO-NH-SO₂-C₁-C₁₂-Alkyl; und
R² ist Wasserstoff oder eine Hydroxyschutzgruppe;
wobei das Verfahren folgendes umfaßt:
(a) Behandlung einer Erythromycin A Verbindung mit der Formel: 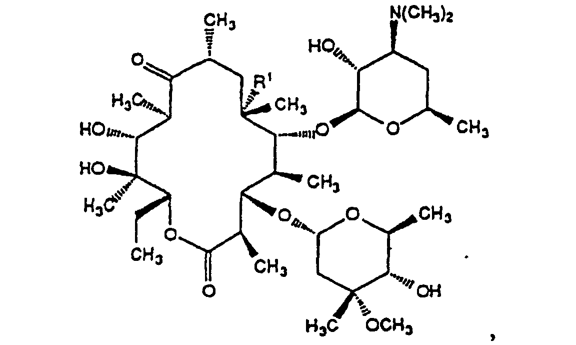 worin R¹ so ist wie oben definiert, mit dehydrierenden Reagenzien, bestehend aus einem Organcarbonat in der Anwesenheit einer Base bei Rückflußtemperatur in einem aprotischen Lösungsmittel, um eine Intermediatverbindung mit der Formel zu bilden: 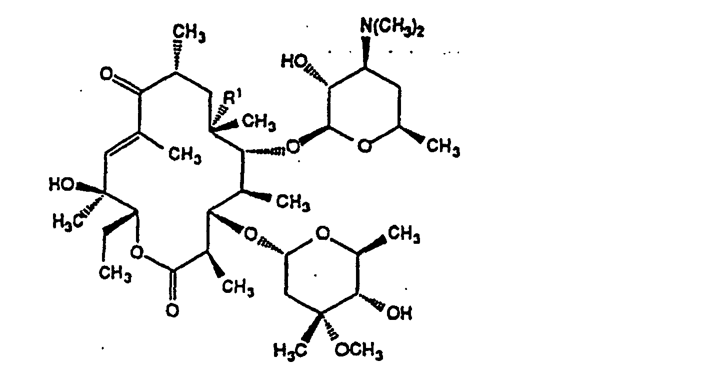
(b) hydrolytische Entfernung des Cladinoseanteils aus der intermediären Verbindung aus Schritt (a) durch Behandlung in einer wässerigen Alkoholsuspension mit einer verdünnten Konzentration einer starken Säure bei Raumtemperatur für etwa 0,5 bis etwa 24 Stunden Extraktion und wahlweise Isolierung der Verbindung mit der Strukturformel: 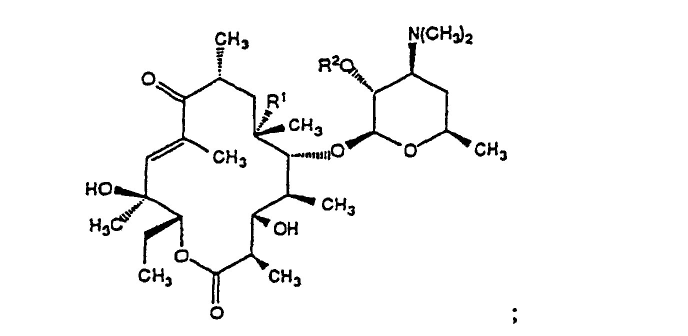
(c) Behandlung der Verbindung aus Schritt (b) mit einem geeigneten Hydroxygruppen Schutzreagenz in einem aprotischen Lösungsmittel, und die extraktive Isolierung der Verbindung,
worin R² eine Hydroxyschutzgruppe ist;
(d) Behandlung einer Lösung der Verbindung aus Schritt (c) mit einem sulfonylierenden Reagenz bei von etwa 0°C bis Raumtemperatur für etwa 1 bis etwa 24 Stunden und extraktive Isolierung der Verbindung mit der Strukturformel: 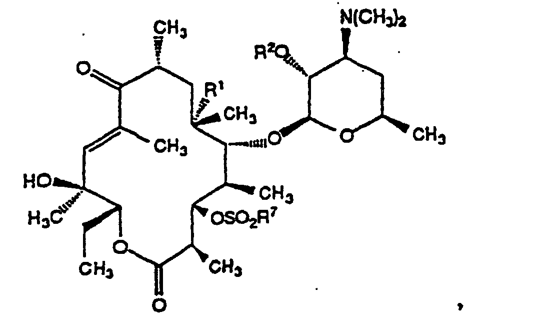 worin R⁷ Alkyl oder Aryl ist;
(e) Dehydrieren der Verbindung aus Schritt (d) mit einer Hydridbase in Anwesenheit von Carbonyldiimidazol in einem aprotischen Lösungsmittel bei einer Temperatur von etwa -20°C bis etwa 70°C für von etwa 0,5 Stunden bis etwa 10 Tagen und Extraktion, wahlweise Schutzaufhebung, und Isolierung der gewünschten Verbindung.

38. Ein Verfahren gemäß Anspruch 36, worin in Schritt (a) die dehydrierenden Reagenzen bestehen aus einer Organocarbonatverbindung gewählt aus der Gruppe bestehend aus Ethylencarbonat, Propylencarbonat, Trimethylencarbonat, Dipropylcarbonat, Dibenzylcarbonat, Isobutylcarbonat, Dimethylcarbonat und Diethylcarbonat in der Anwesenheit von einer Base gewählt aus der Gruppe bestehend aus Triethylamin, Diisopropylethylamin, Pyridin, 2,6-Dimethylpyrimidin, 1,8-Diazabicyclo[5.4.0]undec-7-en, N-Methylmorpholin, N-Methylpyrrolidin, N-Methylpiperidin, Natriumcarbonat, Kaliumcarbonat, Natriumbicarbonat und Kaliumcarbonat; in Schritt (b) ist der Alkohol gewählt aus der Gruppe bestehend aus Methanol, Ethanol, Propanol, iso-Propanol, Butanol, iso-Butanol und t-Butanol, und die Säure ist gewählt aus der Gruppe bestehend aus Salzsäure, Schwefelsäure, Dichloressigsäure und Trichloressigsäure; in Schitt (c) ist das Hydroxygruppen Schutzreagenz gewählt aus der Gruppe bestehend aus Acetylchlorid, Essigsäureanhydrid, Benzoesäureanhydrid, Benzylchlorformat, Trimethylsilylchlorid und Triethylsilylchlorid und das aprotische Lösungsmittel ist gewählt aus der Gruppe bestehend aus Methylenchlorid, Chloroform, Dimethylformamid, Tetrahydrofuran, N-Methylpyrrolidinon und Mischungen davon; in Schritt (d) ist das sulfonylierende Mittel gewählt aus der Gruppe bestehend aus Methansulfonylanhydrid, Methansulfonylchlorid, Ethansulfonylchlorid und p-Toluensulfonylchlorid und die Base ist gewählt aus der Gruppe definiert in Schritt (a) wie oben; in Schritt (e) ist die Hydridbase gewählt aus der Gruppe bestehend aus Natriumhydrid, Kaliumhydrid und Lithiumhydrid und das aprotische Lösungsmittel ist wie definiert für Schritt (c).

39. Ein Verfahren gemäß Anspruch 37, worin R¹ H ist und die Schritte (d) und (e) werden ersetzt durch einen einzelnen Schritt (d') bestehend aus (d') Behandlung der Verbindung aus Schritt (c) mit Natriumhexamethyldisilazan bei von etwa -50 bis etwa -28°C unter einer inerten Atmosphäre gefolgt durch den Zusatz von Carbonyldiimidazol bei von etwa 0°C bis etwa Ratumtemperatur für etwa 15 Minuten bis etwa 6 Stunden und Extraktion, wahlweise Schutzaufhebung, und Isolierung der gewünschten Verbindung.

40. Ein Verfahren für die Herstellung einer Verbindung mit der Strukturformel: 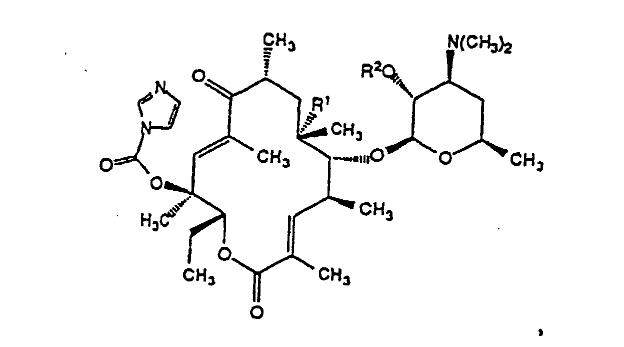 worin
R¹ gewählt ist aus der Gruppe bestehend aus:
(a) Wasserstoff;
(b) geschütztem Hydroxy;
(c) O-C₁-C₁₂-Alkyl;
(d) O-CO-C₁-C₆-Alkyl;
(e) O-CO-NH₂;
(f) O-CO-NH-CO-C₁-C₁₂-Alkyl; und
(g) O-CO-NH-SO₂-C₁-C₁₂-Alkyl; und
R² ist Wasserstoff oder eine Hydroxyschutzgruppe;
wobei das Verfahren folgendes umfaßt:
(a) Hydrolytische Entfernung des Cladinoseanteils aus einer Erythromycin A Verbindung mit der Strukturformel: 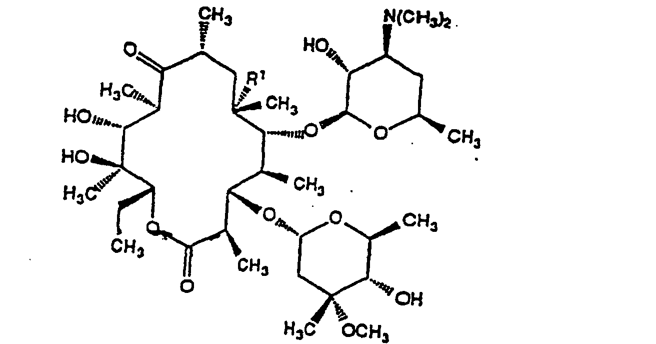 worin R¹ so ist wie oben definiert, durch Behandlung in einer wässerigen Alkoholsuspension mit einer verdünnten Konzentration von einer starken Säure bei Raumtemperatur für etwa 0,5 bis etwa 24 Stunden, Extraktion und wahlweise Isolierung der ersten intermediären Verbindung mit der Strukturformel: 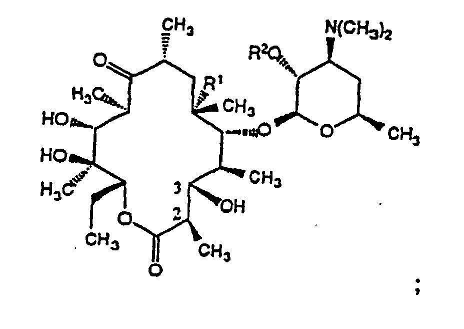
(b) wahlweise Behandlung mit der ersten intermediären Verbindung mit einem geeigneten Hydroxygruppen Schutzreagenz und die extraktive Isolierung der zweiten intermediären Verbindung mit der Strukturformel der Verbindung aus Schritt (a) worin R² eine Hydroxyschutzgruppe ist;
(c) Behandlung der zweiten intermediären Verbindung mit einem Überschuß eines carbonylierenden Reagenzes und Isolierung durch wässerige Aufarbeitung der dritten intermediären Verbindung mit der Strukturformel: 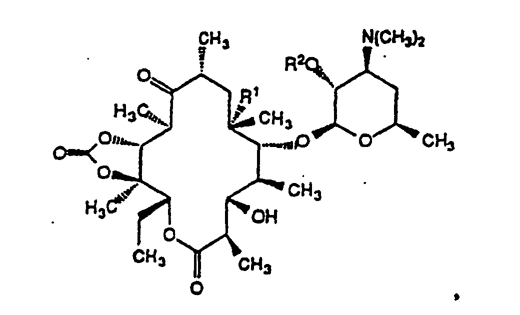 worin R¹ nicht Wasserstoff sein muß, sondern anders wie oben definiert ist;
(d) Behandlung der dritten intermediären Verbindung mit einem sulfonylierenden Wirkstoff bei von etwa 0°C bis Raumtemperatur für etwa 1 bis etwa 24 Stunden und extraktive Isolierung der vierten intermediären Verbindung mit der Strukturformel: 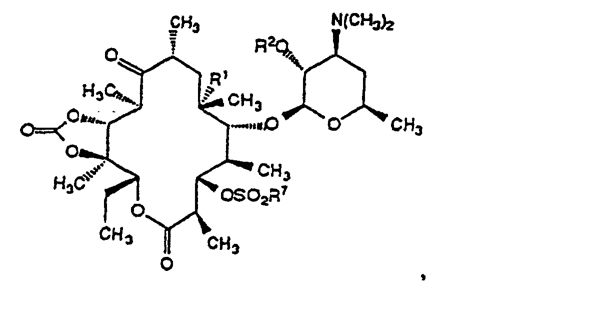 worin R⁷ Alkyl oder Aryl ist;
(e) Behandlung der vierten intermediären Verbindung mit einer starken Base, Extraktion und wahlweise Isolierung, um die fünfte intermediäre Verbindung zu ergeben mit der Formel: 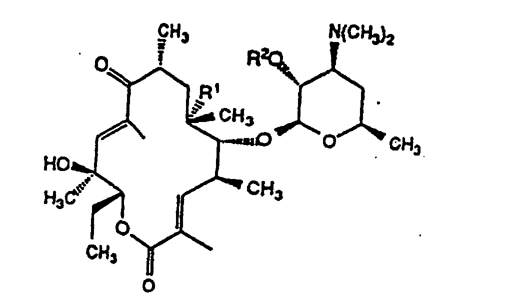
(f) Behandlung der fünften intermediären Verbindung mit einer Hydridbase und Carbonyldiimidazol in einem aprotischen Lösungsmittel bei einer Temperatur von etwa -20°C bis etwa 70°C für von etwa 0,5 Stunden bis etwa 10 Tagen und Extraktion, wahlweise Schutzaufhebung und Isolierung der gewünschten Verbindung.

41. Ein Verfahren gemäß Anspruch 40, worin in Schritt (a) der Alkohol gewählt ist aus der Gruppe bestehend aus Methanol, Ethanol, Propanol, iso-Propanol, Butanol, iso-Butanol und t-Butanol und die Säure ist gewählt aus der Gruppe bestehend aus Salzsäure, Schwefelsäure, Dichloressigsäure und Trichloressigsäure; in Schritt (b) ist das Hydroxygruppen Schutzreagenz gewählt aus der Gruppe bestehend aus Acetylchlorid, Essigsäureanhydrid, Benzoesäureanhydrid, Benzylchlorformat, Trimethylsilylchlorid und Triethylsilylchlorid und das aprotische Lösungsmittel ist gewählt aus der Gruppe bestehend aus Methylenchlorid, Chloroform, Dimethylformamid, Tetrahydrofuran, N-Methylpyrrolidinon und Mischungen davon; in Schritt (c) ist das carbonylierende Reagenz gewählt aus der Gruppe bestehend aus Phosgen, Diphosgen und Triphosgen; in Schritt (d) das sulfonylierende Reagenz ist gewählt aus der Gruppe bestehend aus Methansulfonylanhydrid, Methansulfonylchlorid, Ethansulfonylchlorid und p-Toluensulfonylchlorid; in Schritt (e) ist die Base gewählt aus der Gruppe bestehend aus Triethylamin, Diisopropylethylamin, Pyridin, 2,6-Dimethylpyridin, 1,8-Diazabicyclo[5.4.0]undec-7-en, N-Methylmorpholin, N-Methylpyrrolidin, N-Methylpiperidin, natriumcarbonat, Kaliumcarbonat, Natriumbicarbonat und Kaliumcarbonat; in Schritt (f) ist die Hydridbase gewählt aus der Gruppe bestehend aus Natriumhydrid, Kaliumhydrid und Lithiumhydrid.

42. Ein Verfahren gemäß Anspruch 40, worin in Schritt (b) die Hydroxyschutzgruppe Benzoesäureanhydrid ist und R² ist Benzoyl, und Schritte (c), (d) und (e) werden ersetzt durch einen einzelnen Schritt (c') bestehend aus:
(c') Behandlung der Verbindung aus Schritt (b) mit Natriumhexamethyldisilazan von aus etwa -50 bis etwa -28°C unter einer inerten Atmosphäre gefolgt durch Zusatz von Carbonyldiimidazol bei von etwa 0°C bis etwa Raumtemperatur für etwa 15 Minuten bis etwa 6 Stunden und Extraktion, wahlweise Schutzaufhebung und Isolierung der gewünschten Verbindung.

43. Eine pharmazeutische Zusammensetzung zur Behandlung bakterieller Infektionen die eine therapeutisch wirksame Menge einer Verbindung von Anspruch 1 in Kombination mit einem pharmazeutisch verträglichen Träger umfaßt.

44. Verwendung einer pharmazeutischen Zusammensetzung, die eine therapeutisch wirksame Menge einer Verbindung gemäß Anspruch 1 umfaßt, zur Herstellung eines Medikaments zur Behandlung bakterieller Infektionen durch Verabreichung an einem Säugetier, welches einer solchen Behandlung bedarf.

## Revendications

1. Composé, ou un sel ou ester pharmaceutiquement acceptable de celui-ci, choisi dans le groupe constitué par : 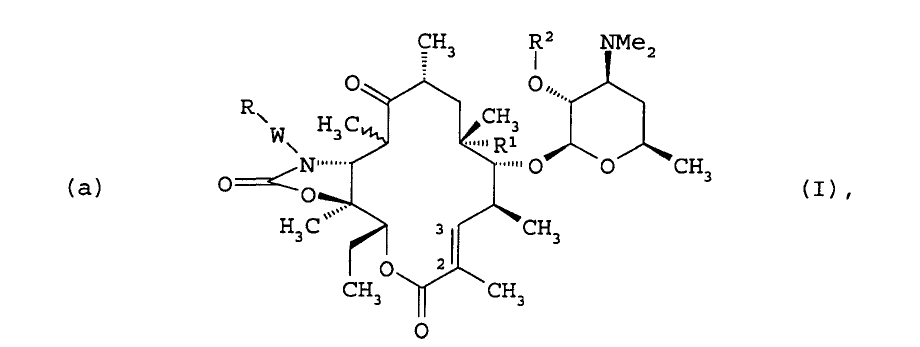 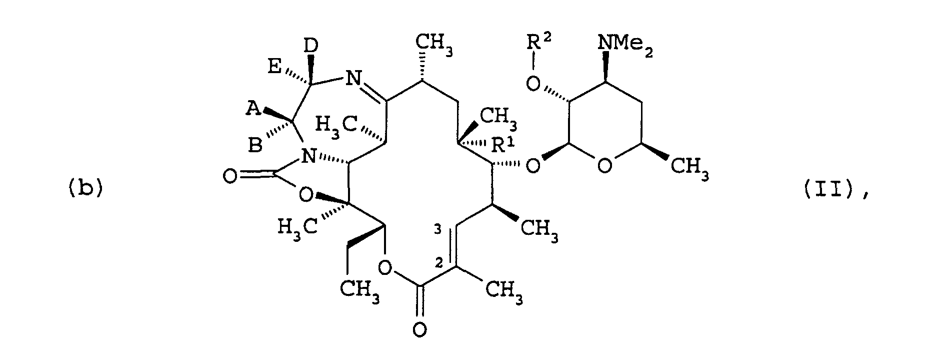 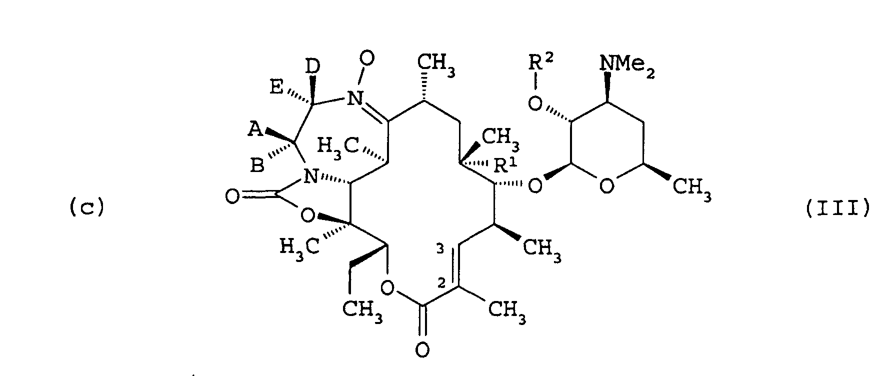 et 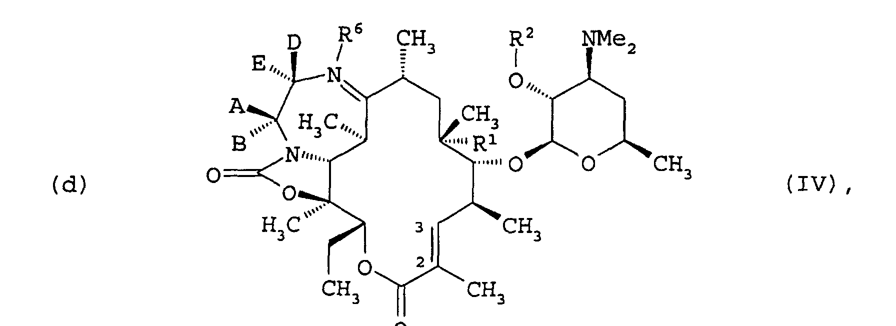 où :
R¹ est choisi dans le groupe constitué par :
(a) hydrogène ;
(b) hydroxy ;
(c) O-alkyle en C₁ à C₁₂ ;
(d) O-CO-alkyle en C₁ à C₆ ;
(e) O-CO-NH₂ ;
(f) O-CO-NH-CO-alkyle en C₁ à C₁₂ ; et
(g) O-CO-NH-SO₂-alkyle en C₁ à C₁₂ ;
R² est hydrogène ou un groupe hydroxyprotecteur ;
R⁶ est hydrogène ou alkyle en C₁ à C₆ ;
R est choisi dans le groupe constitué par :
(a) hydrogène ;
(b) alkyle en C₁ à C₆ le cas échéant substitué par un
ou plusieurs substituants choisis dans le groupe constitué par :
(i) aryle ;
(ii) aryle substitué ;
(iii) hétéroaryle ;
(iv) hétéroaryle substitué ;
(v) hydroxy ;
(vi) alcoxy en C₁ à C₆ ;
(vii) NR³R⁴, où R³ et R⁴ sont choisis indépendamment parmi hydrogène et alkyle en C₁ à C₆, ou bien R³ et R⁴ sont pris avec l'atome d'azote auquel ils sont liés pour former un noyau cyclique de 3 à 7 termes qui, lorsque le noyau est un noyau de 5 à 7 termes, peut contenir le cas échéant une fonction hétéro comprenant-O-, -NH-, -N(alkyle en C₁ à C₆)-, -N(aryle)-, -N-(arylalkyle en C₁ à C₆)-, -N(arylalkyle en C₁ à C₆ substitué)-, -N(hétéroaryle)-, -N-(hétéroarylalkyle en C₁ à C₆)-, -N(hétéro- arylalkyle en C₁ à C₆ substitué)-, -S-
ou -S(O)ₙ-, où n vaut 1 ou 2 ;
(viii) -CH₂-M-R⁵,
où M est choisi dans le groupe constitué par :
(aa) -C(O)-NH- ;
(bb) -NH-C(O)- ;
(CC) -NH- ;
(dd) -N= ;
(ee) -N(CH₃)- ;
(ff) -O- ;
(gg) -S(O)ₙ-, où n vaut 0, 1 ou 2 ;
(hh) -CO-O- ;
(ii) -O-CO- ;
(jj) -CO- ; et
R⁵ est choisi dans le groupe constitué par :
(aaa) alkyle en C₁ à C₆ le cas échant substitué par un substituant choisi dans le groupe constitué par :
(i) aryle ;
(ii) aryle substitué ;
(iii) hétéroaryle ; et
(iv) hétéroaryle substitué ;
(bbb) aryle ;
(ccc) aryle substitué ;
(ddd) hétéroaryle ;
(eee) hétéroaryle substitué ; et
(fff) hétérocycloalkyle ; et
(c) cycloalkyle en C₃ à C₇ ;
(d) aryle ;
(e) aryle substitué ;
(f) hétéroaryle ;
(g) hétéroaryle substitué ;
W est absent ou choisi dans le groupe constitué par -O-, -NH-CO-, -N=CH-, -NH- et -N(alkyle en C₁ à C₆)- ;
A, B, D et E sont choisis indépendamment dans le groupe constitué par :
(a) hydrogène ;
(b) alkyle en C₁ à C₆ le cas échéant substitué par un ou plusieurs substituants choisis dans le groupe constitué par :
(i) aryle ;
(ii) aryle substitué ;
(iii) hétéroaryle ;
(iv) hétéroaryle substitué ;
(v) hétérocycloalkyle ;
(vi) hydroxy ;
(vii) alcoxy en C₁ à C₆ ;
(viii) halogène comprenant Br, Cl, F ou I ; et
(ix) NR³R⁴, où R³ et R⁴ sont choisis indépendamment parmi hydrogène et alkyle en C₁ à C₆, ou bien R³ et R⁴ sont pris avec l'atome d'azote auquel ils sont liés pour former un noyau cyclique de 3 à 7 termes contenant le cas échéant une fonction hétéro comprenant -O-, -NH-,-N(alkyle en C₁ à C₆)-, -N(arylalkyle en C₁ à C₆)-, -N-(arylalkyle en C₁ à C₆ substitué), -S-, -N(hétéroarylalkyle en C₁ à C₆), -N(hétéro- arylalkyle en C₁ à C₆ substitué)-, -S- ou -S(O)ₙ-, où n vaut 1 ou 2 ;
(c) cycloalkyle en C₃ à C₇ ;
(d) aryle :
(e) aryle substitué ;
(f) hétéroaryle ;
(g) hétéroaryle substitué ;
(h) hétérocycloalkyle ; et
(i) un groupe choisi parmi ceux de l'option (b) ci-dessus, substitué en plus par -M-R⁵, où M et R⁵ sont tels que définis plus haut ;
ou une paire de substituants quelconque comprenant AB, AD, AE, BD, BE ou DE est prise ensemble avec le ou les atomes auxquels ils sont rattachés pour former un noyau cyclique de 3 à 7 termes contenant le cas échéant une fonction hétéro comprenant :
-O- ;
-NH- ;
-N(alkyle en C₁ à C₆)- ;
-N(arylalkyle en C₁ à C₆)- ;
-N(arylalkyle en C₁ à C₆ substitué)- ;
-N(hétéroarylalkyle en C₁ à C₆)- ;
-N(hétéroarylalkyle en C₁ à C₆ substitué)- ;
-S- ou -S(O)ₙ,-, où n vaut 1 ou 2 ;
-C(O)-NH- ;
-C(O)-NR⁵-, où R⁵ est tel que défini plus haut ;
-NH-C(O)- ;
-NR⁵-C(O)-, où R⁵ est tel que défini plus haut ; et
-C( = NH)-NH-.

2. Composé selon la revendication 1, ayant la formule (I).

3. Composé selon la revendication 2, dans lequel W est absent ou -NH-.

4. Composé selon la revendication 1, ayant la formule (II).

5. Composé selon la revendication 1, ayant la formule (III).

6. Composé selon la revendication 1, ayant la formule (IV).

7. Composé selon la revendication 1, ou un sel ou ester pharmaceutiquement acceptable de celui-ci, qui est :
Composé de la formule (I) ; R¹ = H ; R² = H ; W est absent ; R = 4-phénylbutyle ;
Composé de la formule (I) ; R¹ = méthoxy ; R² = H ; W est absent ; R = 4-phénylbutyle ;
Composé de la formule (I) ; R¹ = méthoxy ; R² = H ; W est absent ; R = 3-phénoxypropyle ;
Composé de la formule (I) ; R¹ = méthoxy ; R² = H ; W est absent ; R = 2-((phénylméthyl)amino)éthyle ;
Composé de la formule (I) ; R¹ = méthoxy ; R² = H ; W est absent ; R = 3-(N-méthyl-N-phénylamino)propyle ;
Composé de la formule (I) ; R¹ = méthoxy ; R² = H ; W est absent ; R = 3-(4-chlorophénoxy)propyle ;
Composé de la formule (II) ; R¹ = méthoxy ; R² = H ; A = B = C = D = H ;
Composé de la formule (I) ; R¹ = méthoxy ; R² = H ; W est absent ; R = 3-(1-quinoléyloxy)propyle ;
Composé de la formule (I) ; R¹ = méthoxy ; R² = H ; W est absent ; R = 3-(4 chlorophényl)-3(Z)-butényle ;
Composé de la formule (I) ; R¹ = méthoxy ; R² = H ; W est absent, R = 2-phényléthyle ;
Composé de la formule (I) ; R¹ = méthoxy ; R² = H ; W est absent ; R = 2-(3,4-dichlorophényl)éthyle ;
Composé de la formule (I) ; R¹ = méthoxy ; R² = H ; W est absent ; R = phénylméthyle ;
Composé de la formule (I) ; R¹ = méthoxy ; R² = H ; W est -NH- ; R = 3-phénylpropyle ;
Composé de la formule (I) ; R¹ = méthoxy ; R² = H ; W est absent ; R = 3-(4-phénoxyphényl)éthyle ;
Composé de la formule (I) ; R¹ = méthoxy ; R² = H ; W est absent ; R = 3-phénylpropyle ;
Composé de la formule (I) ; R¹ = méthoxy ; R² = H ; W est absent ; R = 2,2-diphényléthyle ;
Composé de la formule (I) ; R¹ = méthoxy ; R² = H ; W est absent ; R = H ;
Composé de la formule (IV) ; R¹ = méthoxy ; R² = H ; A = B = C = D = H ; R = H ;
Composé de la formule (I) ; R¹ = méthoxy ; R² = H ; W est -NH- ; R = H ; le méthyle en C₁₀ est l'isomère *épi* ;
Composé de la formule (I) ; R¹ = méthoxy ; R² = H ; W est -NH- ; R = H ; le méthyle en C₁₀ est l'isomère naturel ;
Composé de la formule (I) ; R¹ = méthoxy ; R² = H ; W est -NH- ; R = (4-quinolinyl)propyle ; le méthyle en C₁₀ est l'isomère naturel ;
Composé de la formule (I) ; R¹ = méthoxy ; R² = H ; W est absent ; R = 3-(2-naphtyloxy)propyle ;
Composé de la formule (I) ; R¹ = méthoxy ; R² = H ; W est absent ; R = 3-(3-pyridyloxy)propyle ;
Composé de la formule (I) ; R¹ = méthoxy ; R² = H ; W est absent ; R = 3-(2-pyridyloxy)propyle ;
Composé de la formule (I) ; R¹ = OH ; R² = H ; W est absent ; R = 1-phénylbutyle ;
Composé de la formule (I) ; R¹ = CONH₂ ; R² = H ; W est absent ; R = 4-phénylbutyle ;
Composé de la formule (I) ; R¹ = OCONHCO-méthyle ; R² = H ; W est absent ; R = 4-phénylbutyle ;
Composé de la formule (I) ; R¹ = OCONHSO₂-méthyle ; R² = H ; W est absent ; R = 4-phénylbutyle ;
Composé de la formule (I) ; R¹ = OMe ; R² = H ; W est absent ; R = phényle ;
Composé de la formule (I) ; R¹ = OMe ; R² = H ; W est absent ; R = 3-pyridyle ;
Composé de la formule (I) ; R¹ = OMe ; R² = H ; W est -O- ; R = H ;
Composé de la formule (I) ; R¹ = OMe ; R² = H ; W est -O- ; R = Me ;
Composé de la formule (I) ; R¹ = OMe ; R² = H ; W est -NH--CO- ; R = phényle ;
Composé de la formule (II) ; R¹ = OMe ; R² = H ; A = benzyle ; B, D, E = H ;
Composé de la formule (II) ; R¹ = OMe ; R² = H ; A, D = 3,4-pyrrolidinyle ; B, E = H ;
Composé de la formule (III) ; R¹ = OMe ; R² = H ; A, B, D, E = H ;
Composé de la formule (IV) ; R¹ = OMe ; R² = H ; A = benzyle ; B, D, E = H ; R = H ;
Composé de la formule (IV) ; R¹ = OMe ; R² = H ; A, D = 3,4-pyrrolidinyle ; B, E = H ; R = H ;
Composé de la formule (IV) ; R¹ = OMe ; R² = H ; A, B, D, E = H ; R = CH₂CH₂CH₂C₆H₅ ;
Composé de la formule (IV) ; R¹ = OMe ; R² = H ; A, B, D, E = H, R = 2,4-dinitrobenzène ;
Composé de la formule (IV) ; R¹ = OMe ; R² = H ; A, B, D, E = H, R = 4-quinoléyle ;
Composé de la formule (I) ; R¹ = méthoxy, R² = H ; W est absent ; R = (4H-4-oxo-1-quinoléyl)propyle ;
Composé de la formule (I) ; R¹ = méthoxy ; R² = H ; W est absent ; R = 2-(4-nitrophényl)éthyle ;
Composé de la formule (I) ; R¹ = méthoxy ; R² = H ; W est absent ; R = 2-(4-aminophényl)éthyle ;
Composé de la formule (I) ; R¹ = méthoxy ; R² = H ; W est absent ; R = 3-éthoxypropyle ;
Composé de la formule (I) ; R¹ = méthoxy, R² = H ; W est absent ; R = isopropyle ;
Composé de la formule (I) ; R¹ = méthoxy ; R² = H ; W est absent ; R = 2-(4-bromophényl)éthyle ;
Composé de la formule (I) ; R¹ = méthoxy ; R² = H ; W est absent ; R = 2-(4-hydroxyphényl)éthyle ;
Composé de la formule (I) ; R¹ = méthoxy ; R² = H ; W est absent ; R = 2-(4-fluorophényl)éthyle ;
Composé de la formule (I) ; R¹ = méthoxy ; R² = H ; W est absent ; R = 2-(3-méthoxyphényl)éthyle ;
Composé de la formule (I) ; R¹ = méthoxy ; R² = H ; W est absent ; R = 3-vinyloxypropyle ;
Composé de la formule (I) ; R¹ = méthoxy ; R² = H ; W est absent ; R = 2-(3-trifluorométhyl)phényléthyle ;
Composé de la formule (I) ; R¹ = méthoxy ; R² = H ; W est absent ; R = 2-thiényléthyle ;
Composé de la formule (I) ; R¹ = méthoxy ; R² = H ; W est absent ; R = 2-(3,4-dibenzyloxyphényl)éthyle ;
Composé de la formule (I) ; R¹ = méthoxy ; R² = H ; W est absent ; R = 2-(4-méthylphényl)éthyle ;
Composé de la formule (I) ; R¹ = méthoxy ; R² = H ; W est absent ; R = allyle ;
Composé de la formule (I) ; R¹ = méthoxy ; R² = H ; W est absent ; R = 1,3-dihydroxypropyle ;
Composé de la formule (I) ; R¹ = méthoxy ; R² = H ; W est absent ; R = 1,3-dihydroxypropyle (10-*épi*) ;
Composé de la formule (I) ; R¹ = méthoxy ; R² = H ; W est absent ; R = 3-hydroxypropyle ;
Composé de la formule (I) ; R¹ = méthoxy ; R² = H ; W est absent ; R = 3-hydroxypropyle (10-*épi*) ;
Composé de la formule (I) ; R¹ = méthoxy ; R² = H ; W est absent ; R = propyle ;
Composé de la formule (I) ; R¹ = méthoxy ; R² = H ; W est absent ; R = isobutyle ;
Composé de la formule (I) ; R¹ = méthoxy ; R² = H ; W est absent ; R = 2-(benzoylamino)éthyle ;
Composé de la formule (I) ; R¹ = méthoxy ; R² = H ; W est absent ; R = 3-(benzoylamino)propyle ;
Composé de la formule (I) ; R¹ = méthoxy ; R² = H ; W est absent ; R = 3-(acétylamino)propyle ;
Composé de la formule (I) ; R¹ = méthoxy ; R² = H ; W est absent ; R = H (10-*épi*) ;
Composé de la formule (I) ; R¹ = méthoxy ; R² = H ; W est absent ; R = 3-phénylpropyle (10-*épi*) ;
Composé de la formule (I) ; R¹ = méthoxy, R² = H ; W est absent ; R = 3-(4-phénoxyphényl)éthyle (10-*épi*) ;
Composé de la formule (I) ; R¹ = méthoxy ; R² = H ; W est -NH- ; R = 3-(4-chlorophényl)propyle ;
Composé de la formule (I) ; R¹ = méthoxy, R² = H ; W est -NH- ; R = 3-(3-chlorophényl)propyle ;
Composé de la formule (I) ; R¹ = méthoxy ; R² = H ; W est -NH- ; R = 3-(2-chlorophényl)propyle ;
Composé de la formule (I) ; R¹ = méthoxy ; R² = H ; W est -NH- ; R = 3-(2,4-dichlorophényl)propyle ;
Composé de la formule (I) ; R¹ = méthoxy ; R² = H ; W est -NH- ; R = 3-(4-hydroxyphényl)propyle ;
Composé de la formule (I) ; R¹ = méthoxy ; R² = H ; W est -NH- ; R = 3-(3-hydroxyphényl)propyle ;
Composé de la formule (I) ; R¹ = méthoxy ; R² = H ; W est -NH- ; R = 3-(2-hydroxyphényl)propyle ;
Composé de la formule (I) ; R¹ = méthoxy ; R² = H ; W est -NH- ; R = 3-(4-méthoxyphényl)propyle ;
Composé de la formule (I) ; R¹ = méthoxy ; R2 = H ; W est -NH- ; R = 3-(4-nitrophényl)propyle ;
Composé de la formule (I) ; R¹ = méthoxy ; R² = H ; W est -NH- ; R = 3-(3-nitrophényl)propyle ;
Composé de la formule (I) ; R¹ = méthoxy ; R² = H ; W est -NH- ; R = 3-(2-nitrophényl)propyle ;
Composé de la formule (I) ; R¹ = méthoxy ; R² = H ; W est -NH- ; R = 3-((4-(acétylamino)phényl)propyle ;
Composé de la formule (I) ; R¹ = méthoxy ; R² = H W est -NH- ; R = *trans*-3-phénylprop-2-ényle ;
Composé de la formule (I) ; R¹ = méthoxy ; R² = H W est -NH- ; R = 2-phényléthyle ;
Composé de la formule (I) ; R¹ = méthoxy ; R² = H ; W est -NH- ; R = phénylméthyle ;
Composé de la formule (I) ; R¹ = méthoxy ; R² = H ; W est -NH- ; R = (3-indolyl)méthyle ;
Composé de la formule (I) ; R¹ = méthoxy ; R² = H ; W est -NH- ; R = (4-méthoxyphényl)méthyle ;
Composé de la formule (I) ; R¹ = méthoxy ; R² = H ; W est -NH- ; R = (4-acétylaminophényl)méthyle ;
Composé de la formule (I) ; R¹ = méthoxy ; R² = H ; W est -NH- ; R = (4-chlorophényl)méthyle ;
Composé de la formule (I) ; R¹ = méthoxy ; R² = H ; W est -NH- ; R = (4-diméthylaminophényl)méthyle ;
Composé de la formule (I) ; R¹ = méthoxy ; R² = H ; W est -NH- ; R = *trans*-3-(4-nitrophényl)prop-2-ényle ;
Composé de la formule (I) ; R¹ = méthoxy ; R² = H ; W est -NH- ; R = (4-nitrophényl)méthyle ;
Composé de la formule (I) ; R¹ = méthoxy ; R² = H ; W est -NH- ; R = (3,4-dihydroxyphényl)méthyle ;
Composé de la formule (I) ; R¹ = méthoxy ; R² = H ; W est -NH- ; R = (2,5-dihydroxyphényl)méthyle ;
Composé de la formule (I) ; R¹ = méthoxy ; R² = H ; W est -NH- ; R = (2-hydroxy-5-nitrophényl)méthyle ;
Composé de la formule (I) ; R¹ = méthoxy ; R² = H ; W est -NH- ; R = (4-hydroxyméthylphényl)méthyle ;
Composé de la formule (I) ; R¹ = méthoxy ; R² = H ; W est -NH- ; R = *trans*-3-(5-nitro-2-furanyl)prop-2-ényle ; R¹R²
Composé de la formule (I) ; R¹ = méthoxy, R² = H ; W est -NH- ; R = (4-hydroxyphényl)méthyle ;
Composé de la formule (I) ; R¹ = méthoxy ; R² = H ; W est -NH- ; R = (3-hydroxyphényl)méthyle ;
Composé de la formule (I) ; R¹ = méthoxy ; R² = H ; W est -NH- ; R = (2-hydroxyphényl)méthyle ;
Composé de la formule (I) ; R¹ = méthoxy ; R² = H1 ; W est -NH- ; R = (4-trifluorométhylphényl)méthyle ;
Composé de la formule (I) ; R¹ = méthoxy ; R² = H ; W est -NH- ; R = (4-cyanophényl)méthyle ;
Composé de la formule (I) ; R¹ = méthoxy ; R² = H ; W est -NH- ; R = (2-pyridyl)méthyle ;
Composé de la formule (I) ; R¹ = méthoxy ; R² = H ; W est -NH- ; R = (3-pyridyl)méthyle ;
Composé de la formule (I) ; R¹ = méthoxy ; R² = H ; W est -NH- ; R = (4-pyridyl)méthyle ;
Composé de la formule (I) ; R¹ = méthoxy ; R² = H ; W est -NH- ; R = (2-hydroxy-1-naphtyl)méthyle ;
Composé de la formule (I) ; R¹ = méthoxy ; R² = H ; W est -NH- ; R = (4-diméthylamino-1-naphtyl)méthyle ;
Composé de la formule (I) ; R¹ = méthoxy ; R² = H ; W est -NH- ; R = (4-(méthylthio)phényl)méthyle ;
Composé de la formule (I) ; R¹ = méthoxy ; R² = H ; W est -NH- ; R = 4-phénoxyphényl)méthyle ;
Composé de la formule (I) ; R¹ = méthoxy ; R² = H ; W est -NH- ; R = 3-(4-fluorophényl)propyle ;
Composé de la formule (I) ; R¹ = méthoxy ; R² = H ; W est -NH- ; R = (*trans*-3-(4-nitrophényl)prop-2-ényle ;
Composé de la formule (I) ; R¹ = méthoxy ; R² = H ; W est -NH- ; R = (4-aminophényl)méthyle ;
Composé de la formule (I) ; R¹ = méthoxy ; R² = H ; W est -NH- ; R = 3-(4-aminophényl)propyle ;
Composé de la formule (I) ; R¹ = méthoxy ; R² = H ; W est -NH- ; R = 3-(3-aminophényl)propyle ;
Composé de la formule (I) ; R¹ = méthoxy ; R² = H ; W est -NH- ; R = 3-(2-aminophényl)propyle ;
Composé de la formule (I) ; R¹ = méthoxy ; R² = H ; W est -NH- ; R = trans-3-(4-acétylaminophényl)prop-2-ényle ;
Composé de la formule (I) ; R1 = méthoxy ; R2 = H ; W est -NH- ; R = trans-3-(4-(4-nitrobenzoylamino)phényl)-prop-2-ényle ;
Composé de la formule (I) ; R¹ = méthoxy ; R² = H ; W est -NH- ; R = 3-(2-benztriazolyl)propyle ;
Composé de la formule (I) ; R¹ = méthoxy ; R² = H ; W est -NH- ; R = 3-(1-benztriazolyl)propyle ;
Composé de la formule (I) ; R¹ = méthoxy ; R² = H ; W est -NH- ; R = 3-(4-phénylimidazolyl)propyle ;
Composé de la formule (I) ; R¹ = méthoxy ; R² = H ; W est -NH- ; R = 3-(1-anhydro-1-cladinosyl)propyle ;
Composé de la formule (I) ; R¹ = méthoxy ; R² = H ; W est -NH- ; R = 3-phénylpropyle (10-*épi*) ;
Composé de la formule (I) ; R¹ = méthoxy ; R² = H ; W est -NH- ; R = isopropyle ;
Composé de la formule (I) ; R¹ = méthoxy ; R² = H ; W est -NH- ; R = 1,3-diphényl-2-propyle ; ou
Composé de la formule (I) ; R¹ = méthoxy ; R² = H ; W est -NH- ; R = 3-pentyle.

8. Procédé de préparation d'un composé ayant la formule (I) : 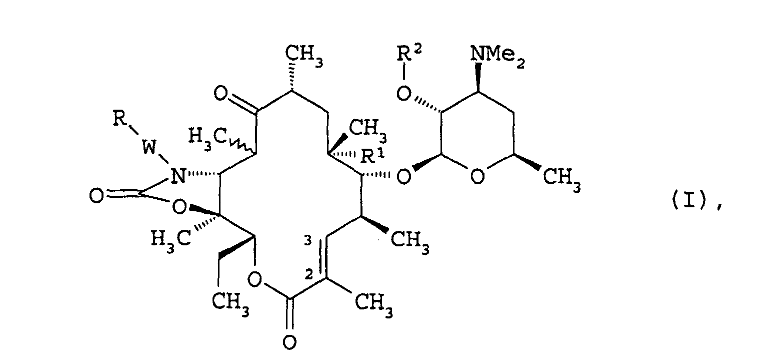 dans laquelle
R¹ est choisi dans le groupe constitué par :
(a) hydrogène ;
(b) hydroxy ;
(c) O-alkyle en C₁ à C₁₂
(d) O-CO-alkyle en C₁ à C₆ ;
(e) O-CO-NH₂ ;
(f) O-CO-NH-CO-alkyle en C₁ à C₁₂ ; et
(g) O-CO-NH-SO₂-alkyle en C₁ à C₁₂ ;
R² est hydrogène ou un groupe hydroxyprotecteur ;
R est choisi dans le groupe constitué par :
(a) hydrogène ;
(b) alkyle en C₁ à C₆ le cas échéant substitué par un ou plusieurs substituants choisis dans le groupe constitué par :
(i) aryle ;
(ii) aryle substitué ;
(iii) hétéroaryle ;
(iv) hétéroaryle substitué ;
(v) hydroxy ;
(vi) alcoxy en C₁ à C₆ ;
(vii) NR³R⁴, où R³ et R⁴ sont choisis indépendamment parmi hydrogène et alkyle en C₁ à C₆, ou bien R³ et R⁴ sont pris avec l'atome d'azote auquel ils sont liés pour former un noyau cyclique de 3 à 7 termes contenant le cas échéant une fonction hétéro comprenant -O-, -NH-,-N(alkyle en C₁ à C₆)-, -N(arylalkyle en C₁ à C₆)-, -N-(arylalkyle en C₁ à C₆ substitué)-, -N-(hétéroarylalkyle en C₁ à C₆)-, -N-(hétéro- arylalkyle en C₁ à C₆ substitué)--S- ou -S(O)ₙ-, où n vaut 1 ou 2 ;
(viii) -CH₂-M-R⁵,
où M est choisi dans le groupe constitué par :
(aa) -C(O)-NH- ;
(bb) -NH-C(O)- ;
(cc) -NH- ;
(dd) -N= ;
(ee) -N(CH₃)- ;
(ff) -O- ;
(gg) -S(O)ₙ-, où n vaut 0, 1 ou 2 ;
(hh) -CO-O- ;
(ii) -O-CO ;
(jj) -CO- ; et
R⁵ est choisi dans le groupe constitué par :
(aaa) alkyle en C₁ à C₆ le cas échant substitué par un substituant choisi dans le groupe constitué par :
(i) aryle ;
(ii) aryle substitué ;
(iii) hétéroaryle ; et
(iv) hétéroaryle substitué ;
(bbb) aryle ;
(ccc) aryle substitué ;
(ddd) hétéroaryle ;
(eee) hétéroaryle substitué ; et
(fff) hétérocycloalkyle ; et
(c) cycloalkyle en C3 à C7 ;
(d) aryle ;
(e) aryle substitué ;
(f) hétéroaryle ;
(g) hétéroaryle substitué ; et
W est absent ;
ledit procédé comprenant les étapes consistant à :
(a) traiter un composé ayant la formule : 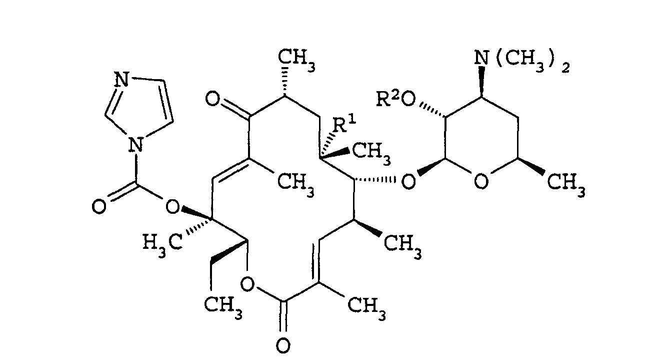
où R¹ est choisi dans le groupe constitué par hydrogène, hydroxy protégé, O-CO-alkyle en C₁ à C₆, O-alkyle en C₁ à C₁₂, O-CO-NH₂, O-CO-NH-CO-alkyle en C₁ à C₁₂ et O-CO-NH-SO₂-alkyle en C₁ à C₁₂ ; et R² est un groupe hydroxy- protecteur, avec une amine primaire RNH₂, où R est tel que défini plus haut, dans un solvant organique approprié à une température allant de l'ambiante à la température de reflux pendant environ 4 à environ 48 heures, et extraire, le cas échéant déprotéger, et isoler le composé recherché.

9. Procédé selon la revendication 8, dans lequel R est hydrogène, alkyle en C₁ à C₆, cycloalkyle en C₃ à C₇, aryle, aryle substitué, hétéroaryle ou hétéroaryle substitué, et le solvant est choisi dans le groupe constitué par le chlorure de méthylène, le tétrahydrofurane, la N-méthylpyrrolidinone, le diéthyléther, le bis-méthoxyméthyléther, le diméthylformamide, l'acétonitrile, l'acétone et des mélanges aqueux de ceux-ci.

10. Procédé de préparation d'un composé ayant la formule (I) : 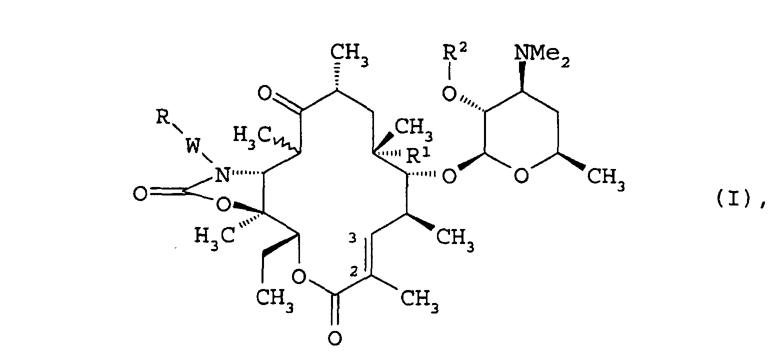 dans laquelle
R¹ est choisi dans le groupe constitué par :
(a) hydrogène ;
(b) hydroxy ;
(c) O-alkyle en C₁ à C₁₂ ;
(d) O-CO-alkyle en C₁ à C₆ ;
(e) O-CO-NH₂ ;
(f) O-CO-NH-CO-alkyle en C₁ à C₁₂ ; et
(g) O-CO-NH-SO₂-alkyle en C₁ à C₁₂ ;
R² est hydrogène ou un groupe hydroxyprotecteur ;
R est choisi dans le groupe constitué par :
(a) hydrogène ;
(b) alkyle en C₁ à C₆ le cas échéant substitué par un ou plusieurs substituants choisis dans le groupe constitué par :
(i) aryle ;
(ii) aryle substitué ;
(iii) hétéroaryle ;
(iv) hétéroaryle substitué ;
(v) hydroxy ;
(vi) alcoxy en C₁ à C₆
(vii) NR³R⁴, où R³ et R⁴ sont choisis indépendamment parmi hydrogène et alkyle en C₁ à C₆, ou bien R³ et R⁴ sont pris avec l'atome d'azote auquel ils sont liés pour former un noyau cyclique de 3 à 7 termes qui, lorsque le noyau est un noyau de 5 à 7 termes, peut contenir le cas échéant une fonction hétéro comprenant -O-, -NH-, -N-(alkyle en C₁ à C₆)-, -N(aryle)-, -N- (arylalkyle en C₁ à C₆)-, -N(arylalkyle en C₁ à C₆ substitué) -,-N(hétéroaryle)-, -N- (hétéroarylalkyle en C₁ à C₆)-,-N(hétéro- arylalkyle en C₁ à C₆ substitué)-, -S- ou-S(O)ₙ-, où n vaut 1 ou 2 ;
(viii) -CH₂-M-R⁵,
où M est choisi dans le groupe constitué par :
(aa) -C(O)-BH- ;
(bb) -NH-C(O)- ;
(cc) -NH- ;
(dd) -N= ;
(ee) -N(CH₃)- ;
(ff) -O- ;
(gg) -S(O)ₙ-, où n vaut 0, 1 ou 2 ;
(hh) -CO-O- ;
(ii) -O-CO- ;
(jj) -CO- ; et
R⁵ est choisi dans le groupe constitué par :
(aaa) alkyle en C₁ à C₆ le cas échant substitué par un substituant choisi dans le groupe constitué par :
(i) aryle ;
(ii) aryle substitué ;
(iii) hétéroaryle ; et
(iv) hétéroaryle substitué ;
(bbb) aryle ;
(ccc) aryle substitué ;
(ddd) hétéroaryle ;
(eee) hétéroaryle substitué ; et
(fff) hétérocycloalkyle ; et
(c) cycloalkyle en C₃ à C₇ ;
(d) aryle ;
(e) aryle substitué ;
(f) hétéroaryle ;
(g) hétéroaryle substitué ; et
W est choisi dans le groupe constitué par -NH-CO-,-N=CH-, -NH- et -N(alkyle en C₁ à C₆)- ;
ledit procédé comprenant les étapes consistant à :
(a) traiter un composé ayant la formule : 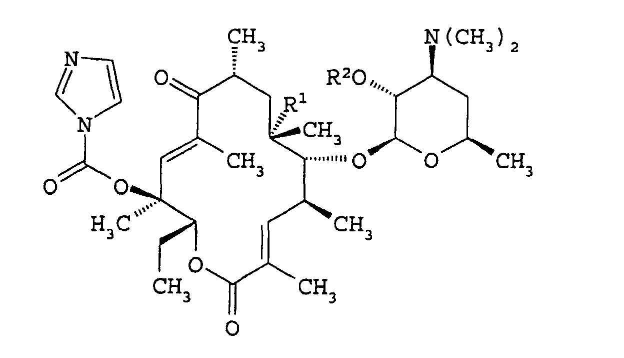 où R¹ est choisi dans le groupe constitué par hydrogène, hydroxy protégé, O-CO-alkyle en C₁ à C₆, O-alkyle en C₁ à C₁₂, O-CO-NH₂, O-CO-NH-CO-alkyle en C₁ à C₁₂
ou O-CO-NH-SO₂-alkyle en C₁ à C₁₂ ; et R² est un groupe hydroxy-protecteur, avec un réactif choisi dans le groupe constitué par l'hydrazine, une hydrazine substituée, l'hydroxylamine et l'hydroxylamine O-substituée dans un solvant organique approprié à une température allant de l'ambiante à la température de reflux pendant environ 4 à environ 48 heures pour donner le composé recherché ;
(b) le cas échéant acyler le composé de la formule (I) obtenu à l'étape (a), dans lequel W est -NH- et R est H, avec un agent acylant pour donner un composé de la formule (I) dans laquelle W est -NH-CO- ;
(c) le cas échéant condenser le composé de la formule (I) obtenu à l'étape (a), dans lequel W est -H-et R est H, avec un aldéhyde pour donner un composé de la formule (I) dans laquelle W est -N=CH- ;
(d) le cas échéant réduire le composé de la formule (I) obtenu à l'étape (c), dans lequel W est -N=CH-, avec un agent réducteur pour donner un composé de la formule (I) dans laquelle W est -NH- ;
(e) et extraire, le cas échéant déprotéger, et isoler le composé recherché.

11. Procédé selon la revendication 10, dans lequel le solvant est choisi dans le groupe constitué par le méthanol, l'éthanol, le propanol, l'isopropanol, le butanol, t-butanol, le chlorure de méthylène, le tétrahydrofurane, la N-méthylpyrrolidinone, le diéthyléther, le bis-méthoxyméthyléther, le diméthylformamide, l'acétone, l'acétonitrile aqueux, le DMF aqueux et l'acétone aqueuse.

12. Procédé selon la revendication 10, dans lequel le produit est un composé de la formule (I) dans laquelle W est -NH- et R est H, et le réactif hydrazinique est l'hydrazine.

13. Procédé selon la revendication 10, dans lequel le produit est un composé de la formule (I) dans laquelle W est -N(alkyle en C₁ à C₆)-, le réactif hydrazinique est une hydrazine substituée RR⁴NNH₂, où R est tel que défini pour la formule (I), et R⁴ est alkyle en C₁ à C₆.

14. Procédé selon la revendication 10, dans lequel le produit est un composé de la formule (I) dans laquelle W est -NH-CO-, le réactif hydrazinique est l'hydrazine et le produit obtenu à l'étape (a), ayant la formule (I) dans laquelle W est -NH- et R est H, est traité avec un agent acylant R-acyle, où R est tel que défini pour la formule (I).

15. Procédé selon la revendication 10, dans lequel l'agent acylant est choisi dans le groupe constitué par un chlorure d'acide, un fluorure d'acide, un anhydride d'acide, un acide carboxylique en présence de carbonyldiimidazole et un acide carboxylique en présence de chlorhydrate de 1-(3-diméthylaminopropyl)-3-éthylcarbo-diimide.

16. Procédé selon la revendication 10, dans lequel le produit est un composé de la formule (I) dans laquelle W est -N=CH-, le réactif hydrazinique est l'hydrazine et le produit obtenu à l'étape (a), ayant la formule (I) dans laquelle W est -NH- et R est H, est traité avec un aldéhyde ayant la formule R-CHO, où R est tel que défini pour la formule (I).

17. Procédé selon la revendication 10, dans lequel le produit est un composé de la formule (I) dans laquelle W est -NH- et R n'est pas H, le réactif hydrazinique est l'hydrazine, le produit obtenu à l'étape (a), ayant la formule (I) dans laquelle W est -NH- et R est H, est traité avec un aldéhyde ayant la formule R-CHO, où R est tel que défini pour la formule (I), et le produit obtenu à l'étape (c), ayant la formule (I) dans laquelle W est -N=CH-, est traité avec un agent réducteur.

18. Procédé selon la revendication 17, dans lequel l'agent réducteur est choisi dans le groupe constitué par le cyanoborohydrure de sodium, le borohydrure de sodium, le triacétoxyborohydrure de sodium, un complexe de borane/ tétrahydrofurane et un complexe de borane/pipéridine.

19. Procédé de préparation d'un composé ayant la formule (I) : 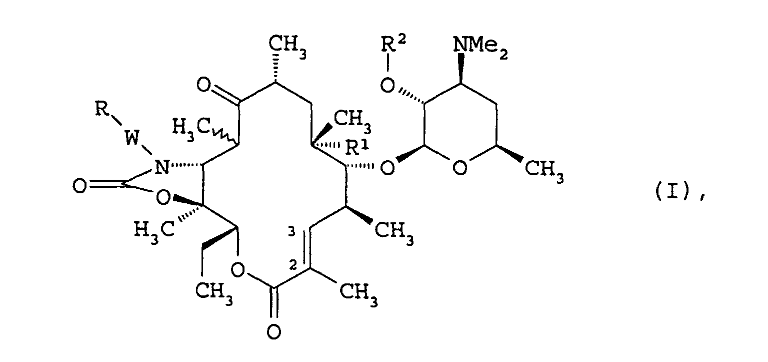 dans laquelle
R¹ est choisi dans le groupe constitué par :
(a) hydrogène ;
(b) hydroxy ;
(c) O-alkyle en C₁ à C₁₂ ;
(d) O-CO-alkyle en C₁ à C₆ ;
(e) O-CO-NH₂ ;
(f) O-CO-NH-CO-alkyle en C₁ à C₁₂ ; et
(g) O-CO-NH-SO₂-alkyle en C₁ à C₁₂ ;
R² est hydrogène ou un groupe hydroxyprotecteur ;
R est choisi dans le groupe constitué par :
(a) hydrogène ;
(b) alkyle en C₁ à C₆ le cas échéant substitué par un ou plusieurs substituants choisis dans le groupe constitué par :
(i) aryle ;
(ii) aryle substitué ;
(iii) hétéroaryle ;
(iv) hétéroaryle substitué ;
(v) hydroxy ;
(vi) alcoxy en C₁ à C₆ ;
(vii) NR³R⁴, où R³ et R⁴ sont choisis indépendamment parmi hydrogène et alkyle en C₁ à C₆, ou bien R³ et R⁴ sont pris avec l'atome d'azote auquel ils sont liés pour former un noyau cyclique de 3 à 7 termes qui, lorsque le noyau est un noyau de 5 à 7 termes, peut contenir le cas échéant une fonction hétéro comprenant -O-, -NH-,-N(alkyle en C₁ à C₆), -N(aryle)-, -N- (arylalkyle en C₁ à C₆)-, -N(arylalkyle en C₁ à C₆ substitué) -,-N(hétéroaryle)-, -N-(hétéroarylalkyle en C₁ à C₆)-, N(hétéro- arylalkyle en C₁ à C₆ substitué), -S- ou -S(O)ₙ -
où n vaut 1 ou 2 ;
(viii) -CH₂-M-R⁵,
où M est choisi dans le groupe constitué par :
(aa) -C(O)-NH- ;
(bb) -NH-C(O)- ;
(cc) -NH- ;
(dd) -N= ;
(ee) -N(CH₃)- ;
(ff) -O- ;
(gg) -S(O)ₙ-, où n vaut 0, 1 ou 2 ;
(hh) -CO-O- ;
(ii) -O-CO- ;
(jj) -CO- ; et
R⁵ est choisi dans le groupe constitué par :
(aaa) alkyle en C₁ à C₆ le cas échant substitué par un substituant choisi dans le groupe constitué par :
(i) aryle ;
(ii) aryle substitué ;
(iii) hétéroaryle ; et
(iv) hétéroaryle substitué ;
(bbb) aryle ;
(ccc) aryle substitué ;
(ddd) hétéroaryle ;
(eee) hétéroaryle substitué ; et
(fff) hétérocycloalkyle ; et
(c) cycloalkyle en C₃ à C₇ ;
(d) aryle :
(e) aryle substitué ;
(f) hétéroaryle ;
(g) hétéroaryle substitué ; et
W est -O- ;
ledit procédé comprenant les étapes consistant à :
(a) traiter un composé ayant la formule : 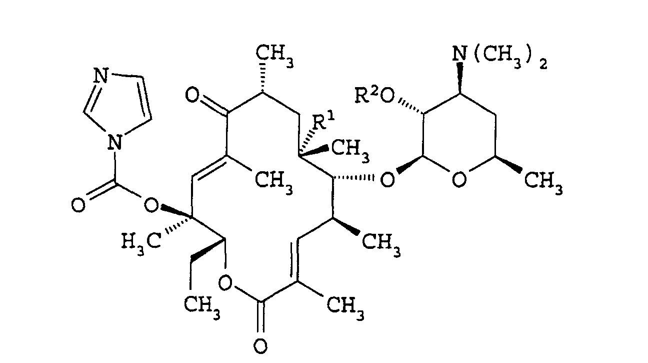 où R¹ est choisi dans le groupe constitué par hydrogène, hydroxy protégé, O-alkyle en C₁ à C₁₂, O-CO-alkyle en C₁ à C₆, -O-CO-NH₂-, O-CO-NH-CO-alkyle en C₁ à C₁₂ ou O-CO-NH-SO₂-alkyle en C₁ à C₁₂ ; et R² est un groupe hydroxy- protecteur, avec un réactif hydroxylamine choisi dans le groupe constitué par l'hydroxylamine non substituée et une hydroxylamine O-C₁-C₆-alkylée dans un solvant organique approprié à une température allant de l'ambiante à la température de reflux pendant environ 4 à environ 48 heures, pour donner le composé recherché ;
(b) le cas échéant traiter le produit obtenu à l'étape (a), ayant la formule (I) dans laquelle W est -O-et R est H, avec une base et un agent électrophile approprié ayant la formule R-L, où R est choisi dans le groupe constitué par alkyle en C₁ à C₆, cycloalkyle en C₃ à C₇, aryle, aryle substitué, hétéroaryle et hétéroaryle substitué, tels que définis pour les composés de la formule (I) ci-dessus, et L est un groupe partant approprié, pour donner le composé recherché de la formule (I) dans laquelle W est -O- et R est choisi dans le groupe constitué par alkyle en C₁ à C₆, cycloalkyle en C₃ à C₇, aryle, aryle substitué, hétéroaryle et hétéroaryle substitué ; et
(c) extraire, le cas échéant déprotéger, et isoler le composé recherché.

20. Procédé selon la revendication 19, dans lequel le solvant est choisi dans le groupe constitué par le méthanol, l'éthanol, le propanol, isopropanol, le butanol, le t-butanol, le chlorure de méthylène, le tétrahydrofurane, la N-méthylpyrrolidinone, le diéthyléther, le bis-méthoxyméthyléther, le diméthylformamide, l'acétone, l'acétonitrile aqueux, le DMF aqueux et l'acétone aqueuse.

21. Procédé selon la revendication 20, dans lequel le produit est un composé de la formule (I) dans laquelle W est -O- et R est H et le réactif hydroxylamine est de l'hydroxylamine non substituée.

22. Procédé selon la revendication 20, dans lequel le produit est un composé de la formule (I) dans laquelle W est -O- et R est O-alkyle en C₁ à C₆ et le réactif hydroxylamine est une hydroxylamine O-C₁-C₆-alkylée.

23. Procédé selon la revendication 20, dans lequel le produit final est un composé de la formule (I) dans laquelle W est -O- et R est choisi dans le groupe constitué par alkyle en C₁ à C₆, cycloalkyle en C₃ à C₇, aryle, aryle substitué, hétéroaryle ou hétéroaryle substitué, et le produit obtenu à l'étape (a), ayant la formule (I) dans laquelle W est -O- et R est H, est traité avec une base appropriée et un agent électrophile ayant la formule R-L, où R est tel que défini plus haut et L est un groupe partant approprié.

24. Procédé selon la revendication 22, dans lequel la base est choisie dans le groupe constitué par l'hydrure de sodium, l'hydrure de potassium, l'hydrure de lithium, le diéthylamide de lithium et le butyllithium, et L est choisi dans le groupe constitué par halogénure, méthanesulfonyle et p-toluènesulfonyle.

25. Procédé de préparation d'un composé ayant la formule (II) : 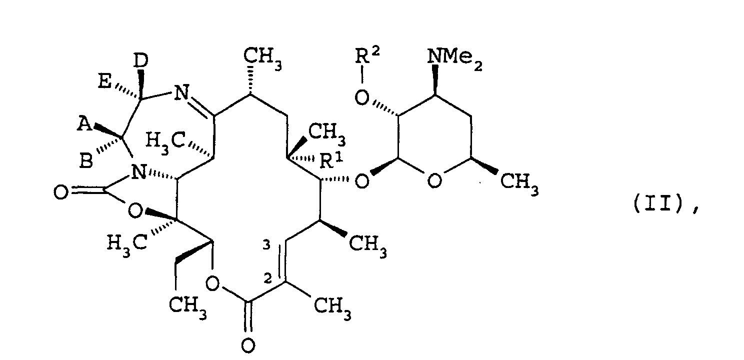 dans laquelle
R¹ est choisi dans le groupe constitué par :
(a) hydrogène ;
(b) hydroxy ;
(c) O-alkyle en C₁ à C₁₂ ;
(d) O-CO-alkyle en C₁ à C₆ ;
(e) -O-CO-NH₂ ;
(f) O-CO-NH-CO-alkyle en C₁ à C₁₂ ; et
(g) O-CO-NH-SO₂-alkyle en C₁ à C₁₂ ;
R² est hydrogène ou un groupe hydroxyprotecteur ;
A, B, D et E sont choisis indépendamment dans le groupe constitué par :
(a) hydrogène ;
(b) alkyle en C₁ à C₆ le cas échéant substitué par un ou plusieurs substituants choisis dans le groupe constitué par :
(i) aryle ;
(ii) aryle substitué ;
(iii) hétéroaryle ;
(iv) hétéroaryle substitué ;
(v) hétérocycloalkyle ;
(vi) hydroxy ;
(vii) alcoxy en C₁ à C₆ ;
(viii) halogène comprenant Br, Cl, F ou I ; et
(ix) NR³R⁴, où R³ et R⁴ sont choisis indépendamment parmi hydrogène et alkyle en C₁ à C₆, bien ou R³ et R⁴ sont pris avec l'atome d'azote auquel ils sont liés pour former un noyau cyclique de 3 à 7 termes qui, lorsque le noyau est un noyau de 5 à 7 termes, peut contenir le cas échéant une fonction hétéro comprenant -O-, -NH-,-N(alkyle en C₁ à C₆)-, -N(aryle)-, -N-(arylalkyle en C₁ à C₆)-, -N(arylalkyle en C₁ à C₆ substitué) -,-N(hétéroaryle)-, -N-(hétéroarylalkyle en C₁ à C₆)-, -N-(hétéro- arylalkyle en C₁ à C₆ substitué)-, -S- ou -S(O)ₙ-
où n vaut 1 ou 2 ;
(c) cycloalkyle en C₃ à C₇ ;
(d) aryle ;
(e) aryle substitué ;
(f) hétéroaryle ;
(g) hétéroaryle substitué ;
(h) hétérocycloalkyle ; et
(i) un groupe choisi parmi ceux de l'option (b) ci-dessus, substitué en plus par -M-R⁵, où M est choisi dans le groupe constitué par :
(aa) -C(O)-NH- ;
(bb) -NH-C(O)- ;
(cc) -NH- ;
(dd) -N(CH₃)- ;
(ee) -O- ;
(ff) -S(O)ₙ-, où n vaut 0, 1 ou 2 ;
(gg) -C(=NH)-NH- ;
(hh) -CO-O- ;
(ii) -O-CO- ;
(jj) -CO- ;
et R⁵ est choisi dans le groupe constitué par :
(aaa) alkyle en C₁ à C₆ le cas échant substitué par un substituant choisi dans le groupe constitué par :
(i) aryle ;
(ii) aryle substitué ;
(iii) hétéroaryle ; et
(iv) hétéroaryle substitué ;
(bbb) aryle ;
(ccc) aryle substitué ;
(ddd) hétéroaryle ;
(eee) hétéroaryle substitué ; et
(fff) hétérocycloalkyle ;
ou une paire de substituants quelconque comprenant AB, AD, AE, BD, BE ou DE est prise ensemble avec le ou les atomes auxquels ils sont rattachés pour former un noyau cyclique de 3 à 7 termes contenant le cas échéant une fonction hétéro comprenant :
-O- ;
-NH- ;
-N(alkyle en C₁ à C₆)- ;
-N(arylalkyle en C₁ à C₆)- ;
-N(arylalkyle en C₁ à C₆ substitué)- ;
-N(hétéroarylalkyle en C₁ à C₆)- ;
-N(hétéroarylalkyle en C₁ à C₆ substitué)- ;
-S- ou -S(O)ₙ-, où n vaut 1 ou 2 ;
-C(O)-NH- ;
-C(O)-NR⁵, où R⁵ est tel que défini plus haut ;
-NR⁵-C(O)-, où R⁵ est tel que défini plus haut ; et
-C(=NH)-NH- ;
ledit procédé comprenant les étapes consistant à :
(a) traiter un composé ayant la formule : 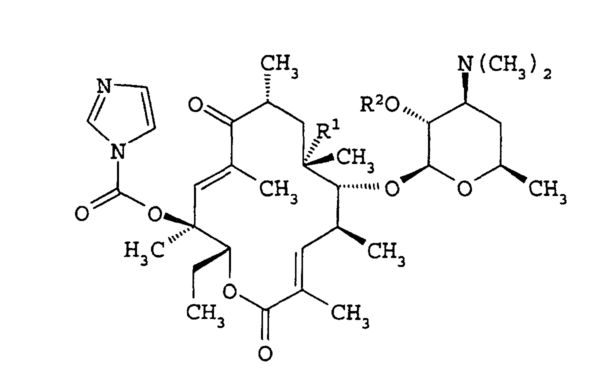 où R¹ est choisi dans le groupe constitué par hydrogène, hydroxy protégé, O-alkyle en C₁ à C₁₂, O-CO-alkyle en C₁ à C₆, O-CO-NH₂, O-CO-NH-CO-alkyle en C₁ à C₁₂
ou O-CO-NH-SO₂-alkyle en C₁ à C₁₂ ; et R² est un groupe hydroxy- protecteur, avec un composé ayant la formule : 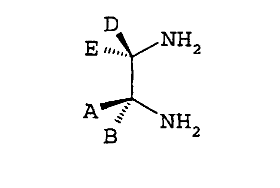 où A, B, D et E sont tels que définis pour les composés de la formule (I) ci-dessus, dans un solvant approprié à une température allant de l'ambiante à la température de reflux pendant environ 4 à environ 48 heures pour donner le composé intermédiaire bicyclique ayant la formule : 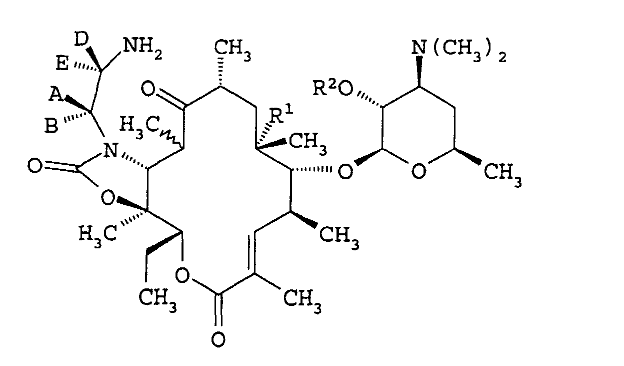
(b) déprotéger lesdits composés intermédiaires bicycliques pour donner les deuxièmes composés intermédiaires ayant la formule : 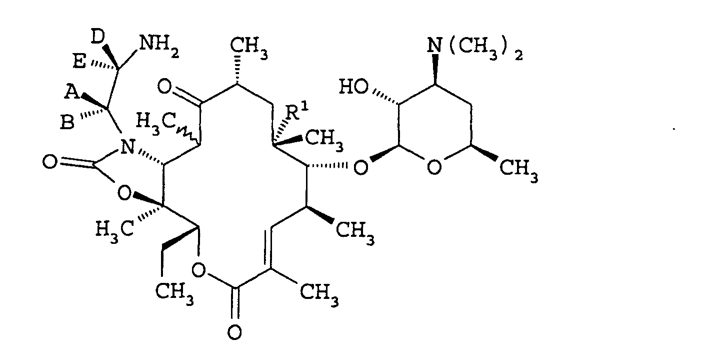
(c) cycliser lesdits deuxièmes composés intermédiaires par traitement avec une concentration diluée d'un acide fort dans un solvant organique approprié pendant une durée d'environ 4 heures à environ 10 jours à une température allant de l'ambiante à la température de reflux du solvant pour donner le composé recherché ; et
(d) extraire, le cas échéant déprotéger, et isoler le composé recherché.

26. Procédé selon la revendication 25, dans lequel, à l'étape (a), le solvant est choisi dans le groupe constitué par le méthanol, l'éthanol, le propanol, l'isopropanol, le butanol, le t-butanol, le chlorure de méthylène, le tétrahydrofurane, la N-méthylpyrrolidinone, le diéthyléther, le bis-méthoxyméthyléther, le diméthylformamide, l'acétone, l'acétonitrile aqueux, le DMF aqueux et l'acétone aqueuse ; et à l'étape (c) le solvant est choisi dans le groupe constitué par le méthanol, l'éthanol, le propanol, l'isopropanol, le butanol, l'isobutanol et le t-butanol.

27. Procédé de préparation d'un composé ayant la formule (II) : 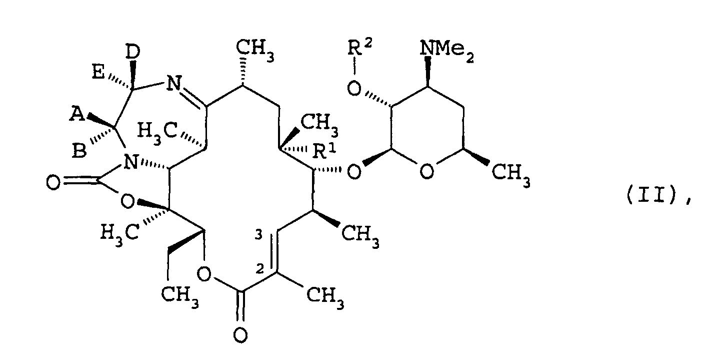 dans laquelle
R¹ est choisi dans le groupe constitué par :
(a) hydrogène ;
(b) hydroxy ;
(c) O-alkyle en C₁ à C₁₂ ;
(d) O-CO-alkyle en C₁ à C₆ ;
(e) O-CO-NH₂ ;
(f) O-CO-NH-CO-alkyle en C₁ à C₁₂ ; et
(g) O-CO-NH-SO₂-alkyle en C₁ à C₁₂ ;
R² est hydrogène ou un groupe hydroxyprotecteur ;
A, B, D et E sont choisis indépendamment dans le groupe constitué par :
(a) hydrogène ;
(b) alkyle en C₁ à C₆ le cas échéant substitué par un ou plusieurs substituants choisis dans le groupe constitué par :
(i) aryle ;
(ii) aryle substitué ;
(iii) hétéroaryle ;
(iv) hétéroaryle substitué ;
(v) hétérocycloalkyle ;
(vi) hydroxy ;
(vii) alcoxy en C₁ à C₆ ;
(viii) halogène comprenant Br, Cl, F ou I ; et
(ix) NR³R⁴, où R³ et R⁴ sont choisis indépendamment parmi hydrogène et alkyle en C₁ à C₆, ou bien R³ et R⁴ sont pris avec l'atome d'azote auquel ils sont liés pour former un noyau cyclique de 3 à 7 termes qui, lorsque le noyau est un noyau de 5 à 7 termes, peut contenir le cas échéant une fonction hétéro comprenant -O-, -NH-,-N(alkyle en C₁ à C₆)-, -N(aryle)-, -N-(arylalkyle en C₁ à C₆)-, -N(arylalkyle en C₁ à C₆ substitué) -,-N(hétéroaryle)-, -N-(hétéroarylalkyle en C₁ à C₆)-,-N(hétéro- arylalkyle en C₁ à C₆ substitué), -S- ou -S(O)ₙ-
où n vaut 1 ou 2 ;
(c) cycloalkyle en C₃ à C₇ ;
(d) aryle ;
(e) aryle substitué ;
(f) hétéroaryle ;
(g) hétéroaryle substitué ;
(h) hétérocycloalkyle ;
et
(i) un groupe choisi parmi ceux de l'option (b) ci-dessus, substitué en plus par -M-R⁵, où M est choisi dans le groupe constitué par :
(aa) -C(O)-NH- ;
(bb) -NH-C(O)- ;
(cc) -NH- ;
(dd) -N(CH₃)- ;
(ee) -O- ;
(ff) -S(O)ₙ-, où n vaut 0, 1 ou 2 ;
(gg) -C(=NH)-NH- ;
(hh) -CO-O- ;
(ii) -O-CO- ;
(jj) -CO- ;
et R⁵ est choisi dans le groupe constitué par :
(aaa) alkyle en C₁ à C₆ le cas échant substitué par un substituant choisi dans le groupe constitué par :
(i) aryle ;
(ii) aryle substitué ;
(iii) hétéroaryle ; et
(iv) hétéroaryle substitué ;
(bbb) aryle ;
(ccc) aryle substitué ;
(ddd) hétéroaryle ;
(eee) hétéroaryle substitué ; et
(fff) hétérocycloalkyle ;
ou une paire de substituants quelconque comprenant AB, AD, AE, BD, BE ou DE est prise ensemble avec le ou les atomes auxquels ils sont rattachés pour former un noyau cyclique de 3 à 7 termes contenant le cas échéant une fonction hétéro comprenant :
-O- ;
-NH- ;
-N(alkyle en C₁ à C₆)- ;
-N(arylalkyle en C₁ à C₆)- ;
-N(arylalkyle en C₁ à C₆ substitué)- ;
-N(hétéroarylalkyle en C₁ à C₆)- ;
-N(hétéroarylalkyle en C₁ à C₆ substitué)- ;
-S- ou -S(O)ₙ-. où n vaut 1 ou 2 ;
-C(O)-NH- ;
-C(O)-NR⁵-, où R⁵ est tel que défini plus haut ;
-NH-C(O)-
-NR⁵-C(O), où R⁵ est tel que défini plus haut ; et
-C(=NH)-NH- ;
ledit procédé comprenant les étapes consistant à :
(a) traiter un composé ayant la formule : 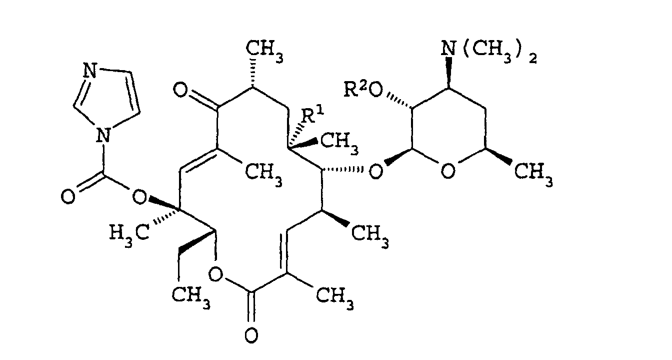 où R¹ est choisi dans le groupe constitué par hydrogène, hydroxy protégé, O-alkyle en C₁ à C₁₂, O-CO-alkyle en C₁ à C₆, O-CO-NH₂, O-CO-NH-CO-alkyle en C₁ à C₁₂
ou O-CO-NH-SO₂-alkyle en C₁ à C₁₂ ; et R² est un groupe hydroxy- protecteur, avec un composé ayant la formule : 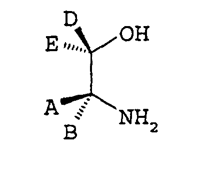 où A, B, D, et E sont tels que définis plus haut, dans un solvant approprié à une température de 0 à 70°C pendant environ 4 à environ 48 heures pour donner un composé bicyclique intermédiaire ayant la formule : 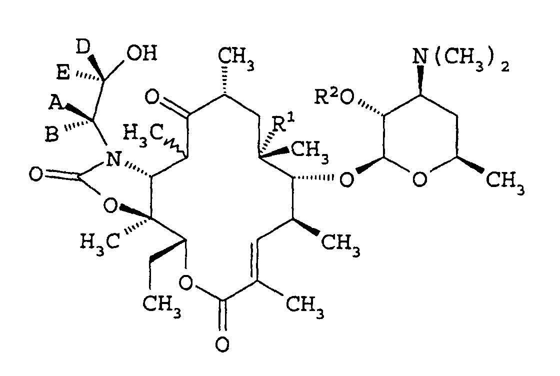
(b) traiter le composé intermédiaire bicyclique de l'étape (a) avec de la triphénylphosphine et de l'azide de diphénylphosphoryle/azodicarboxylate de diéthyle dans du tétrahydrofurane, dans les conditions de la réaction de Mitsunobu, afin de préparer le deuxième composé azide intermédiaire ayant la formule : 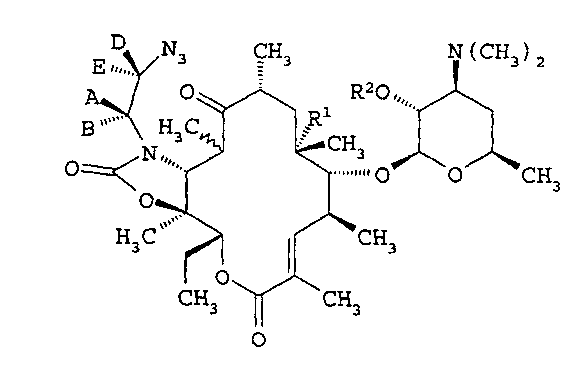
(c) réduire le deuxième composé azide intermédiaire afin de préparer le troisième composé intermédiaire ayant la formule : 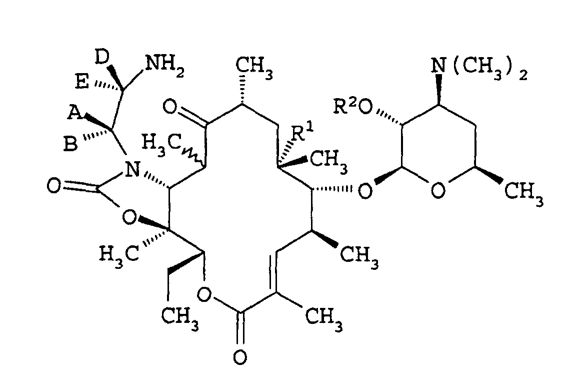
(d) cycliser ledit troisième composé intermédiaire par traitement avec une concentration diluée d'un acide fort à une température allant de l'ambiante à la température de reflux pendant environ 4 heures à environ 10 jours dans un solvant alcoolique aqueux pour donner le composé recherché ; et
(e) extraire, le cas échéant déprotéger, et isoler le composé recherché.

28. Procédé selon la revendication 27, dans lequel, à l'étape (a), le solvant est choisi dans le groupe constitué par le méthanol, l'éthanol, le propanol, l'isopropanol, le butanol, le t-butanol, le chlorure de méthylène, le tétrahydrofurane, la N-méthylpyrrolidinone, le diéthyléther, le bis-méthoxyméthyléther, le diméthylformamide, l'acétone, l'acétonitrile aqueux, le DMF aqueux et l'acétone aqueuse ; à l'étape (c), l'agent réducteur est choisi dans le groupe constitué par la triphénylphosphine/eau, l'hydrogène avec un catalyseur, le borohydrure de sodium et l'hydrure de dialkyl-aluminium ; et, à l'étape (d), le solvant est choisi dans le groupe constitué par le méthanol, l'éthanol, le propanol, l'isopropanol, le butanol, l'isobutanol et le t-butanol.

29. Procédé selon la revendication 27, dans lequel l'étape (b) de celui-ci est remplacée par deux étapes consistant à :
(b') faire réagir le groupe hydroxy du composé intermédiaire bicyclique avec un agent sulfonant choisi dans le groupe constitué par le chlorure de sulfonyle, un anhydride alkylsulfonique, un anhydride arylsulfonique et un anhydride trifluorométhanesulfonique, dans un solvant aprotique à une température allant de -78°C à l'ambiante pour donner un composé intermédiaire dans lequel le groupe hydroxyle a été remplacé par un fragment ester sulfonate ; et
(b") faire réagir l'ester sulfonate de l'étape (b') avec un oxyde de métal alcalin dans un solvant aprotique à une température allant d'environ 0°C à environ 100°C pour donner le deuxième composé oxyde intermédiaire.

30. Procédé de préparation d'un composé ayant la formule (III) : 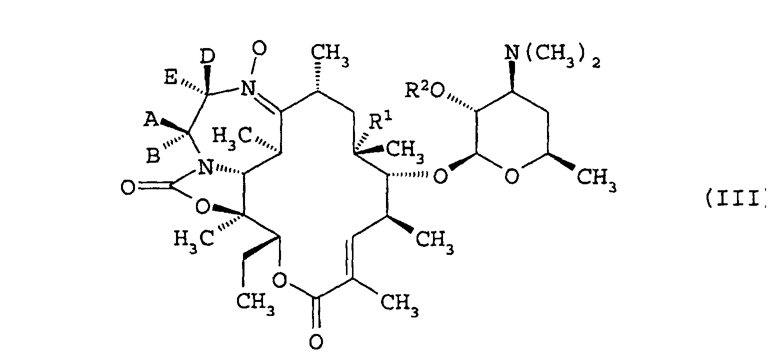 et
dans laquelle
A, B, D et E sont choisis indépendamment dans le groupe constitué par :
(a) hydrogène ;
(b) alkyle en C₁ à C₆ le cas échéant substitué par un ou plusieurs substituants choisis dans le groupe constitué par :
(i) aryle ;
(ii) aryle substitué ;
(iii) hétéroaryle ;
(iv) hétéroaryle substitué ;
(v) hétérocycloalkyle ;
(vi) hydroxy ;
(vii) alcoxy en C₁ à C₆ ;
(viii) halogène comprenant Br, Cl, F ou I ; et
(ix) NR³R⁴, où R³ et R⁴ sont choisis indépendamment parmi hydrogène et alkyle en C₁ à C₆, ou bien R³ et R⁴ sont pris avec l'atome d'azote auquel ils sont liés pour former un noyau cyclique de 3 à 7 termes qui, lorsque le noyau est un noyau de 5 à 7 termes, peut contenir le cas échéant une fonction hétéro comprenant -O-, -NH-, N(alkyle en C₁ à C₆)-, -N(aryle)-, -N-(arylalkyle en C₁ à C₆)-, -N(arylalkyle en C₁ à C₆ substitué) -,-N(hétéroaryle)-, -N-(hétéroarylalkyle en C₁ à C₆)-,-N(hétéro- arylalkyle en C₁ à C₆ substitué)-, -S- ou-S(O)ₙ-, où n vaut 1 ou 2 ;
(c) cycloalkyle en C₃ à C₇ ;
(d) aryle ;
(e) aryle substitué ;
(f) hétéroaryle ;
(g) hétéroaryle substitué ;
(h) hétérocycloalkyle ; et
(i) un groupe choisi parmi ceux de l'option (b) ci-dessus, substitué en plus par -M-R⁵, où M est choisi dans le groupe constitué par :
(aa) -C(O)-NH- ;
(bb) -NH-C(O)- ;
(cc) -NH- ;
(dd) -N(CH₃)- ;
(ee) -O- ;
(ff) -S(O)ₙ-, où n vaut 0, 1 ou 2 ; et
(gg) -C(=NH)-NH- ;
et R⁵ est choisi dans le groupe constitué par :
(aaa) alkyle en C₁ à C₆ le cas échant substitué par un substituant choisi dans le groupe constitué par :
(i) aryle ;
(ii) aryle substitué ;
(iii) hétéroaryle ; et
(iv) hétéroaryle substitué ;
(bbb) aryle ;
(ccc) aryle substitué ;
(ddd) hétéroaryle ;
(eee) hétéroaryle substitué ; et
(fff) hétérocycloalkyle ;
ou une paire de substituants quelconque comprenant AB, AD, AE, BD, BE ou BE est prise ensemble avec le ou les atomes auxquels ils sont rattachés pour former un noyau cyclique de 3 à 7 termes contenant le cas échéant une fonction hétéro comprenant :
-O- ;
-NH- ;
-N(alkyle en C₁ à C₆)- ;
-N(arylalkyle en C₁ à C₆)- ;
-N(arylalkyle en C₁ à C₆ substitué)- ;
-N(hétéroarylalkyle en C₁ à C₆)- ;
-N(hétéroarylalkyle en C₁ à C₆ substitué),
-S- ou -S(O)ₙ-, où n vaut 1 ou 2 ;
-C(O)-NH- ;
-C(O)-NR⁵-, où R⁵ est tel que défini plus haut ;
-NH-C(O) ;
-NR⁵-C(O)-, où R⁵ est tel que défini plus haut ; et
-C(=NH)-NH- ;
R¹ est choisi dans le groupe constitué par :
(a) hydrogène ;
(b) hydroxy ;
(c) O-alkyle en C₁ à C₁₂ ;
(d) O-CO-alkyle en C₁ à C₆ ;
(e) O-CO-NH₂ ;
(f) O-CO-NH-CO-alkyle en C₁ à C₁₂ ; et
(g) O-CO-NH-SO₂-alkyle en C₁ à C₁₂ ; et
R² est hydrogène ou un groupe hydroxyprotecteur ; ledit procédé comprenant les étapes consistant à :
(a) faire réagir un composé ayant la formule (II) : 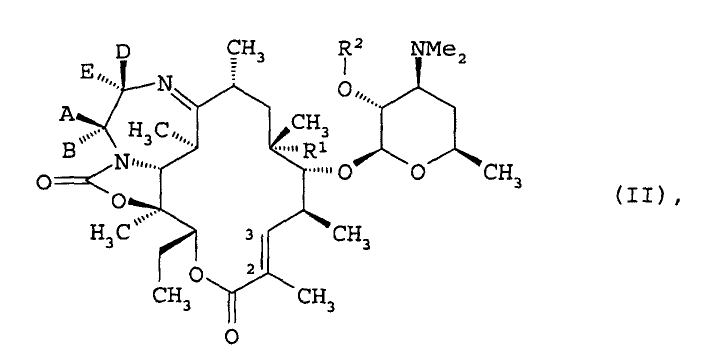 où R¹ est tel que défini plus haut ou est un groupe hydroxyprotecteur et R², A, B, D, et E sont tels que définis plus haut, avec un agent oxydant approprié pour oxyder l'azote de l'imine en nitrone et l'atome d'azote du fragment désosamine en N-oxyde afin de donner un intermédiaire N-oxydé ; et
(b) traiter l'intermédiaire N-oxydé avec un agent réducteur afin de réduire le N-oxyde de désosamine et extraire, le cas échéant déprotéger, et isoler le composé recherché.

31. Procédé selon la revendication 30, dans lequel, à l'étape (a), l'agent oxydant est choisi dans le groupe constitué par le peroxyde d'hydrogène et un peracide carboxylique ; et, à l'étape (b), l'agent réducteur est choisi dans le groupe constitué par la triphénylphosphine et l'hydrogène en présence d'un catalyseur.

32. Procédé de préparation d'un composé ayant la formule (IV) 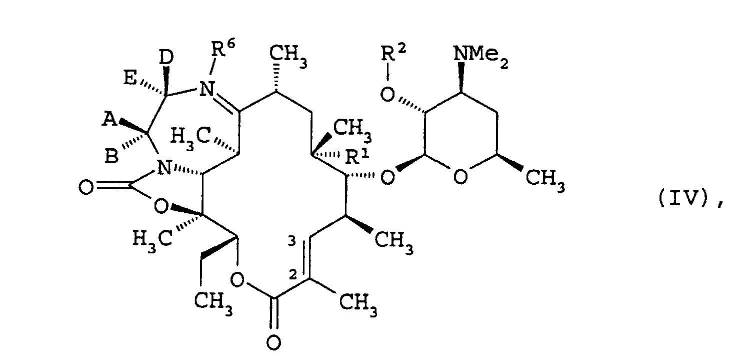 dans laquelle
A, B, D et E sont choisis indépendamment dans le groupe constitué par :
(a) hydrogène ;
(b) alkyle en C₁ à C₆ le cas échéant substitué par un ou plusieurs substituants choisis dans le groupe constitué par :
(i) aryle ;
(ii) aryle substitué ;
(iii) hétéroaryle ;
(iv) hétéroaryle substitué ;
(v) hétérocycloalkyle ;
(vi) hydroxy ;
(vii) alcoxy en C₁ à C₆ ;
(viii) halogène comprenant Br, Cl, F ou I ; et
(ix) NR³R⁴, où R³ et R⁴ sont choisis indépendamment parmi hydrogène et alkyle en C₁ à C₆, ou bien R³ et R⁴ sont pris avec l'atome d'azote auquel ils sont liés pour former un noyau cyclique de 3 à 7 termes qui, lorsque le noyau est un noyau de 5 à 7 termes, peut contenir le cas échéant une fonction hétéro comprenant -O-, -NH-,-N(alkyle en C₁ à C₆)-, -N(aryle)-, -N-(arylalkyle en C₁ à C₆)-, -N(arylalkyle en C₁ à C₆ substitué)-,-N(hétéroaryle)-, -N-(hétéroarylalkyle en C₁ à C₆)-,-N(hétéro- arylalkyle en C₁ à C₆ substitué)-, -S- ou-S(O)ₙ-, où n vaut 1 ou 2 ;
(c) cycloalkyle en C₃ à C₇ ;
(d) aryle ;
(e) aryle substitué ;
(f) hétéroaryle ;
(g) hétéroaryle substitué ;
(h) hétérocycloalkyle ; et
(i) un groupe choisi parmi ceux de l'option (b) ci-dessus, substitué en plus par -M-R⁵, où M est choisi dans le groupe constitué par
(aa) -C(O)-NH- ;
(bb) -NH-C(O)- ;
(cc) -NH- ;
(dd) -N(CH₃)- ;
(ee) -O- ;
(ff) -S(O)ₙ-, où n vaut 0, 1 ou 2 ; et
(gg) -C(=N)-NH- ;
et R⁵ est choisi dans le groupe constitué par :
(aaa) alkyle en C₁ à C₆ le cas échant substitué par un substituant choisi dans le groupe constitué par :
(i) aryle ;
(ii) aryle substitué ;
(iii) hétéroaryle ; et
(iv) hétéroaryle substitué ;
(bbb) aryle ;
(ccc) aryle substitué ;
(ddd) hétéroaryle ;
(eee) hétéroaryle substitué ; et
(fff) hétérocycloalkyle ;
ou une paire de substituants quelconque comprenant AB, AD, AE, BD, BE ou DE est prise ensemble avec le ou les atomes auxquels ils sont rattachés pour former un noyau cyclique de 3 à 7 termes contenant le cas échéant une fonction hétéro comprenant :
-O- ;
-NH- ;
-N(alkyle en C₁ à C₆)- ;
-N(arylalkyle en C₁ à C₆) ;
-N(arylalkyle en C₁ à C₆ substitué)- ;
-N(hétéroarylalkyle en C₁ à C₆)- ;
-N(hétéroarylalkyle en C₁ à C₆ substitué)- ;
-S- ou -S(O)ₙ-, où n vaut 1 ou 2 ;
-C(O)-NH- ;
-C(O)-NR⁵-, où R⁵ est tel que défini plus haut ;
-NH-C(O) ;
-NR⁵-C(O)-, où R⁵ est tel que défini plus haut ; et
-C(=NH)-NH- ;
R¹ est choisi dans le groupe constitué par :
(a) hydrogène ;
(b) hydroxy ;
(c) O-alkyle en C₁ à C₁₂ ;
(d) O-CO-alkyle en C₁ à C₆ ;
(e) O-CO-NH₂ ;
(f) O-CO-NH-CO-alkyle en C₁ à C₁₂ ; et
(g) O-CO-NH-SO₂-alkyle en C₁ à C₁₂ ; et
R² est hydrogène ou un groupe hydroxyprotecteur ;
R⁶ est hydrogène ou alkyle en C₁ à C₆ ;
ledit procédé comprenant les étapes consistant à :
(a) faire réagir un composé ayant la formule : 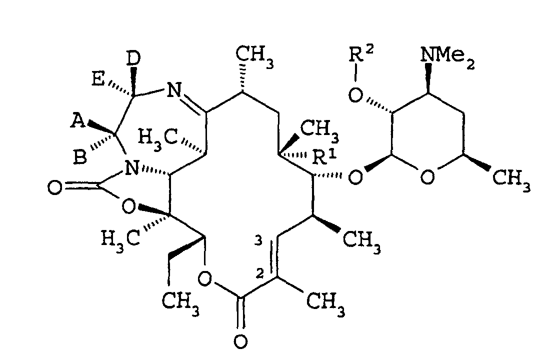 où R¹ est tel que défini plus haut ou est un groupe hydroxyprotecteur et R², A, B, D et E sont tels que définis plus haut, avec un agent réducteur dans un solvant organique approprié pour donner le composé recherché dans lequel R⁶ est H ;
(b) le cas échéant alkyler en conditions réductrices l'amino du produit de l'étape (a) avec un réactif réducteur en présence d'un précurseur de groupe alkyle en C₁ à C₆ pour donner le composé recherché dans lequel R⁶ est alkyle en C₁ à C₆ ; et
(c) extraire, le cas échéant déprotéger, et isoler le composé recherché.

33. Procédé selon la revendication 31, dans lequel, à l'étape (a) et à l'étape facultative (b), l'agent réducteur est choisi dans le groupe constitué par le cyanoborohydrure de sodium, le borohydrure de sodium, le triacétoxyborohydrure de sodium, un complexe de borane et tétrahydrofurane et un complexe de borane et pipéridine.

34. Composé ayant la formule : 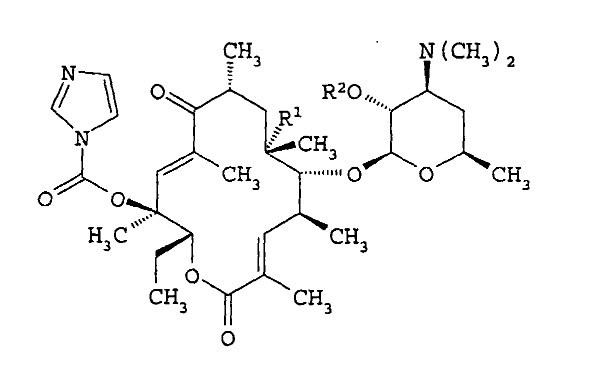 dans laquelle
R¹ est choisi dans le groupe constitué par hydrogène, hydroxy protégé, O-alkyle en C₁ à C₁₂, O-CO-alkyle en C₁ à C₆, O-CO-NH₂, O-CO-NH-CO-alkyle en C₁ à C₁₂ ou O-CO-NH-SO₂-alkyle en C₁ à C₁₂ ; et
R² est hydrogène ou un groupe hydroxyprotecteur.

35. Composé selon la revendication 33, dans lequel R¹ est O-alkyle en C₁ à C₁₂.

36. Composé selon la revendication 34, dans lequel R¹ est méthoxy.

37. Procédé de préparation d'un composé ayant la formule : 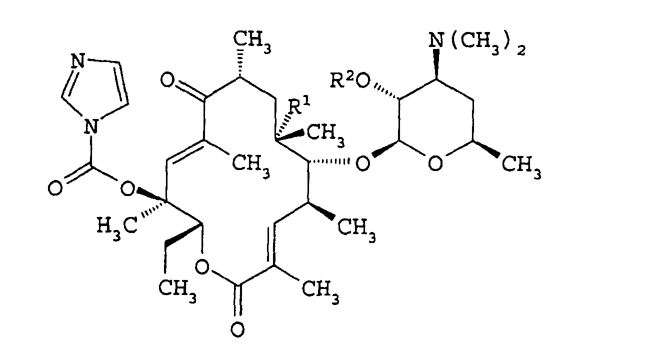 dans laquelle
R¹ est choisi dans le groupe constitué par :
(a) hydrogène ;
(b) hydroxy protégé ;
(c) O-alkyle en C₁ à C₁₂ ;
(d) O-CO-alkyle en C₁ à C₆ ;
(e) O-CO-NH₂ ;
(f) O-CO-NH-CO-alkyle en C₁ à C₁₂ ; et
(g) O-CO-NH-SO₂-alkyle en C₁ à C₁₂ ; et
R² est hydrogène ou un groupe hydroxyprotecteur ; ledit procédé comprenant les étapes consistant à :
(a) traiter un composé d'érythromycine A ayant la formule : 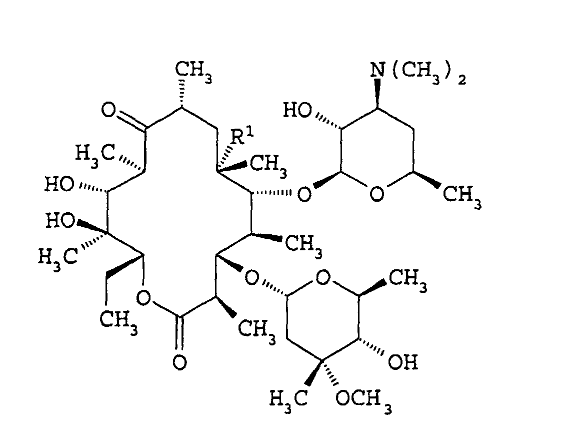 où R¹ est tel que défini plus haut, avec des réactifs de déshydrogénation comprenant un organocarbonate en présence d'une base à la température de reflux dans un solvant aprotique afin de former un composé intermédiaire ayant la formule : 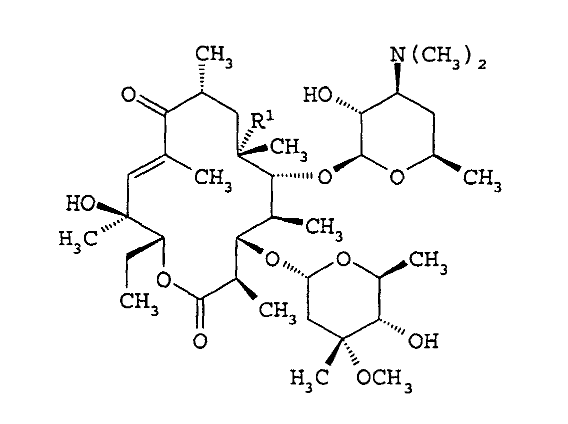
(b) enlever hydrolytiquement le fragment cladinose du composé intermédiaire de l'étape (a) par traitement dans une suspension aqueuse d'un alcool avec une concentration diluée d'un acide fort à la température ambiante pendant environ 0,5 à environ 24 heures, extraire et le cas échéant isoler le composé ayant la formule : 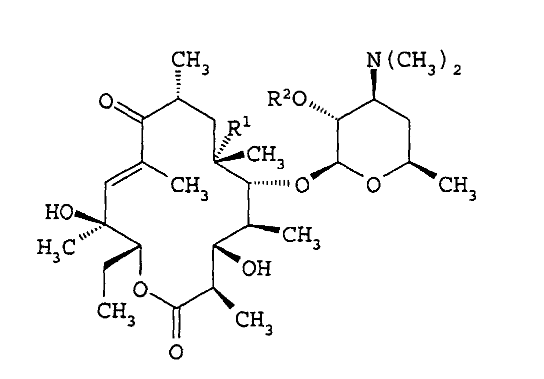
(c) traiter le composé de l'étape (b) avec un réactif approprié protégeant les groupes hydroxy dans un solvant aprotique, et isoler par voie extractive le composé dans lequel R² est un groupe hydroxyprotecteur ;
(d) traiter une solution du composé de l'étape (c) avec un agent sulfonylant à une température allant d'environ 0°C à l'ambiante pendant environ 1 à environ 24 heures et isoler par voie extractive le composé ayant la formule : 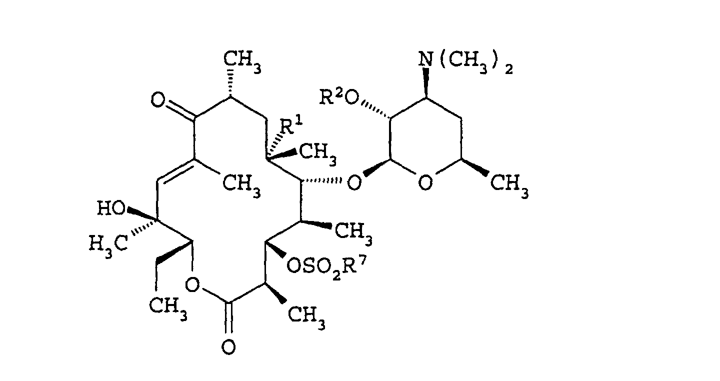 où R⁷ est alkyle ou aryle ;
(e) déshydrogéner le composé de l'étape (d) avec une base hydrure en présence de carbonyldiimidazole dans un solvant aprotique à une température allant d'environ -20°C à environ 70°C pendant environ 0,5 heures à environ 10 jours et extraire, le cas échéant déprotéger, et isoler le composé recherché.

38. Procédé selon la revendication 36, dans lequel, à l'étape (a), les réactifs de déshydrogénation sont constitués par un composé organocarbonate choisi dans le groupe constitué par le carbonate d'éthylène, le carbonate de propylène, le carbonate de triméthylène, le carbonate de dipropyle, le carbonate de dibenzyle, le carbonate d'isobutyle, le carbonate de diméthyle et le carbonate de diéthyle, en présence d'une base choisie dans le groupe constitué par la triéthylamine, la diisopropyléthylamine, la pyridine, la 2,6-diméthylpyridine, le 1,8-diazabicyclo-[5.4.0]undéc-7-ène, la N-méthylmorpholine, la N-méthylpyrrolidine, la N-méthylpipéridine, le carbonate de sodium, le carbonate de potassium, le bicarbonate de sodium et le carbonate de potassium ; à l'étape (b), l'alcool est choisi dans le groupe constitué par le méthanol, l'éthanol, le propanol, l'isopropanol, le butanol, l'isobutanol et le t-butanol, et l'acide est choisi dans le groupe constitué par l'acide chlorhydrique, l'acide sulfurique, l'acide dichloracétique et l'acide trichloracétique ; à l'étape (c), le réactif protégeant les groupes hydroxy est choisi dans le groupe constitué par le chlorure d'acétyle, l'anhydride acétique, l'anhydride benzoïque, le chloroformiate de benzyle, le chlorure de triméthylsilyle et le chlorure de triéthylsilyle, et le solvant aprotique est choisi dans le groupe constitué par le chlorure de méthylène, le chloroforme, le diméthylformamide, le tétrahydrofurane, la N-méthylpyrrolidinone ainsi que des mélanges de ceux-ci ; à l'étape (d), l'agent sulfonylant est choisi dans le groupe constitué par l'anhydride méthanesulfonylique, le chlorure de méthanesulfonyle, le chlorure d'éthanesulfonyle et le chlorure de p-toluènesulfonyle, et la base est choisie dans le groupe défini à l'étape (a) ci-dessus ; à l'étape (e), la base hydrure est choisie dans le groupe constitué par l'hydrure de sodium, l'hydrure de potassium et l'hydrure de lithium et le solvant aprotique est tel que défini pour l'étape (c).

39. Procédé selon la revendication 37, dans lequel R¹ est H et les étapes (d) et (e) sont remplacées par une étape (d') unique consistant à :
(d') traiter le composé de l'étape (c) avec de l'hexaméthyldisilazane de sodium à une température allant d'environ -30 à environ -28°C sous une atmosphère inerte, suivi de l'ajout de carbonyldiimidazole à une température allant d'environ 0°C à l'ambiante pendant environ 15 minutes à environ 6 heures, et extraire, le cas échéant déprotéger, et isoler le composé recherché.

40. Procédé de préparation d'un composé ayant la formule : 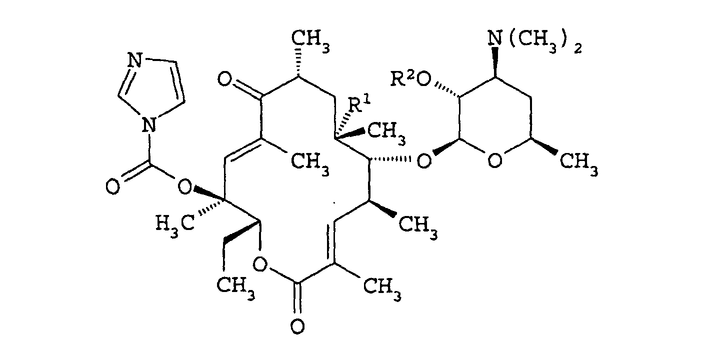 dans laquelle
R¹ est choisi dans le groupe constitué par :
(a) hydrogène ;
(b) hydroxy protégé ;
(c) O-alkyle en C₁ à C₁₂ ;
(d) O-CO-alkyle en C₁ à C₆ ;
(e) O-CO-NH₂ ;
(f) O-CO-NH-CO-alkyle en C₁ à C₁₂ ; et
(g) O-CO-NH-SO₂-alkyle en C₁ à C₁₂ ; et
R² est hydrogène ou un groupe hydroxyprotecteur ; ledit procédé comprenant les étapes consistant à :
(a) enlever hydrolytiquement le fragment cladinose d'un composé d'érythromycine A ayant la formule : 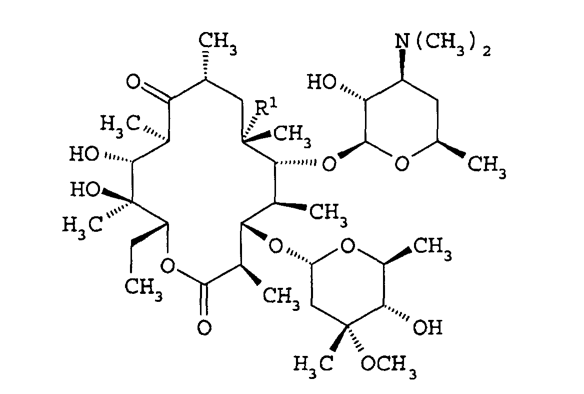 où R¹ est tel que décrit plus haut, par traitement dans une suspension aqueuse d'alcool avec une concentration diluée d'un acide fort à la température ambiante pendant environ 0,5 à environ 24 heures, extraire et le cas échéant isoler le premier composé intermédiaire ayant la formule : 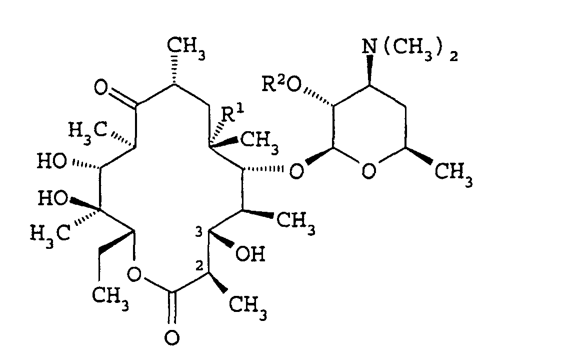
(b) le cas échéant traiter le premier composé intermédiaire avec un réactif approprié protégeant les groupes hydroxy et isoler par voie extractive le deuxième composé intermédiaire ayant la formule du composé de l'étape (a)
dans laquelle R² est un groupe hydroxyprotecteur ;
(c) traiter le deuxième composé intermédiaire avec un excès d'un réactif carbonylant et isoler par un traitement aqueux le troisième composé intermédiaire ayant la formule : 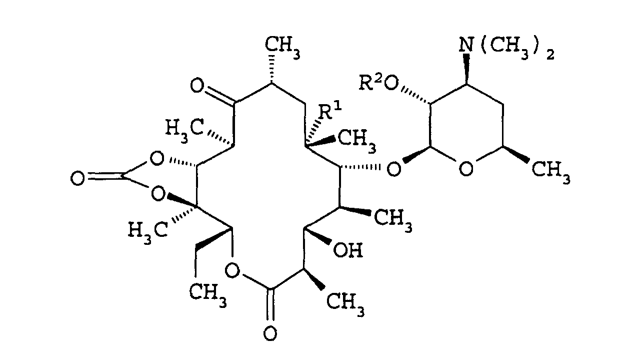 où R¹ ne peut pas être hydrogène mais est par ailleurs tel que défini plus haut ;
(d) traiter le troisième composé intermédiaire avec un agent sulfonylant à une température allant d'environ 0°C à l'ambiante pendant environ 1 à environ 24 heures et isoler par voie extractive le quatrième composé intermédiaire ayant la formule : 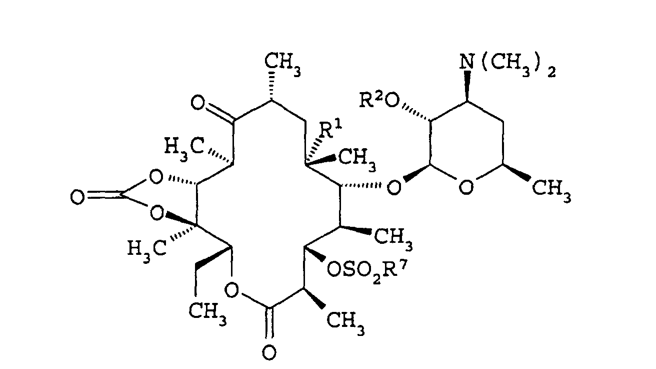 où R⁷ est alkyle ou aryle ;
(e) traiter le quatrième composé intermédiaire avec une base forte, extraire et le cas échéant isoler le quatrième composé intermédiaire pour donner le cinquième composé intermédiaire ayant la formule : 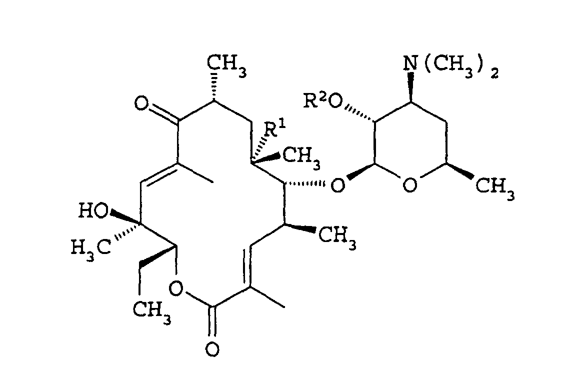
(f) traiter le cinquième composé intermédiaire avec une base hydrure et du carbonyldiimidazole dans un solvant aprotique à une température allant d'environ -20°C à environ 70°C pendant environ 0,5 heures à environ 10 jours et extraire, le cas échéant déprotéger, et isoler le composé recherché.

41. Procédé selon la revendication 40, dans lequel, à l'étape (a), l'alcool est choisi dans le groupe constitué par le méthanol, l'éthanol, le propanol, l'isopropanol, le butanol, l'isobutanol et le t-butanol, et l'acide est choisi dans le groupe constitué par l'acide chlorhydrique, l'acide sulfurique, l'acide dichloracétique et l'acide trichloracétique ; à l'étape (b), le réactif protégeant les groupes hydroxy est choisi dans le groupe constitué par le chlorure d'acétyle, l'anhydride acétique, l'anhydride benzoïque, le chloroformiate de benzyle, le chlorure de triméthylsilyle et le chlorure de triéthylsilyle, et le solvant aprotique est choisi dans le groupe constitué par le chlorure de méthylène, le chloroforme, le diméthylformamide, le tétrahydrofurane, la N-méthylpyrrolidinone ainsi que des mélanges de ceux-ci ; à l'étape (c), le réactif carbonylant est choisi dans le groupe constitué par le phosgène, le diphosgène et le triphosgène ; à l'étape (d), l'agent sulfonylant est choisi dans le groupe constitué par l'anhydride méthanesulfonylique, le chlorure de méthanesulfonyle, le chlorure d'éthanesulfonyle et le chlorure de p-toluènesulfonyle ; à l'étape (e), la base est choisie dans le groupe constitué par la triéthylamine, la diisopropyléthylamine, la pyridine, la 2,5-diméthylpyridine, le 1,8-diazabicyclo[5.4.0]undéc-7-ène, la N-méthylmorpholine, la N-méthylpyrrolidine, la N-méthylpipéridine, le carbonate de sodium, le carbonate de potassium, le bicarbonate de sodium et le carbonate de potassium ; à l'étape (f), la base hydrure est choisie dans le groupe constitué par l'hydrure de sodium, l'hydrure de potassium et l'hydrure de lithium.

42. Procédé selon la revendication 40, dans lequel, à l'étape (b), le réactif hydroxyprotecteur est l'anhydride benzoïque et R² est benzoyle, et les étapes (c), (d) et (e) sont remplacées par une étape (c') unique consistant à :
(c') traiter le composé de l'étape (6) avec de l'hexaméthyldisilazane de sodium à une température d'environ -50 à environ -28°C sous une atmosphère inerte, suivi de l'ajout de carbonyldiimidazole à une température d'environ 0°C à environ l'ambiante pendant environ 15 minutes à environ 6 heures, et extraire, le cas échéant déprotéger, et isoler le composé recherché.

43. Composition pharmaceutique pour traiter des infections bactériennes, comprenant une quantité thérapeutiquement efficace d'un composé selon la revendication 1 en combinaison avec un véhicule pharmaceutiquement acceptable.

44. Utilisation d'une composition pharmaceutique contenant une quantité thérapeutiquement efficace d'un composé selon la revendication 1 pour fabriquer un médicament destiné à traiter des infections bactériennes par administration à un mammifère nécessitant un tel traitement.
